# EUROPEAN PATENT SPECIFICATION

(11) **EP 2 004 182 B1**
(45) Date of publication and mention of the grant of the patent: **08.05.2013**
(21) Application number: 07727115.3
(22) Date of filing: 20.03.2007
(51) Int. Cl.: A61K 31/4184

(54) **BENZIMIDAZOLES WHICH HAVE ACTIVITY AT M1 RECEPTOR AND THEIR USES IN MEDICINE**
BENZIMIDAZOLE MIT WIRKUNG AM M1-REZEPTOR UND IHRE VERWENDUNGEN IN DER MEDIZIN
BENZIMIDAZOLES PRÉSENTANT UNE ACTIVITÉ AU NIVEAU DU RÉCEPTEUR M1 ET LEURS UTILISATIONS EN MÉDECINE

(30) Priority: 22.03.2006 GB 0605786
(43) Date of publication of application: 24.12.2008
(73) Proprietor: Glaxo Group Limited, Brentford, Middlesex TW8 9GS (GB)
(72) Inventor: COOPER, David, Gwyn, Harlow Essex CM19 5AW (GB); FORBES, Ian, Thomson, Harlow Essex CM19 5AW (GB); GARZYA, Vincenzo, Harlow Essex CM19 5AW (GB); JIN, Jian, Collegeville, Pennsylvania 19426 (US); LOUCHART, Yann, Harlow Essex CM19 5AW (GB); WALKER, Graham, Harlow Essex CM19 5AW (GB); WYMAN, Paul, Adrian, Harlow Essex CM19 5AW (GB)
(74) Representative: Kondo, Rie
(86) International application number: PCT/EP2007/052638
(87) International publication number: WO 2007/107565

(56) References cited:
- EP-A1- 0 491 212
- WO-A-96/13262
- WO-A-97/16186
- WO-A-03/105781
- WO-A-2004/054974
- WO-A-2007/036711

## Description

This invention relates to novel compounds, pharmaceutical compositions containing them and their use in therapy, in particular as antipsychotic agents.

Muscarinic acetylcholine receptors are members of the G protein coupled receptor superfamily which mediate the actions of the neurotransmitter acetylcholine in both the central and peripheral nervous system. Five muscarinic receptor subtypes have been cloned, M₁ to M₅. The muscarinic M₁ receptor is predominantly expressed in the cerebral cortex and hippocampus, although it is also expressed in the periphery e.g. exocrine glands.

Muscarinic receptors in the central nervous system, especially M₁, play a critical role in mediating higher cognitive processing. Diseases associated with cognitive impairments, such as Alzheimer's disease, are accompanied by loss of cholinergic neurons in the basal forebrain. Furthermore, in animal models, blockade or lesion of central cholinergic pathways results in profound cognitive deficits.

Cholinergic replacement therapy has largely been based on the use of acetylcholinesterase inhibitors to prevent the breakdown of endogenous acetylcholine. These compounds have shown efficacy versus symptomatic cognitive decline in the clinic, but give rise to side effects resulting from stimulation of peripheral muscarinic receptors including disturbed gastrointestinal motility and nausea.

The dopamine hypothesis of schizophrenia suggests that excess dopaminergic stimulation is responsible for the positive symptoms of the disease, hence the utility of dopamine receptor antagonists to reduce psychotic symptoms. However, conventional dopamine receptor antagonists can cause extrapyramidal side effects (EPS) in patients, including tremor and tardive dyskinesias.

M₁ receptor agonists have been sought for the symptomatic treatment of cognitive decline. More recently, a number of groups have shown that muscarinic receptor agonists display an atypical antipsychotic-like profile in a range of pre-clinical paradigms. The muscarinic agonist, xanomeline, reverses a number of dopamine driven behaviours, including amphetamine induced locomotion in rats, apomorphine induced climbing in mice, dopamine agonist driven turning in unilateral 6-OH-DA lesioned rats and amphetamine-induced motor unrest in monkeys (without EPS liability). It also has been shown to inhibit A10, but not A9, dopamine cell firing and conditioned avoidance and induces *c-fos* expression in prefrontal cortex and nucleus accumbens, but not in striatum in rats. These data are all suggestive of an atypical antipsychotic-like profile.

Xanomeline has also been shown to reduce psychotic symptoms such as suspiciousness, hallucinations and delusions in Alzheimer's patients. However, the relatively non-selective nature of the compound gives rise to dose-limiting peripheral cholinergic side effects.

Certain M₁ receptor agonists are known, for example in WO2007036711. We have now found a novel group of compounds which are M₁ receptor agonists.

In a first aspect therefore, the invention provides a compound of formula (I) or a salt or solvate thereof: 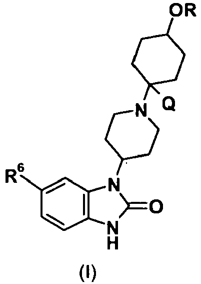 wherein:
- R⁶ is selected from hydrogen, cyano, halogen, C₁₋₆alkyl, C₁₋₆alkyl substituted with one or more fluorine atoms, C₁₋₆alkylsulfonyl, C₃₋₆cycloalkyl, C₃₋₆cycloalkyl substituted with one or more fluorine atoms, C₁₋₆alkoxy, and C₁₋₆alkoxy substituted with one or more fluorine atoms;
- R is selected from C₁₋₆alkyl, C₃₋₆cycloalkyl, C₃₋₆cycloalkylC₁₋₆alkyl and C₂₋₆alkynyl, any alkyl or cycloalkyl group being optionally substituted by one or more fluorine atoms; and
- Q is hydrogen or C₁₋₆alkyl.

The present invention also provides a compound of formula (Ia): 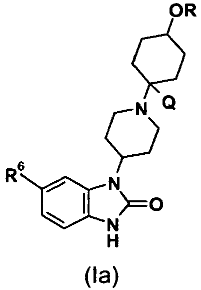 wherein:
R⁶ is selected from hydrogen, halogen, C₁₋₆alkyl, C₁₋₆alkyl substituted with one or more fluorine atoms, C₃₋₆cycloalkyl, C₃₋₆cycloalkyl substituted with one or more fluorine atoms, C₁₋₆alkoxy and C₁₋₆alkoxy substituted with one or more fluorine atoms;
R is selected from C₁₋₆alkyl, C₃₋₆cycloalkyl, C₃₋₆cycloalkylC₁₋₆alkyl, any alkyl or cycloalkyl group being optionally substituted with one or more fluorine atoms; and
Q is hydrogen or C₁₋₆alkyl;
or a pharmaceutically acceptable salt or solvate thereof.

As used herein, the term "alkyl" refers to straight or branched hydrocarbon chains containing the specified number of carbon atoms. For example, C₁₋₆alkyl means a straight or branched alkyl containing at least 1, and at most 6, carbon atoms. Examples include, but are not limited to, methyl, ethyl, n-propyl, n-butyl, n-pentyl, n-hexyl, isobutyl, isopropyl, t-butyl and 1,1-dimethylpropyl.

As used herein, the term "alkoxy" refers to a straight or branched alkoxy group containing the specified number of carbon atoms. For example, C₁₋₆alkoxy means a straight or branched alkoxy group containing at least 1, and at most 6, carbon atoms. Examples of "alkoxy" as used herein include, but are not limited to, methoxy, ethoxy, propoxy, prop-2-oxy, butoxy, but-2-oxy, 1-methylethyl-oxy, 2-methylprop-1-oxy, 2-methylprop-2-oxy, pentoxy or hexyloxy.

As used herein, the term "cycloalkyl" refers to a non-aromatic hydrocarbon ring containing the specified number of carbon atoms. For example, C₃₋₆cycloalkyl means a non-aromatic carbocyclic ring containing at least three, and at most six, ring carbon atoms. Examples of "cycloalkyl" as used herein include, but are not limited to, cyclopropyl, cyclobutyl, cyclopentyl and cyclohexyl.

As used herein, the term "alkynyl" refers to a linear or branched hydrocarbon group containing one or more carbon-carbon triple bonds and the specified number of carbon atoms. For example, C₂₋₆alkynyl means a linear or branched hydrocarbon group containing one or more carbon-carbon triple bonds and at least two, and at most six, carbon atoms. Examples of "alkynyl" as used herein include, but are not limited to, include ethynyl, propynyl, butynyl, pentynyl and hexynyl.

As used herein, the term "halogen" (or the abbreviated form "halo") refers to the elements fluorine (which may be abbreviated to "fluoro" or "F"), chlorine (which may be abbreviated to "chloro" or "Cl"), bromine (which may be abbreviated to "bromo" or "Br") and iodine (which may be abbreviated to "iodo" or "I"). Examples of halogens are fluorine, chlorine and bromine.

As used herein, the term "solvate" refers to a complex of variable stoichiometry formed by a solute (in this invention, a compound of formula (I) or a salt thereof) and a solvent. Such solvents for the purpose of the invention may not interfere with the biological activity of the solute. Examples of suitable solvents include water, methanol, ethanol and acetic acid. The solvent used may be water and the solvate may also be referred to as a hydrate.

As used herein, the term "substituted" refers to substitution with the named substituent or substituents, multiple degrees of substitution being allowed unless otherwise stated. For example, there may be 1, 2, 3 or 4 substituents on a given substituted group. For example, if R⁶ is a C₁₋₆alkyl group, it may be substituted by 1, 2, 3 or 4 fluoro groups; and if R⁶ is a C₁₋₆alkoxy group, it may be substituted by 1, 2, 3 or 4 fluoro groups. For example, R⁶ may be a C₁₋₆alkyl group substituted by 3 fluoro groups; and R⁶ may be a C₁₋₆alkoxy group substituted by 3 fluoro groups. For example, R⁶ may be CF₃. Similarly, if R is a C₁₋₆alkyl group substituted by one or more fluoro groups, it may be substituted by 1, 2, 3 or 4 fluoro groups; and if R is a C₃₋₆cycloalkyl group, it may be substituted by 1, 2, 3 or 4 fluoro groups. For example, R may be a C₁₋₆alkyl group substituted by 3 fluoro groups; and R may be a C₃₋₆cycloalkyl group substituted by 3 fluoro groups. For example, R may be CF₃ or CH₂F.

In one embodiment of the invention, R⁶ is selected from hydrogen, halogen, C₁₋₆alkyl, C₁₋₆alkyl substituted with one or more fluorine atoms, C₃₋₆cycloalkyl, C₁₋₆alkylsulfonyl, C₁₋₆alkoxy, and C₁₋₆alkoxy substituted with one or more fluorine atoms..

In one embodiment, R⁶ is cyano.

In one embodiment of the invention, R⁶ is selected from hydrogen, cyano, halogen, C₁₋₆alkyl, C₁₋₆alkyl substituted with one, two or three fluorine atoms, C₃₋₆cycloalkyl, C₁₋₆alkylsulfonyl, C₁₋₆alkoxy, and C₁₋₆alkoxy substituted with one, two or three fluorine atoms.

In one embodiment, R⁶ is selected from hydrogen, halogen, C₁₋₃alkyl, C₁₋₃alkyl substituted with one or more fluorine atoms, C₃₋₄cycloalkyl, C₁₋₃alkylsulfonyl, C₁₋₃alkoxy, and C₁₋₃alkoxy substituted with one or more fluorine atoms.

In one embodiment, R⁶ is selected from hydrogen, halogen, C₁₋₃alkyl, C₁₋₃alkyl substituted with one or two fluorine atoms, C₃₋₄cycloalkyl, C₁₋₃alkylsulfonyl, C₁₋₃alkoxy, and C₁₋₃alkoxy substituted with one, two or three fluorine atoms.

In one embodiment of the invention, R⁶ is selected from halogen, C₁₋₆alkyl, C₁₋₆alkyl substituted with one or more fluorine atoms, C₃₋₆cycloalkyl, C₃₋₆cycloalkyl substituted with one or more fluorine atoms, C₁₋₆alkoxy and C₁₋₆alkoxy substituted with one or more fluorine atoms.

In one embodiment of the invention, R⁶ is selected from hydrogen, cyano, fluoro, bromo, methyl, ethyl, methoxy, trifluoromethoxy, methylsulfonyl, trifluoromethyl and cyclopropyl.

In one embodiment of the invention, R⁶ is selected from fluoro, chloro, bromo, methyl, ethyl, isopropyl, methoxy, trifluoromethoxy and trifluoromethyl. In a further embodiment of the invention, R⁶ is selected from chloro, bromo, methyl, ethyl, isopropyl, methoxy, trifluoromethoxy and trifluoromethyl. For example, R⁶ is methyl.

In one embodiment, R is selected from C₁₋₆alkyl, C₃₋₆cycloalkyl, and C₃₋₆cycloalkylC₁₋₆alkyl, any alkyl or cycloalkyl group being optionally substituted by one, two or three fluorine atoms.

In one embodiment, R is selected from C₁₋₃alkyl, C₃₋₆cycloalkyl, C₃₋₆cycloalkylC₁₋₃alkyl and C₂₋₆alkynyl, any alkyl or cycloalkyl group being optionally substituted by one, two or three fluorine atoms.

In one embodiment, R is C₂₋₆alkynyl. In one embodiment, R is C₂₋₄alkynyl. In one embodiment, R is propynyl.

In a further embodiment of the invention, R is selected from C₁₋₃alkyl, and C₃₋₆cycloalkylC₁₋₃alkyl, any alkyl or cycloalkyl group (for example any alkyl group) being optionally substituted with one or more fluorine atoms.

In a further embodiment, R is selected from methyl, ethyl, propyl, isopropyl and cyclopropylmethyl. In one embodiment, R is selected from methyl, ethyl, propyl and isopropyl.

In one embodiment of the invention, R is selected from C₁₋₆alkyl, C₃₋₆cycloalkyl, C₃₋₆cycloalkylC₁₋₆alkyl, any alkyl group being optionally substituted with one or more fluorine atoms.

In one embodiment of the invention, R is selected from C₁₋₆alkyl, C₃₋₆cycloalkyl, C₃₋₆cycloalkylC₁₋₆alkyl and C₂₋₆alkynyl, any alkyl or cycloalkyl group being optionally substituted by one, two or three fluorine atoms.

In a further embodiment, R is selected from methyl, ethyl, propyl, isopropyl, CF₃, cyclopropylmethyl, propynyl and cyclobutyl.

In an embodiment of the invention, Q is selected from hydrogen and C₁₋₃alkyl. In a further embodiment, Q is selected from hydrogen, methyl, ethyl and propyl. In one embodiment, Q represents hydrogen or methyl. In one embodiment, Q is hydrogen.

In one embodiment the salt or solvate of the compound of formula (I) is a pharmaceutically acceptable salt or solvate. In one embodiment, the invention provides a compound of formula (I), or a pharmaceutically acceptable salt or solvate thereof.

It will be appreciated that for use in medicine the salts of formula (I) should be pharmaceutically acceptable. Suitable salts will be apparent to those skilled in the art and include for example mono- or di- basic salts formed with inorganic acids e.g. hydrochloric, hydrobromic, sulfuric, nitric, sulfamic phosphoric, hydroiodic, phosphoric or metaphosphoric acid; and with organic acids, such as tartaric, acetic, trifluoroacetic, citric, malic, lactic, fumaric, benzoic, formic, propionic, glycolic, gluconic, maleic, succinic, (1 S)-(-)-10-camphorsulphonic, (1S)-(+)-10-camphorsulphonic, isothionic, mucic, gentisic, isonicotinic, saccharic, glucuronic, furoic, glutamic, ascorbic, anthranilic, salicylic, phenylacetic, mandelic, embonic (pamoic), methanesulfonic, ethanesulfonic, pantothenic, stearic, sulfinilic, alginic, galacturonic and arylsulfonic, for example naphthalene-1,5-disulphonic, naphthalene-1,3-disulphonic, benzenesulfonic, and p-toluenesulfonic, acids. Other non-pharmaceutically acceptable salts e.g. oxalates, may be used, for example in the isolation of compounds of formula (I) and are included within the scope of this invention. The compounds of the present invention may be in the form of their free base or pharmaceutically acceptable salts thereof, particularly the monohydrochloride, monoformate or monotrifluoroacetate salts.

Certain of the compounds of formula (I) may form acid addition salts with less than one (for example, 0.5 equivalent of a dibasic acid) or one or more equivalents of an acid. The present invention includes within its scope all possible stoichiometric and non-stoichiometric forms thereof.

It will be appreciated that compounds of formula (I) can exist in *cis* or *trans* isomeric forms (the OR group on the cyclohexane ring in relation to the piperidine substituent).

*Cis* form:

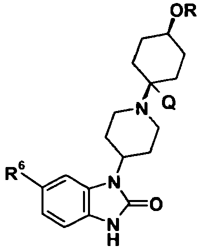

It will be appreciated that the *trans* form may be drawn in the following different ways, although both represent the same isomeric form:

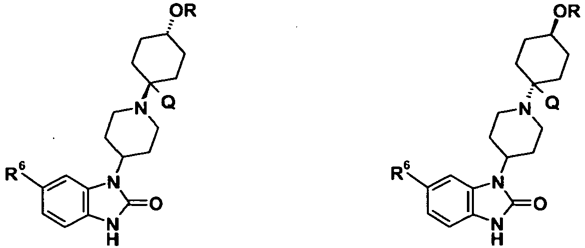

The individual isomers (*cis* and *trans)* and mixtures of these are included within the scope of the present invention. The isomers may be separated one from the other by the usual methods or by methods detailed for the example compounds below. Any given isomer may also be obtained by stereospecific or asymmetric synthesis. The invention also extends to any tautomeric forms and mixtures thereof.

In one embodiment, the compounds of formula (I) are *trans* isomers.

In another embodiment, the compounds of formula (I) are *cis* isomers.

Mixtures of *cis*- and *trans-* compounds, or compounds in which the cis/trans conformation have not been determined, are drawn herein as shown below:

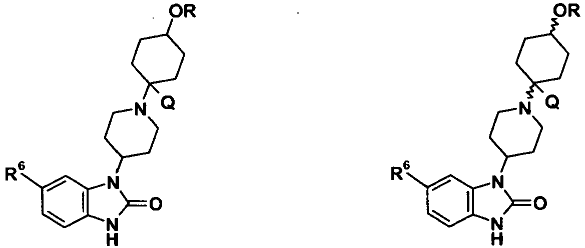

Compounds according to the invention include those specifically exemplified in the Examples section and named hereinafter including, without limitation:-
cis-6-Methyl-1-[1-(*cis*-4-methoxycyclohexyl)-4-piperidinyl]-1,3-dihydro-2*H*-benzimidazol-2-one
trans-6-Methyl-1-[1-(*cis*-4-methoxycyclohexyl)-4-piperidinyl]-1,3-dihydro-2*H*-benzimidazol-2-one
1-{1-[*trans*-4-(Ethyloxy)cyclohexyl]-4-piperidinyl}-6-methyl-1,3-dihydro-2*H*-benzimidazol-2-one
1-{1-[*cis*-4-(Ethyloxy)cyclohexyl]-4-piperidinyl}-6-methyl-1,3-dihydro-2*H*-benzimidazol-2-one
6-Methyl-1-{1-[*cis*-4-(propyloxy)cyclohexyl]-4-piperidinyl}-1,3-dihydro-2*H*-benzimidazol-2-one
6-Methyl-1-{1-[*trans*-4-(propyloxy)cyclohexyl]-4-piperidinyl}-1,3-dihydro-2*H*-benzimidazol-2-one
6-Methyl-1-(1-{*trans*-4-[(1-methylethyl)oxy]cyclohexyl}-4-piperidinyl)-1,3-dihydro-2*H-*benzimidazol-2-one
6-Methyl-1-{1-[*cis*-1-methyl-4-(methyloxy)cyclohexyl]-4-piperidinyl}-1,3-dihydro-2*H-*benzimidazol-2-one
6-Methyl-1-{1-[*trans*-1-methyl-4-(methyloxy)cyclohexyl]-4-piperidinyl}-1,3-dihydro-2*H-*benzimidazol-2-one
6-Methyl-1-(1-{*cis*-4-[(1-methylethyl)oxy]cyclohexyl}-4-piperidinyl)-1,3-dihydro-2*H-*benzimidazol-2-one
or a salt or solvate thereof, for example the hydrochloride salt, the trifluoroacetate salt or the formate salt.

In one embodiment, the salt of a compound listed above is a pharmaceutically acceptable salt.

Examples of the present invention includes:
1. 6-Methyl-1-[1-(*cis*-4-methoxycyclohexyl)-4-piperidinyl]-1,3-dihydro-2*H*-benzimidazol-2-one
2. 6-Methyl-1-[1-(*trans*-4-methoxycyclohexyl)-4-piperidinyl]-1,3-dihydro-2*H*-benzimidazol-2-one
3. 1-{1-[*trans*-4-(Ethyloxy)cyclohexyl]-4-piperidinyl}-6-methyl-1,3-dihydro-2*H-*benzimidazol-2-one
4. 1-{1-[*cis*-4-(Ethyloxy)cyclohexyl]-4-piperidinyl}-6-methyl-1,3-dihydro-2*H*-benzimidazol-2-one
5. 6-Methyl-1-{1-[*cis*-4-(propyloxy)cyclohexyl]-4-piperidinyl}-1,3-dihydro-2*H-*benzimidazol-2-one
6. 6-Methyl-1-{1-[*trans*-4-(propyloxy)cyclohexyl]-4-piperidinyl}-1,3-dihydro-2*H-*benzimidazol-2-one
7. 6-Methyl-1-(1-{*trans*-4-[(1-methylethyl)oxy]cyclohexyl}-4-piperidinyl)-1,3-dihydro-2*H-*benzimidazol-2-one
8. 6-Methyl-1-{1-[*cis*-1-methyl-4-(methyloxy)cyclohexyl]-4-piperidinyl}-1,3-dihydro-2*H-*benzimidazol-2-one
9. 6-Methyl-1-{1-[*trans*-1-methyl-4-(methyloxy)cyclohexyl]-4-piperidinyl}-1,3-dihydro-2*H-*benzimidazol-2-one
10. 6-Methyl-1-(1-{*cis*-4-[(1-methylethyl)oxy]cyclohexyl}-4-piperidinyl)-1,3-dihydro-2*H-*benzimidazol-2-one
11. *cis* -(1-[4-(Ethoxyl)cyclohexyl]-4-piperidinyl)-1,3-dihydro-2H-benzimidazol-2-one
*12. trans* 1-(1-[4-(Ethoxyl)cyclohexyl]-4-piperidinyl)-1,3-dihydro-2H-benzimidazol-2-one
13. 1-{1-[*trans*-4-(Ethyloxy)cyclohexyl]-4-piperidinyl}-6-fluoro-1,3-dihydro-2*H-*benzimidazol-2-one
14. 6-Bromo-1-{1-[trans-4-(ethyloxy)cyclohexyl]-4-piperidinyl}-1,3-dihydro-2*H-*benzimidazol-2-one
15. 1-{1-[*cis*-4-(Ethyloxy)cyclohexyl]-4-piperidinyl}-6-(trifluoromethyl)-1,3-dihydro-2*H-*benzimidazol-2-one
16. 6-Ethyl-1-{1-[*trans*-4-(ethyloxy)cyclohexyl]-4-piperidinyl}-1,3-dihydro-2*H*-benzimidazol-2-one
17. 6-Cyclopropyl-1-{1-[*trans*-4-(ethyloxy)cyclohexyl]-4-piperidinyl}-1,3-dihydro-2*H-*benzimidazol-2-one
18. 1-{1-[*trans*-4-(Ethyloxy)cyclohexyl]-4-piperidinyl}-6-(methyloxy)-1,3-dihydro-2*H-*benzimidazol-2-one
19. *cis*-1-{1-[4-(Ethoxy)cyclohexyl]-4-piperidinyl}-6-[(trifluoromethyl)oxy]-1,3-dihydro-2H-benzimidazol-2-one
20. *trans*-1-{1-[4-(Ethoxy)cyclohexyl]-4-piperidinyl}-6-[(trifluoromethyl)oxy]-1,3-dihydro-2H-benzimidazol-2-one
21. 6-Methyl-1-[1-(4-trifluoromethoxycyclohexyl)-4-piperidinyl]-1,3-dihydro-2*H-*benzimidazol-2-one
22. 1-(1-{*cis*-4-[(Cyclopropylmethyl)oxy]cyclohexyl}-4-piperidinyl)-6-methyl-1,3-dihydro-2*H-*benzimidazol-2-one
23. 1-(1-{*trans*-4-[(Cyclopropylmethyl)oxy]cyclohexyl}-4-piperidinyl)-6-methyl-1,3-dihydro-2*H*-benzimidazol-2-one
24. *trans*-6-Methyl-1-[1-(4-cyclopropyloxycyclohexyl)-4-piperidinyl]-1,3-dihydro-2*H-*benzimidazol-2-one
25. *cis* -1-(1-[4-(Propyloxy)cyclohexyl]-4-piperidinyl)-1,3-dihydro-2H-benzimidazol-2-one
26. *trans*-1-(1-[4-(Propyloxy)cyclohexyl]-4-piperidinyl)-1,3-dihydro-2H-benzimidazol-2-one
27. 6-Bromo-1-{1-[*trans*-4-(propyloxy)cyclohexyl]-4-piperidinyl}-1,3-dihydro-2*H-*benzimidazol-2-one
28. 6-Ethyl-1-{1-[*trans*-4-(propyloxy)cyclohexyl]-4-piperidinyl}-1,3-dihydro-2*H-*benzimidazol-2-one
29. 6-Cyclopropyl-1-{1-[trans-4-(propyloxy)cyclohexyl]-4-piperidinyl}-1,3-dihydro-2*H-*benzimidazol-2-one
30. *cis*-1-{1-[4-(Propyloxy)cyclohexyl]-4-piperidinyl}-6-[(trifluoromethyl)oxy]-1,3-dihydro-2*H*-benzimidazol-2-one
31. *trans*-1-{1-[4-(Propyloxy)cyclohexyl]-4-piperidinyl}-6-[(trifluoromethyl)oxy]-1,3-dihydro-2*H*-benzimidazol-2-one
32. 1-{1-[*cis*-4-(Ethyloxy)-1-methylcyclohexyl]-4-piperidinyl}-6-methyl-1,3-dihydro-2*H-*benzimidazol-2-one
33. 1-{1-[*trans*-4-(ethyloxy)-1-methylcyclohexyl]-4-piperidinyl}-6-methyl-1,3-dihydro-2*H-*benzimidazol-2-one
34. 6-Methyl-1-{1-[*cis*-1-methyl-4-(propyloxy)cyclohexyl]-4-piperidinyl}-1,3-dihydro-2*H-*benzimidazol-2-one
35. 6-Methyl-1-{1-[*trans*-1-methyl-4-(propyloxy)cyclohexyl]-4-piperidinyl}-1,3-dihydro-2H-benzimidazol-2-one
36. *trans*-6-Methyl-1-[1-(1-ethyl-4-propoxycyclohexyl)-4-piperidinyl]-1,3-dihydro-2*H-*benzimidazol-2-one
37. 3-{1-[*trans*-4-(Ethyloxy)cyclohexyl]-4-piperidinyl}-2-oxo-2,3-dihydro-1*H*-benzimidazole-5-carbonitrile
38. 3-{1-[*trans*-4-(Ethyloxy)-1-methylcyclohexyl]-4-piperidinyl}-2-oxo-2, 3-dihydro-1*H-*benzimidazole-5-carbonitrile
39. 1-{1-[*trans*-4-(Ethyloxy)cyclohexyl]-4-piperidinyl}-6-(methylsulfonyl)-1,3-dihydro-2*H-*benzimidazol-2-one
40. 6-Methyl-1-{1-[*cis*-4-(2-propyn-1-yloxy)cyclohexyl]-4-piperidinyl}-1,3-dihydro-2*H-*benzimidazol-2-one
41. 6-Methyl-1-{1-[*trans*-4-(2-propyn-1-yloxy)cyclohexyl]-4-piperidinyl}-1,3-dihydro-2*H-*benzimidazol-2-one
42. 1-{1-[*trans*-4-(Ethyloxy)-1-methylcyclohexyl]-4-piperidinyl}-6-fluoro-1,3-dihydro-2*H-*benzimidazol-2-one
43. 6-Fluoro-1-{1-[*trans*-1-methyl-4-(methyloxy)cyclohexyl]-4-piperidinyl}-1,3-dihydro-2*H-*benzimidazol-2-one
44. 6-Chloro-1-{1-[*trans*-1-methyl-4-(methyloxy)cyclohexyl]-4-piperidinyl}-1,3-dihydro-2*H-*benzimidazol-2-one
45. 6-(Ethyloxy)-1-{1-[*trans*-1-methyl-4-(methyloxy)cyclohexyl]-4-piperidinyl}-1,3-dihydro-2*H*-benzimidazol-2-one
46. 6-(Ethyloxy)-1-{1-[*trans*-4-(ethyloxy)-1-methylcyclohexyl]-4-piperidinyl}-1,3-dihydro-2*H-*benzimidazol-2-one
47. 6-Chloro-1-{1-[*trans*-4-(ethyloxy)-1-methylcyclohexyl]-4-piperidinyl}-1,3-dihydro-2*H-*benzimidazol-2-one
and salts and solvates thereof, for example the hydrochloride salt, the trifluoroacetate salt or the formate salt.

It will be appreciated by those skilled in the art that certain protected derivatives of compounds of formula (I), which may be made prior to a final deprotection stage, may not possess pharmacological activity as such, but may, in certain instances, be administered orally or parenterally and thereafter metabolised in the body to form compounds of the invention which are pharmacologically active. Such derivatives may therefore be described as "prodrugs". Further, certain compounds of the invention may act as prodrugs of other compounds of the invention. Examples of suitable protecting groups for the compounds of the present invention are described in Drugs of Today, Volume 19, Number 9, 1983, pp 499 - 538 and in Topics in Chemistry, Chapter 31, pp 306 - 316 and in "Design of Prodrugs" by H. Bundgaard, Elsevier, 1985, Chapter 1. It will further be appreciated by those skilled in the art, that certain moieties, known to those skilled in the art as "pro-moieties", for example as described by H. Bundgaard in "Design of Prodrugs" may be placed on appropriate functionalities when such functionalities are present within compounds of the invention. Suitable prodrugs for compounds of the invention include : esters, carbonate esters, hemi-esters, phosphate esters, nitro esters, sulfate esters, sulfoxides, amides, carbamates, azo-compounds, phosphamides, glycosides, ethers, acetals and ketals.

In a further aspect, the invention provides a general process (A1) for preparing compounds of formula (I) in which Q =H, which process comprises:
coupling a compound of formula (II): 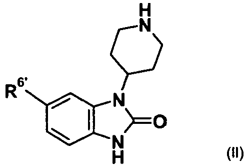
with a compound of formula (III) 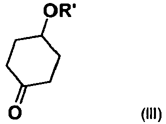
wherein R^{6'} is a group R⁶ as previously defined, or a group convertible to R⁶, and R' is a group R as previously defined, or a group convertible to R.

The reaction is carried out under conditions suitable for reductive alkylation. The reductive alkylation reaction is typically carried out using sodium triacetoxyborohydride in dichloroethane, optionally in the presence of triethylamine, and optionally in the presence of titanium tetraisopropoxide. Alternatively sodium cyanoborohydride can be used as the reducing reagent in solvents such as methanol or ethanol, or the reductive alkylation can be effected under catalytic hydrogenation conditions using a palladium catalyst. In a further variation, the compounds (II) and (III) can be condensed under dehydrating conditions e.g. molecular sieves or magnesium sulfate, and the resultant imine or enamine reduced using for example sodium borohydride or by catalytic hydrogenation.

This reaction can generate a mixture of *cis* and *trans* isomers which can be separated by chromatography or crystallisation.

A modification of general process (A1) is required where Q is C₁₋₆ alkyl. Thus, in general process (A2), a compound of formula (II) can be reacted with a compound of formula (III) in the presence of a source of cyanide, e.g. acetone cyanohydrin, to form the cyano intermediate (XXXX) which can be reacted with an alkyl Grignard reagent QMgX to form compounds of formula (I) in which Q is C₁₋₆ alkyl. 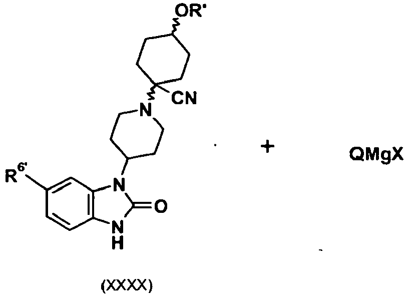 wherein R^{6'} is a group R⁶ as previously defined, or a group convertible to R⁶, and R' is a group R as previously defined, or a group convertible to R, Q is C₁₋₆ alkyl, and X is bromo or iodo or chloro.

This reaction can generate a mixture of *cis* and *trans* isomers which can be separated by chromatography or crystallisation.

In a further aspect, the invention provides a general process (B) for preparing compounds of formula (I) which process comprises:
coupling a compound of formula (IV) 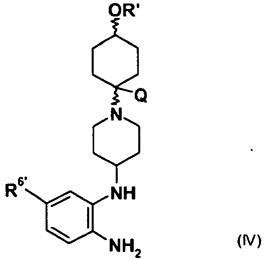
with a compound of formula (V) 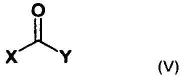
wherein R^{6'} is a group R⁶ as previously defined, or a group convertible to R⁶, R' is a group R as previously defined, or a group convertible to R, Q is as previously defined, and X and Y both represent leaving groups. X and Y can be the same or different and examples are Cl, PhO, EtO, imidazole. When X and Y are both Cl, i.e. phosgene, this reagent can be generated in situ e.g. from diphosgene or triphosgene.

The above reaction is carried out using standard methodology e.g. reacting the diamine (IV) with the reagent (V) in an inert solvent for example dichloromethane or toluene, optionally in the presence of a base such as triethylamine or potassium carbonate, and optionally with heating.

It will be appreciated that compounds of formula (IV) can be pure *cis* or *trans* isomers, or a mixture of isomers. If necessary, separation of pure *cis* and *trans* isomers after the reaction with (V) can be achieved by chromatography or crystallisation.

In a further aspect, the invention provides a general process (C) for preparing compounds of formula (I) which process comprises:
treatment of a compound of formula (VI) 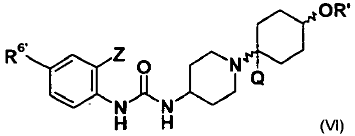
with a palladium or copper catalyst (VII) to effect an intramolecular cyclisation; wherein R^{6'} is a group R⁶ as previously defined, or a group convertible to R⁶, R' is a group R as previously defined, or a group convertible to R, Q is as previously defined, and Z is a leaving group such as bromo, iodo, chloro or triflate.

The cyclisation reaction can be carried out using a variety of palladium or copper reagents as described in the literature (JACS, 2003, 125, 6653, Tet. Lett., 2004, 45, 8535, or JACS, 2002, 124, 7421.)

It will be appreciated that compounds of formula (VI) can be pure *cis* or *trans* isomers, or a mixture of isomers. If necessary, separation of pure *cis* and *trans* isomers after the intramolecular cyclisation can be achieved by chromatography or crystallisation.

In a further aspect, the invention provides a general process (D) for preparing compounds of formula (I) which process comprises:
coupling a compound of formula (VIII) 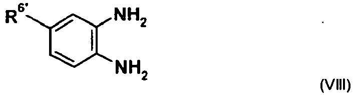
with a compound of formula (IX) 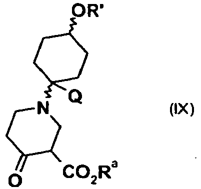 wherein R^{6'} is a group R⁶ as previously defined, or a group convertible to R⁶, R' is a group R as previously defined, or a group convertible to R, Q is as previously defined, and R^{a} is a C₁₋₅ alkyl group.

The condensation and cyclisation reactions can be carried out under reaction conditions similar to those described in the literature for an analogous process (see eg US 3161645) (for example heating in an inert solvent such as xylene) followed by reduction of the piperidine double bond using for example catalytic hydrogenation over palladium or Raney nickel.

It will be appreciated that compounds of formula (IX) can be pure *cis* or *trans* isomers, or a mixture of isomers. If necessary, separation of pure *cis* and *trans* isomers after the intramolecular cyclisation can be achieved by chromatography or crystallisation.

In a further aspect, the invention provides a general process (E) for preparing compounds of formula (I) which process comprises:
reaction of a compound of formula (X) 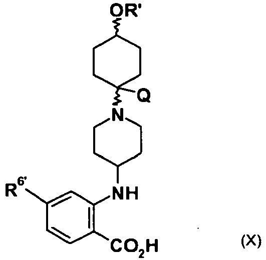
wherein R^{6'} is a group R⁶ as previously defined, or a group convertible to R⁶, R' is a group R as previously defined, or a group convertible to R, and Q is as previously defined; with diphenylphosphoryl azide or other reagent/combination of reagents to effect the Curtius rearrangement of compound (X), followed by intramolecular cyclisation.

The Curtius rearrangement is typically carried out by mixing the two reactants in an inert solvent such as toluene, optionally with heating.

It will be appreciated that compounds of formula (X) can be pure *cis* or *trans* isomers, or a mixture of isomers. If necessary, separation of pure *cis* and *trans* isomers after the intramolecular cyclisation can be achieved by chromatography or crystallisation.

In a further aspect, the invention provides a general process (F) for preparing compounds of formula (I) which process comprises:
coupling a compound of formula (XI): 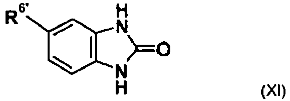 with a compound of formula (XII) 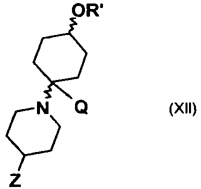
wherein R^{6'} is a group R⁶ as previously defined, or a group convertible to R⁶, R' is a group R as previously defined, or a group convertible to R, Q is as previously defined, and Z is hydroxy or a leaving group such as chloro, bromo or iodo, or alkyl/aryl sulfonate.

The alkylation reaction can be carried out under classical alkylation (Z = a leaving group) or Mitsunobu reaction (Z = OH) conditions. Using classical alkylation conditions, the benzimidazolone intermediate (XI) can be deprotonated using a base such as sodium hydride in an inert solvent such as dimethylformamide, and then treated with the alkylating reagent (XII), optionally with heating. The Mitsunobu reaction with (XII) Z = OH can be carried out using standard conditions e.g. triphenylphosphine and diethylazodicarboxylate in an inert solvent such as dichloromethane or tetrahydrofuran at room temperature.

It will be appreciated that compounds of formula (X) can be pure *cis* or *trans* isomers, or a mixture of isomers. If necessary, separation of pure *cis* and *trans* isomers after the intramolecular cyclisation can be achieved by chromatography or crystallisation.

Conversion of R^{6'} to R⁶ or interconversions of R⁶ may be accomplished as indicated below.

For example, when R^{6'} is a halogen, it can be converted to an alkoxy, trifluoromethyl or methylsulphonyl group by copper catalysed reaction, using an alcohol, methyl fluorosulfonyl(difluoro)acetate or sodium methanesulphinate respectively. It may also be converted to an alkyl group with an organometallic reagent, for example an alkylstannane.

As another example, when R^{6'} is hydroxy, it may be converted to alkoxy by reaction with an alkyl halide or sulfonate, or to trifluoromethoxy by conversion to the xanthate followed by oxidation in the presence of fluoride ion.

As a further example, when R^{6'} is methyl, it may be converted to trifluoromethyl by chlorination or bromination followed by displacement of the introduced halogens with fluoride.

Conversion of R' to R or interconversions of R may be accomplished as indicated below.

For example when R' is benzyl, the benzyl group can be removed using standard methodology, e.g. catalytic hydrogenation over palladium on carbon, to provide the alcohol. Alkylation of the resultant alcohol using a strong base e.g. sodium hydride and a C₁₋₆ alkylating agent e.g. methyl iodide or ethyl iodide or propyl iodide, will afford the desired product. It will be appreciated that protection of any NH functionality present in the molecule may be necessary.

As another example, when R is methyl, the methyl group can be removed by treatment with a dealkylating agent such as boron tribromide to afford the alcohol intermediate, which can be alkylated in a similar manner to that described above. Alternatively the alcohol intermediate can be converted to R is trifluoromethyl by conversion to the xanthate followed by oxidation in the presence of fluoride ion.

Compounds of formula (II) are generally known in the literature or can be prepared by a range of different processes for example:
(a) displacement of an ortho-fluoro or ortho-chloro nitrobenzene intermediate (XIII) with the amine (XIV), wherein R^{6'} is a group R⁶ as previously defined, or a group convertible to R⁶, and P represents a nitrogen protecting group e.g. Boc, acetyl, trifluoroacetyl, ethoxycarbonyl, benzyloxycarbonyl, to give (XXIII), followed by reduction of the nitro group, cyclisation using phosgene or a phosgene equivalent, and deprotection of the piperidine nitrogen using standard literature conditions (Scheme 1). 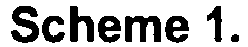 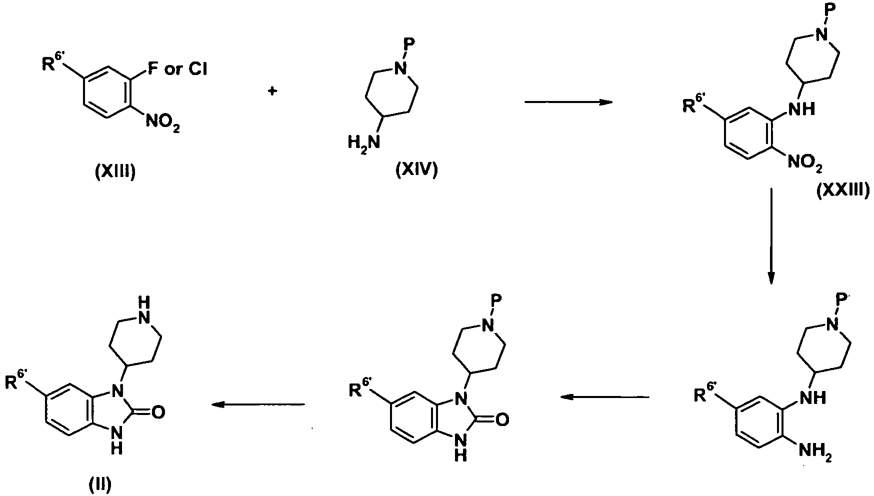 Compounds of formula (XIII) are commercially available or can be prepared by standard methodology. The compound (XIV) in which P = Boc is commercially available
(b) metal catalysed cyclisation of an intermediate (XV) followed by deprotection of the piperidine nitrogen, wherein R^{6'} is a group R⁶ as previously defined, or a group convertible to R⁶, P represents a nitrogen protecting group e.g. Boc, acetyl, trifluoroacetyl, benzyloxycarbonyl, and Z represents a leaving group such as bromo, iodo, chloro or triflate. Reaction conditions for the metal catalysed cyclisation are summarised in Process C. The urea (XV) can be prepared using any of the classical methods for urea formation as illustrated in Scheme 2. The starting materials for this process are commercially available or can be prepared using standard methodology. 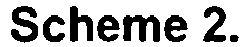 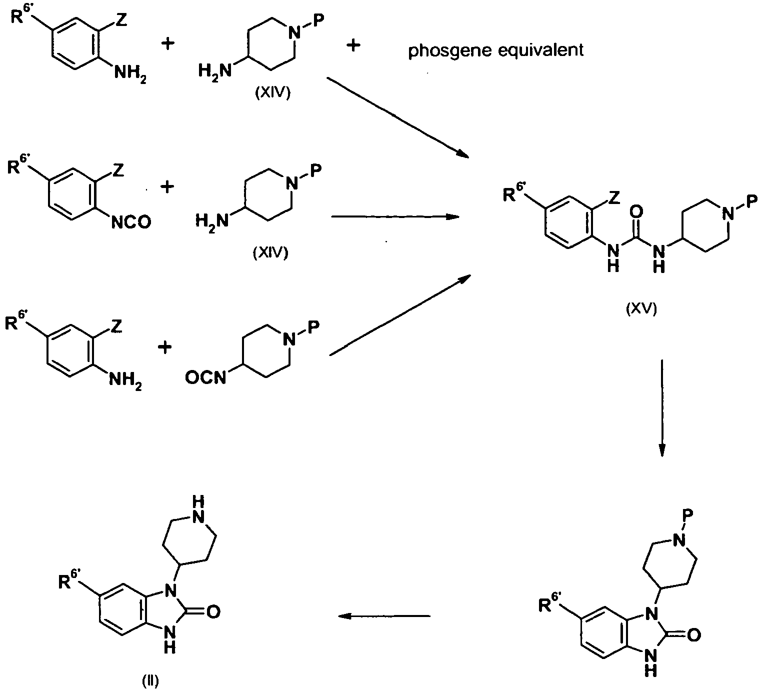
(c) Curtius rearrangement of an intermediate (XVI), wherein R^{6'} is a group R⁶ as previously defined, or a group convertible to R⁶, P represents a nitrogen protecting group e.g. Boc, acetyl, trifluoroacetyl, benzyloxycarbonyl, and R^{b} represents H or a C₁₋₅ alkyl group e.g. methyl or ethyl, followed by intramolecular cyclisation and deprotection of the piperidine nitrogen (Scheme 3). The anthranilic acid or ester starting materials (XVII) are commercially available or can be made by standard methodology. The piperidone starting material (P = Boc or benzyl) is commercially available. The Curtius rearrangement can be effected using the conditions described under process E. 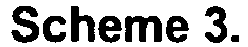 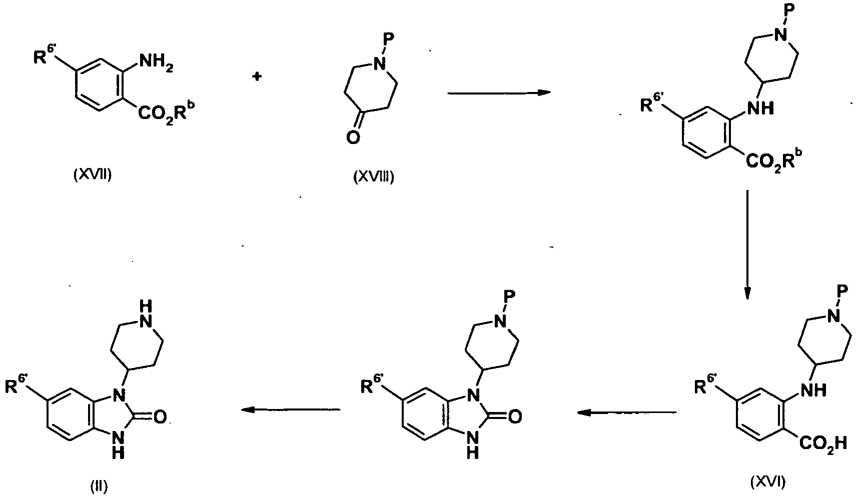
(d) Condensation of an orthophenylenediamine (VIII) with a 3-alkoxycarbonyl-4-piperidone (XX), wherein R^{6'} is a group R⁶ as previously defined, or a group convertible to R⁶, P represents a nitrogen protecting group e.g. Boc, acetyl, trifluoroacetyl, benzyloxycarbonyl and R^{b} is a C₁₋₅ alkyl group (Scheme 4), by heating in an inert solvent at elevated temperature, to afford the tetrahydropyridine intermediate (XXI). Hydrogenation of the double bond and deprotection of the piperidine nitrogen can be accomplished separately or concomitantly dependent on the precise nature of the protecting group P, to afford the desired product (II). Compounds of formula (VIII) are commercially available or can be prepared by standard methodology. Compounds of formula (XX) are commercially available or can be prepared by standard methodology. 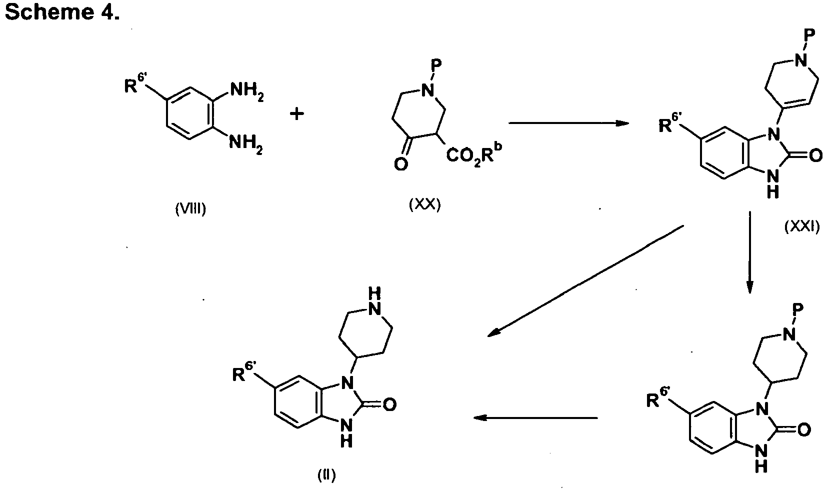
(e) Reductive alkylation of an ortho nitroaniline (XXII) with an N-protected 4-piperidone (XVIII), wherein R^{6'} is a group R⁶ as previously defined, or a group convertible to R⁶, and P represents a nitrogen protecting group e.g. Boc, acetyl, trifluoroacetyl, benzyloxycarbonyl, using for example sodium triacetoxyborohydride to give the intermediate (XXIII). Reduction of the nitro group, followed by cyclisation and deprotection as described hereinbefore provides the desired product (II) (Scheme 5). Compounds of formula (XXII) and (XVIII) are commercially available or can be prepared by standard methodology 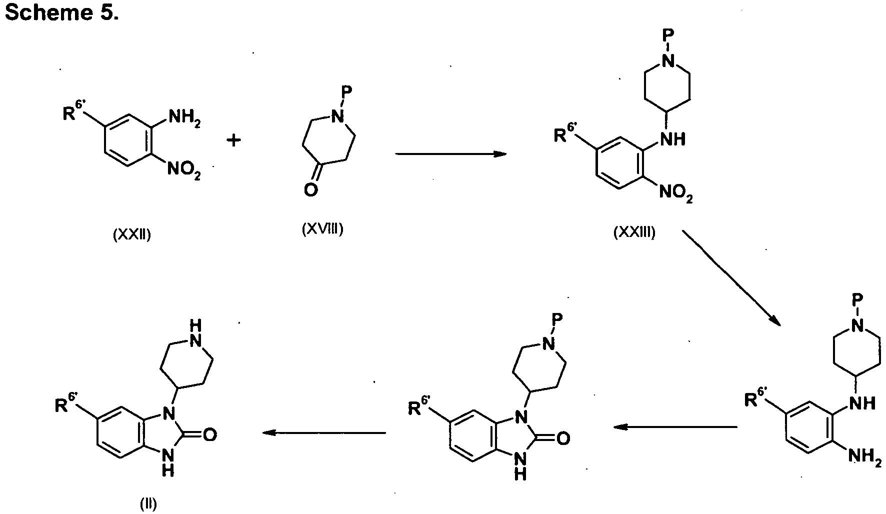
(f) metal catalysed reaction between the amine (XIV) and a suitably substituted nitrobenzene compound (XXIV) wherein R^{6'} is a group R⁶ as previously defined, or a group convertible to R⁶, P represents a nitrogen protecting group e.g. Boc, acetyl, trifluoroacetyl, benzyloxycarbonyl, and Z represents a leaving group such as bromo, iodo, chloro or triflate (Scheme 6). This process generates intermediates of formula (XXIII) and subsequent reactions are similar to that for Scheme 5. Compounds of formula (XXIV) are commercially available or can be prepared by known methodology. The compound (XIV) in which P = Boc is commercially available 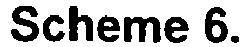 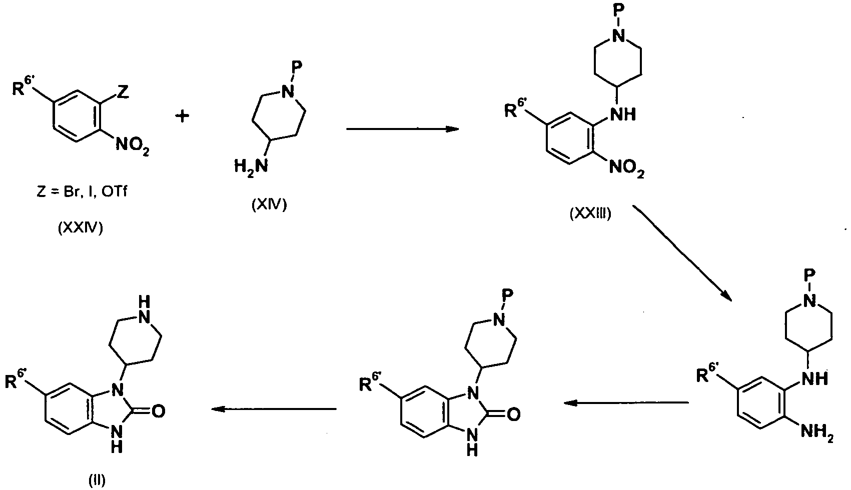
(g) metal catalysed reaction between the amine (XIV) and the protected aniline (XXV), wherein R^{6'} is a group R⁶ as previously defined, or a group convertible to R⁶, P and P' independently represent a nitrogen protecting group e.g. Boc, acetyl, trifluoroacetyl, benzyloxycarbonyl, and Z represents a leaving group such as bromo, iodo, chloro or triflate, to give the intermediate (XXVI) (Scheme 7). Deprotection of the aniline followed by the same reaction sequence as in Scheme 6 affords the desired intermediate (II). Compounds of formula (XXV) are commercially available or can be prepared by known methodology e.g. halogenation ortho to the optionally protected aniline group. The compound (XIV) in which P = Boc is commercially available 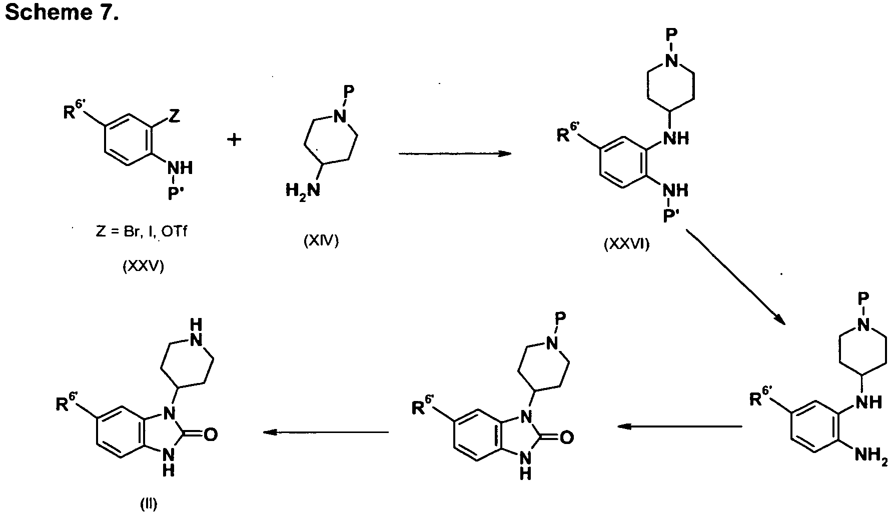 The compounds of formula (III) can be prepared by standard literature methodology.
   Compounds of formula (IV) can be prepared by a number of different processes e.g.
(h) displacement of an ortho-fluoro or ortho-chloro nitrobenzene intermediate (XIII) with the amine (XXVII) wherein R^{6'} is a group R⁶ as previously defined, or a group convertible to R⁶, R' is a group R as previously defined, or a group convertible to R, and Q is as previously defined, to afford compound (XXVIII) followed by reduction of the nitro group using standard conditions e.g. hydrogenation over palladium or Raney nickel (Scheme 8). Compounds of formula (XIII) are commercially available or can be prepared by standard methodology. It will be appreciated that separation of the *cis* and *trans* isomers can be achieved at any suitable stage in the synthesis. 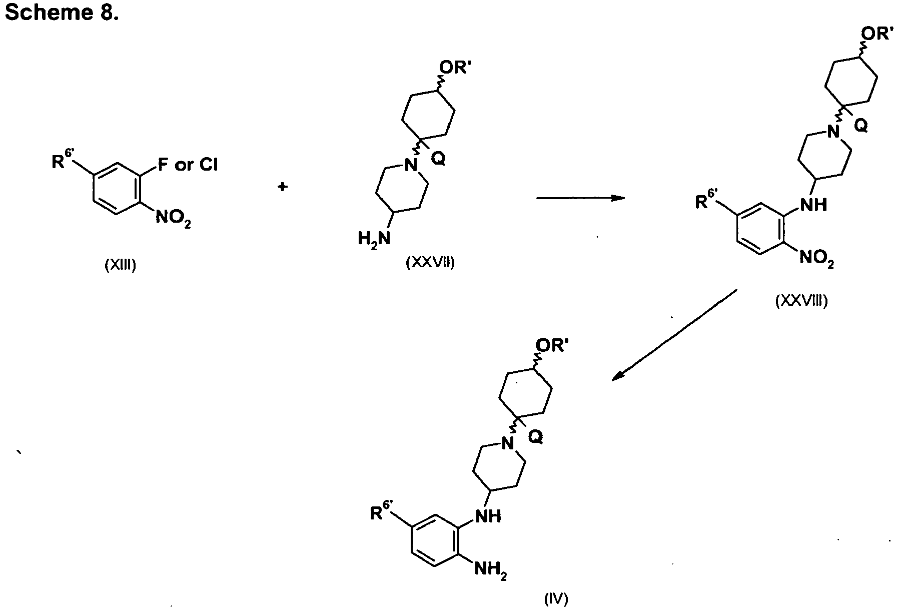
(i) metal catalysed reaction of the amine (XXVII) with the ortho substituted nitrobenzene (XXIX), wherein R^{6'} is a group R⁶ as previously defined, or a group convertible to R⁶, R' is a group R as previously defined, or a group convertible to R, and Q is as previously defined, to afford compound (XXVIII) (Scheme 9) followed by the same reactions as illustrated in Scheme 8. Compounds of formula (XXIX) are commercially available or can be prepared by standard methodology. It will be appreciated that separation of the *cis* and *trans* isomers can be achieved at any suitable stage in the synthesis. 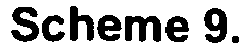 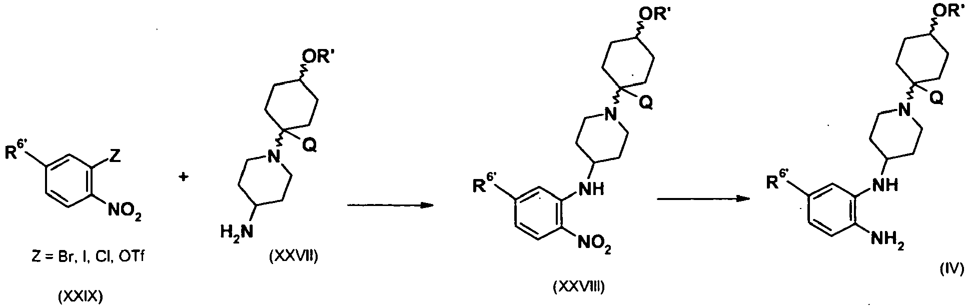
(j) metal catalysed reaction of the amine (XXVII) with the protected aniline derivative (XXV), wherein wherein R^{6'} is a group R⁶ as previously defined, or a group convertible to R⁶, R' is a group R as previously defined, or a group convertible to R, and Q is as previously defined, and P' represents a nitrogen protecting group such as acetyl, trifluoroacetyl, Boc, phthalimide, to afford compound (XXXI) (Scheme 10) followed by deprotection of the aniline group. Compounds of formula (XXV) are commercially available or can be prepared by standard methodology. It will be appreciated that separation of the *cis* and *trans* isomers can be achieved at any suitable stage in the synthesis. 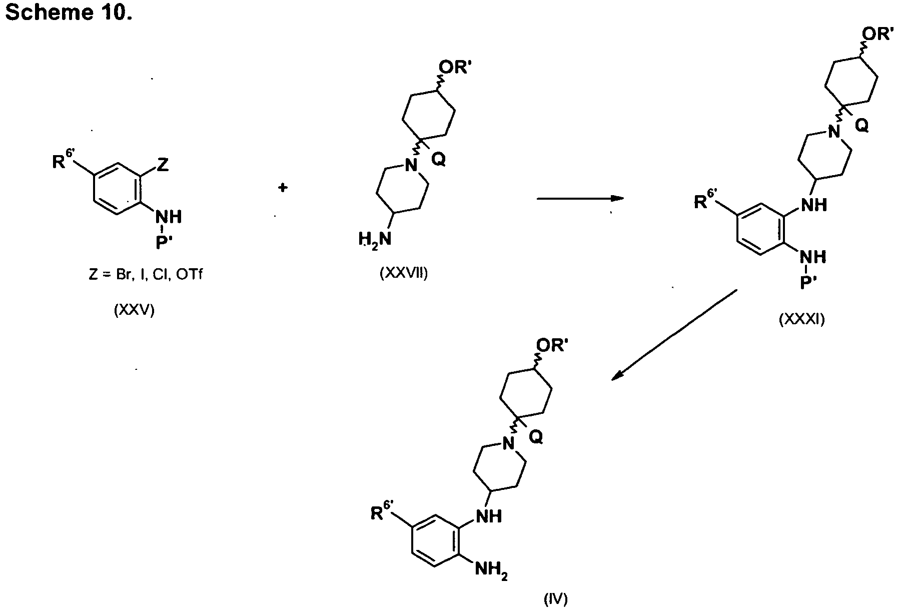
(k) Reductive alkylation of an ortho nitroaniline (XXII) with the piperidone (XXXII) wherein R^{6'} is a group R⁶ as previously defined, or a group convertible to R⁶, R' is a group R as previously defined, or a group convertible to R, and Q is as previously defined, using for example sodium triacetoxyborohydride in dichloroethane to give the intermediate (XXVIII) (Scheme 11). Reduction of the nitro group using, for example, palladium on carbon or Raney nickel affords the desired intermediate (IV). It will be appreciated that separation of the *cis* and *trans* isomers can be achieved at any suitable stage in the synthesis.

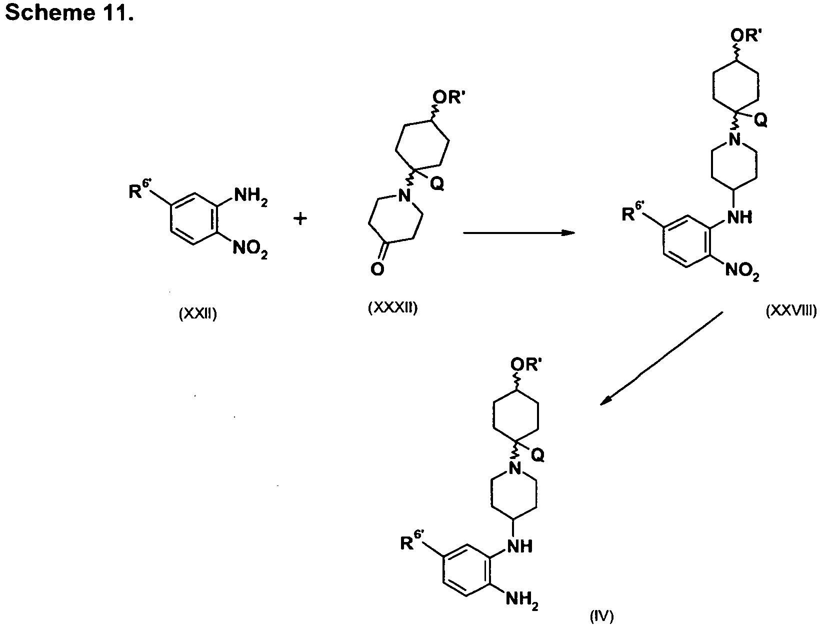

Compounds of formula (V) are commercially available e.g. carbonyl diimidazole, phosgene, phosgene solution in toluene, diphosgene, triphosgene, phenyl chloroformate, diethyl carbonate.

Compounds of formula (VI) can be prepared by a variety of processes e.g. urea formation can be achieved as shown in Scheme 12 by
- combining the two amines (XXXIV) and (XXVII) with phosgene or a phosgene equivalent using standard conditions Phosgene equivalents include carbonyl diimidazole, diphosgene, triphosgene, phenyl chloroformate
- reacting the amine (XXVII) with the isocyanate (XXXV)
- reacting the amine (XXXIV) with the isocyanate (XXXVI)

Both isocyanates can be prepared from the corresponding amines using standard methodology for isocyanate formation.
It will be appreciated that separation of the *cis* and *trans* isomers can be achieved at any suitable stage in the synthesis.

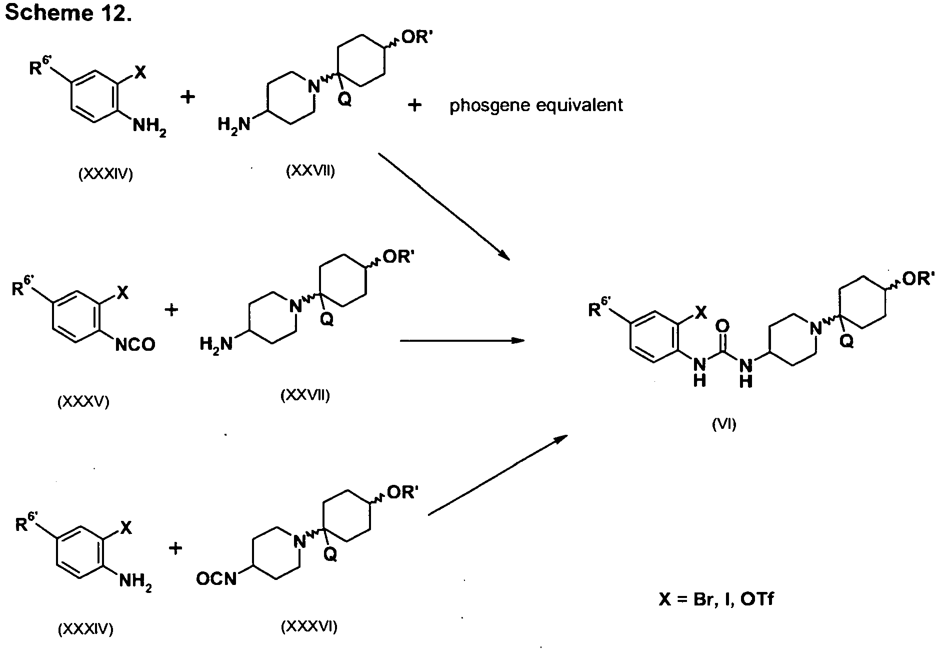

Palladium and copper catalysts (VII) are commercially available or can be prepared as described in the literature (see references in Process C).

Compounds of formula (VIII) are commercially available or can be prepared by known literature routes e.g. reduction of a mono or dinitrobenzene precursor.

Compounds of formula (IX) can be prepared by reductive alkylation of the 3-alkoxycarbonyl-4-piperidone with cyclohexanone (III).

Compounds of formula (X) can be prepared as shown in Scheme 13. Reductive alkylation of an anthranilic acid or ester (XVII) with the ketone (XXXII), followed if appropriate by hydrolysis of the ester group. It will be appreciated that separation of the *cis* and *trans* isomers can be achieved at any suitable stage in the synthesis.

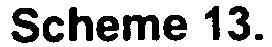

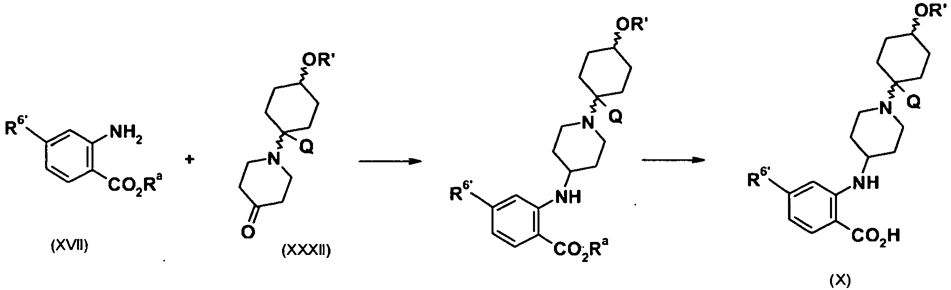

Compounds of formula (XI) are commercially available or can be prepared by literature processes.

Compounds of formula (XII) where Q = H can be prepared as shown in Scheme 14, by reductive alkylation of (XXXVII) where Z' represents Z or a group convertible to Z with the ketone (III). Conversion of a Z' hydroxy group to Z = chloro or bromo can be accomplished using standard methodology e.g. treatment with thionyl chloride or triphenylphosphine/carbon tetrabromide. It will be appreciated that separation of the *cis* and *trans* isomers can be achieved at any suitable stage in the synthesis.

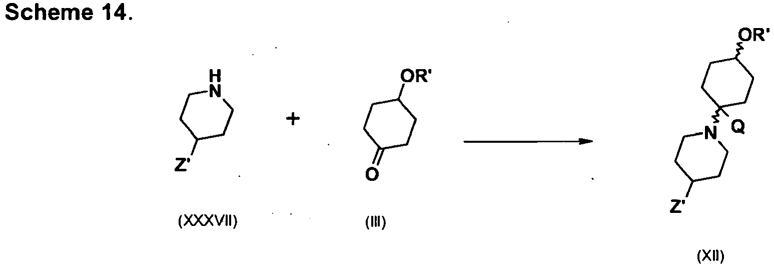

The compound (XXVII) where Q = H can be prepared as shown in Scheme 15. Reductive alkylation of the commercially available amine (XXXVIII) with cyclohexanone (III) using for example sodium triacetoxyborohydride in dichloroethane provides the intermediate (XXXIX) which is deprotected using HCl in ethanol or trifluoroacetic acid to afford the primary amine (XXVII). It will be appreciated that separation of the *cis* and *trans* isomers can be achieved at any suitable stage in the synthesis.

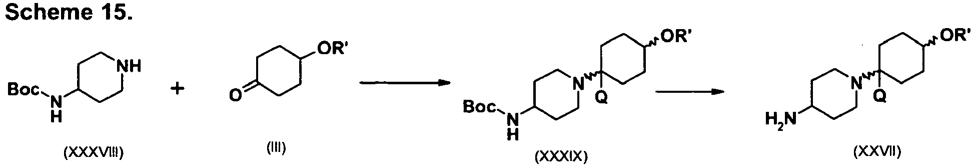

The compound (XXVII) where Q = alkyl can be prepared as in process A2, followed by deprotection.

Alternatively the compound (XXVII) where Q = H can be prepared as shown in Scheme 16. Reductive amination of cyclohexanone (III) using for example ammonia solution under catalytic hydrogenation conditions provides intermediate amine (XL), which can be converted into piperidinone (XLII) by reaction with quaternary piperidine salt (XLI). Reductive amination, for example using ammonia and catalytic hydrogenation, affords primary amine (XXVII). It will be appreciated that separation of the *cis* and *trans* isomers can be achieved at any suitable stage in the synthesis.

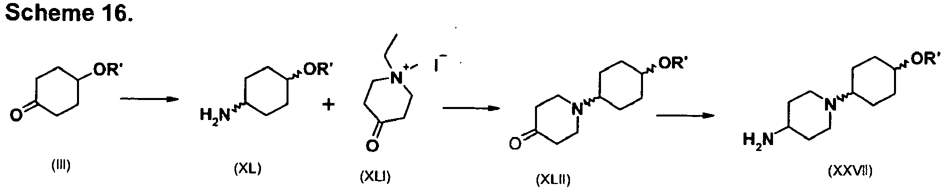

Selective preparation of the cis and trans isomers of compound (XXVII) where Q =H can be carried out via the process which is illustrated in scheme 17 for the trans isomer. The commercially available amine (XLIII) is initially N-protected for example by incorporation in a phthalimide ring to give (XLIV), then the hydroxyl function converted into a silyl protected group, for example tert-butyldimethylsifyl, to give (XLV), which upon treatment with the appropriate aldehyde or ketone in presence of triethylsilane and bismuth tribromide affords (XLVI), which after deprotection gives intermediate amine (XLVII). Conversion of amine (XLVII) to the piperidinone (XLVIII) by reaction with quaternary piperidine salt (XLI) followed by reductive amination, for example using ammonia and catalytic hydrogenation, affords primary amine (XXVII).

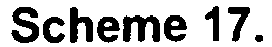

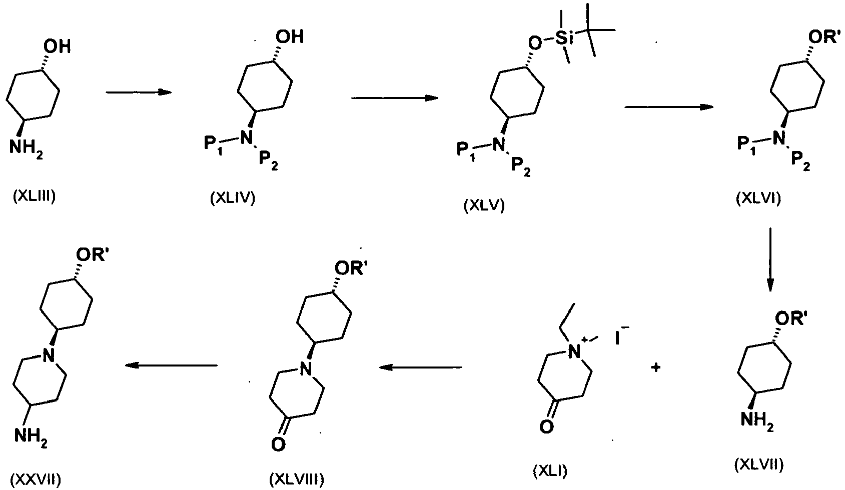

The cis isomer of compound (XXVII) can be prepared by a similar procedure from the appropriate cis amine (XLIII).

A selective preparation of the trans isomer of compound (XXVII) where Q = Me is shown in scheme 18. A suitable ester of 4-hydroxycyclohexane carboxylic acid such as methyl or ethyl (XLIX) is protected as a silyl ether, for example tert-butyldimethylsilyl, to give (L), which on treatment with the appropriate aldehyde or ketone in presence of triethylsilane and bismuth tribromide affords ether (LI). Hydrolysis to acid (LII) followed by alkylation using a base such as lithium diisopropylamide with iodomethane affords mixture of cis and trans 1-methylcyclohexane carboxylic acid (LIIII). The trans isomer (LIV) can be isolated by conversion of the mixture to the acid chloride, for example with thionyl chloride, followed by selective hydrolysis of the products by treatment with weak aqueous base such as sodium hydrogen carbonate solution. The acid can be converted to the isocyanate (LV) by Curtius rearrangement at elevated temperature of an intermediate azide prepared using for example diphenylphosphoryl azide, then the isocyanate hydrolysed to amine (LVI) under acidic conditions. Conversion of amine (LVI) to the piperidinone (LVII) by reaction with quaternary piperidine salt (XLI) followed by reductive amination, for example using ammonia and catalytic hydrogenation, affords primary amine (XXVII).

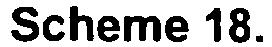

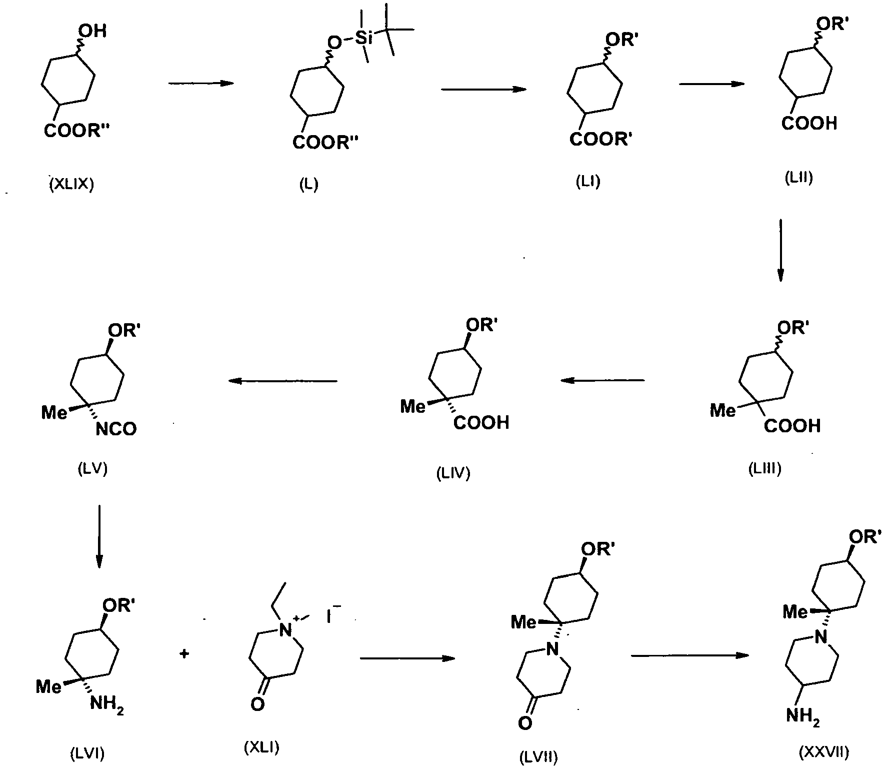

The cis isomer of (XXVII) where Q = Me can be obtained as shown in scheme 19. The mixture of acids (LIII) is converted to a mixture of isocyantes (LVIII) via Curtius rearrangement of an intermediate azide at elevated temperature prepared using for example diphenylphosphoryl azide. The cis isomer (LIX) can be isolated from this mixture by chromatographic separation, then converted through to amine (XXVII) using a similar procedure to that as shown for the trans isomer in scheme 17 through intermediates (LX) and (LXI).

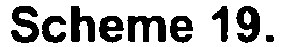

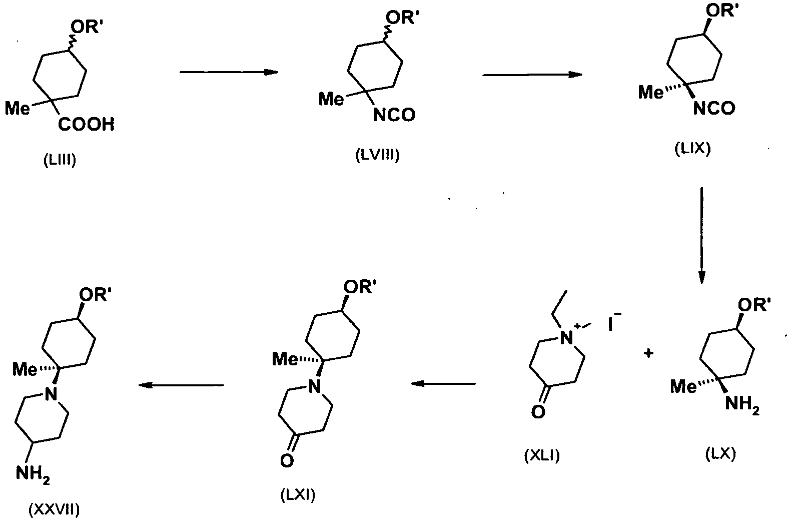

Compounds of the present invention are M₁ receptor agonists. Selective M₁ receptor agonists are said to be useful to ameliorate positive and cognitive symptoms of psychotic disorders such as schizophrenia, schizo-affective disorders, schizophreniform diseases, psychotic depression, mania, acute mania, paranoid and delusional disorders, and cognitive impairment including memory disorders such as Alzheimer's disease without peripheral cholinergic side effects mediated predominantly through M₂ and M₃ receptors. M₁ receptor agonists may also be suitable for combination with other typical and atypical antipsychotics and other actives such as mood stabilisers, antidepressants, anxiolytics, drugs for extrapyrimidal side effects and cognitive enhancers, to provide improved treatment of psychotic disorders.

Thus in a further aspect, the invention provides a compound of formula (I) as hereinbefore described or a salt or solvate thereof for use in therapy.

In another aspect, the invention provides a compound of formula (I) or a salt or solvate thereof for use in the treatment of a condition which requires agonism of a muscarinic M₁ receptor.

The terms describing the indications used herein are classified in the Diagnostic and Statistical Manual of Mental Disorders, 4th Edition, published by the American Psychiatric Association (DSM-IV) and/or the International Classification of Diseases, 10th Edition (ICD-10). The various subtypes of the disorders mentioned herein are contemplated as part of the present invention. Numbers in brackets after the listed diseases below refer to the classification code in DSM-IV.

Within the context of the present invention, the term psychotic disorder includes Schizophrenia including the subtypes Paranoid Type (295.30), Disorganised Type (295.10), Catatonic Type (295.20), Undifferentiated Type (295.90) and Residual Type (295.60); Schizophreniform Disorder (295.40); Schizoaffective Disorder (295.70) including the subtypes Bipolar Type and Depressive Type; Delusional Disorder (297.1) including the subtypes Erotomanic Type, Grandiose Type, Jealous Type, Persecutory Type, Somatic Type, Mixed Type and Unspecified Type; Brief Psychotic Disorder (298.8); Shared Psychotic Disorder (297.3); Psychotic Disorder Due to a General Medical Condition including the subtypes With Delusions and With Hallucinations; Substance-Induced Psychotic Disorder including the subtypes With Delusions (293.81) and With Hallucinations (293.82); and Psychotic Disorder Not Otherwise Specified (298.9);

Other conditions the treatment of which require agonism of a muscarinic M₁ receptor include:

Depression and mood disorders including Major Depressive Episode, Manic Episode, Mixed Episode and Hypomanic Episode; Depressive Disorders including Major Depressive Disorder, Dysthymic Disorder (300.4), Depressive Disorder Not Otherwise Specified (311); Bipolar Disorders including Bipolar I Disorder, Bipolar II Disorder (Recurrent Major Depressive Episodes with Hypomanic Episodes) (296.89), Cyclothymic Disorder (301.13) and Bipolar Disorder Not Otherwise Specified (296.80); Other Mood Disorders including Mood Disorder Due to a General Medical Condition (293.83) which includes the subtypes With Depressive Features, With Major Depressive-like Episode, With Manic Features and With Mixed Features), Substance-Induced Mood Disorder (including the subtypes With Depressive Features, With Manic Features and With Mixed Features) and Mood Disorder Not Otherwise Specified (296.90);

Anxiety disorders including Social Anxiety Disorder, Panic Attack, Agoraphobia, Panic Disorder, Agoraphobia Without History of Panic Disorder (300.22), Specific Phobia (300.29) including the subtypes Animal Type, Natural Environment Type, Blood-Injection-Injury Type, Situational Type and Other Type), Social Phobia (300.23), Obsessive-Compulsive Disorder (300.3), Posttraumatic Stress Disorder (309.81), Acute Stress Disorder (308.3), Generalized Anxiety Disorder (300.02), Anxiety Disorder Due to a General Medical Condition (293.84), Substance-Induced Anxiety Disorder and Anxiety Disorder Not Otherwise Specified (300.00);

Substance-related disorders including Substance Use Disorders such as Substance Dependence, Substance Craving and Substance Abuse; Substance-Induced Disorders such as Substance Intoxication, Substance Withdrawal, Substance-Induced Delirium, Substance-Induced Persisting Dementia, Substance-Induced Persisting Amnestic Disorder, Substance-Induced Psychotic Disorder, Substance-Induced Mood Disorder, Substance-Induced Anxiety Disorder, Substance-Induced Sexual Dysfunction, Substance-Induced Sleep Disorder and Hallucinogen Persisting Perception Disorder (Flashbacks); Alcohol-Related Disorders such as Alcohol Dependence (303.90), Alcohol Abuse (305.00), Alcohol Intoxication (303.00), Alcohol Withdrawal (291.81), Alcohol Intoxication Delirium, Alcohol Withdrawal Delirium, Alcohol-Induced Persisting Dementia, Alcohol-Induced Persisting Amnestic Disorder, Alcohol-Induced Psychotic Disorder, Alcohol-Induced Mood Disorder, Alcohol-Induced Anxiety Disorder, Alcohol-Induced Sexual Dysfunction, Alcohol-Induced Sleep Disorder and Alcohol-Related Disorder Not Otherwise Specified (291.9); Amphetamine (or Amphetamine-Like)-Related Disorders such as Amphetamine Dependence (304.40), Amphetamine Abuse (305.70), Amphetamine Intoxication (292.89), Amphetamine withdrawal (292.0), Amphetamine Intoxication Delirium, Amphetamine Induced Psychotic Disorder, Amphetamine-Induced Mood Disorder, Amphetamine-Induced Anxiety Disorder, Amphetamine-Induced Sexual Dysfunction, Amphetamine-Induced Sleep Disorder and Amphetamine-Related Disorder Not Otherwise Specified (292.9); Caffeine Related Disorders such as Caffeine Intoxication (305.90), Caffeine-Induced Anxiety Disorder, Caffeine-Induced Sleep Disorder and Caffeine-Related Disorder Not Otherwise Specified (292.9); Cannabis-Related Disorders such as Cannabis Dependence (304.30), Cannabis Abuse (305.20), Cannabis Intoxication (292.89), Cannabis Intoxication Delirium, Cannabis-Induced Psychotic Disorder, Cannabis-Induced Anxiety Disorder and Cannabis-Related Disorder Not Otherwise Specified (292.9); Cocaine-Related Disorders such as Cocaine Dependence (304.20), Cocaine Abuse (305.60), Cocaine Intoxication (292.89), Cocaine Withdrawal (292.0), Cocaine Intoxication Delirium, Cocaine-Induced Psychotic Disorder, Cocaine-Induced Mood Disorder, Cocaine-Induced Anxiety Disorder, Cocaine-Induced Sexual Dysfunction, Cocaine-Induced Sleep Disorder and Cocaine-Related Disorder Not Otherwise Specified (292.9); Hallucinogen-Related Disorders such as Hallucinogen Dependence (304.50), Hallucinogen Abuse (305.30), Hallucinogen Intoxication (292.89), Hallucinogen Persisting Perception Disorder (Flashbacks) (292.89), Hallucinogen Intoxication Delirium, Hallucinogen-Induced Psychotic Disorder, Hallucinogen-Induced Mood Disorder, Hallucinogen-Induced Anxiety Disorder and Hallucinogen-Related Disorder Not Otherwise Specified (292.9); Inhalant-Related Disorders such as Inhalant Dependence (304.60), Inhalant Abuse (305.90), Inhalant Intoxication (292.89), Inhalant Intoxication Delirium, Inhalant-Induced Persisting Dementia, Inhalant-Induced Psychotic Disorder, Inhalant-Induced Mood Disorder, Inhalant-Induced Anxiety Disorder and Inhalant-Related Disorder Not Otherwise Specified (292.9); Nicotine-Related Disorders such as Nicotine Dependence (305.1), Nicotine Withdrawal (292.0) and Nicotine-Related Disorder Not Otherwise Specified (292.9); Opioid-Related Disorders such as Opioid Dependence (304.00), Opioid Abuse (305.50), Opioid Intoxication (292.89), Opioid Withdrawal (292.0), Opioid Intoxication Delirium, Opioid-Induced Psychotic Disorder, Opioid-Induced Mood Disorder, Opioid-Induced Sexual Dysfunction, Opioid-Induced Sleep Disorder and Opioid-Related Disorder Not Otherwise Specified (292.9); Phencyclidine (or Phencyclidine-Like)-Related Disorders such as Phencyclidine Dependence (304.60), Phencyclidine Abuse (305.90), Phencyclidine Intoxication (292.89), Phencyclidine Intoxication Delirium, Phencyclidine-Induced Psychotic Disorder, Phencyclidine-Induced Mood Disorder, Phencyclidine-Induced Anxiety Disorder and Phencyclidine-Related Disorder Not Otherwise Specified (292.9); Sedative-, Hypnotic-, or Anxiolytic-Related Disorders such as Sedative, Hypnotic, or Anxiolytic Dependence (304.10), Sedative, Hypnotic, or Anxiolytic Abuse (305.40), Sedative, Hypnotic, or Anxiolytic Intoxication (292.89), Sedative, Hypnotic, or Anxiolytic Withdrawal (292.0), Sedative, Hypnotic, or Anxiolytic Intoxication Delirium, Sedative, Hypnotic, or Anxiolytic Withdrawal Delirium, Sedative-, Hypnotic-, or Anxiolytic-Persisting Dementia, Sedative-, Hypnotic-, or Anxiolytic- Persisting Amnestic Disorder, Sedative-, Hypnotic-, or Anxiolytic-Induced Psychotic Disorder, Sedative-, Hypnotic-, or Anxiolytic-Induced Mood Disorder, Sedative-, Hypnotic-, or Anxiolytic-Induced Anxiety Disorder Sedative-, Hypnotic-, or Anxiolytic-Induced Sexual Dysfunction, Sedative-, Hypnotic-, or Anxiolytic-Induced Sleep Disorder and Sedative-, Hypnotic-, or Anxiolytic-Related Disorder Not Otherwise Specified (292.9); Polysubstance-Related Disorder such as Polysubstance Dependence (304.80); and Other (or Unknown) Substance-Related Disorders such as Anabolic Steroids, Nitrate Inhalants and Nitrous Oxide;

Sleep disorders including primary sleep disorders such as Dyssomnias such as Primary Insomnia (307.42), Primary Hypersomnia (307.44), Narcolepsy (347), Breathing-Related Sleep Disorders (780.59), Circadian Rhythm Sleep Disorder (307.45) and Dyssomnia Not Otherwise Specified (307.47); primary sleep disorders such as Parasomnias such as Nightmare Disorder (307.47), Sleep Terror Disorder (307.46), Sleepwalking Disorder (307.46) and Parasomnia Not Otherwise Specified (307.47); Sleep Disorders Related to Another Mental Disorder such as Insomnia Related to Another Mental Disorder (307.42) and Hypersomnia Related to Another Mental Disorder (307.44); Sleep Disorder Due to a General Medical Condition; and Substance-induced Sleep Disorder including the subtypes Insomnia Type, Hypersomnia Type, Parasomnia Type and Mixed Type;

Eating disorders such as Anorexia Nervosa (307.1) including the subtypes Restricting Type and Binge-Eating/Purging Type; Bulimia Nervosa (307.51) including the subtypes Purging Type and Nonpurging Type; Obesity; Compulsive Eating Disorder; and Eating Disorder Not Otherwise Specified (307.50);

Autistic Disorder (299.00); Attention-Deficit /Hyperactivity Disorder including the subtypes Attention-Deficit /Hyperactivity Disorder Combined Type (314.01), Attention-Deficit /Hyperactivity Disorder Predominantly Inattentive Type (314.00), Attention-Deficit /Hyperactivity Disorder Hyperactive-Impulse Type (314.01) and Attention-Deficit /Hyperactivity Disorder Not Otherwise Specified (314.9); Hyperkinetic Disorder; Disruptive Behaviour Disorders such as Conduct Disorder including the subtypes childhood-onset type (321.81), Adolescent-Onset Type (312.82) and Unspecified Onset (312.89), Oppositional Defiant Disorder (313.81) and Disruptive Behaviour Disorder Not Otherwise Specified; and Tic Disorders such as Tourette's Disorder (307.23);

Personality Disorders including the subtypes Paranoid Personality Disorder (301.0), Schizoid Personality Disorder (301.20), Schizotypal Personality Disorder (301,22), Antisocial Personality Disorder (301.7), Borderline Personality Disorder (301,83), Histrionic Personality Disorder (301.50), Narcissistic Personality Disorder (301,81), Avoidant Personality Disorder (301.82), Dependent Personality Disorder (301.6), Obsessive-Compulsive Personality Disorder (301.4) and Personality Disorder Not Otherwise Specified (301.9); and

Sexual dysfunctions including Sexual Desire Disorders such as Hypoactive Sexual Desire Disorder (302.71), and Sexual Aversion Disorder (302.79); sexual arousal disorders such as Female Sexual Arousal Disorder (302.72) and Male Erectile Disorder (302.72); orgasmic disorders such as Female Orgasmic Disorder (302.73), Male Orgasmic Disorder (302.74) and Premature Ejaculation (302.75); sexual pain disorder such as Dyspareunia (302.76) and Vaginismus (306.51); Sexual Dysfunction Not Otherwise Specified (302.70); paraphilias such as Exhibitionism (302.4), Fetishism (302.81), Frotteurism (302.89), Pedophilia (302.2), Sexual Masochism (302.83), Sexual Sadism (302.84), Transvestic Fetishism (302.3), Voyeurism (302.82) and Paraphilia Not Otherwise Specified (302.9); gender identity disorders such as Gender Identity Disorder in Children (302.6) and Gender Identity Disorder in Adolescents or Adults (302.85); and Sexual Disorder Not Otherwise Specified (302.9).

The compounds of formula (I) may also be useful for the enhancement of cognition, including both the treatment of cognitive impairment on its own and the treatment of cognition impairment in other diseases such as schizophrenia, bipolar disorder, depression, other psychiatric disorders and psychotic conditions associated with cognitive impairment.

Within the context of the present invention, the term cognitive impairment includes, for example, impairment of cognitive functions including attention, orientation, learning disorders, memory (i.e. memory disorders, amnesia, amnesic disorders, transient global amnesia syndrome and age-associated memory impairment) and language function; cognitive impairment as a result of stroke, Alzheimer's disease, Huntington's disease, Pick disease, Aids-related dementia or other dementia states such as Multiinfarct dementia, alcoholic dementia, hypotiroidism-related dementia, and dementia associated to other degenerative disorders such as cerebellar atrophy and amyotropic lateral sclerosis; other acute or sub-acute conditions that may cause cognitive decline such as delirium or depression (pseudodementia states) trauma, head trauma, age related cognitive decline, stroke, neurodegeneration, drug-induced states, neurotoxic agents, mild cognitive impairment, age related cognitive impairment, autism related cognitive impairment, Down's syndrome, cognitive deficit related to psychosis, and post-electroconvulsive treatment related cognitive disorders; and dyskinetic disorders such as Parkinson's disease, neuroleptic-induced parkinsonism, and tardive dyskinesias.

The therapy of the present invention may also be used as a memory and/or cognition enhancer in healthy humans with no cognitive and/or memory deficit.

In another aspect, the invention provides a compound of formula (I) as hereinbefore described or a salt or solvate thereof for use in the treatment of a psychotic disorder. In one embodiment, the invention provides a compound of formula (I) as hereinbefore described or a salt or solvate thereof for use in the treatment of schizophrenia.

The invention also provides a compound of formula (I) as hereinbefore described or a salt or solvate thereof for use in the treatment of cognitive impairment.

In another aspect, the invention provides the use of a compound of formula (I) as hereinbefore described or a salt or solvate thereof in the manufacture of a medicament for the treatment of a condition which requires agonism of a muscarinic M₁ receptor.

In another aspect, the invention provides the use of a compound of formula (I) as hereinbefore described or a salt or solvate thereof in the manufacture of a medicament for the treatment of a psychotic disorder. In one embodiment, the invention provides the use of a compound of formula (I) as hereinbefore described or a salt or solvate thereof in the manufacture of a medicament for the treatment of schizophrenia.

The invention also provides the use of a compound of formula (I) as hereinbefore described or a salt or solvate thereof in the manufacture of a medicament for the treatment of cognitive impairment.

The compounds of formula (I) and their salts and solvates thereof may alsbo be suitable for combination with other actives, such as typical and atypical antipsychotics, mood stabilisers, antidepressants, anxiolytics, drugs for extrapyrimidal side effects and cognitive enhancers to provide improved treatment of psychotic disorders.

The combination therapies of the invention are, for example, administered adjunctively. By adjunctive administration is meant the coterminous or overlapping administration of each of the components in the form of separate pharmaceutical compositions or devices. This regime of therapeutic administration of two or more therapeutic agents is referred to generally by those skilled in the art and herein as adjunctive therapeutic administration; it is also known as add-on therapeutic administration. Any and all treatment regimes in which a patient receives separate but coterminous or overlapping therapeutic administration of the compounds of formula (I) or a salt or solvate thereof and at least one antipsychotic agent, a mood stabiliser, an antidepressant, an anxiolytic, a drug for extrapyrimidal side effects or a cognitive enhancer are within the scope of the current invention. In one embodiment of adjunctive therapeutic administration as described herein, a patient is typically stabilised on a therapeutic administration of one or more of the components for a period of time and then receives administration of another component. The compounds of formula (I) or a salt or solvate thereof may be administered as adjunctive therapeutic treatment to patients who are receiving administration of at least one antipsychotic agent, a mood stabiliser, an antidepressant, an anxiolytic, a drug for extrapyrimidal side effects or a cognitive enhancer, but the scope of the invention also includes the adjunctive therapeutic administration of at least one antipsychotic agent, a mood stabiliser, an antidepressant, an anxiolytic, a drug for extrapyrimidal side effects or a cognitive enhancer to patients who are receiving administration of compounds of formula (I) or a salt or solvate thereof.

The combination therapies of the invention may also be administered simultaneously. By simultaneous administration is meant a treatment regime wherein the individual components are administered together, either in the form of a single pharmaceutical composition or device comprising or containing both components, or as separate compositions or devices, each comprising one of the components, administered simultaneously. Such combinations of the separate individual components for simultaneous combination may be provided in the form of a kit-of-parts.

In a further aspect, the invention provides the use of compounds of formula (I) or a salt or solvate thereof in the manufacture of a medicament for adjunctive therapeutic administration for the treatment of a psychotic disorder in a patient receiving therapeutic administration of at least one antipsychotic agent. The invention further provides compounds of formula (I) or a salt or solvate thereof for use for adjunctive therapeutic administration for the treatment of a psychotic disorder in a patient receiving therapeutic administration of at least one antipsychotic agent.

In a further aspect, the invention provides the use of at least one antipsychotic agent in the manufacture of a medicament for adjunctive therapeutic administration for the treatment of a psychotic disorder in a patient receiving therapeutic administration of compounds of formula (I) or a salt or solvate thereof. The invention further provides at least one antipsychotic agent for adjunctive therapeutic administration for the treatment of a psychotic disorder in a patient receiving therapeutic administration of compounds of formula (I) or a salt or solvate thereof.

The invention further provides the use of a combination of compounds of formula (I) or a salt or solvate thereof and at least one antipsychotic agent in the manufacture of a medicament for simultaneous therapeutic administration in the treatment of a psychotic disorder. The invention further provides the use of compounds of formula (I) or a salt thereof in the manufacture of a medicament for simultaneous therapeutic administration with at least one antipsychotic agent in the treatment of a psychotic disorder. The invention further provides compounds of formula (I) or a salt thereof for use for simultaneous therapeutic administration with at least one antipsychotic agent in the treatment of a psychotic disorder. The invention further provides the use of at least one antipsychotic agent in the manufacture of a medicament for simultaneous therapeutic administration with compounds of formula (I) or a salt thereof in the treatment of a psychotic disorder.

In a further aspect, the invention provides a kit-of-parts for use in the treatment of a psychotic disorder comprising a first dosage form comprising compounds of formula (I) or a salt or solvate thereof and one or more further dosage forms each comprising a antipsychotic agent for simultaneous therapeutic administration.

In a further aspect, the invention provides the use of a compound of the present invention in the manufacture of a medicament for adjunctive therapeutic administration for the treatment of a psychotic disorder in a patient receiving therapeutic administration of an active ingredient selected from the group consisting of: a mood stabiliser, an antidepressant, an anxiolytic, a drug for extrapyrimidal side effects and a cognitive enhancer.

In a further aspect, the invention provides the use of an active ingredient selected from the group consisting of: a mood stabiliser, an antidepressant, an anxiolytic, a drug for extrapyrimidal side effects and a cognitive enhancer in the manufacture of a medicament for adjunctive therapeutic administration for the treatment of a psychotic disorder in a patient receiving therapeutic administration of a compound of the present invention.

The invention further provides the use of a combination of a compound of the present invention and an active ingredient selected from the group consisting of: a mood stabiliser, an antidepressant, an anxiolytic, a drug for extrapyrimidal side effects and a cognitive enhancer in the manufacture of a medicament for simultaneous therapeutic administration in the treatment of a psychotic disorder.

The invention further provides the use of a compound of the present invention in the manufacture of a medicament for simultaneous therapeutic administration with an active ingredient selected from the group consisting of: a mood stabiliser, an antidepressant, an anxiolytic, a drug for extrapyrimidal side effects and a cognitive enhancer in the treatment of a psychotic disorder.

The invention further provides a compound of the present invention for use for simultaneous therapeutic administration with an active ingredient selected from the group consisting of: a mood stabiliser, an antidepressant, an anxiolytic, a drug for extrapyrimidal side effects and a cognitive enhancer in the treatment of a psychotic disorder.

The invention further provides the use of an active ingredient selected from the group consisting of: a mood stabiliser, an antidepressant, an anxiolytic, a drug for extrapyrimidal side effects and a cognitive enhancer in the manufacture of a medicament for simultaneous therapeutic administration with a compound of the present invention in the treatment of a psychotic disorder.

In a further aspect, the invention provides a kit-of-parts for use in the treatment of a psychotic disorder comprising a first dosage form comprising a compound of the present invention and one or more further dosage forms each comprising an active ingredient selected from the group consisting of: a mood stabiliser, an antidepressant, an anxiolytic, a drug for extrapyrimidal side effects and a cognitive enhancer for simultaneous therapeutic administration.

In one embodiment, the patient is a human.

Examples of antipsychotic drugs that may be useful in the present invention include, but are not limited to: sodium channel blockers; mixed 5HT/dopamine receptor antagonists; mGluR5 positive modulators; D3 antagonists; 5HT6 angatonists; nicotinic alpha-7 modulators; glycine transporter GlyT1 inhibitors; D2 partial agonist/D3 antanogist/H3 antagonists; AMPA modulators; NK3 antagonists such as osanetant and talnetant; an atypical antipsychotic, for example clozapine, olanzapine, risperidone, quetiapine, aripirazole, ziprasidone and amisulpride; butyrophenones, such as haloperidol, pimozide, and droperidol; phenothiazines, such as chlorpromazine, thioridazine, mesoridazine, trifluoperazine, perphenazine, fluphenazine, thiflupromazine, prochlorperazine, and acetophenazine; thioxanthenes, such as thiothixene and chlorprothixene; thienobenzodiazepines; dibenzodiazepines; benzisoxazoles; dibenzothiazepines; imidazolidinones; benzisothiazolyl-piperazines; triazine such as lamotrigine; dibenzoxazepines, such as loxapine; dihydroindolones, such as molindone; aripiprazole; and derivatives thereof that have antipsychotic activity.

Examples of tradenames and suppliers of selected antipsychotic drugs that may be suitable for use in the present invention are as follows : clozapine (available under the tradename CLOZARIL®, from Mylan, Zenith Goldline, UDL, Novartis); olanzapine (available under the tradename ZYPREXA®, from Lilly ; ziprasidone (available under the tradename GEODON®, from Pfizer); risperidone (available under the tradename RISPERDAL®, from Janssen); quetiapine fumarate (available under the tradename SEROQUEL®, from AstraZeneca); sertindole (available under the tradename SERLECT®); amisulpride (available under the tradename SOLION®, from Sanofi-Synthelabo); haloperidol (available under the tradename HALDOL®, from Ortho-McNeil); haloperidol decanoate (available under the tradename HALDOL decanoate®); haloperidol lactate (available under the tradenames HALDOL® and INTENSOL®); chlorpromazine (available under the tradename THORAZINE®, from SmithKline Beecham (GSK); fluphenazine (available under the tradename PROLIXIN®, from Apothecon, Copley, Schering, Teva, and American Pharmaceutical Partners, Pasadena); fluphenazine decanoate (available under the tradename PROLIXIN decanoate®); fluphenazine enanthate (available under the tradename PROLIXIN®); fluphenazine hydrochloride (available under the tradename PROLIXIN®); thiothixene (available under the tradename NAVANE®;, from Pfizer); thiothixene hydrochloride (available under the tradename NAVANE®); trifluoperazine (10-[3-(4-methyl-1-piperazinyl)propyl]-2-(trifluoromethyl)phenothiazine dihydrochloride, available under the tradename STELAZINE®, from SmithKline Beckman; perphenazine (available under the tradename TRILAFON®; from Schering); perphenazine and amitriptyline hydrochloride (available under the tradename ETRAFON TRILAFON®); thioridazine (available under the tradename MELLARIL®; from Novartis, Roxane, HiTech, Teva, and Alpharma) ; molindone (available under the tradename MOBAN®, from Endo); molindone hydrochloride (available under the tradename MOBAN®); loxapine (available under the tradename LOXITANE®; from Watson); loxapine hydrochloride (available under the tradename LOXITANE®); and loxapine succinate (available under the tradename LOXITANE®). Furthermore, benperidol (Glianimon®), perazine (Taxilan®) or melperone (Eunerpan®)) may be used.

Other suitable antipsychotic drugs include promazine (available under the tradename SPARING®), triflurpromazine (available under the tradename VESPRIN®), chlorprothixene (available under the tradename TARACTAN®), droperidol (available under the tradename INAPSINE®), acetophenazine (available under the tradename TINDAL®;), prochlorperazine (available under the tradename COMPAZINE®), methotrimeprazine (available under the tradename NOZINAN®), pipotiazine (available under the tradename PIPOTRIL®), iloperidone, pimozide and flupenthixol.

The antipsychotic drugs listed above by Tradename may also be available from other suppliers under a different Tradename.

In one further aspect of the invention, suitable antipsychotic agents include olanzapine, risperidone, quetiapine, aripiprazole, haloperidol, clozapine, ziprasidone, talnetant and osanetant.

Mood stabilisers which may be used in the therapy of the present invention include lithium, sodium valproate/valproic acid/divalproex, carbamazepine, lamotrigine, gabapentin, topiramate, oxcarbazepine and tiagabine.

Antidepressant drugs which may be used in the therapy of the present invention include serotonin antagonists, CRF-1 antagonists, Cox-2 inhibitor/SSRI dual antagonists; dopamine/noradrenaline/serotonin triple reuptake inhibitors; NK1 antagonists; NK1 and NK2 dual antagonists; NK1/SSRI dual antagonists; NK2 antagonists; serotonin agonists (such as rauwolscine, yohimbine and metoclopramide); serotonin reuptake inhibitors (such as citalopram, escitalopram, fluoxetine, fluvoxamine, femoxetine, indalpine, zimeldine, paroxetine and sertraline); dual serotonin/noradrenaline reuptake inhibitors (such as venlafaxine, reboxetine, duloxetine and milnacipran); Noradrenaline reuptake inhibitors (such as reboxetine); tricyclic antidepressants (such as amitriptyline, clomipramine, imipramine, maprotiline, nortriptyline and trimipramine); monoamine oxidase inhibitors (such as isocarboxazide, moclobemide, phenelzine and tranylcypromine); 5HT3 antagonists (such as example ondansetron and granisetron); and others (such as bupropion, amineptine, radafaxine, mianserin, mirtazapine, nefazodone and trazodone).

Anxiolytics which may be used in the therapy of the present invention include V1b antagonists, 5HT7 antagonists and benzodiazepines such as alprazolam and lorazepam.

Drugs for extrapyramidal side effects which may be used in the therapy of the present invention include anticholinergics (such as benztropine, biperiden, procyclidine and trihexyphenidyl), antihistamines (such as diphenhydramine) and dopaminergics (such as amantadine).

Cognitive enhancers which may be used in the therapy of the present invention include example cholinesterase inhibitors (such as tacrine, donepezil, rivastigmine and galantamine), H3 antagonists and muscarinic M1 agonists (such as cevimeline).

In one embodiment, the active ingredient for use in combination with a compound of the present invention, is an atypical antipsychotic, for example clozapine, olanzapine, risperidone, quetiapine, aripirazole, ziprasidone or amisulpride.

In one embodiment, the active ingredient for use in combination with a compound of the present invention is a typical antipsychotic, for example chlorpromazine, thioridazine, mesoridazine, fluphenazine, perphenazine, prochlorperazine, trifluoperazine, thiothixine, haloperidol, thiflurpromazine, pimozide, droperidol, chlorprothixene, molindone or loxapine.

In another embodiment, the active ingredient for use in combination with a compound of the present invention is a mood stabiliser, for example lithium, sodium valproate/valproic acid/divalproex, carbamazepine, lamotrigine, gabapentin, topiramate, oxcarbazepine or tiagabine.

In another embodiment, the active ingredient for use in combination with a compound of the present invention is an antidepressant, for example a serotonin agonist (such as rauwolscine, yohimbine or metoclopramide); a serotonin reuptake inhibitor (such as citalopram, escitalopram, fluoxetine, fluvoxamine, femoxetine, indalpine, zimeldine, paroxetine or sertraline); a dual serotonin/noradrenaline reuptake inhibitor (such as venlafaxine, reboxetine, duloxetine or milnacipran); a noradrenaline reuptake inhibitors (such as reboxetine); a tricyclic antidepressants (such as amitriptyline, clomipramine, imipramine, maprotiline, nortriptyline or trimipramine); a monoamine oxidase inhibitor (such as isocarboxazide, moclobemide, phenelzine or tranylcypromine); or other (such as bupropion, amineptine, radafaxine, mianserin, mirtazapine, nefazodone or trazodone).

In another embodiment, the active ingredient for use in combination with a compound of the present invention is an anxiolytic, for example a benzodiazepine such as alprazolam or lorazepam.

For use in medicine, the compounds of the present invention are usually administered as a standard pharmaceutical composition. The present invention therefore provides in a further aspect a pharmaceutical composition comprising a compound of formula (I) as hereinbefore described or a salt or solvate thereof and a pharmaceutically acceptable carrier. The pharmaceutical composition can be for use in the treatment of any of the conditions described herein.

The compounds of formula (I) may be administered by any convenient method, for example by oral, parenteral (e.g. intravenous), buccal, sublingual, nasal, rectal or transdermal administration and the pharmaceutical compositions adapted accordingly.

The compounds of formula (I) as hereinbefore described and their salts or solvates which are active when given orally can be formulated as liquids or solids, for example syrups, suspensions or emulsions, tablets, capsules and lozenges.

A liquid formulation will generally consist of a suspension or solution of the compound or salt or solvate in a suitable liquid carrier(s) for example an aqueous solvent such as water, ethanol or glycerine, or a non-aqueous solvent, such as polyethylene glycol or an oil. The formulation may also contain a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

Typical parenteral compositions consist of a solution or suspension of the compound or salt or solvate in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

Compositions for nasal administration may conveniently be formulated as aerosols, drops, gels and powders. Aerosol formulations typically comprise a solution or fine suspension of the active substance in a pharmaceutically acceptable aqueous or non-aqueous solvent and are usually presented in single or multidose quantities in sterile form in a sealed container, which can take the form of a cartridge or refill for use with an atomising device. Alternatively the sealed container may be a unitary dispensing device such as a single dose nasal inhaler or an aerosol dispenser fitted with a metering valve which is intended for disposal once the contents of the container have been exhausted. Where the dosage form comprises an aerosol dispenser, it will contain a propellant which can be a compressed gas such as compressed air or an organic propellant such as a fluorochloro-hydrocarbon. The aerosol dosage forms can also take the form of a pump-atomiser.

Compositions suitable for buccal or sublingual administration include tablets, lozenges and pastilles, wherein the active ingredient is formulated with a carrier such as sugar and acacia, tragacanth, or gelatin and glycerin.

Compositions for rectal administration are conveniently in the form of suppositories containing a conventional suppository base such as cocoa butter.

Compositions suitable for transdermal administration include ointments, gels and patches. The composition may be in unit dose form such as a tablet, capsule or ampoule.

Each dosage unit for oral administration contains, for example, from 1 to 250 mg (and for parenteral administration contains, for example, from 0.1 to 25 mg) of a compound of the formula (I) or a salt thereof calculated as the free base.

The antipsychotic agent component or components used in the adjunctive therapy of the present invention may also be administered in their basic or acidic forms as appropriate or, where appropriate, in the form of a salt or other derivative. All solvates and all alternative physical forms of the antipsychotic agent or agents or their salts or derivatives as described herein, including but not limited to alternative crystalline forms, amorphous forms and polymorphs, are also within the scope of this invention. In the case of the antipsychotic agent or agents, the forms and derivatives are, for example, those which are approved for therapeutic administration as monotherapies, including those mentioned above, but all references to antipsychotic agents herein include all salts or other derivatives thereof, and all solvates and alternative physical forms thereof.

For adjunctive therapeutic administration according to the invention, compounds of formula (I) or salts or solvates and the antipsychotic agent or agents or their salts, derivatives or solvates may each be administered in pure form, but each of the components will, for example, be formulated into any suitable pharmaceutically acceptable and effective composition which provides effective levels of the respective component in the body. The choice of the most appropriate pharmaceutical compositions for each component is within the skill of the art, and may be the same form or different forms for each of the components. Suitable formulations include, but are not limited to tablets, capsules, powders, granules, lozenges, suppositories, reconstitutable powders, or liquid preparations such as oral or sterile parenteral solutions or suspensions.

For simultaneous administration as a combined composition of compounds of formula (I) and the antipsychotic agent or agents according to the invention, compounds of formula (I) or their salts or solvates and the antipsychotic agent or agents and their salts, derivatives or solvates may be administered together in pure form, but the combined components will, for example, be formulated into any suitable pharmaceutically acceptable and effective composition which provides effective levels of each of the components in the body. The choice of the most appropriate pharmaceutical compositions for the combined components is within the skill of the art. Suitable formulations include, but are not limited to tablets, sub-lingual tablets, buccal compositions, capsules, powders, granules, lozenges, suppositories, reconstitutable powders, or liquid preparations such as oral or sterile parenteral solutions or suspensions.

In order to obtain consistency of adjunctive administration, the compositions of each of the components, or of the combination of the components is, for example, in the form of a unit dose.

The term "treatment" includes prophylaxis, where this is appropriate for the relevant condition(s).

### Biological Test Methods

### FLIPR experiments on M₁ receptor to determine agonist/antagonist potency

### Assay A

Compounds of the invention were characterized in a functional assay to determine their ability to activate the intracellular calcium pathway in CHO cells with stable expression of human muscarinic receptors using FLIPR (Fluorometric Imaging Plate Reader) technology. Briefly, CHO-M1 cells were plated (20,000/well) and allowed to grow overnight at 37 degrees. Media was removed and 30uL loading buffer (HBSS with 20mM HEPES, pH 7.4) containing FLIPR Calcium 3 dye (Molecular Devices Co., Sunnyvale, CA) was added according to manufacturer's instructions. After incubation at 37 degrees for 45-60 minutes, 10uL of the assay buffer (HBSS with 20mM HEPES and 2.5 mM probenecid, pH 7.4) containing test compounds was added to each well on FLIPR instrument. Calcium response was monitored to determine agonism. Plates were then incubated for another 30 minutes before 10uL of assay buffer containing acetylcholine was added at an EC₈₀, as the agonist challenge. Calcium response was then monitored again to determine compound's antagonism to acetylcholine. Concentration-response curves of both agonism and antagonism on M1 receptors were performed for each compound. Results were imported into ActivityBase data analysis suite (ID Business Solution Inc., Parsippany, NJ) where the curves were analysed by non-linear curve fitting and the resulting pEC₅₀/plC₅₀ were calculated. The intrinsic activities of agonist compounds were calculated as percentage of maximum FLIPR response induced by acetylcholine (ie using acetylcholine at EC₁₀₀ as the control).

### Assay B

Compounds of the invention were characterized in a functional assay to determine their ability to activate the intracellular calcium pathway in CHO cells with stable expression of human muscarinic receptors using FLIPR (Fluorometric Imaging Plate Reader) technology. Briefly, CHO-M1 cells were plated (15,000/well) and allowed to grow overnight at 37 degrees. Media was removed and 30uL loading buffer (HBSS with 2.5mM probenicid, 2uM Fluo-4, 500uM Brilliant Black, pH 7.4) was added. After incubation at 37 degrees for 90 minutes, 10uL of the assay buffer (HBSS with 2.5 mM probenecid, pH 7.4) containing test compounds was added to each well on the FLIPR instrument. Calcium response was monitored to determine agonism. Plates were then incubated for another 30 minutes before 10uL of assay buffer containing acetylcholine was added at an EC80, as the agonist challenge. Calcium response was then monitored again to determine compound's antagonism to acetylcholine. Concentration-response curves of both agonism and antagonism on M1 receptors were performed for each compound. Results were imported into ActivityBase data analysis suite (ID Business Solution Inc., Parsippany, NJ) where the curves were analysed by non-linear curve fitting and the resulting pEC50/fpKi were calculated. The intrinsic activities of agonist compounds were calculated as percentage of maximum FLIPR response induced by acetylcholine added as control on the same compound plates, and converted to a fraction between 0 and 1 (ie calculated using a 100% max response from a fitted acetylcholine standard curve, containing multiple concentrations, as control).

The example compounds below were tested in one or both of the above assays and were found to have a pEC₅₀ value of > 6.0 at the muscarinic M₁ receptor, and intrinsic activity > 50%.

### FLIPR experiments on M₁ receptor to determine agonist intrinsic activity

### Assay A

To determine the intrinsic activities of M1 agonist compounds, compounds of the invention were characterized in FLIPR experiments on U2OS cells with transient expression of human muscarinic M1 receptors. Briefly, U2OS cells were transduced with M1 BacMam virus (Ames, R S; Fornwald, J A; Nuthulaganti, P; Trill, J J; Foley, J J; Buckley, P T; Kost, T A; Wu, Z and Romanos, M A. (2004) Use of BacMam recombinant baculoviruses to support G protein-coupled receptor drug discovery. Receptors and Channels 10 (3-4): 99-109) in 2x10e⁵/mL cell suspension with 0.1% virus/cell ratio (v/v). The virus to cell ratio was determined in separate experiments by functional titration to be most appropriate to measure intrinsic activities of partial agonists. After mixing with virus in suspension, cells were then plated (10,000/well) and allowed to grow overnight at 37 degrees. FLIPR experiment was then carried out next day using the same protocol as described above for CHO-M1 cells. Results were imported into ActivityBase data analysis suite where the curves were analysed by non-linear curve fitting and the resulting pEC50 values were calculated. The intrinsic activities of agonist compounds were calculated as percentage of maximum FLIPR response induced by acetylcholine added as control on the same compound plates, and converted to a fraction between 0 and 1 (ie calculated using a 100%max response from a fitted acetylcholine standard curve, containing multiple concentrations, as control).

### Assay B

To determine the intrinsic activities of M1 agonist compounds, compounds of the invention were characterized in FLIPR experiments on CHO cells with transient expression of human muscarinic M1 receptors. Briefly, CHO cells were transduced with M1 BacMam virus (Ames, R S; Fornwald, J A; Nuthulaganti, P; Trill, J J; Foley, J J; Buckley, P T; Kost, T A; Wu, Z and Romanos, M A. (2004) Use of BacMam recombinant baculoviruses to support G protein-coupled receptor drug discovery. Receptors and Channels 10 (3-4): 99-109) at a multiplicity of infection of 6. The virus to cell ratio was determined in separate experiments by functional titration to be most appropriate to measure intrinsic activities of partial agonists. After mixing with virus in suspension, cells were then plated (15,000/well) and allowed to grow overnight at 37 degrees. Alternatively, cells were then frozen in 1ml vials at a concentration of 4.8x10e7 cells/ml in 90% dialysed Foetal Bovine Serum, 10% DimethylSulphoxide at -140 degrees. Cells could then be thawed on the day prior to assay, plated (15,000/well) and allowed to grow overnight at 37 degrees.

The FLIPR experiment was carried out on the day following plating using the same protocol as described above for CHO-M1 cells. Results were imported into ActivityBase data analysis suite where the curves were analysed by non-linear curve fitting and the resulting pEC50 values were calculated. The intrinsic activities of agonist compounds were calculated as percentage of maximum FLIPR response induced by acetylcholine added as control on the same compound plates, and converted to a fraction between 0 and 1 (ie calculated using a 100% max response from a fitted acetylcholine standard curve, containing multiple concentrations, as control).

The example compounds below were tested in one or both of the above assays, and were found to have a pEC₅₀ value of > 6.0 at the muscarinic M₁ receptor, and intrinsic activity of greater than or equal to 0.3.

### FLIPR experiments on M₂₋₅ receptor to determine receptor subtype selectivity

### Assay A

To determine selectivity of compounds of the invention against other muscarinic receptor subtypes, compounds were characterized in FLIPR experiments in CHO cells with stable expression of human muscarinic receptors, M2, M3, M4 or M5. In the case of M2 and M4 receptors, chimeric G-protein Gqi5 was also co-expressed to couple receptors to the calcium signaling pathway. Briefly, cells were plated (20,000/well) and allowed to grow overnight at 37 degrees. FLIPR experiment was then carried out next day using the same protocol as described above for CHO-M1 cells. Results were imported into ActivityBase data analysis suite where the curves were analysed by non-linear curve fitting and the resulting pEC50/plC50 values were calculated.

### Assay B

To determine selectivity of compounds of the invention against other muscarinic receptor subtypes, compounds were characterized in FLIPR experiments in CHO cells with stable expression of human muscarinic receptors, M2, M3, M4 or M5. In the case of M2 and M4 receptors, chimeric G-protein Gqi5 was also co-expressed to couple receptors to the calcium signaling pathway. Briefly, cells were plated (15,000/well) and allowed to grow overnight at 37 degrees. The FLIPR experiment was then carried out on the next day using the same protocol as described above for CHO-M1 cells. Results were imported into ActivityBase data analysis suite where the curves were analysed by non-linear curve fitting and the resulting pEC50/fpKi values were calculated.

The example compounds below were tested in one or both of the above assays and were found to be selective for the M1 receptor over M2, M3, M4 and M5 receptors, with typical selectivity (ratio of pEC50's) of ≥10-fold, and in certain cases ≥ 100-fold.

The invention is further illustrated by the following non-limiting examples. In the procedures that follow, after each starting material, reference to a Description by number is typically provided. This is provided merely for assistance to the skilled chemist. The starting material may not necessarily have been prepared from the batch referred to. SCX refers to a sulfonic acid ion exchange resin supplied by Varian.

All reactions were either done under argon or can be done under argon, unless stated otherwise (for example hydrogenation reactions).

### Description 1. 1,4-Dioxaspiro[4.5]decan-8-ol (D1)

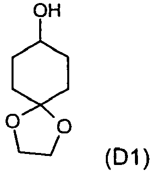

1,4-Dioxaspiro[4.5]decan-8-one (64 mmol, 10 g) was dissolved in ethanol (125 ml) and treated with NaBH₄ (1.2eq., 76.8 mmol, 2.9 g), at 0°C portionwise and the mixture was stirred at room temperature for 1 hour. Reaction was quenched with NaOH (25 ml, 2N aqueous solution). The aqueous solution was extracted with dichloromethane (2x). The organics were combined, dried over Na₂SO₄, filtered and the solvent was evaporated to afford the title compound, 8.3 g, 82%, as a colourless oil.
¹H NMR δ (d⁶DMSO, 400 MHz) 1.44 (4H, m), 1.64 (4H, m), 3.54 (1H, d broad), 3.82 (4H, m), 4.48 (1 H, d).

### Description 2. 8-(Methyloxy)-1,4-dioxaspiro[4.5]decane (D2)

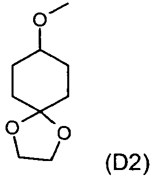

1,4-dioxaspiro[4.5]decan-8-ol (D1, 52.5 mmol, 8.3g) was dissolved in dimethylformamide (100 ml) and NaH (2eq., 105 mmol, 4.2 g) was added at 0°C portionwise. The mixture was stirred at 0°C for 10 minutes and iodomethane (2 eq., 6.5 ml) was added at room temperature. The mixture was stirred at room temperature for 2 hours, then it was quenched with methanol, partitioned between ethyl acetate and water and the two phases were separated. The aqueous phase was extracted with ethyl acetate (2x), the combined organics were washed with brine, dried over Na₂SO₄, filtered and the solvent was evaporated to afford the crude compound. Chromatography (ethyl acetate/n-hexane) afforded title compound, 7.35 g, 80%, as a colourless oil.
¹H NMR δ (d⁶DMSO, 400 MHz) 1.46 (2H, m),1.56 (2H, m), 1.64 (2H, m), 1.73 (2H, m), 3.21 (3H, s), 3.24 (1H, m), 3.83 (4H, m)

### Description 3. 4-(Methyloxy)cyclohexanone (D3)

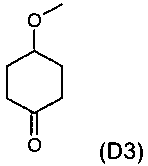

8-(Methyloxy)-1,4-dioxaspiro[4.5]decane (D2, 62.2 mmol, 11.9g) was dissolved in 10 ml of tetrahydrofuran and HCl (50ml of 5M aqueous solution) was added at room temperature; the mixture was stirred at room temperature overnight. Tetrahydrofuran was then evaporated, mixture was basified to pH=10 and it was extracted with ethyl acetate (3x); the organic phases were combined and washed with brine, dried over Na₂SO₄, filtered and the solvent was evaporated to afford the title compound, 8.2g, complete conversion.
¹H NMR δ (d⁶DMSO, 400 MHz) 1.92 (4H, m), 2.20 (2H, m), 2.35 (2H, m), 3.29 (3H, s), 3.53 (1H, m)

### Description 4. cis/trans-1,1-Dimethylethyl [1-(4-methoxycyclohexyl)-4-piperidinyl]carbamate (D4)

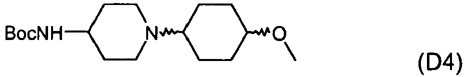

A mixture of 4-methoxycyclohexanone (D3, 4.5g, 40mmol), dichloromethane (200ml), 1,1-dimethylethyl 4-piperidinylcarbamate (9.0g; 45mmol) and sodium triacetoxyborohydride (16.0g, 80mmol) was stirred at room temperature for 18h, then partitioned between dichloromethane and water at pH9. Drying, evaporation, and chromatography (50g silica, 0-10% methanol in dichloromethane + NH₃) gave the title compound as a mixture of cis and trans isomers, 7.0g.

### Description 5. cis/trans-1-(4-Methoxycyclohexyl)-4-piperidinamine dihydrochloride (D5)

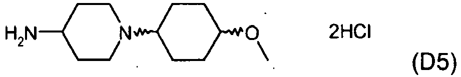

A mixture of cis/trans 1,1-dimethylethyl [1-(4-methoxycydohexyl)-4-piperidinyl]carbamate (D4, 7.0g; 22mmol), dichloromethane (50ml) and 4M HCl in dioxane (25ml; 100mmol) was maintained at room temperature for 4h. Evaporation gave the title compound, 6.3g.

### Description 6. cis and trans-N-(5-Methyl-2-nitrophenyl)-1-(4-methoxycyclohexyl)-4- piperidinamine (D6a, D6b)

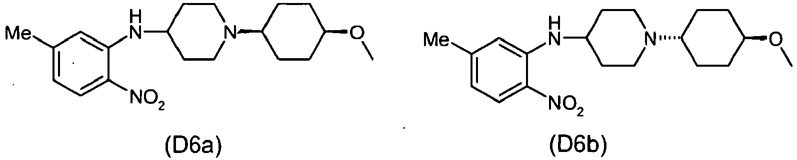

A stirred solution of 3-fluoro-4-nitrotoluene (0.87g) in dimethylformamide (25ml) at room temperature under argon was treated with diisopropylethylamine (2.9ml) and 1-[4-(methoxy)cyclohexyl]-4-piperidinamine dihydrochloride (D5, cis:trans mixture, 1.2g) and heated at 55°C over a weekend. The cooled reaction was diluted with ethyl acetate and washed three times with water and once with brine, then dried, evaporated and chromatographed (Biotage KP-NH^{™}-silica column eluting with 0-25% ethyl acetate/hexane) to give the *cis* (D6a, 300mg) and *trans* (D6b, 100mg) isomers of the title compound.

### Description 7. cis-5-Methyl-N-[1-(4-methoxycyclohexyl)-4-piperidinyl]-1,2-benzenediamine (D7)

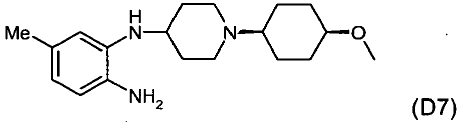

A stirred suspension of cis *N*-(5-methyl-2-nitrophenyl)-1-(4-methoxycyclohexyl)-4-piperidinamine (D6a, 300mg; 0.9mmol) in EtOH (20ml) at 50°C was treated with Raney nickel (1.8ml of 50% aqueous suspension) followed by dropwise addition of hydrazine hydrate (0.45ml). The mixture was heated at the same temperature for 1h, then filtered through Celite and the filtrate evaporated, and re-evaporated from first toluene and then diethyl ether, to afford the title compound, 320mg.

### Description 8. trans-5-Methyl-N-[1-(4-methoxycyclohexyl)-4-piperidinyl]-1,2-benzenediamine (D8)

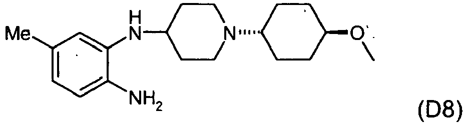

A stirred suspension of trans *N*-(3-fluoro-5-methyl-2-nitrophenyl)-(4-methoxycyclohexyl)-4-piperidinamine (D6b, 100mg, 0.3 mmol) in ethanol (6ml) at 50°C was treated with Raney nickel (0.6ml of 10% aqueous suspension) followed by dropwise addition of hydrazine hydrate (0.15ml, 10eq). The mixture was heated at the same temperature for 1h, then filtered through Kieselguhr and the filtrate evaporated and re-evaporated from toluene then Et₂O to afford the title compound, 100mg.

### Description 9. 8-(Ethyloxy)-1,4-dioxaspiro[4.5]decane (D9)

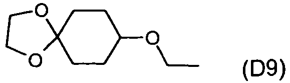

A stirred solution of 1,4-dioxaspiro[4.5]decan-8-ol (D1, 4.0g, 0.025mol) in N-methylpyrrolidinone (35ml) at 10°C under argon was treated portionwise with NaH (1.1g of 60% oil dispersion, 0.028mol) and maintained for 1h, then iodoethane (2.6ml, 0.032mol) was added. The mixture was allowed to warm to room temperature and stir for 18h. Further NaH (0.5g of 60% oil dispersion; 0.012mol) was added and the mixture stirred for 0.5h, then more iodoethane (2.0ml; 0.025mol) was added and the mixture maintained at room temperature for 3h. The mixture was cautiously treated with ethanol (1 ml) to destroy excess NaH and then water (300ml) was added and the mixture extracted with hexane (2 x 200ml). The combined extract was washed with water (2 x 200ml), then dried (Na₂SO₄) and concentrated under vacuum to afford a colourless oil (4.7g) containing the title compound contaminated with mineral oil residues.
¹HNMR δ (CDCl₃, 400 MHz): 1.20 (3H, t), 1.50-1.60 (2H, m), 1.63-1.77 (2H, m), 1.77-1.90 (4H, m), 3.35-3.43 (1H, m), 3.49 (2H, q), 3.90-4.00 (4H, m).

### Description 10. 4-(Ethyloxy)cyclohexanone (D10)

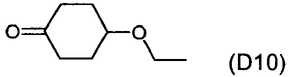

A solution of 8-(ethyloxy)-1,4-dioxaspiro[4.5]decane (D9, 4.7g, 0.025mol) in tetrahydrofuran (10ml) at room temperature under argon was treated with 5M HCl acid (40ml) and stirred for 18h, at which stage conc. HCl acid (5ml) was added and the mixture stirred at 40°C for 3.5h to complete the reaction. The resulting mixture was diluted with water (75ml) and extracted with dichloromethane (2 x 80ml). The combined extract was dried (Na₂SO₄)and carefully concentrated under partial vacuum at <20°C to afford the title compound as a pale yellow oil (3.8g, ∼90% purity).
¹H NMR □(CDCl₃, 400 MHz): 1.25 (3H, t), 1.90-2.00 (2H, m), 2.01-2.13 (2H, m), 2.20-2.32 (2H, m), 2.53-2.65 (2H, m), 3.55 (2H, q), 3.58-3.70 (1H, m).

### Description 11. 1,1-Dimethylethyl {1-[cis-4-(ethyloxy)cyclohexyl]-4-piperidinyl}carbamate (D11a) and 1,1-dimethylethyl {1-[trans-4-(ethyloxy)cyclohexyl]-4-piperidinyl}carbamate (D11b)

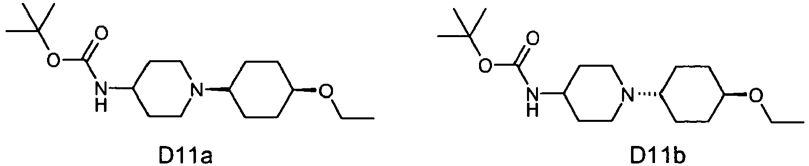

A stirred solution of 4-(ethyloxy)cyclohexanone (D10, 3.5g, ≤0.025 mol) in dichloromethane (100ml) at room temperature under argon was treated with 1,1-dimethylethyl 4-piperidinylcarbamate (5.0g, 0.025 mol), followed by portionwise addition over 5 minutes of sodium triacetoxyborohydride (7.4g, 0.035mol), then the resulting mixture stirred well at room temperature for 20h. The reaction mixture was treated with methanol (8ml) and the resulting solution allowed to stand for 3 days at room temperature, then 5% Na₂CO₃ solution (100ml) was added and the mixture extracted with dichloromethane (200ml). The extract was washed with brine, then dried (Na₂SO₄) and concentrated under vacuum. The residual yellow oil was dissolved in 60-80 petrol ether (100ml) and allowed to stand at room temperature overnight whereupon the crystals which had formed were filtered off, washed with petrol and dried to give 1.7g of ∼45:55 mixture of title compound trans isomer and starting 1,1-dimethylethyl 4-piperidinylcarbamate. Purification by column chromatography on silica gel eluting with 0-5% methanol/dichloromethane afforded the pure title compound *trans* isomer (D11b) as a white solid (930mg).
¹H NMR δ (CDCl₃, 400 MHz): 1.15-1.50 (6H, m), 1.18 (3H, t), 1.44 (9H, s), 1.82-2.00 (4H, m), 2.04-2.14 (2H, m), 2.20-2.32 (3H, m), 2.85 (2H, br d), 3.10-3.20 (1H, m), 3.38-3.50 (1H, m), 3.50 (2H, q), 4.40 (1H, br d).
Concentration under vacuum of the mother liquors from the crystallisation afforded 6g of a yellow oil containing the title compound *cis* isomer (D11a) as ∼4:1 mixture with the *trans* isomer.

### Description 12. 1-(trans-4-Ethoxycyclohexyl)piperidin-4-amine (D12)

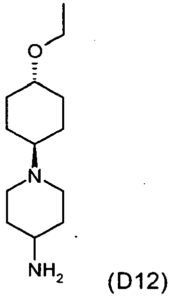

### Method A

1,1-Dimethylethyl {1-[*trans*-4-(ethyloxy)cyclohexyl]-4-piperidinyl}carbamate (D11b, 920mg, 4.299 mmol) was dissolved in dichloromethane (6ml) and treated with HCl (21 ml of a 4M solution in 1,4-dioxane) at room temperature. The mixture was stirred at room temperature for 1 hour and the solvent was evaporated. The crude was passed through an SCX cartridge to yield the free base title compound as a pale brown powder (865mg, 4.0 mmol, 89%). M⁺ + H = 227.

### Method B

A mixture of *trans*-1-(4-ethoxycyclohexyl)-4-piperidone (D45, 13.2g), 2M ammonia in methanol (300ml) and 10% palladium on carbon paste (4g) was hydrogenated at 50psi at room temperature overnight, then more 10% palladium on carbon paste (1g) was added and hydrogenated at 50psi over a weekend, then filtered and evaporated to give the title compound (9.5g) as an oil.

### Description 13. 1-[trans-4-(Ethyloxy)cyclohexyl]-N-(5-methyl-2-nitrophenyl)-4-piperidinamine (D13)

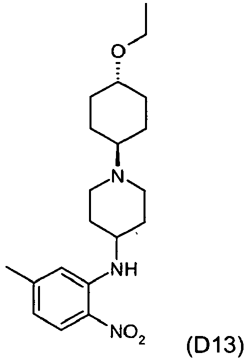

To a solution of 1-(*trans*-4-ethoxycyclohexyl)piperidin-4-amine (D12, 150mg, 0.70 mmol) in dry dimethylformamide (6 ml), diisopropylethylamine (0.11ml, 1eq., 0.70 mmol) and 3-fluoro-4-nitrotoluene (103mg, 1eq., 0.70 mmol) were added at room temperature and the mixture was refluxed at 80°C for 24 hours. The reaction showed to be incomplete by TLC. The reaction was then completed by microwave at 150°C for a total of 30 minutes. The crude mixture was then cooled to room temperature and the crude was poured onto water; the aqueous solution was extracted with ethyl acetate (2x) and the organics were alternatively washed with brine and water (2x). The organics were combined, dried over Na₂SO₄, filtered and the solvent was evaporated to afford the crude product which was purified by chromatography (ethyl acetate/n-hexane) on a Biotage KP-NH^{™}-silica column to yield the title compound as a yellow gum (118 mg, 50%). M⁺ + H = 362.

### Description 14. (2-Amino-5-methylphenyl){1-[trans-4-(ethyloxy)cyclohexyl]-4-piperidinyl}amine (D14)

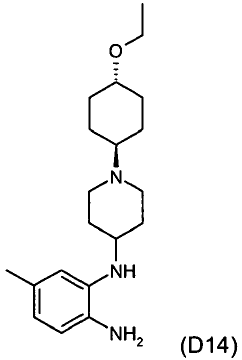

1-[*trans*-4-(Ethyloxy)cyclohexyl]-*N*-(5-methyl-2-nitrophenyl)-4-piperidinamine (D13, 118mg, 0.33 mmol) was dissolved in ethanol (3 ml) and an aqueous suspension of Raney Nickel (1 ml of a 50% suspension) was added at room temperature. The mixture was stirred for 30 minutes at room temperature before adding hydrazine monohydrate (0.15ml, 15eq., 5.0 mmol) over 30 minutes. The reaction was left overnight. The reaction mixture was filtered through celite and the solvent was evaporated to yield the title compound as a brown solid (110mg, 100%). M⁺ + H = 332.

### Description 15. cis/trans-1-(4-Ethoxycyclohexyl)piperidin-4-amine dihydrochloride salt (D15)

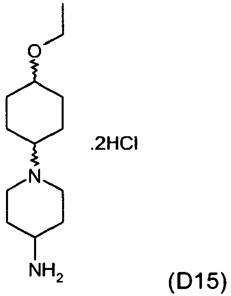

cis/trans-1,1-Dimethylethyl {1-[4-(ethyloxy)cyclohexyl]-4-piperidinyl}carbamate (D11a, 2.6g, 8.0 mmol) was dissolved in dichloromethane (12 ml) and treated with HCl (35 ml of a 4M solution in 1,4-dioxane). The mixture was stirred at room temperature for 1 hour and the solvent was evaporated to yield the title compound as a mixture of the *cisltrans* isomers (2.17g, 9.631 mmol, 100%). M⁺ + H = 227.

### Description 16. 1-[cis-4-(Ethyloxy)cyclohexyl]-N-(5-methyl-2-nitrophenyl)-4-piperidinamine (D16)

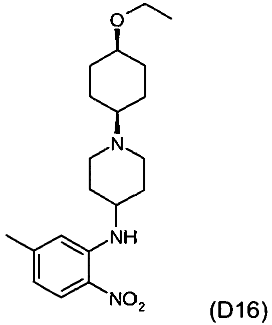

2-Fluoro-4-methyl-1-nitrobenzene (300mg, 1 eq.), *cis*/*trans* 1-[4-(ethyloxy)cyclohexyl]-4-piperidinamine dihydrochloride (D15, 600mg, 1 eq., 2 mmol) and diisopropylethylamine (1ml, 3eq.) were partially dissolved in dimethylformamide (2.5 ml) at room temperature and the mixture was heated to 200°C by microwave for 25 minutes. The crude mixture was then cooled to room temperature and poured onto water/brine; the aqueous solution was extracted with ethyl acetate (2x) and the organics were alternatively washed with brine and water (2x). The organics were combined, dried over Na₂SO₄, filtered and the solvent was evaporated to afford the crude product which was purified by chromatography on a Biotage KP-NH^{™}-silica column to yield the title compound (cis isomer) (160 mg). M⁺ +H=362.

### Description 17. (2-Amino-5-methylphenyl){1-[cis-4-(ethyloxy)cyclohexyl]-4-piperidinyl}amine (D17)

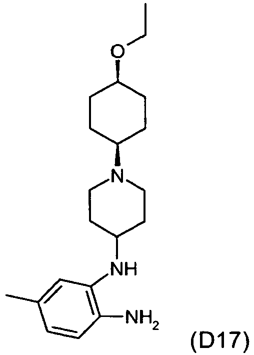

1-[*cis*-4-(Ethyloxy)cyclohexyl]-*N*-(5-methyl-2-nitrophenyl)-4-piperidinamine (D16, 160mg, 0.44 mmol) was dissolved in ethanol (3 ml) and an aqueous suspension of Raney Nickel (1 ml of a 50% suspension) was added at room temperature. The mixture was stirred for 30 minutes before adding hydrazine monohydrate (0.21ml, 15eq., 6.6 mmol) over 30 minutes. The reaction was left overnight. The reaction mixture was filtered through celite and the solvent was evaporated to yield the title compound as a brown solid (147 mg, 100%). M⁺ + H = 332.

### Description 18. 8-(Propyloxy)-1,4-dioxaspiro[4.5]decane (D18)

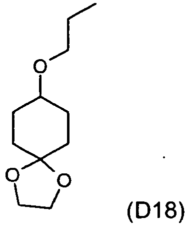

A stirred solution of 1,4-dioxaspiro[4.5]decan-8-ol (D1, 4g, 25.3mmol) and 1-bromopropane (2.78ml, 30.36mmol) in N-methylpyrrolidin-2-one (35ml) at 0°C under argon was treated with NaH (60%, 1.1g) then warmed up to room temperature and stirred for 3h. 1-bromopropane (1ml, 10.92mmol) was added and the reaction stirred overnight. Water was added and the mixture was extracted with hexane (20ml x 3). The organic fractions were collected, dried on MgSO4, then concentrated to leave the title compound as a colourless liquid (3.4g, 68% yield).
1H NMR δ (CDCl3 400MHz): 0.8-0.9 (3H, t), 1.5-1.85 (10H, m), 3.4 (3H, m), 3.9 (4H, m).

### Description 19. 4-(Propyloxy)cyclohexanone (D19)

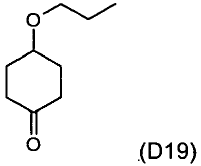

A stirred solution of 8-(propyloxy)-1,4-dioxaspiro[4.5]decane (D18, 3.4g, 17mmol) in tetrahydrofuran (35ml) was treated with 5M HCl acid (35ml) and stirred overnight under argon at room temperature. The tetrahydrofuran was partially removed under vacuum, water (∼20ml) was added and the solution was extracted with dichloromethane (∼20ml x 3), and the extract dried on MgSO₄ then concentrated under vacuum to leave the title compound as a pale yellow oil (2.7g, 98%yield). This crude was used for the next step without any further purification.

### Description 20. cis/trans-1,1-Dimethylethyl {1-[4-(propyloxy)cyclohexyl]-4-piperidinyl}carbamate (D20)

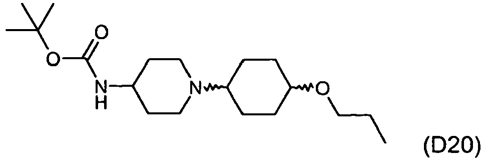

A stirred solution of 4-propoxycyclohexanone (D19, 2.7g, 0.017 mol) and 1,1-dimethylethyl 4-piperidinylcarbamate (3.6g, 0.017 mol) in dichloromethane (30ml) at room temperature under argon was treated with sodium triacetoxyborohydride (3.8g, 0.02 mol), then the resulting mixture stirred well at room temperature overnight. The mixture was treated with water and extracted with dichloromethane (30ml x3) and the organics were collected and dried on MgSO₄ then concentrated under vacuum. The crude was eluted on SCX cartridge, firstly with dichloromethane to remove impurities and then with 2M NH₃ in methanol for collect the isomer mixture, plus a small amount of starting material. This mixture was chromatographed on a silica column (100g for 3.4g of crude: 5% to 5% 3CV, 5% to 20% 10CV, 20% to 20% 2CV of 0.4M NH₃ in methanol/dichloromethane) to afford 2.1g of the title compound mixture (36% yield).

### Description 21. cis/trans-1-[4-(Propyloxy)cyclohexyl]-4-piperidinamine dihydrochloride (D21)

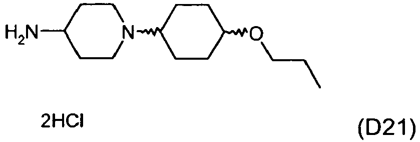

A mixture of *cis*/*trans*-1,1-dimethylethyl {1-[4-(propyloxy)cyclohexyl]-4-piperidinyl}carbamate (D20, 2.1g; 6.17mmol) in diethyl ether (15ml) was treated with a 1M HCl solution in diethyl ether (15ml) under argon at room temperature and stirred overnight. The mixture containing a white precipitate was collected by filtration then dried to afford the title mixture as a white solid (1.2g, 81 %yield).

### Description 22. N-(5-Methyl-2-nitrophenyl)-1-[cis-4-(propyloxy)cyclohexyl]-4-piperidinamine (D22a) and N-(5-methyl-2-nitrophenyl)-1-[trans-4-(propyloxy)cyclohexyl]-4-piperidinamine (D22b)

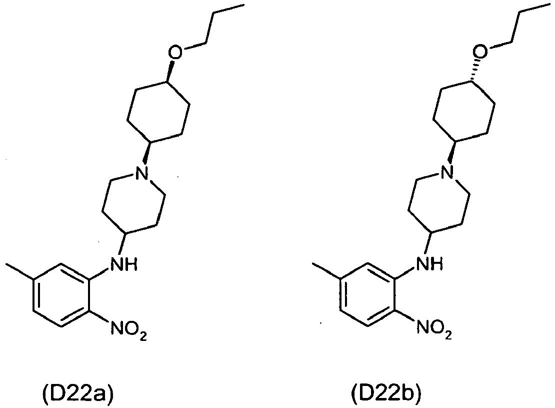

A mixture of *cis*/*trans*-1-[4-(propyloxy)cyclohexyl]-4-piperidinamine dihydrochloride (D21, 1.2g, 3.8mmol), 3-fluoro-4-nitrotoluene (570mg, 3.8mmol) and diisopropylethylamine (2.5ml 14.7mmol; 3eq) in dimethylformamide (20ml) was stirred and heated at 80°C overnight, under argon. The mixture was washed with water and extracted with dichloromethane. The organic layer was eluted through a SCX cartridge and the *cis*/*trans* mixture was collected by elution of 2M NH₃ in methanol. Separation of isomers was obtained by chromatography on a Biotage KP-NH^{™}-silica column (100g for 1.4gr of crude: 0% to 20% ethyl acetate/hexane, 20CV). The chromatography was repeated twice affording 410mg of *cis* isomer and 340mg of *trans* isomer (55% yield).

*Cis* (D22a): ¹H NMR δ (CDCl₃ 400MHz): 0.9 (3H, t), 1.2-1.35 (4H, m), 1.55-17 (4H, m), 1.9 (2H, m), 2.1 (4H, m), 2.35 (3H, s), 2.35 (1H, m), 2.4 (2H, m), 2.9 (2H, m), 3.1-3.2 (1H, m), 3.4 (2H, t), 3.55 (1H, m), 6.4 (1H, d), 6.6 (1H, s), 8.05 (1H, d), 8.2 (1H, d).
MH⁺ 376 and 377.
*Trans* (D22b): ¹H NMR δ (CDCl₃ 400MHz): 0.9 (3H, t), 1.4 (2H, m), 1.55-175 (8H, m), 2.0 (2H, m), 2.1 (2H, m), 2.3 (3H, s), 2.4 (1H, m), 2.5 (2H, m), 2.9 (2H, m), 3.35 (2H, t), 3.5 (1H, m), 3.55 (1H, m), 6.4 (1H, d), 6.6 (1H, s), 8.05 (1H, d), 8.2 (1H, d).
MH⁺ 376 and 377.

### Description 23. (2-Amino-5-methylphenyl){1-[cis-4-(propyloxy)cyclohexyl]-4-piperidinyl}amine (D23)

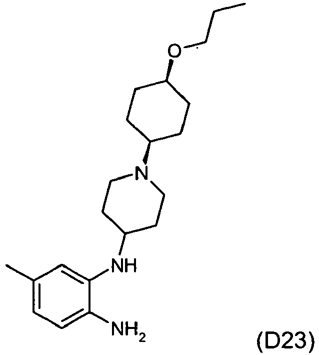

A stirred solution of *N*-(5-methyl-2-nitrophenyl)-1-[*cis*-4-(propyloxy)cyclohexyl]-4-piperidinamine (D22a, 440mg, 1.17mmol) in ethanol (30ml) at room temperature under argon was treated with Raney nickel (∼50mg) followed by a dropwise addition of hydrazine hydrate (2ml, 30.4mmol) and heated at 50°C for 3h. The solution was filtered through a Kieselguhr pad and then concentrated under vacuum to leave 400mg (98% yield) of the title compound as a pale yellow oil.
MH⁺ 346 and 347.

### Description 24. (2-Amino-5-methylphenyl){1-[trans-4-(propyloxy)cyclohexyl]-4-piperidinyl}amine (D24)

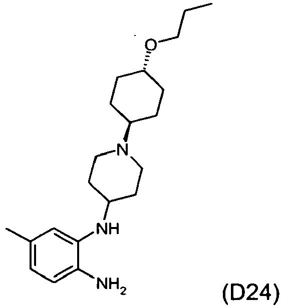

A stirred suspension of *N*-(5-methyl-2-nitrophenyl)-1-[*trans*-4-(propyloxy)cyclohexyl]-4-piperidinamine (D22b, 340mg, 0.9mmol) in ethanol (20ml) at room temperature under argon was treated with Raney nickel (-30mg) followed by a dropwise addition of hydrazine hydrate (0.28ml, 9.5mmol) and heated at 50°C for 3h. The solution was filtered through a Kieselguhr pad and then concentrated under vacuum to leave 310mg (99% yield) of title compound as a pale yellow oil.
MH⁺ 346 and 347.

### Description 25. 8-[(1-Methylethyl)oxy]-1,4-dioxaspiro[4.5]decane (D25)

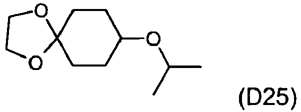

A mixture of 1,4-dioxaspiro[4.5]decan-8-ol (D1, 5.0g, 0.032mol), 2-iodopropane (25ml) and silver (I) oxide (14g, 0.060mol) at room temperature under argon was stirred in the dark for 6 days, followed by 6 days standing. The mixture was treated with diethyl ether (40ml) and filtered through Kieselguhr washing well with diethyl ether. The filtrate was concentrated under vacuum and the residue dissolved in hexane (150ml), washed with water (150ml), then dried (Na₂SO₄)and concentrated under vacuum to afford a colourless oil (4.89g) containing approx. 80% of the title compound together with 4-[(1-methylethyl)oxy]cyclohexanone from ketal hydrolysis. This mixture was used in the next step without purification.
¹H NMR δ (CDCl₃, 400 MHz): 1.13 (6H, d), 1.5-1.59 (2H, m), 1.60-1.74 (2H, m), 1.75-1.88 (4H, m), 3.43-3.50 (1H, m), 3.62-3.72 (1H, m), 3.90-4.00 (4H, m).

### Description 26. 4-[(1-Methylethyl)oxy]cyclohexanone (D26)

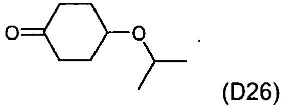

A solution of the mixture of 8-[(1-methylethyl)oxy]-1,4-dioxaspiro[4.5]decane and 4-[(1-methylethyl)oxy]cyclohexanone (∼4:1) (D25 , 4.89g, ∼0.025mol) in tetrahydrofuran (10ml) at room temperature under argon was treated with 5M HCl acid (50ml) and stirred well for 18h. The reaction mixture was treated with water (150ml) and extracted with dichloromethane (2 x 80ml). The combined extract was washed with brine, dried (Na₂SO₄)and concentrated under vacuum to afford the title compound as a colourless oil (3.9g, 100%).
¹H NMR δ (CDCl₃, 400 MHz): 1.19 (6H, d), 1.88-2.08 (4H, m), 2.22-2.32 (2H, m), 2.54-2.65 (2H, m), 3.70-3.84 (2H, m).

### Description 27. 1,1-Dimethylethyl (1-{cis-4-[(1-methylethyl)oxy]cyclohexyl}-4-piperidinyl)carbamate (D27a) and 1,1-dimethylethyl (1-{trans-4-[(1-methylethyl)oxy]cyclohexyl}-4-piperidinyl)carbamate (D27b)

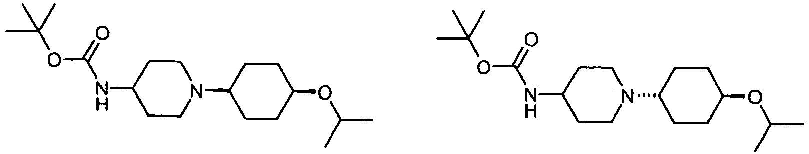

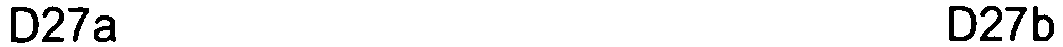

A stirred solution of 4-[(1-methylethyl)oxy]cyclohexanone (D26, 3.9g, 0.025 mol) in dichloromethane (100ml) at room temperature under argon was treated with 1,1-dimethylethyl 4-piperidinylcarbamate (5.0g, 0.025 mol), followed by portionwise addition over 10 minutes of sodium triacetoxyborohydride (7.4g, 0.035mol). Further dichloromethane (50ml) was added to aid stirring which was continued at room temperature for 18h. The reaction mixture was carefully treated with methanol (5ml) and stirred for 20 minutes, then 2% Na₂CO₃ solution (200ml) was added and the mixture extracted with dichloromethane (2 x 150ml). The combined extract was washed with brine, then dried (Na₂SO₄)and concentrated under vacuum. The residual yellow oil (8.6g) was triturated with 60-80 petrol ether (80ml) which caused immediate crystallisation of a white solid. This was filtered off, washed with 60-80 petrol ether and dried, then recrystallised from 9:1 60-80 petrol ether/dichloromethane to afford the title compound *trans* isomer (D27b) as a white solid (0.85g).
¹H NMR δ (CDCl₃, 400 MHz): 1.12 (6H, d), 1.18-1.50 (6H, m), 1.44 (9H, s), 1.80-2.05 (6H, m), 2.20-2.32 (3H, m), 2.80-2.90 (2H, m), 3.15-3.26 (1H, m), 3.38-3.50 (1H, m), 3.64-3.73 (1H, m), 4.37-4.47 (1H, m).

Concentration under vacuum of the mother liquors from the first crystallisation afforded 7.3g of a yellow oil containing the title compound *cis* isomer (D27a) as ∼4:1 mixture with the *trans* isomer.

### Description 28. 1-{trans-4-[(1-Methylethyl)oxy]cyclohexyl}-4-piperidinamine dihydrochloride (D28)

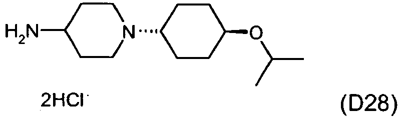

A stirred solution of 1,1-dimethylethyl (1-{*trans*-4-[(1-methylethyl)oxy]cyclohexyl}-4-piperidinyl)carbamate (D27b, 850mg, 2.5mmol) in dichloromethane (10ml) at room temperature under argon was treated with 4M HCl/dioxane (5ml; 20mmol) and maintained for 18h, then concentrated under vacuum. The residue was treated with diethyl ether (20ml), stirred for 5 minutes, then the solid filtered off, washed with diethyl ether and then dried to afford the title compound as a white solid (730mg, 93%).
¹H NMR δ (d⁶DMSO, 400 MHz): 1.05 (6H, d), 1.08-1.22 (2H, m), 1.43-1.60 (2H, m), 1.93-2.20 (8H, m), 2.98-3.15 (3H, m), 3.20-3.55 (5H, m), 3.62-3.72 (1H, m), 8.46 & 8.60 (together 3H, 2 x brs), 10.84 & 10.95 (together 1H, 2 x brs).

### Description 29. cis/trans-1-{4-[(1-Methylethyl)oxy]cyclohexyl}-4-piperidinamine dihydrochloride (D29)

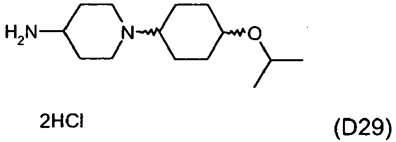

A solution of cis/trans 1,1-dimethylethyl (1-{cis-4-[(1-methylethyl)oxy]cyclohexyl}-4-piperidinyl)carbamate (D27a, ∼4:1 *cis:trans*, 7.3g, 0.021mol) in dichloromethane (50ml) at room temperature under argon was treated with 4M HCl/dioxane (30ml; 0.12mol) and stirred well for 18h. The mixture containing a white precipitate was concentrated under vacuum and the residue treated with diethyl ether (100ml), stirred well for 5 minutes, then the solid filtered off, washed with diethyl ether and dried to afford the title compound (∼4:1 mixture of *cis:trans* isomers) as a white solid (4.46g, 67%).

### Description 30. 1-{trans-4-[(1-Methylethyl)oxy]cyclohexyl}-N-(5-methyl-2-nitrophenyl)-4-piperidinamine (D30)

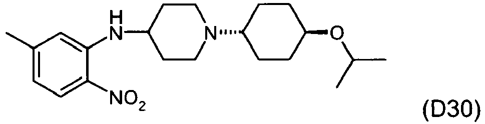

A stirred mixture of 3-fluoro-4-nitrotoluene (155mg, 1.0mmol), diisopropylethylamine (0.53ml, 3.0mmol) and 1-{*trans*-4-[(1-methylethyl)oxy]cyclohexyl}-4-piperidinamine dihydrochloride (D28, 250mg, 0.80mmol) in dimethylformamide (5ml) under argon was heated at 200°C for 10 minutes in a microwave reactor. The resulting mixture was concentrated under vacuum and the residue treated with 10% Na₂CO₃ solution and extracted with dichloromethane. The extract was dried, concentrated under vacuum and the residue loaded onto a SCX cartridge (10g), which was washed with dichloromethane followed by methanol to remove non-basic contaminants, then with 2M NH₃/methanol to remove the product. Further purification by chromatography on silica gel (20g) eluting with 0-5% methanol/dichloromethane afforded the title compound as a dark orange solid (220mg, 74%).
¹H NMR δ (CDCl₃, 400 MHz): 1.14 (6H, d), 1.20-1.38 (4H, m), 1.58-1.72 (2H, m), 1.86-1.96 (2H, m), 2.00-2.12 (4H, m), 2.27-2.37 (1H, m), 2.33 (3H, s), 2.38-2.48 (2H, m), 2.83-2.93 (2H, m), 3.19-3.29 (1H, m), 3.50-3.60 (1H, m), 3.65-3.77 (1H, m), 6.43 (1H, dd), 6.61 (1H, s), 8.06 (1H, d), 8.19 (1H, d).

### Description 31. (2-Amino-5-methylphenyl)(1-{trans-4-[(1-methylethyl)oxy]cyclohexyl}-4-piperidinyl)amine (D31)

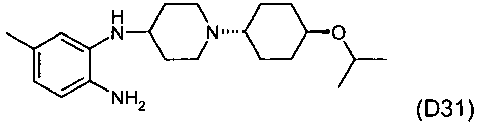

A stirred suspension of 1-{*trans*-4-[(1-methylethyl)oxy]cyclohexyl}-*N*-(5-methyl-2-nitrophenyl)-4-piperidinamine (D30, 220mg, 0.59mmol) in ethanol (15ml) at room temperature under argon was treated with Raney nickel (∼20mg) followed by dropwise addition of hydrazine hydrate (0.18ml, 5.0mmol). The mixture was maintained at room temperature for 3h, then filtered through Kieselguhr to remove the catalyst and the filtrate concentrated under vacuum to afford the title compound as a pale grey solid (187mg, 92%).
¹H NMR δ (CDCl₃, 400 MHz): 1.14 (6H, d), 1.18-1.37 (4H, m), 1.42-1.56 (2H, m), 1.87-1.97 (2H, m), 1.98-2.12 (4H, m), 2.25 (3H, s), 2.27-2.42 (3H, m), 2.85-2.95 (2H, m), 3.00-3.30 (4H, br m), 3.63-3.75 (1H, m), 6.43-6.49 (2H, m), 6.62 (1H, dd).

### Description 32. cis/trans-1,1-Dimethylethyl [1-(1-cyano-4-methoxycyclohexyl)-4-piperidinyl]carbamate (D32)

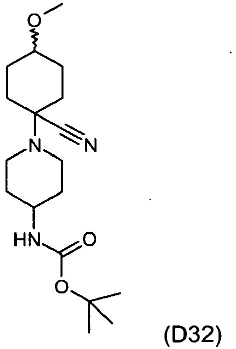

Potassium cyanide (320mg; 5 mmol) was added to a mixture of 1,1-dimethylethyl 4-piperidinylcarbamate (1.0g), 4-(methyloxy)cyclohexanone (D3; 640mg; 5 mmol), and 2.5M hydrochloric acid (2.0ml; 5 mmol). After 2h at room temperature the mixture was partitioned between water and dichloromethane. Drying and evaporation gave the title compound as a mixture of cis and *trans* isomers, 1.1 g.

### Description 33. cis/trans-1,1-Dimethylethyl [1-(1-methyl-4-methoxycyclohexyl)-4-piperidinyl]carbamate (D33)

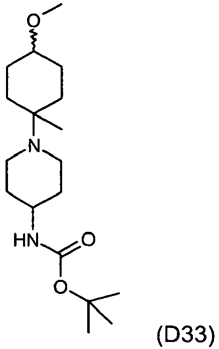

Methyl magnesium bromide (5.0ml of 3M solution in diethyl ether) was added to a solution of *cis*/*trans*-1,1-dimethylethyl [1-(1-cyano-4-methoxycyclohexyl)-4-piperidinyl]carbamate (D32, mixture of isomers) (1.1g; 3.3 mmol) in tetrahydrofuran (25ml). After 2h at room temperature the solution was partitioned between an aqueous solution of Rochelle salt and dichloromethane. Drying, evaporation and chromatography (50g silica, 0 to 10% methanol in dichloromethane) gave the title compound as a mixture of cis and trans isomers, 400mg.

### Description 34. cis/trans-1-[1-Methyl-4-(methyloxy)cyclohexyl]-4-piperidinamine (D34)

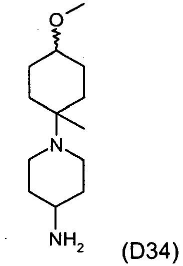

*cis*/*trans*-1,1-Dimethylethyl {1-[1-methyl-4-(methyloxy)cyclohexyl]-4-piperidinyl}carbamate (D33, 360 mg, 1.1 mmol), was dissolved in dichloromethane (5 ml) and HCl (10 eq., 11 mmol, 2.8 ml of a 4M solution in 1,4-dioxane) was added at room temperature; the mixture was stirred at room temperature overnight. The solvent was subsequently evaporated to afford the crude product which was purified by SCX to afford the title compound, 200 mg, 60%.
¹H NMR δ (d⁶DMSO, 400 MHz) 0.750 (3H, d), 1.100(3H, m), 1.522 (9H, m), 1.838 (1H, d), 1.973 (2H, m), 2.433 (1H, m), 2.770 (2H, t), 3.121 (1H, m), 3.195 (3H, d), 3.329 (1H, s br).

### Description 35. cis and trans-1-[1-Methyl-4-(methyloxy)cyclohexyl]-N-(5-methyl-2-nitrophenyl)-4-piperidinamine (cis = D35a, trans = D35b)

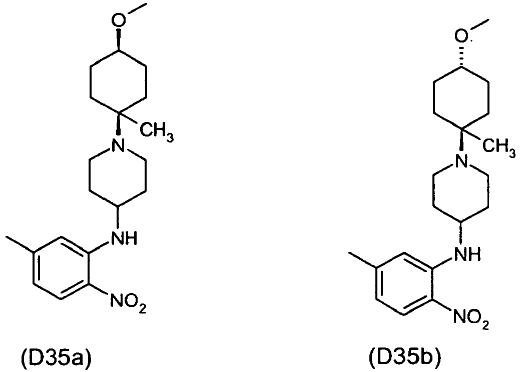

2-Fluoro-4-methyl-1-nitrobenzene (1 eq., 136 mg), *cis*/*trans* 1-[1-methyl-4-(methyloxy)cyclohexyl]-4-piperidinamine (D34, 1 eq., 200 mg, 0.88 mmol), Hunig's base diisopropylethylamine (1eq., 150 µl), were all dissolved in dimethylformamide (3 ml) at room temperature and the mixture was refluxed at 100 °C overnight. The crude mixture was then cooled to room temperature and poured onto brine; the aqueous solution was extracted with ethyl acetate (3x) and the organics were alternatively washed with brine and water. The organics were combined, dried over Na₂SO₄, filtered and the solvent was evaporated to afford the crude product which was purified by chromatography on silica column (methanol-NH₃-dichloromethane) to yield the two separate isomers, (*cis*, D35a, 118 mg) and (*trans,* D35b, 61 mg), 56% total yield, M⁺ + H = 362.

### Description 36. (2-Amino-5-methylphenyl){1-[cis-1-methyl-4-(methyloxy)-cyclohexyl]-4-pipdridinyl}amine (D36)

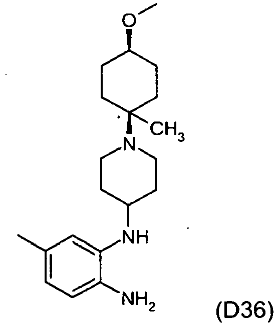

1-[*cis*-1-Methyl-4-(methyloxy)cyclohexyl]-*N*-(5-methyl-2-nitrophenyl)-4-piperidinamine (D35a, 118 mg, 0.327 mmol,) was dissolved into ethanol (8ml) and Raney-Ni (0.3 ml of a 50% aqueous suspension) was added at room temperature; the mixture was heated to 50°C and hydrazine hydrate (10 eq., 3.2 mmol, 0.1 ml) was added dropwise at 50°C. Reaction was stirred at 50°C until no more starting material was observed by TLC. The mixture was cooled to room temperature and filtered through celite; the solvent was subsequently evaporated to afford the title compound, 91 mg, 84%, M⁺ + H = 332.

### Description 37. (2-Amino-5-methylphenyl){1-[trans-1-methyl-4-(methyloxy)-cyclohexyl]-4-piperidinyl}amine (D37)

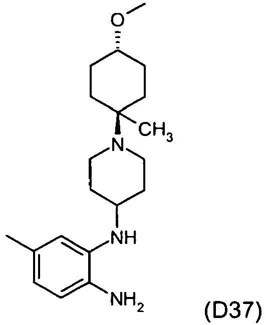

1-[*trans*-1-Methyl-4-(methyloxy)cyclohexyl]-*N*-(5-methyl-2-nitrophenyl)-4-piperidinamine (D35b, 60 mg, 0.166 mmol,) was dissolved into ethanol (4 ml) and Raney-Ni (0.2 ml of a 50% aqueous suspension) was added at room temperature; the mixture was heated to 50°C and hydrazine hydrate (10 eq., 1.6 mmol, 0.05 ml) was added dropwise at 50°C. Reaction was stirred at 50°C until no more starting material was observed by TLC. The mixture was cooled to room temperature and filtered through celite; the solvent was subsequently evaporated to afford the title compound, 30 mg, 55%, M⁺ + H = 332.

### Description 38. 1-{cis-4-[(1-Methylethyl)oxy]cyclohexyl}-N-(5-methyl-2-nitrophenyl)-4-piperidinamine (D38)

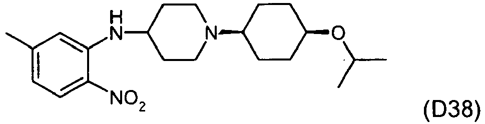

A stirred mixture of 3-fluoro-4-nitrotoluene (250mg, 1.6mmol), diisopropylethylamine (0.86ml, 4.8mmol) and cis/trans-1-{4-[(1-methylethyl)oxy]cyclohexyl}-4-piperidinamine dihydrochloride (D29, -4:1 cis/trans, 400mg, 1.3mmol) in dimethylformamide (8ml) under argon was heated at 200°C for 12 minutes in a microwave reactor. The resulting mixture was concentrated under vacuum and the residue treated with 10% Na₂CO₃ solution and extracted with dichloromethane. The extract was dried, concentrated under vacuum and the residue loaded onto a SCX cartridge (20g), which was eluted with dichloromethane followed by methanol to remove non-basic contaminants, then with 2M NH₃/methanol to remove the product. The product solution was concentrated under vacuum and the residue chromatographed on Biotage KP-NH^{™}-silica column (100g) eluting with 0-20% ethyl acetate/hexane to obtain the title compound as the fastest eluting isomer. This was obtained as an orange oil (186mg, 38%).
¹HNMR δ (CDCl₃, 400 MHz): 1.14 (6H, d), 1.33-1.46 (2H, m), 1.52-1.78 (6H, m), 1.80-1.96 (2H, m), 2.02-2.12 (2H, m), 2.28-2.40 (1H, m), 2.33 (3H, s), 2.40-2.50 (2H, m), 2.82-2.93 (2H, m), 3.48-3.68 (3H, m), 6.42 (1H, dd), 6.62 (1H, s), 8.05 (1H, d), 8.19 (1H, d).

### Description 39. (2-Amino-5-methylphenyl)(1-{cis-4-[(1-methylethyl)oxy]-cyclohexyl}-4-piperidinyl)amine (D39)

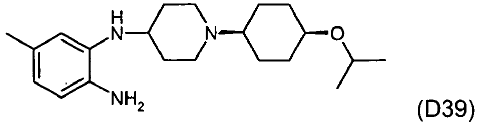

A stirred suspension of 1-{cis-4-[(1-methylethyl)oxy]cyclohexyl}-*N*-(5-methyl-2-nitrophenyl)-4-piperidinamine (D38, 186mg, 0.50mmol) in ethanol (15ml) at room temperature under argon was treated with Raney nickel (∼20mg) followed by dropwise addition of hydrazine hydrate (0.15ml, 5.0mmol). The mixture was maintained at room temperature for 2h, then filtered through Kieselguhr to remove the catalyst and the filtrate concentrated under vacuum to afford the title compound as a grey oil (170mg, 99%).
¹H NMR δ (CDCl₃, 400 MHz): 1.13 (6H, d), 1.33-1.80 (8H, m), 1.85-1.96 (2H, m), 2.02-2.12 (2H, m), 2.25 (3H, s), 2.29-2.42 (3H, m), 2.88-2.98 (2H, m), 3.05-3.40 (4H, br m), 3.53-3.70 (2H, m), 6.41-6.50 (2H, m), 6.61 (1H, d).

### Description 40. trans-N-(4-Hydroxycyclohexyl)phthalimide (D40)

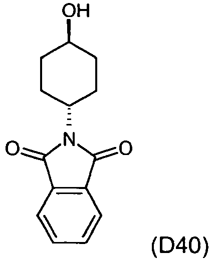

N-Ethoxycarbonylphthalimide (100g; 0.46 mol) was added to a mixture of *trans*-4-hydroxycyclohexylamine hydrochloride (69g; 0.46 mol), potassium carbonate (158g; 1.15mol) and water (1l) at room temperature. After stirring for 3h the title compound was isolated by filtration, washing with water then ethyl acetate, 95g.

### Description 41. trans-N-(4-tert-Butyldimethylsilyloxycyclohexyl)phthalimide (D41)

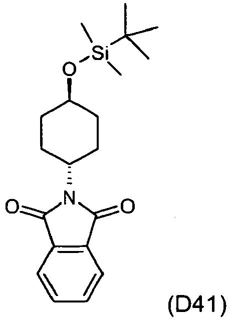

Tert-butyldimethylsilyl chloride (60g; 0.39mol) was added in portions to a mixture of trans N-(4-hydroxycyclohexyl)phthalimide (D40, 95g; 0.39mol), imidazole (55g; 0.78mol), and DMF (200ml) at 20-30°C (internal, ice cooling). After stirring for 3h more at 40°C the mixture was partitioned water/hexane. Drying and evaporation of the organic layer gave the title compound crystallised from pentane, 92g.

### Description 42. trans-N-(4-Ethoxycyclohexyl)phthalimide (D42)

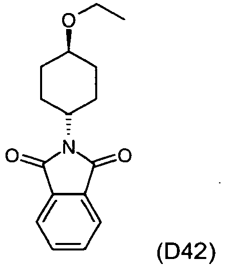

Acetaldehyde (10ml; 0.17mol) in acetonitrile (50ml) was added over 30 minutes to a solution of *trans*-N-(4-tert-butyldimethylsilyloxycyclohexyl)phthalimide (D41; 50.0g; 0.14ml), bismuth tribromide (6.7g; 0.015mol), triethylsilane (27ml; 0.18mol) in acetonitrile (500ml) stirred at ice bath temperature and the mixture was allowed to warm to room temperature overnight. The mixture was filtered and the resulting grey solid and filtrate were worked up separately. The filtrate was evaporated and treated with hexane to give the title compound as a white solid (16.35g). The grey solid washed with dichloromethane, the dichloromethane extract was evaporated and the residue was stirred with hexane (200ml) to give a second crop of the title compound as a white solid (17.47g).

### Description 43. trans-4-Ethoxycyclohexylamine (D43)

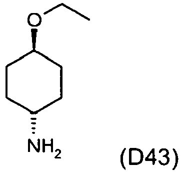

A solution of *trans*-N-(4-ethoxycyclohexyl)phthalimide (D42, 16.35g), hydrazine hydrate (12ml) in ethanol (300ml) and methanol (200ml) was stirred at reflux for 3 hours. The solvent was removed to give a slurry, which was treated with diethyl ether and filtered. The filtrate was evaporated to afford the title compound as a viscous oil contaminated with ether (8.16g).

### Description 44. 1-Ethyl-1-methyl-4-oxopiperidinium iodide (D44)

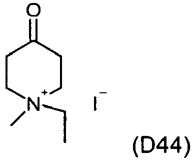

Iodomethane (65ml; 1.00mol) was added in portions to a solution of 1-ethyl-4-piperidone (100g; 0.79mol) in acetone (1 L) at 20-30°C (internal, ice cooling). After stirring for 3h more the title compound was obtained by filtration, and washing with acetone (189g).

### Description 45. trans-1-(4-Ethoxycyclohexyl)-4-piperidone (D45)

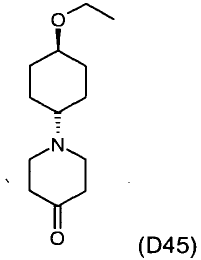

A mixture of 1-ethyl-4-piperidone methiodide (D44, 27g), *trans*-4-ethoxycyclohexylamine (D43, 8.16g, 0.065mol), potassium carbonate (13.5g), water (100ml), and ethanol (200ml) was heated for 3 hours at 80°C, then cooled overnight. The mixture was partitioned with aqueous sodium bicarbonate and dichloromethane. The dichloromethane layer was separated, washed with brine and solvent removed to give the title compound as a amber coloured oil (13.2g).

### Description 46. trans-N-(4-Propoxycyclohexyl)phthalimide (D46)

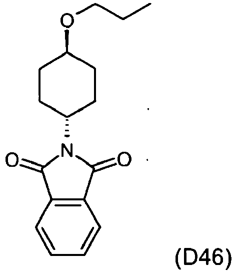

A solution of *trans*-N-(4-tert-butyldimethylsilyloxycyclohexyl)phthalimide (D41, 45g) in acetonitrile (500ml) at room temperature was treated sequentially with triethylsilane (24ml), bismuth tribromide (6g), and (dropwise) propanal (11ml) at 20-30°C (internal, ice cooling). After 30min more the solution was partitioned aqueous sodium bicarbonate /ethyl acetate. Drying and evaporation of the organic layer gave the title compound crystallised from pentane, 20g.

### Description 47. trans-4-Propoxycyclohexylamine (D47)

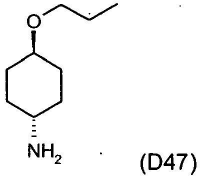

A mixture of *trans*-N-(4-propoxycyclohexyl)phthalimide (D46, 20g), hydrazine hydrate (15ml), and ethanol (400ml) was heated at at 80°C for 2h then cooled and filtered. The filtrate was evaporated and the resulting residue redissolved in diethyl ether, filtered and again evaporated to give the title compound, 11g.

### Description 48. trans-1-(4-Propoxycyclohexyl)-4-piperidone (D48)

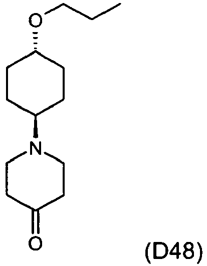

1-Ethyl-4-piperidone methiodide (D44, 26g) was added to a refluxing mixture of *trans*-4-propoxycyclohexylamine (D47, 11g), , potassium carbonate (1g), water (75ml), and ethanol (150ml) over 30min, and the mixture was then heated for another 30min at 80°C, then cooled, partitioned aqueous sodium bicarbonate / dichloromethane, and purified by chromatography (40+M Biotage silica column, 0 to 10% methanol in dichloromethane containing 0.2M ammonia) to give the title compound, 8g.

### Description 49. trans-1-(4-Propoxycyclohexyl)-4-piperidinamine dihydrochloride (D49)

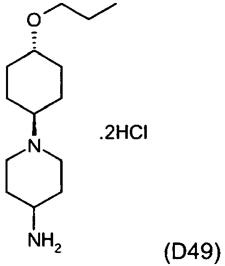

A mixture of *trans*-1-(4-propoxycyclohexyl)-4-piperidone (D48, 7.5g), 2M ammonia in methanol (100ml), and 10% palladium on carbon paste (100mg) was hydrogenated at 50psi at room temperature for 18h then filtered and evaporated. The residue was converted to the dihydrochloride salt to give the title compound, crystallised from diethyl ether, 7.5g.

### Description 50. cis/trans-Ethyl 4-{[(1,1-dimethylethyl)(dimethyl)silyl]oxy}cyclohexanecarboxylate (D50)

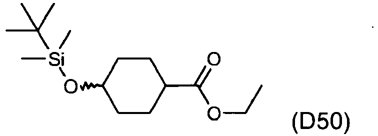

Chloro(1,1-dimethylethyl)dimethylsilane (115g; 0.76mol) was added in portions over 1 hour to a solution of ethyl 4-hydroxycyclohexanecarboxylate (118g; 0.68mol), imidazole (103g; 1.52mol) and dimethylformamide (400ml) stirred under an atmosphere of argon. A small exotherm was observed resulting in the reaction mixture temperature increasing to -40°C. The mixture was stirred at room temperature overnight then poured into 10% citric acid solution (2L) and extracted with diethyl ether (2 x 800ml). The ether extracts were washed with water, brine and then dried (Na₂SO₄) and the solvent was removed to give the title compound as a oil (198.4g)
¹H NMR δ (CDCl₃, 400 MHz): 0.01 (6H, m), 0.85 (9H, s), 1.2 (3H, m), 1.3-1.5 (2H, m), 1.6 (2H, m), 1.85-2 (3H, m), 2.15-2.3 (1H, m) 3.5 (0.4H, m) 3.86 (1H, m) 4.1 (1H, m).

### Description 51. cis/trans-Ethyl 4-(ethyloxy)cyclohexanecarboxylate (D51)

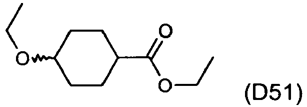

*cis*/*trans* ethyl 4-{[(1,1-dimethylethyl)(dimethyl)silyl]oxy}cyclohexanecarboxylate (D50, 35g, 122 mmol) was dissolved in CH₃CN (250 ml) and Et₃SiH (1.2 eq., 146 mmol, 23ml), BiBr₃ (4% mol, 4.9 mmol, 2.2 g) were added dropwise at room temperature followed by acetaldehyde (1.2 eq., 8.2 ml) that was slowly added at 25°C. The mixture was stirred at room temperature for 1 hour. The mixture was subsequently poured onto an aqueous saturated solution of NaHCO₃ and the mixture obtained was then extracted with EtOAc (3x). Organics were combined, washed with brine, dried over Na₂SO₄, filtered and the solvent was evaporated to afford the crude mixture that was purified by silica chromatography (Biotage 65i column, EtOAc-nhex) to afford the title compound, 21 g, 87%.
¹H NMR δ (DMSO, 400 MHz): 1.1 (3H, m), 1.15 (3H, m), 1.492-3.212 (assume 10 H, set of broad signals and multiplets), 3.312 (2 H, m), 4.041 (2H, m).

### Description 52. cis/trans-4-(Ethyloxy)cyclohexanecarboxylic acid (D52)

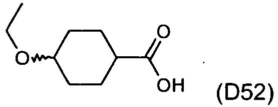

*cis*/*trans*-ethyl 4-(ethyloxy)cyclohexanecarboxylate (D51, 21 g, 105 mmol) was dissolved in MeOH-THF (100 ml-100 ml) and NaOH (5 eq., 0.5 mol, 40 ml, 12.5N aqueous solution) was slowly added at room temperature. The mixture was stirred at room temperature overnight. The THF/MeOH was then evaporated and the crude was washed with Et₂O. The aqueous phase was acidified and extracted with EtOAc (2x); organics were dried over Na₂SO₄, filtered and the solvent was evaporated to afford the title compound as a pale-yellow oil, 17.3 g, 96%.
¹H NMR δ (DMSO, 400 MHz): 1.1 (3H, m), 1.3-3.201 (assume 10 H, set of broad signals and multiplets), 3.417 (2H, m), 12.1 (1H, s broad).

### Description 53. cis/trans-4-(Ethyloxy)-1-methylcyclohexanecarboxylic acid (D53)

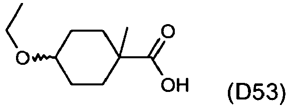

A stirred solution of diisopropylamine (2.3 eq., 0.231 mol, 33 ml) in dryTHF (400ml) at - 20°C under argon was treated over 10 min with 2.5M n-butyllithium in hexane (2.3 eq., 93 ml; 0.231 mol), then allowed to warm to 0°C and stir for 15 mins. The mixture was recooled to -10°C and treated over 10 min with a solution of *cis*/*trans*-4-(ethyloxy)cyclohexanecarboxylic acid (D52, 17.3g, 0.1 mol) in -50 ml of dry THF. The resulting yellow solution was heated at 50°C for 2.5hrs, then cooled to 0°C and treated with iodomethane (3 eq., 0.3 mol, 19 ml). The mixture was allowed to warm to room temperature and stir overnight. The mixture was cooled to 10°C, treated with 10% citric acid solution (200ml), and then solvent evaporated to half volume concentrated under vacuum. The residual mixture was diluted with water (200ml) and extracted with Et₂O (2x). The combined extracts were washed with water (150ml x 2), dried (Na₂SO₄), filtered and concentrated under vacuum to leave the title compound as a yellow oil, 16.2 g, 87%, a mixture of *cis:trans* isomers.
¹H NMR δ (DMSO, 400 MHz): 1.086 (assume 8H, m), 1.510 (3H, m), 1.698 (1H, m), 1.781 (1H, m), 2.005 (1H, m), 3.191 (1/2H, m), 3.392 (2H, m), 3.606 (1/2H, m), 12.2 (1H, s br).

### Description 54. trans-4-(Ethyloxy)-1-methylcyclohexanecarboxylic acid (D54)

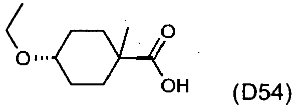

*cis*/*trans*-4-(ethyloxy)-1-methylcyclohexanecarboxylic acid (D53, 16.2 g, 87.1 mmol) was dissolved in thionyl chloride (15 eq., 1.31 mol, ∼95 ml) at at room temperature and it was subsequently reluxed at 85°C for 4 hrs. The mixture was then allowed to cool to room temperature and the thionyl chloride was azeotropically evaporated with toluene. The residue was dissolved in THF (100ml), treated with a 5 % aqueous solution of Na₂CO₃ (500 ml) and stirred well at room temperature for 20 minutes. The aqueous residue washed with Et₂O (2), then acidified and extracted with EtOAc (3x). The combined extracts were dried (Na₂SO₄), filtered and concentrated under vacuum to leave the title compound, 6.5 g, 40%
¹H NMR δ (DMSO, 400 MHz): 1.076 (6H, m), 1.505 (6H, m), 1.693 (2H, m), 3.331 (1H, m), 3.394 (2H, q), 12.1 (1H, s br).

### Description 55. trans-4-(Ethyloxy)-1-isocyanato-1-methylcyclohexane (D55)

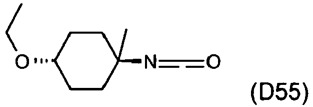

A stirred solution of *trans*-4-(ethyloxy)-1-methylcyclohexanecarboxylic acid (D54, 5.5g, 29.5mmol) in toluene (120ml) at room temperature under argon was treated with triethylamine (1.3 eq., 37.7 mmol, 5.3 ml) and diphenylphosphoryl azide (1eq., 29.5 mmol, 6.4 ml) and heated at 85°C for 1 hr. The mixture was allowed to cool to room temperature, then treated with 1 M NaOH solution (300ml) and extracted with Et₂O (3x). The combined extract was dried (Na₂SO₄), filtered and concentrated under vacuum to leave the title compound, 5 g, 94 %.
¹H NMR δ (DMSO, 400 MHz): 1.092 (3H, t), 1.330 (3H, s), 1.487-1.691 (8H, m), 3.392 (2H, q), 3.477 (1H, m).

### Description 56. [trans-4-(Ethyloxy)-1-methylcyclohexyl]amine mono hydrochloride (D56)

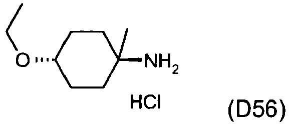

A solution of *trans*-4-(ethyloxy)-1-isocyanato-1-methylcyclohexane (D55, 5.0g, 27.3 mmol) in THF (100ml) was treated with 5M aqueous HCl acid (5.5 eq., 150 mmol, 30 ml) and stirred at room temperature under argon overnight, then concentrated under vacuum. The residual semi-solid was triturated with Et₂O to give a first batch of title compound as a white solid, 2.8 g. The mother liquors were evaporated, dissolved in THF again (50 ml) and treated with 5M aqueous HCl acid (15 ml) and the mixture stirred over a weekend at room temperature. The solvent was then evaporated and the solid obtained was dried in the oven at 50°C before trituration with Et₂O. This final trituration afforded further 646 mg of title compound.
¹H NMR δ (DMSO, 400 MHz): 1.084 (3H, t), 1.256 (3H, s), 1.378 (2H, m), 1.619 (4H, m), 1.847 (2H, m), 3.243 (1H, m), 3.424 (2H, q), 8.090 (3H, br s).

### Description 57. 1-[trans-4-(Ethyloxy)-1-methylcyclohexyl]-4-piperidinone (D57)

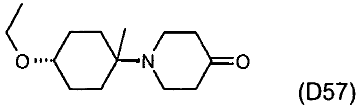

A stirred solution of [*trans*-4-(ethyloxyyl-methylcydohexyl]amine mono hydrochloride (D56, 3.44 g, 17.8 mmol) in a mixture of ethanol (216 ml) and water (108 ml) at room temperature under argon was treated with potassium carbonate (1.1 eq., 19.6 mmol, 2.7g) followed by 1-ethyl-1-methyl-4-oxopiperidinium iodide (D44, 1.5 eq., 26.7 mmol, 7.1g), then heated at 80°C for 2 hours. The mixture was allowed to cool to room temperature then the aqueous residue was treated with sat. NaHCO₃ solution and extracted with dichloromethane (3x). The combined extracts were dried (Na₂SO₄) and concentrated under vacuum to leave the crude compound which was chromatographed on silica gel (Biotage 65i column) eluting with 0-10% MeOH/NH₃/DCM to afford the title compound, 2.3 g, 54%.
¹H NMR δ (DMSO, 400 MHz): 0.855 (3H, s), 1.099 (3H, t), 1.421 (2H, m), 1.544 (4H, m), 1.793 (2H, m), 2.297 (4H, t), 2.726 (4H, t), 3.377-3.430 (3H, t + m).

### Description 58. 1-[trans-4-(Ethyloxy)-1-methylcyclohexyl]-4-piperidinamine dihydrochloride (D58)

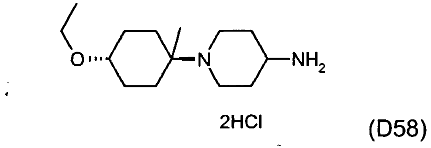

A solution of 1-[*trans*-4-(ethyloxy)-1-methylcyclohexyl]-4-piperidinone (2.3g, 9.62 mmol) in 2M NH₃/MeOH (200ml) was treated with 10% Pd/C (510 mg) and shaken under hydrogen atmosphere at 50psi initial pressure overnight at room temperature. The mixture was filtered through Kieselguhr to remove catalyst and the filtrate concentrated under vacuum to leave the free base of the title compound. This was treated with 10 ml of HCl (1M solution of in Et₂O) and stirred in MeOH (20 ml) for 10 min. Solvent was subsequently evaporated to afford the title compound as a white solid 2.8 g, complete conversion.
¹H NMR δ (DMSO, 250 MHz, at 352.2K): 1.091 (6H, m br), 1.336 (2H, m), 1.695-1.865 (7H, m), 2.067 (2H, d br), 2.531 (1H, br), 3.187-3.317 (assume 6H, set of broad signals and multiplets), 3.434 (2H, q), 8.496 (2H, s br).

### Description 59. cis-4-(Ethyloxy)-1-isocyanato-1-methylcyclohexane (D59)

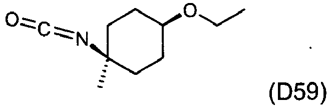

A stirred solution of *cis*/*trans*-4-(ethoxy)-1-methylcyclohexanecarboxylic acid (D53, 2.0g, 10.7mmol) in toluene (40ml) at room temperature under argon was treated with triethylamine (1.95ml, 14.0mmol) and diphenylphosphoryl azide (2.3ml, 10.7mmol) and heated at 80°C for 45 mins. The mixture was allowed to cool to room temperature, then treated with 1 M NaOH solution (50ml) and Et₂O (50ml), shaken well and the organic solution separated, dried (Na₂SO₄) and concentrated under vacuum to leave a yellow oil (1.7g), which was chromatographed on silica gel (40g) eluting with 0-10% EtOAc/hexane. This afforded the title compound as the lower rf component (215mg) along with mixed fractions containing both isomers.
¹H NMR (cis isomer) δ (CDCl₃, 400 MHz): 1.20 (3H, t), 1.32-1.50 (5H, m), 1.5-1.66 (2H, m), 1.78-1.96 (4H, m), 3.16-3.26 (1H, m), 3.48-3.58 (2H, q)

### Description 60. [cis-4-(Ethyloxy)-1-methylcyclohexyl]amine hydrochloride (D60)

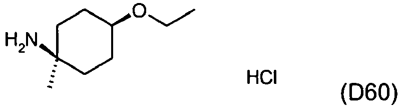

A solution of *cis*-4-(ethyloxy)-1-isocyanato-1-methylcyclohexane (D59, 215mg, 1.17mmol) in THF (5ml) was treated with 5M HCl acid (1ml) and stirred at room temperature for 18hrs, then concentrated under vacuum and the residue azeotroped with toluene to remove traces of water. The residual colourless oil was dried under vacuum for 2 days. Trituration of the remaining semi-solid with Et₂O (5ml) afforded the title compound as a white solid (213mg, 94%).
¹H NMR δ (CDCl₃, 400 MHz): 1.19 (3H, t), 1.50 (3H, s), 1.55-1.77 (4H, m), 1.79-1.91 (2H, m), 2.02-2.15 (2H, m), 3.34-3.40 (1H, m), 3.47 (2H, q), 8.40 (3H, br s).

### Description 61. 1-[cis-4-(Ethyloxy)-1-methylcyclohexyl]-4-piperidinone (D61)

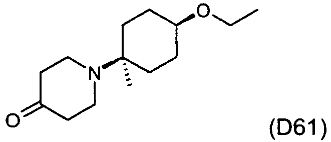

A stirred solution of [*cis*-4-(ethyloxyfl-methylcyclohexyl]amine hydrochloride (D60, 200mg, 1.03mmol) in a mixture of ethanol (12ml) and water (6ml) at room temperature under argon was treated with potassium carbonate (156mg, 1.13mmol) followed by 1-ethyl-1-methyl-4-oxopiperidinium iodide (D44, 404mg, 1.50mmol), then heated at 80°C for 1.5hrs. The mixture was allowed to cool, concentrated under vacuum to approx. 7ml, then treated with NaHCO₃ solution (15ml) and extracted with dichloromethane (3x20ml). The combined extract was dried (Na₂SO₄) and concentrated under vacuum to leave an brown oil (250mg), which was chromatographed on silica gel eluting with 0-5% MeOH/dichloromethane to afford the title compound as a yellow oil (140mg, 57%).
¹H NMR δ (CDCl₃, 400 MHz): 0.89 (3H, s), 1.13-1.27 (2H, m), 1.20 (3H, t), 1.68-1.80 (4H, m), 1.95-2.05 (2H, m), 2.40-2.47 (4H, m), 2.76-2.83 (4H, m), 3.25-3.33 (1H, m), 3.52 (2H, q).

### Description 62. 1-[cis-4-(Ethyloxy)-1-methylcyclohexyl]-4-piperidinamine (D62)

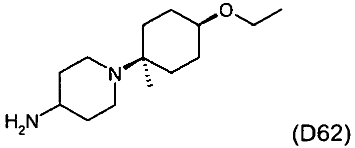

A solution of 1-[*cis*-4-ethoxy-1-methylcyclohexyl]-4-piperidinone (D61, 140mg, 0.585mmol) in 2M NH₃/MeOH (12ml) was treated with 10% Pd/C paste (30mg) and shaken under hydrogen atmosphere at 50psi initial pressure for 2.5 days. The mixture was filtered through Kieselguhr to remove catalyst and the filtrate concentrated under vacuum to leave the title compound as a colourless oil (140mg, 100%).
¹H NMR δ (CDCl₃, 400 MHz): 0.84 (3H, s), 1.04-1.20 (2H, m), 1.20 (3H, t), 1.37-1.50 (2H, m), 1.58-1.72 (4H, m), 1.83-2.00 (4H, m), 2.05-2.20 (2H, m), 2.65-2.75 (1H, m), 2.88 (2H, br d), 3.20-3.30 (1H, m), 3.20-3.80 (assume 4H including 3.50 = 2H, q).

### Description 63 cis/trans-Ethyl 4-(propyloxy)cyclohexanecarboxylate (D63)

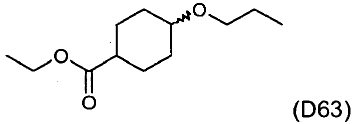

Propionaldehyde (6.4g) in acetonitrile (50ml) was added over 30 minutes to a solution of *cis*/*trans* ethyl 4-{[(1,1-dimethylethyl)(dimethyl)silyl]oxy}cyclohexanecarboxylate (D50, 25.2g), bismuth tribromide (4.4g), triethylsilane (17.5ml) in acetonitrile (300ml) and the mixture was stirred fro a further 1.5 hours. The solvent was partially removed then the residue was treated with ethyl acetate and saturated sodium bicarbonate solution. The organic layer was separated, washed with brine, dried with anhydrous sodium sulphate and the solvent was removed to give the title compound contaminated with silicone residues (39.1g).

### Description 64. cis/trans-4-(Propyloxy)cyclohexanecarboxylic acid (D64)

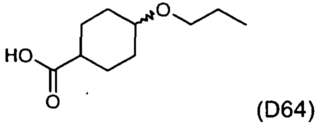

The product from Description 63 (39.1g), 40% w/w sodium hydroxide solution (150ml) tetrahydrofuran (200ml) and methanol (150ml) was stirred for approx. 72 hours. The solvent was partially removed then the resulting mixture was treated with ethyl acetate and water. The aqueous layer was separated, acidified with concentrated hydrochloric acid and extracted with diethyl ether. The ether layer was washed with brine, dried with anhydrous sodium sulphate and the solvent was removed to give the title compound as a oil (15.42g).

### Description 65. cis/trans-1-Methyl-4-(propyloxy)cyclohexanecarboxylic acid (D65)

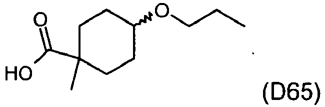

A stirred solution of diisopropylamine (24ml, 0.17mol) in THF (300ml) at -20°C under argon was treated over 10 mins with 2.5M n-butyllithium in hexane (68ml, 0.17mol), then allowed to warm to 0°C and stir for 15 mins. The mixture was recooled to -10°C and treated over 10 mins with a solution of 4-(propyloxy)cyclohexanecarboxylic acid (D64, 13.8g, 0.074mol) in THF. The resulting yellow solution was heated at 50°C for 2.5hrs, then cooled to 0°C and treated with iodomethane (13.8m, 0.22mol). The mixture was allowed to warm to room temperature and stir for 20hrs when a yellow precipitate had formed. The mixture was cooled to 10°C, treated with 10% citric acid solution (200ml), then concentrated under vacuum to approx. 250ml volume. The residual mixture was diluted with water (200ml) and extracted with Et₂O (3x250ml). The combined extract was dried (Na₂SO₄) and concentrated under vacuum to leave a yellow oil (15.0g) which was approx. 60:40 mixture of cis:trans isomers.

### Description 66. trans-1-Methyl-4-(propyloxy)cyclohexanecarboxylic acid (D66)

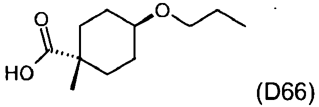

*cis*/*trans*-1-Methyl-4-(propyloxy)cyclohexanecarboxylic acid (D65, 13g, 0.070mol) was added to stirred thionyl chloride (50ml), 0.68mol) at 10°C and then allowed to warm to room temperature, followed by heating at 85°C for 3 hrs. The mixture was concentrated under vacuum and the residue concentrated twice with toluene to remove excess thionyl chloride. The residue was dissolved in THF (100ml), treated with dil. NaHCO₃ solution (250ml) and stirred well at room temperature for 24 hrs, followed by standing at room temperatue for 3 days. The mixture from the same stage of a smaller scale reaction on 2g of *cis*/*trans*-1-methyl-4-(propyloxy)cyclohexanecarboxylic acid was combined at this time. The combined mixture was concentrated under vacuum to approx. 300ml and the aqueous residue washed with Et₂O (2x120ml), then acidified with 2M HCl acid and extracted with EtOAc (2x150ml). The combined extract was dried (Na₂SO₄) and concentrated under vacuum to leave the title compound as a pale yellow solid (5.1g, 34%).
¹H NMR δ (CDCl₃, 400 MHz): 0.92 (3H, t), 1.24 (3H, s), 1.54-1.74 (8H, m), 1.78-1.88 (2H, m), 3.33-3.40 (3H, m). 1H not discernible from spectrum.

### Description 67. trans-1-Isocyanato-1-methyl-4-(propyloxy)cyclohexane (D67)

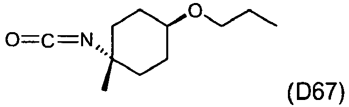

A stirred solution of *trans*-1-methyl-4-(propyloxy)cyclohexanecarboxylic acid (D66, 5.1g, 0.027mol) in toluene (120ml) at room temperature under argon was treated with triethylamine (4,9ml, 0.035mol) and diphenylphosphoryl azide (5.8ml, 0.027mol) and heated at 85°C for 1 hr. The mixture was allowed to cool to room temperature, then treated with 1 M NaOH solution (200ml) and extracted with Et₂O (2x150ml). The combined extract was dried (Na₂SO₄) and concentrated under vacuum to leave the title compound as a yekllow oil (5.0g, 100%).
¹H NMR δ (CDCl₃, 400 MHz): 0.91 (3H, t), 1.36 (3H, s), 1.50-1.60 (4H, m), 1.65-1.80 (6H, m), 3.33 (2H, t), 3.46-3.52 (1H, m).

### Description 68. [trans-1-Methyl-4-(propyloxy)cyclohexyl]amine hydrochloride (D68)

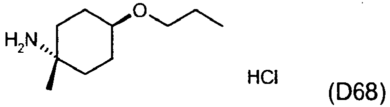

A solution of *trans*-1-isocyanato-1-methyl-4-(propyloxy)cyclohexane (D67, 5.0g, 0.027mol) in THF (100ml) was treated with 5M HCl acid (25ml) and stirred at room temperature under argon for 20hrs, then concentrated under vacuum and the residue azeotroped with toluene to remove traces of water. The residual semi-solid was triturated with Et₂O (120ml) to give a solid, which was filtered off, washed with Et₂O and dried at 50°C under vacuum to afford the title compound as a white solid (4.5g, 85%).
¹H NMR δ (CDCl₃, 400 MHz): 0.91 (3H, t), 1.47 (3H, s), 1-45-1.70 (4H, m), 1.75-2.00 (6H, m), 3.52 (2H, t), 3.40-3.48 (1H, m), 8.38 (3H, br s).

### Description 69. 1-[trans-1-Methyl-4-(propyloxy)cyclohexyl]-4-piperidinone (D69)

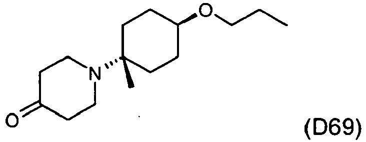

A stirred solution of [*trans*-1-methyl-4-(propyloxy)cyclohexyl]amine hydrochloride (D68, 4.5g, 0.022mol) in a mixture of ethanol (100ml) and water (60ml) at room temperature under argon was treated with potassium carbonate (3.31g, 0.024mol) followed by 1-ethyl-1-methyl-4-oxopiperidinium iodide (D44, 8.91g, 0.033mol), then heated at 80°C for 2.5hrs. The mixture was allowed to cool, concentrated under vacuum to approx. 60ml, then the aqueous residue was treated with sat. NaHCO₃ solution and extracted with DCM (3x80ml). The combined extract was dried (Na₂SO₄) and concentrated under vacuum to leave an orange oil (6.1 g), which was chromatographed on silica gel eluting with 0-10% MeOH/DCM to afford the title compound as a yellow oil (3.45g, 63%).
¹H NMR δ (CDCl₃, 400 MHz): 0.93 (3H, s + 3H, t), 1.48-1.72 (8H, m), 1.80-1.92 (2H, m), 2.41 (4H, t), 2.82 (4H, t), 3.35-3.45 (3H, t + m).

### Description 70. 1-[trans-1-Methyl-4-(propyloxy)cyclohexyl]-4-piperidinamine (D70)

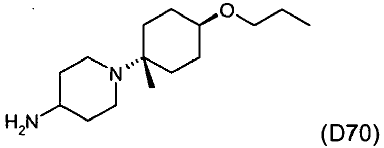

A solution of 1-[*trans*-1-methyl-4-(propyloxy)cyclohexyl]-4-piperidinone (D69, 2.0g, 0.0079mol) in 2M NH₃/MeOH (60ml) was treated with 10% Pd/C (150mg) and shaken under hydrogen atmosphere at 55psi initial pressure for 3.5 days at room temperature. The mixture was filtered through Kieselguhr to remove catalyst and the filtrate concentrated under vacuum to leave the title compound as a white solid (1.82g, 91 %).
¹H NMR δ (CDCl₃, 400 MHz): 0.91 (3H, t), 1.08 (3H, br s), 1.32-1.45 (2H, m), 1.5-1-60 (2H, m), 1.62-2.60 (assume 12H, set of broad signals), 2.70-3.10 (1H, br), 34.10-3.25 (2H, br), 3.30-3.45 (3H, m), 3.40-4.40 (2H, v br).

### Description 71. 1-[trans-4-(Ethyloxy)cyclohexyl]-N-(5-fluoro-2-nitrophenyl)-4-piperidinamine (D71)

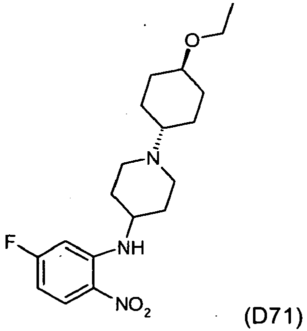

A stirred solution of 2,4-difluoro-1-nitrobenzene (100mg, 0.63mmol) in DMF (3ml) at room temperature under argon was treated with diisopropylethylamine (0.40ml, 2.2mmol) followed by 1-[*trans*-4-(ethyloxy)cyclohexyl]-4-piperidinamine dihydrochloride (D12, 179mg, 0.60mmol) and maintained at room temperature for 20hrs, then heated at 50°C for 7hrs. The mixture was concentrated under vacuum and the residue treated with 10% Na₂CO₃ solution (10ml) and extracted with dichloromethane (2x15ml). The combined extract was dried (Na₂SO₄), concentrated under vacuum and the residue chromatographed on silica gel (10g) eluting with 0-10% methanol/dichloromethane to give a yellow solid which was recrystallised from methanol to afford the title compound as an yellow solid (120mg, 55%). MH⁺ 366.
¹H NMR δ (CDCl₃, 400 MHz): 1.15-1.40 (7H, m), 1.60-1.72 (2H, m), 1.90-1.96 (2H, m), 2.05-2.18 (4H, m), 2.30-2.50 (3H, m), 2.85-2.93 (2H, m), 3.13-3.23 (1H, m), 3.38-3.50 (1H, m), 3.52 (2H, q), 6.31-6.40 (1H, m), 6.48 (1H, dd), 8.18-8.30 (2H, m).

### Description 72. N²-{1-[trans-4-(Ethyloxy)cyclohexyl]-4-piperidinyl}-4-fluoro-1,2-benzenediamine (D72)

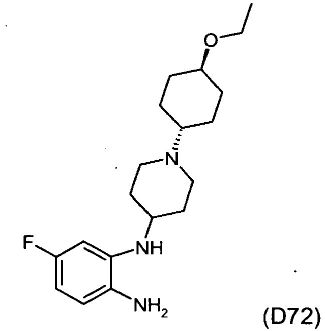

A stirred solution of 1-[*trans*-4-(ethyloxy)cyclohexyl]-*N*-(5-fluoro-2-nitrophenyl)-4-piperidinamine (D71, 120mg, 0.33mmol) in ethanol (15ml) at room temperature under argon was treated with Raney Nickel (∼20mg) followed by hydrazine hydrate (0.10ml, 3.3mmol) and maintained for 2 hrs. The mixture was filtered through Kieselguhr to remove catalyst and the filtrate concentrated under vacuum to afford the title compound as a pale brown solid (110mg, 100%yield). MH⁺ 336.
¹H NMR δ (CDCl₃, 400MHz): 1.17-1.37 (4H, m), 1.20 (3H, t), 1.42-1.54 (2H, m), 1.88-1.96 (2H, m), 2.02-2.15 (4H, m), 2.28-2.42 (3H, m), 2.92 (2H, br d), 3.07 (2H, br s), 3.13-3.25 (2H, m), 3.51 (2H, q), 3.60 (1H, br s), 6.25-6.40 (2H, m), 6.60-6.67 (1H, m),

### Description 73. N-(5-Bromo-2-nitrophenyl)-1-[trans-4-(ethyloxy)cyclohexyl]-4-piperidinamine (D73)

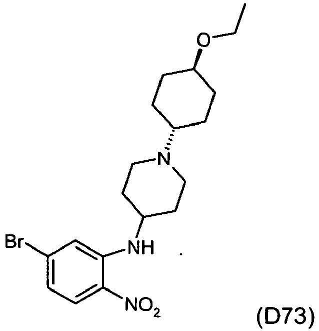

A mixture of 4-bromo-2-fluoro-1-nitrobenzene (600mg, 2.72mmol) and 1-[*trans*-4-(ethoxy)cyclohexyl]-4-piperidinamine dihydrochloride (D12, 807mg, 2.72mmol) in dimethylformamide (20ml) was treated with diisopropylethylamine (1.4ml, 8.16mmol) then heated with stirring at 80°C overnight under argon. The solution was washed with water then extracted with dichloromethane. The extract was dried (Na₂SO₄) and concentrated. The residue was loaded on an SCX cartridge and eluted firstly with dichloromethane (approx. 30ml) and then with 2M NH₃/MeOH (approx. 30ml). The methanolic solution was concentrated under vacuum to leave a yellow/orange solid (1.4g). This was purified by chromatography on an amino silica column (40+M) eluting with 3-25% EtOAc/hexane afford the title compound (1.15g, 99%). MH+ = 427, 428.
¹H NMR δ (CDCl₃, 400MHz): 1.15-1.38 (4H, m), 1.20 (3H, t), 1.58-1.72 (2H, m), 1.88-1.98 (2H, m), 2.02-2.16 (4H, m), 2.3-2.38 (1H, m), 2.402.50 (2H, m), 2.85-2.95 (2H, m), 3.13-3.24 (1H, m), 3.50 (2H, t), 6.73 (1H, dd), 7.00 (1H, d), 8.03 (1H, d), 8.14 (1H, d).

### Description 74. 4-Bromo-N²-{1-[trans-4-(ethyloxy)cyclohexyl]-4-piperidinyl}-1,2-benzenediamine (D74)

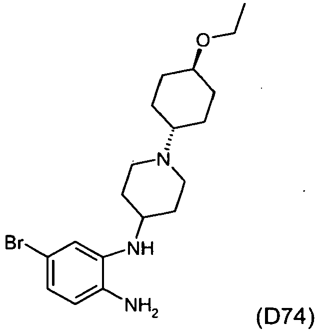

A stirred solution of *N*-(5-bromo-2-nitrophenyl)-1-[*trans*-4-(ethyloxy)cyclohexyl]-4-piperidinamine (D73, 650mg, 1.53mmol) in ethanol (100ml) at room temperature under argon was treated with Raney Nickel (100mg) and dropwise addition of hydrazine hydrate (1.2m, 38.25mmol) and maintained for 3hrs. The mixture was filtered through Kieselguhr and the filtrate concentrated under vacuum to leave the title compound as an amber oil (600mg, 99%). MH+ = 397, 398.
¹H NMR δ (CDCl₃, 400MHz): 1.20 (3H, t), 1.20-1.40 (4H, m), 1.38-1.55 (2H, m), 1.90-2.00 (2H, m), 2.00-2.15 (4H, m), 2.25-2.43 (3H, m), 2.90 (2H, br d), 3.13-3.43 (5H, m), 3.51 (2H, t), 6.57 (1H, d), 6.70-6.78 (2H, m).

### Description 75. N-(5-Ethenyl-2-nitrophenyl)-1-[trans-4-(ethyloxy)cyclohexyl]-4-piperidinamine (D75)

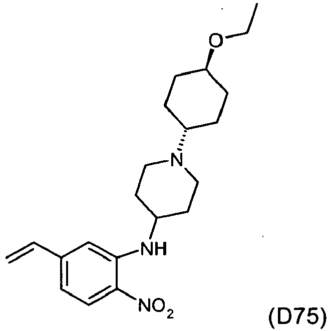

A solution of *N*-(5-bromo-2-nitrophenyl)-1-[*trans*-4-(ethyloxy)cyclohexyl]-4-piperidinamine (D73, 500mg, 1.17mmol) and tributylvinyltin (0.42ml, 1.36mmol) in toluene (15ml) at room temperature under argon was treated with triphenylphosphine (77mg, 0.29mmol) and bis(dibenzylidene)acetone)palladium(0) (102mg, 0.18mmol) and heated at 120°C overnight. The mixture was allowed to cool, then filtered through Kieselguhr. The organic filtrate was washed with 10% aqu. ammonium hydroxide solution and water, then dried (Na₂SO₄) and concentrated under vacuum to leave a brown solid (approx. 550mg). This was dissolved in dichloromethane and eluted through an SCX cartrtidge, then collected by elution with 2M NH₃/MeOH and concentrated. The residue was chromatographed on 40+M Biotage KP-NH^{™}-silica column to afford the title compound as an orange solid (99%). MH+ = 374.
¹H NMR δ (CDCl₃, 400MHz): 1.18-1.40 (4H, m), 1.20 (3H, t), 1.55-1.73 (assume 2H, m), 1.90-2.00 (2H, m), 2.03-2.18 (4H, m), 2.30-2.40 (1H, m), 2.40-2.50 (2H, m), 2.85-2.94 (2H, m), 3.13-3.23 (1H, m), 3.50-3.61 (1H, m), 3.51 (2H, q), 5.46 (1H, d), 5.87 (1H, d), 6.66 (1H, dd), 6.72 (2H, m), 8.14 (1H, dd), 8.22 (1H, d).

### Description 76. 4-Ethyl-N²-{1-[trans-4-(ethyloxy)cyclohexyl]-4-piperidinyl}-1,2-benzenediamine (D76)

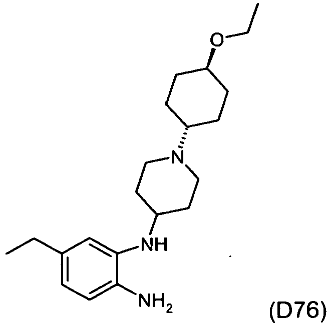

A mixture of *N*-(5-ethenyl-2-nitrophenyl)-1-[*trans*-4-(ethyloxy)cyclohexyl]-4-piperidinamine (D75, 440mg, 1.17mmol) and ammonium formate (734mg, 11.7mmol) in methanol (50ml) was stirred at room temperature under argon for 10mins, then treated with 10% Pd/C (261mg, 0.2equivalents) and stirred for 30mins. The mixture was filtered through Kieselguhr and concentrated to remove solvent. The residue was dissolved in dichloromethane, washed with 2M NaOH solution, dried (Na₂SO₄) and concentrated to leave the title compound as a purple sticky oil (348mg, 86%). MH+ = 346.
¹H NMR δ (CDCl₃, 400MHz): 1.18-1.40 (10H, t + t + m), 1.42-1.58 (2H, m), 1.90-2.00 (2H, m), 2.03-2.16 (4H, m), 2.30-2.42 (3H, m), 2.53 (2H, q), 2.86-2.95 (2H, m), 3.00-3.40 (assume 5H, m), 3.51 (2H, q), 6.50 (2H, m), 6.65 (1H, d).

### Description 77. N-(5-Cyclopropyl-2-nitrophenyl)-1-[trans-4-(ethyloxy)cyclohexyl]-4-piperidinamine (D77)

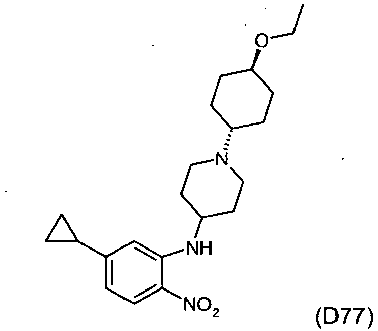

A mixture of 4-cyclopropyl-2-fluoro-1-nitrobenzene (200mg, 1.1mmol) and 1-[*trans*-4-(ethyloxy)cyclohexyl]-4-piperidinamine dihydrochloride (D12, 327mg, 1.1 mmol) in dimethylformamide (20ml) was treated with diisopropylethylamine (0.56ml) then heated under argon at 80°C overnight. The solution was washed with water then extracted with dichloromethane. The extract was dried (Na₂SO₄) and concentrated under vacuum. The residue was dissolved in dichloromethane and eluted through an SCX cartridge, firstly with dichloromethane and then with 2M NH₃/MeOH. The methanolic solution was concentrated under vacuum to leave a dark orange solid (300mg), which was purified by chromatography on a 40+M Biotage KP-NH^{™}-silica column eluting with 5-30% EtOAc/hexane to afford the title compound as an orange solid (240mg, 60%). MH+ = 388.
¹H NMR δ (CDCl₃, 400MHz): 0.77-0.83 (2H, m), 1.03-1.11 (2H, m), 1.17-1.40 (4H, m), 1.20 (3H, t), 1.58-1.72 (2H, m), 1.82-1.98 (3H, m), 2.02-2.15 (4H, m), 2.30-2.40 (1H, m), 2.40-2.50 (2H, m), 2.82-2.92 (2H, m), 3.13-3.24 (1H, m), 3.48-3.62 (3H, t + m), 6.18 (1H, dd), 6.55 (1H, d), 8.05 (1H, d), 8.21 (1H, d).

### Description 78. 4-Cyclopropyl-N²-{1-[trans-4-(ethyloxy)cyclohexyl]-4-piperidinyl}-1,2-benzenediamine (D78)

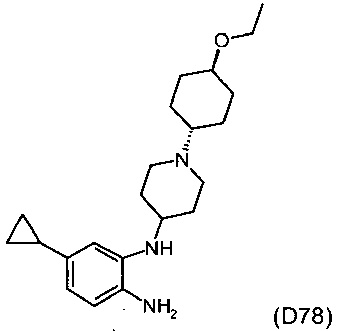

A stirred solution of *N*-(5-cyclopropyl-2-nitrophenyl)-1-[*trans*-4-(ethyloxy)cyclohexyl]-4-piperidinamine (D77, 240mg, 0.62mmol) in ethanol (40ml) at room temperature under argon was treated with Raney Nickel (50mg) and dropwise addition of hydrazine hydrate (0.5ml, 15.5mmol) and maintained for 2hrs. The mixture was filtered through Kieselguhr and the filtrate concentrated under vacuum to leave the title compound as green/yellow oil (200mg, 90%). MH+ = 358.
¹H NMR δ (CDCl₃, 400MHz): 0.57-0.63 (2H, m), 0.83-0.90 (2H, m), 1.14-1.40 (4H, m), 1.20 (3H, t), 1.40-1.60 (assume 2H, m), 1.75-1.85 (1H, m), 1.90-2.00 (2H, m), 2.02-2.16 (4H, m), 2.29-2.42 (3H, m), 2.87-2.93 (2H, m), 3.00-3.40 (5H, m), 3.45-3.57 (2H, q), 6.37 (1H, dd), 6.42 (1H, d), 6.62 (1H, d).

### Description 79. 1-[trans-4-(Ethyloxy)cyclohexyl]-N-[5-(methyloxy)-2-nitrophenyl]-4-piperidinamine (D79)

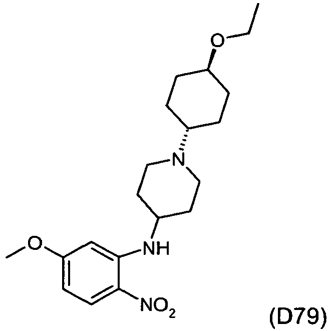

A stirred solution of 3-fluoro-4-nitroanisole (120mg, 0.70mmol) in dimethylformamide (4ml) at room temperature under argon was treated with diisopropylethylamine (0.43ml, 2.4mmol) followed by 1-[*trans*-4-(ethyloxy)cyclohexyl]-4-piperidinamine dihydrochloride (D12, 200mg, 0.67mmol) and heated at 50°C for 8hrs, followed by 90°C for 20hrs. The mixture was concentrated under vacuum and the residue treated with 10% Na₂CO₃ solution (10ml) and extracted with dichloromethane. The extract was dried (Na₂SO₄), concentrated under vacuum and the residue chromatographed on silica gel (5g) eluting with 0-10% methanol/dichloromethane to give a yellow oil, which was crystallised from methanol (6ml) to afford the title compound as an yellow solid (130mg, 52%). MH⁺ 378.
¹H NMR δ (CDCl₃, 400 MHz): 1.15-1.38 (4H, m), 1.20 (3H, t), 1.6-1.75 (2H, m), 1.90-1.96 (2H, m), 2.02-2.15 (4H, m), 2.25-2.38 (1H, m), 2.38-2.48 (2H, m), 2.82-2.92 (2H, m), 3.12-3.23 (1H, m), 3.43-3.56 (3H, m), 3.86 (3H, s), 6.16 (1H, d), 6.2 (1H, dd), 8.15 (1H, d), 8.37 (1H, d).

### Description 80. N²-{1-[trans-4-(Ethyloxy)cyclohexyl]-4-piperidinyl}-4-(methyloxy)-1,2-benzenediamine (D80)

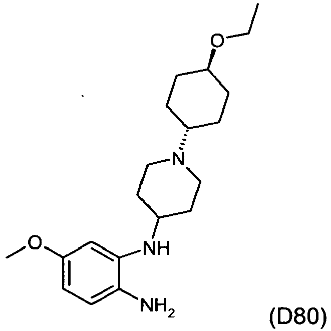

A stirred solution of 1-[*trans*-4-(ethyloxy)cyclohexyl]-*N*-[5-(methyloxy)-2-nitrophenyl]-4-piperidinamine (D79, 130mg, 0.34mmol) in ethanol (15ml) at room temperature under argon was treated with Raney Nickel (∼20mg) followed by hydrazine hydrate (0.10ml, 3.3mmol) and maintained for 2 hrs. The mixture was filtered through Kieselguhr to remove catalyst and the filtrate concentrated under vacuum to afford the title compound as a beige solid (120mg, 100%yield). MH⁺ 348.
¹H NMR δ (CDCl₃, 400MHz): 1.15-1.38 (4H, m), 1.18 (3H, t), 1.40-1.55 (2H, m), 1.90-1.96 (2H, m), 2.03-2.13 (4H, m), 2.23-2.38 (3H, m), 2.85-3.10 (4H, m), 3.13-3.26 (2H, m), 3.51 (2H, q), 3.60 (1H, br s), 3.75 (3H, s), 6.15 (1H, dd), 6.25 (1H, d), 6.65 (1H, d).

### Description 81. 1,1-Dimethylethyl 4-{[({2-bromo-4-[(trifluoromethyl)oxy]phenyl} amino)carbonyl]amino}-1-piperidinecarboxylate (D81)

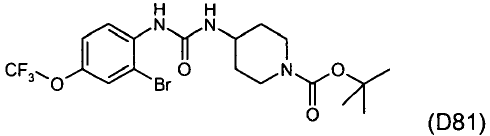

A solution of 2-bromo-4-(trifluoromethyloxy)aniline (5.1 g), in 1,4-dioxane (30 mL) was treated with bis(trichloromethyl) carbonate (2.2g). The mixture was heated to 100°C to give a clear solution then allowed to cool to ambient temperature. The solvent was evaporated and the residue was dissolved in dichloromethane (70ml) and 1,1-dimethylethyl 4-amino-1-piperidinecarboxylate (4.0 g) was added. The mixture was stirred overnight at room temperature. Silica gel was added and the solvent was evaporated. The residue was loaded onto a silica gel chromatography column which was eluted with hexane/ethyl acetate 0-100% followed by chromatography on silica gel eluted with dichloromethane/methanol 0-10% to give the title compound as a white solid from diethyl ether (4.3 g).
¹HNMR δ (DMSO, 400 MHz): 1.25 (2H, m), 1.40 (9H, s), 1.79 (2H, m), 2.93 (2H, broad), 3.64 (1H, m), 3.80 (2H, m), 7.21 (1H, d), 7.35 (1H, d), 7.66 (1H, s), 7.91 (1H, s), 8.19 (1H, d).

### Description 82. 1,1-Dimethylethyl 4-{2-oxo-6-[(trifluoromethyl)oxy]-2,3-dihydro-1H-benzimidazol-1-yl}-1-piperidinecarboxylate (D82)

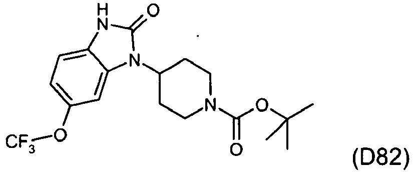

A solution of tris(dibenzylideneacetone)dipalladium (0) (0.33g), 1,1'-bis(diphenylphosphino)ferrocene (0.21g), sodium tert-butoxide (1.1g) in 1,4-dioxane (20 mL) was stirred under an atmosphere of argon for 10 minutes. 1,1-Dimethylethyl 4-{[({2-bromo-4-[(trifluoromethyl)oxy]phenyl}amino)carbonyl]amino}-1-piperidinecarboxylate (D81, 2.7 g) was added and the mixture was heated to 90°C. The mixture was stirred at this temperature overnight. The mixture was poured into dichloromethane which was washed with dilute ammonium chloride, brine and dried over sodium sulphate. The solvent was removed and the residue was chromatographed on silica gel eluted with ethyl acetate/hexane 0-50% to give the title compound as a solid foam (1.54 g).
¹HNMR δ (DMSO, 400 MHz): 1.41 (9H, m), 1.72 (2H, s), 2.24 (2H, m), 2.85 (2H, broad), 4.18 (2H, m), 4.31 (1H, m), 6.88 (1H, d), 7.00 (1H, d), 7.10 (1H, s).

### Description 83. 1-(4-Piperidinyl)-6-[(trifluoromethyl)oxy]-1,3-dihydro-2H-benzimidazol-2-one hydrochloride (D83)

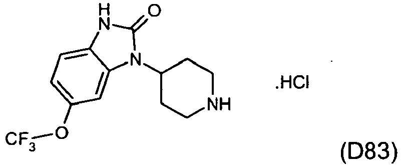

A solution of 1,1-dimethylethyl 4-{2-oxo-6-[(trifluoromethyl)oxy]-2,3-dihydro-1*H-*benzimidazol-1-yl}-1-piperidinecarboxylate (D82, 1.54 g) in methanol (20 ml) was treated with a saturated solution of hydrogen chloride in ethanol (10 ml). The mixture was stirred for 1 hour when the solvent was removed to give the title compound as a white solid (1.28 g).
¹HNMR δ (DMSO, 400 MHz): 1.81 (2H, m), 2.56 (2H, m), 3.04 (2H, m), 3.40 (2H, m), 4.56 (1H, m), 6.99 (1H, m), 7.05 (1H, d), 7.50 (1H, d), 8.97 (2H, br s), 11.2 (1H, s).

### Description 84. O-1,4-Dioxaspiro[4.5]dec-8-yl S-methyl dithiocarbonate (D84)

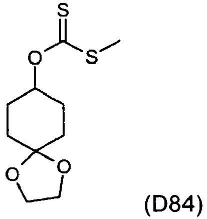

A stirred solution of 1,4-dioxaspiro[4,5]decan-8-ol (D1, 8,7g, 55mmol) in THF (110ml) at 0°C was treated portionwise with sodium hydride (2.7g of 60%, 66mmol). The mixture was stirred for 1 hr at room temperature then cooled to 0°C and treated dropwise with carbon disulphide (7ml, 110mmol). The resulting mixture was stirred at room temperature for 3 hrs before cooling to 0°C and adding dropwise iodomethane (4.12ml, 66mmol). The resulting mixture was stirred for 1 hr at room temperature, then treated with aqueous NH₄Cl solution and extracting with Et₂O. The organic phase was separated and the aqueous extracted twice with Et₂O. The combined organics was washed with sat. NaCl solution containing small portion of sodium hydrogen sulphite, dried (Na₂SO₄) and concentrated under vacuum to afford the title compound as a yellow/orange oil (quantitative).
1H NMR (CDCl3, 400MHz): 1.62-1.72 (2H, m), 1.78-1.92 (2H, m), 1.95-2.06 (4H, m), 2.55 (3H, s), 3.92-4.02 (4H, m), 5.64-5.74 (1H, m).

### Description 85. 8-[(Trifluoromethyl)oxy]-1,4-dioxaspiro[4.5]decane (D85)

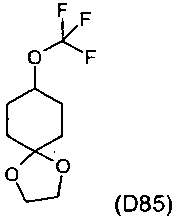

A solution of O-1,4-dioxaspiro[4.5]dec-8-yl S-methyl dithiocarbonate (D84, 500mg) in dichloromethane (5ml) was added dropwise at 0°C to a mixture of dichloromethane (5ml), 70% HF-pyridine (2ml), N-bromosuccinimide (1.8g), and pyridine (0.6ml). After a further 1h at 0°C the reaction was washed with aqueous sodium bicarbonate and aqueous sodium hydroxide at pH9.
An exactly similar reaction in which the N-bromosuccinimide was replaced by 1,3-dibromo-5,5-dimethylhydantoin was then performed and the combined products of the two reactions purified by chromatography (20g silica, 25% ethyl acetate in hexane) to give the title compound, 400mg.

### Description 86. 8-[(Cyclopropylmethyl)oxy]-1,4-dioxaspiro[4.5]decane (D86)

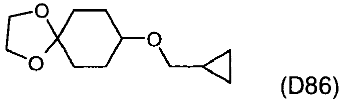

A stirred solution of 1,4-dioxaspiro[4.5]decan-8-ol (D1, 4.0g), cyclopropylmethyl bromide (4.1g) in N-methylpyrrolidinone (20ml) at ice bath temperature under argon and was treated portionwise with sodium hydride (2.0g of 60% oil dispersion). The mixture was allowed to warm to room temperature overnight. The mixture was cooled cautiously treated with methanol and then water was added and the mixture extracted with hexane. The organic extract as washed with water, then dried (Na₂SO₄) and solvent removed to give the title compound as oil (7.49g) contaminated with mineral oil residues.
¹HNMR δ (CDCl₃, 400 MHz): 0.18 (2H, m), 0.52 (2H, m), 0.85 (1H, m), 1.05 (1H, m), 1.55 (2H, m), 1.77-1.90 (4H, m), 3.25 (2H, m), 3.42 (1H, m), 3.95 (4H, m).

### Description 87. 4-[(Cyclopropylmethyl)oxy]cyclohexanone (D87)

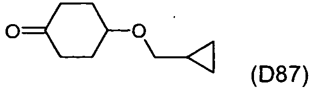

A solution of 8-[(cyclopropylmethyl)oxy]-1,4-dioxaspiro[4.5]decane (D86, 7.46g) in tetrahydrofuran (15ml) was treated with 5M HCl acid (40ml) and stirred for 3h. The resulting mixture was partially concentrated on the rotary evaporator, diluted with water and extracted with dichloromethane (2 x). The combined extract was washed with brine and dried (Na₂SO₄) and concentrated under vacuum to afford the title compound as a clear oil (6.09g).
¹H NMR δ (CDCl₃, 400 MHz): 0.22 (2H, m), 0.55 (2H, m), 0.88 (1H, m), 1.1 (1H, m) 1.90-2.00 (2H, m), 2.05-2.13 (2H, m), 2.20-2.32 (2H, m), 2.53-2.65 (2H, m), 3.54 (2H, m), 3.74 (1H, m).

### Description 88. cis/trans-1,1-Dimethylethyl (1-{4-[(cyclopropylmethyl)oxy]cyclohexyl}-4-piperidinyl)carbamate (D88)

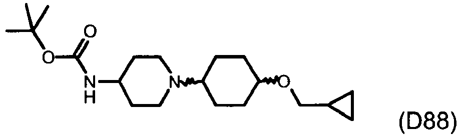

A stirred solution of 4-[(cyclopropylmethyl)oxy]cyclohexanone (D87, 6.09g) in 1,2-dichloroethane (200ml) under argon was treated with 1,1-dimethylethyl 4-piperidinylcarbamate (7.2g), sodium triacetoxyborohydride (9.8g), then the resulting mixture stirred at room temperature for 3 days. The reaction mixture was diluted with dichloromethane then washed with Na₂CO₃ solution (x2), brine (x2), then dried (Na₂SO₄) and concentrated under vacuum. The residual yellow oil was dissolved in boiling 60-80 petrol ether (∼200ml) and allowed to stand at room temperature. The crystals which had formed were filtered off, washed with petrol and dried to give 3.71g of a mixture of trans and cis isomers of the title compound. Further crops were obtained from the mother liquors. M⁺ + H = 353.

### Description 89. cis/trans-1-{4-[(Cyclopropylmethyl)oxy]cyclohexyl}-4-piperidinamine dihydrochloride

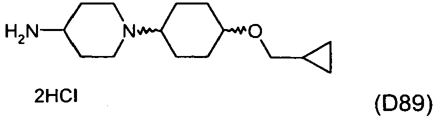

cis/trans-1,1-dimethylethyl (1-{4-[(cyclopropylmethyl)oxy]cyclohexyl}-4-piperidinyl)carbamate (D88, 3.71g) was dissolved in methanol (15ml) and treated with a solution of ethanol saturated with hydrogen chloride (20ml) for 2 hours. The solvent was evaporated to give the title compound as a white solid (3.27g). M⁺ + H = 253.

### Description 90. 1-{trans-4-[(Cyclopropylmethyl)oxy]cyclohexyl}-N-(5-methyl-2-nitrophenyl)-4-piperidinamine (D90a) and 1-{cis-4-[(cyclopropylmethyl)oxy]cyclohexyl}-N-(5-methyl-2-nitrophenyl)-4-piperidinamine (D90b)

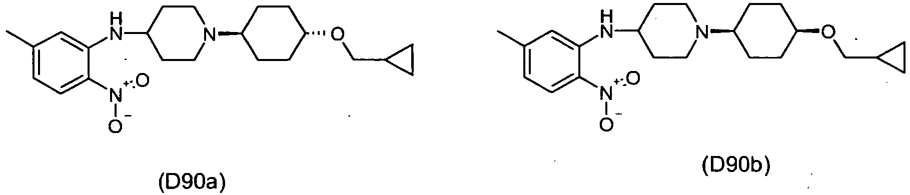

A solution of cis/trans-1-{4-[(cyclopropylmethyl)oxy]cyclohexyl}-4-piperidinamine dihydrochloride (D89, 1.20g), diisopropylethylamine (3ml) and 3-fluoro-4-nitrotoluene (1.1g) in dry dimethylformamide (10 ml), was heated at 200°C for 20 minutes in a microwave reactor. The crude mixture was then cooled to room temperature and loaded onto a SCX cartridge. The cartridge was eluted with methanol followed by 2M methanolic ammonia. The methanolic ammonia fraction was evaporated to afford the crude product which was purified by chromatography (ethyl acetate/n-hexane) on a Biotage KP-NH^{™}-silica column to yield, separately, the cis and trans isomers of the title compound.

Fast eluting isomer (D90a; trans isomer) was obtained as a orange solid (0.616g). MH⁺ = 388.
¹H NMR δ (CDCl₃, 400 MHz): 0.2 (2H, m), 0.55 (2H, m), 1.05 (1H, m), 1.4 (2H, m), 1.55-1.70 (obs, m), 1.97 (2H, m), 2.1 (2H, m), 2.45 (4H, m), 2.5 (2H, m), 2.9 (2H, m), 3.22 (2H, m), 3.52 (2H, m), 6.42 (1H, d), 6.62 (1H, s) 8.06 (1H, d), 8.2 (1H, d).

Slow eluting isomer (D90b; cis isomer) was obtained as a solid (0.417g). MH⁺ = 388.
¹H NMR δ (CDCl₃, 400 MHz): 0.2 (2H, m), 0.5 (2H, m), 1.05 (1H, m), 1.3 (4H, m), 1.55-1.70 (obs, m), 1.9 (2H, m), 2.1 (4H, m), 2.35 (3H, m), 2.45 (2H, m), 3.2 (1H, m), 3.3 (2H, m), 3.56 (1H, m), 6.43 (1H, d), 6.61 (1H, s) 8.08 (1H, d), 8.2 (1H, d).

### Description 91. N²-(1-{cis-4-[(Cyclopropylmethyl)oxy]cyclohexyl}-4-piperidinyl)-4-methyl-1,2-benzenediamine (D91)

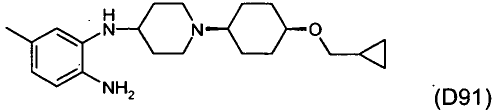

1-{*cis*-4-[(Cyclopropylmethyl)oxy]cyclohexyl}-*N*-(5-methyl-2-nitrophenyl)-4-piperidinamine (D90b, 0.417g) in ethanol (30 ml), and an aqueous suspension of Raney Nickel (1 ml of a 50% suspension) was stirred at 40°C during the addition of hydrazine hydrate (1 ml). The mixture was stirred at 40°C for 30 minutes, cooled and filtered. The filtrate was evaporated and azeotroped with dichloromethane to give the title compound as a white solid (380mg). M⁺ + H = 394

### Description 92. N²-(1-{trans-4-[(Cyclopropylmethyl)oxy]cyclohexyl}-4-piperidinyl)-4-methyl-1,2-benzenediamine (D92)

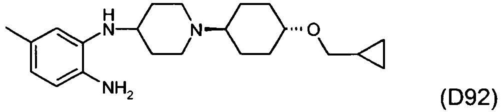

1-{*trans*-4-[(Cyclopropylmethyl)oxy]cyclohexyl}-*N*-(5-methyl-2-nitrophenyl)4-piperidinamine (D90a, 0.613g) in ethanol (30 ml), methanol (40ml) and an aqueous suspension of Raney Nickel (1 ml of a 50% suspension) was stirred at 40°C during the addition of hydrazine hydrate (1ml). The mixture was stirred at 40°C for 30 minutes, cooled and filtered. The filtrate was evaporated to give the title compound as a white solid (579mg). M⁺ + H = 394.

### Description 93. trans N-(4-Cyclobutyloxycyclhexyl)phthalimide (D93)

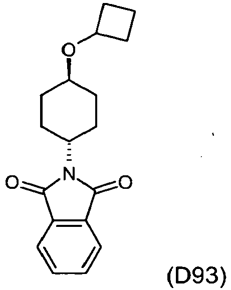

A solution of trans N-(4-tert-butyldimethylsilyloxycyclhexyl)phthalimide (D41, 1.8g, 5mmol) in acetonitrile (20ml) at room temperature was treated sequentially with triethylsilane (1.0ml, 6mmol), bismuth tribromide (240mg, 0.5mmol), and cyclobutanone (0.45ml, 6mmol). After 30min more the solution was partitioned aqueous sodium bicarbonate/ethyl acetate. Drying and evaporation of the organic layer gave the title compound crystallised from hexane, 820mg.

### Description 94. trans 4-Cyclobutyloxycyclohexylamine (D94)

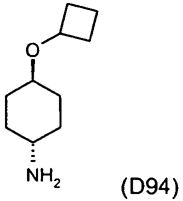

A mixture of trans N-(4-cyclobutyloxycyclhexyl)phthalimide (D93, 820mg, 2.7mmol), hydrazine hydrate (0.5ml, 10.0mmol), and ethanol (5ml) was heated at at 80°C for 2h then cooled and filtered. The filtrate was evaporated and the resulting residue redissolved in diethyl ether, filtered and again evaporated to give the title compound, 180mg.

### Description 95. trans 1-[4-(Cyclobutyloxy)-cyclohexyl]-4-piperidone (D95)

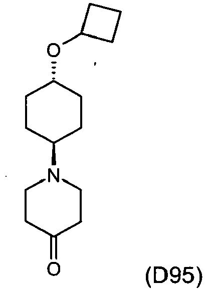

A mixture of trans 4-cyclobutyloxycyclohexylamine (D94, 180mg, 1.1mmol), 1-ethyl-4-piperidone methiodide (D44, 540mg, 2.0mmol), potassium carbonate (15mg, 0.1mmol), water (5ml), and ethanol (10ml) was heated for 1h at 80°C, then cooled, partitioned aqueous sodium bicarbonate / dichloromethane, and purified by chromatography (20g silica, 0 to 10% methanol in dichloromethane containing 0.2M ammonia) to give the title compound, 150mg.

### Description 96. trans 1-[4-(Cyclobutyloxy)-cyclohexyl]-4-piperidinamine (D96)

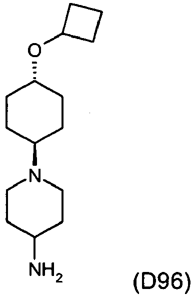

A mixture of trans 1-[4-(cyclobutyloxy)-cyclohexyl]-4-piperidone (D95, 150mg, 0.6mmol), 2M ammonia in methanol (10ml, 20mmol), and 10% palladium on carbon paste (20mg) was hydrogenated at 50psi at room temperature overnight then filtered and evaporated to give the title compound, 130mg.

### Description 97. trans N-(5-Methyl-2-nitrophenyl)-1-(4-cyclobutyloxycyclohexyl)-4-piperidinamine (D97)

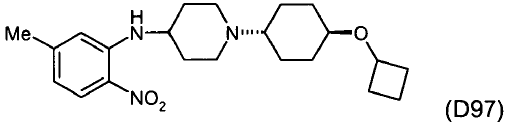

A mixture of 3-fluoro-4-nitrotoluene (80mg), dimethylformamide (2.5ml), diisopropylethylamine (0.17ml), and trans 1-[4-(cyclobutyloxy)-cyclohexyl]-4-piperidinamine (D96, 130mg, 0.5mmol) was heated at 80°C overnight. The cooled reaction was diluted with ethyl acetate and washed three times with water then dried, evaporated and chromatographed (5g silica, 0 to 5% methanol in dichloromethane containing 0.2M ammonia) to give the title compound (60mg).

### Description 98. trans 5-Methyl-N-[1-(4-cyclobutyloxycyclohexyl)-4-piperidinyl]-1,2-benzenediamine (D98)

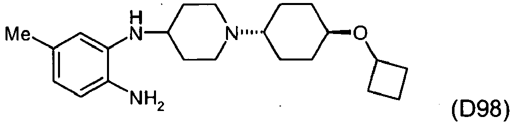

A stirred suspension of trans *N*-(5-methyl-2-nitrophenyl)-1-(4-cycllbutyloxycyclohexyl)-4-piperidinamine (D97, 60mg, 0.16mmol) in ethanol (5ml) at 50°C was treated with Raney nickel (1.0ml of 10% aqueous suspension) followed by dropwise addition of hydrazine hydrate (0.08ml). The mixture was heated at the same temperature for 1h, then filtered through Kieselguhr and the filtrate evaporated and re-evaporated from toluene then diethyl ether to afford the title compound, 50mg.

### Description 99. N-(5-Bromo-2-nitrophenyl)-1-[trans-4-(propyloxy)cyclohexyl]-4-piperidinamine (D99)

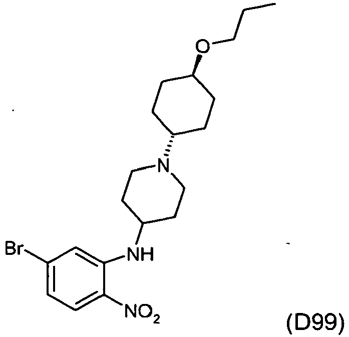

A mixture of 4-bromo-2-fluoro-1-nitrobenzene (300mg, 1.36mmol) and 1-[*trans*-4-(propyloxy)cyclohexyl]-4-piperidinamine dihydrochloride (D49, 422mg, 1.36mmol) in dimethylformamide (20ml) at room temperature was treated with diisopropylethylamine (0.8ml,3equivalents) then heated at 90°C for 5hrs under argon. The mixture was washed with water and sat. NaHCO₃ solution then extracted with dichloromethane. The extract was dried (Na₂SO₄) and concentrated under vacuum. The residue was loaded on an SCX cartridge and eluted first with dichloromethane and then with 2M NH₃/MeOH to remove the product, which was concentrated under vacuum to afford the title compound (610mg, 99%). MH+ = 441, 442.
¹H NMR δ (CDCl₃, 400MHz): 0.91 (3H, t), 1.18-1.40 (4H, m), 1.51-1.72 (assume 4H, m), 1.85-1.97 (2H, m), 2.02-2.18 (4H, m), 2.29-2.39 (1H, m), 2.39-2.50 (2H, m), 2.84-2.94 (2H, m), 3.10-3.20 (1H, m), 3.40 (2H, t), 3.41-3.55 (1H, m), 6.73 (1H, dd), 7.00 (1H, d), 8.03 (1H, d), 8.13 (1H, d).

### Description 100. 4-Bromo-N²-{1-[trans-4-(propyloxy)cyclohexyl]-4-piperidinyl}-1,2-benzenediamine (D100)

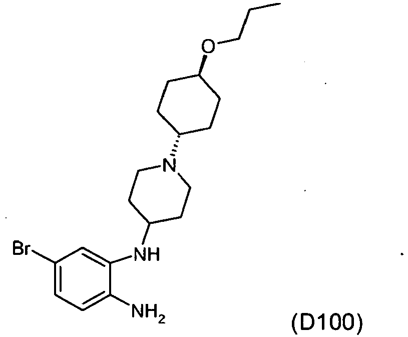

A stirred solution of *N*-(5-bromo-2-nitrophenyl)-1-[*trans*-4-(propyloxy)cyclohexyl]-4-piperidinamine (D99, 620mg, 1.41mmol) in ethanol (100ml) at room temperature under argon was treated with Raney Nickel (80mg) and dropwise addition of hydrazine hydrate (1.1ml), 35.25mmol) and maintained for 3hrs. The mixture was filtered through Kieselguhr and the filtrate concentrated under vacuum to leave the title compound as a brown oil (574mg, 99%). MH+ = 411, 412.
¹H NMR δ (CDCl₃, 400MHz): 0.90 (3H, t), 1.20-1.40 (4H, m), 1.40-1.65 (4H, m), 1.85-1.98 (2H, m), 2.01-2.15 (4H, m), 2.26-2.40 (3H, m), 2.85-2.95 (2H, m), 3.05-3.45 (5H, m), 3.40 (2H, t), 6.57 (1H, m), 6.69-6.76 (2H, m).

### Description 101. N-(5-Ethenyl-2-nitrophenyl)-1-[trans-4-(propyloxy)cyclohexyl]-4-piperidinamine (D101)

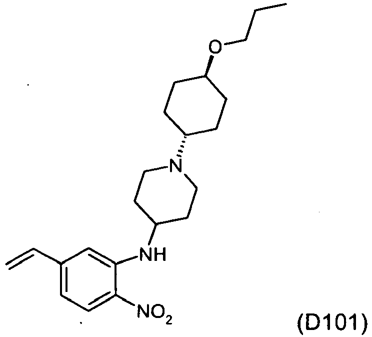

A solution of *N*-(5-bromo-2-nitrophenyl)-1-[*trans*-4-(propyloxy)cyclohexyl]-4-piperidinamine (D99, 340mg, 0.77mmol) and tributylvinyltin (0.28ml, 0.89mmol) in toluene (10ml) at room temperature under argon was treated with triphenylphosphine (51mg, 0.19mmol) and bis(dibenzylidene)acetone)palladium(0) (68mg, 0.12mmol) and heated at 120°C overnight. The mixture was allowed to cool, then filtered through Kieselguhr and concentrated under vacuum. The residue was dissolved in dichloromethane and washed with 10% aqu. ammounium hydroxide solution, then dried (Na₂SO₄) and concentrated under vacuum. The residue was dissolved in dichloromethane and eluted through an SCX cartridge, then collected by elution with 2M NH₃/MeOH and concentrated. The residue was chromatographed on a 25+M Biotage KP-NH^{™}-silica column using 5-30% solvent to afford the title compound as a yellow solid (240mg, 80%). MH+ = 388.
¹H NMR δ (CDCl₃, 400MHz): 0.91 (3H, t), 1.20-1.38 (4H, m), 1.52-1.72 (assume 4H, m), 1.88-1.97 (2H, m), 2.03-2.16 (4H, m), 2.29-2.39 (1H, m), 2.39-2.50 (2H, m), 2.82-2.92 (2H, m), 3.10-3.21 (1H, m), 3.39 (2H, t), 3.52-3.63 (1H, t), 5.46 (1H, d), 5.87 (1H, d), 6.66 (1H, dd), 6.71-6.76 (2H, m), 8.15 (1H, d), 8.22 (1H, d).

### Description 102. 4-Ethyl-N²-{1-[trans-4-(propyloxy)cyclohexyl]-4-piperidinyl}-1,2-benzenediamine (D102)

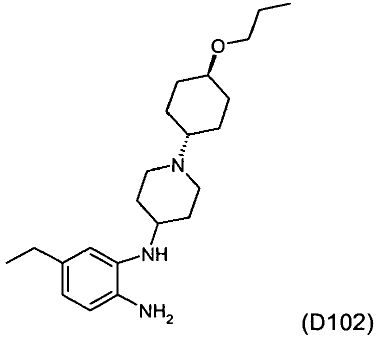

A mixture of *N*-(5-ethenyl-2-nitrophenyl)-1-[*trans*-4-(propyloxy)cyclohexyl]-4-piperidinamine (D101, 240mg, 0.61mmol) and ammonium formate (382.7mg, 6.1mmol) in methanol (25ml) was stirred at room temperature under argon for 10mins, then treated with 10% Pd/C (136mg, 0.2equivalents) and stirred for 30mins. The mixture was filtered through Kieselguhr and concentrated to remove solvent. The residue was dissolved in dichloromethane, washed with 2M NaOH solution, dried (Na₂SO₄) and concentrated under vacuum to leave the title compound as a purple sticky oil (180mg, 82%). MH+ = 360.
¹H NMR δ (CDCl₃, 400MHz): 0.91 (3H, t), 1.16-1.38 (7H, m), 1.42-1.62 (4H, m), 1.88-1.97 (2H, m), 2.01-2.12 (4H, m), 2.25-2.42 (3H, m), 2.53 (2H, q), 2.84-2.94 (2H, m), 2.95-3.40 (5H, m), 3.40 (2H, t), 6.47-6.61 (2H, m), 6.65 (1H, d).

### Description 103. N-(5-Cyclopropyl-2-nitrophenyl)-1-[trans-4-(propyloxy)cyclohexyl]-4-piperidinamine (D103)

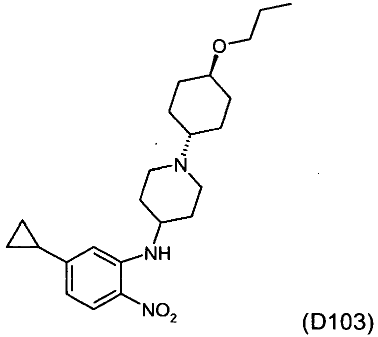

A mixture of 4-cyclopropyl -2-fluoro-1-nitrobenzene (100mg, 0.55mmol) and 1-[*trans*-4-(propyloxy)cyclohexyl]-4-piperidinamine dihydrochloride (D49, 170mg, 1 equivalent) in dimethylformamide (10ml) was treated with diisopropylethylamine (0.28ml) then heated and stirred under argon at 80°C overnight. The solution was washed with water then extracted with dichloromethane. The extract was dried (Na₂SO₄) and concentrated under vacuum. The residue was dissolved in dichloromethane and eluted through an SCX cartridge, firstly with dichloromethane and then with 2M NH₃/MeOH. The methanolic solution was concentrated under vacuum to leave a dark orange solid (140mg), which was purified by chromatography on a 40+M Biotage KP-NH^{™}-silica column eluting with 5-30% EtOAc/hexane to afford the title compound as an orange solid (110mg, 50%). MH+ = 402.
¹H NMR δ (CDCl₃, 400MHz): 0.76-0.82 (2H, m), 0.90 (3H, t), 1.03-1.11 (2H, m), 1.19-1.40 (assume 4H, m), 1.52-1.72 (assume 4H, m), 1.82-1.98 (assume 3H, m), 2.02-2.15 (4H, m), 2.18-2.39 (1H, m), 2.40-2.50 (2H, m), 2.80-2.92 (2H, m), 3.10-3.20 (1H, m), 3.40 (2H, t), 3.50-3.60 (1H, m), 6.19 (1H, dd), 6.55 (1H, d), 8.05 (1H, d), 8.21 (1H, d).

### Description 104. 4-Cyclopropyl-N²-{1-[trans-4-(propyloxy)cyclohexyl]-4-piperidinyl}-1,2-benzenediamine (D104)

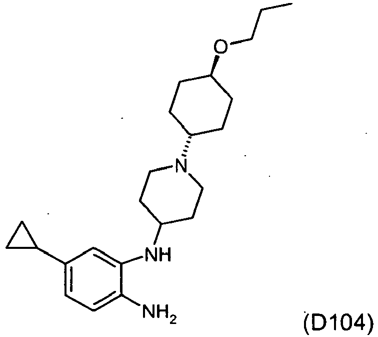

A stirred solution of *N*-(5-cyclopropyl-2-nitrophenyl)-1-[*trans*-4-(propyloxy)cyclohexyl]-4-piperidinamine (D103, 110mg, 0.27mmol) in ethanol (20ml) at room temperature under argon was treated with Raney Nickel (30mg) and dropwise addition of hydrazine hydrate (0.2ml, 6.75mmol) and maintained for 2hrs. The mixture was filtered through Kieselguhr and the filtrate concentrated under vacuum to leave the title compound as a green/yellow oil (70mg, 70%). MH+ = 372.

### Description 105. 1-[cis-4-(Ethyloxy)-1-methylcyclohexyl]-N-(5-methyl-2-nitrophenyl)-4-piperidinamine (D105)

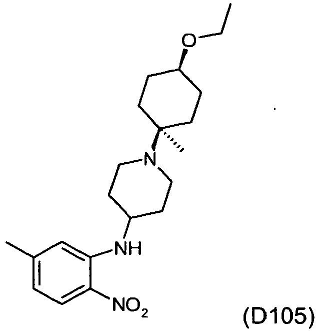

A stirred solution of 1-[*cis*-4-(ethyloxy)-1-methylcyclohexyl]-4-piperidinamine (D62, 140mg, 0.58mmol) in dimethylformamide (3ml) at room temperature under argon was treated with diisopropylethylamine (0.124ml, 0.70mmol) and 3-fluoro-4-nitrotoluene (124mg, 0.80mmol) and heated at 80°C for 18hrs. The mixture was concentrated under vacuum and the residue treated with 10% Na₂CO₃ solution and extracted with dichloromethane. The extract was dried (Na₂SO₄), concentrated under vacuum and the residue chromatographed on silica gel (10g) eluting with 0-10% methanol/dichloromethane to afford the title compound as an yellow oil (170mg, 78%). MH⁺ 376.
¹H NMR δ (CDCl₃, 400 MHz): 0.85 (3H, s), 1.05-1.20 (2H, m), 1.21 (3H, t), 1.60-1.72 (6H, m), 1.90-2.00 (2H, m), 2.00-2.10 (2H, m), 2.28-2.40 (2H, m), 2.33 (3H, s), 2.80-72.90 (2H, m), 3.22-3.32 (1H, m), 3.47-3.57 (3H, t + m), 6.41 (1H, dd), 6.62 (1H, s), 8.06 (1H, d), 8.19 (1H, d).

### Description 106. N²-{1-[cis-4-(Ethyloxy)-1-methylcyclohexyl]-4-piperidinyl}-4 methyl-1,2-benzenediamine (D106)

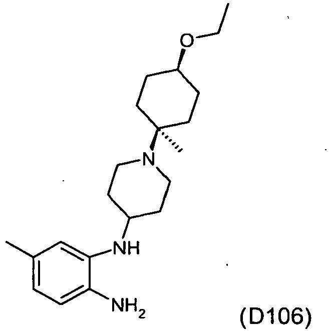

A stirred solution of 1-[*cis*-4-(ethyloxy)-1-methylcyclohexyl]-*N*-(5-methyl-2-nitrophenyl)-4-piperidinamine (D105, 170mg, 0.45mmol) in ethanol (12ml) at room temperature under argon was treated with Raney Nickel (~20mg) followed by hydrazine hydrate (0.15ml, 4.8mmol) and maintained for 2 hrs. The mixture was filtered through Kieselguhr to remove catalyst and the filtrate concentrated under vacuum to afford the title compound as a pale grey oil (150mg, 96%yield). MH⁺ 346.
¹H NMR δ (CDCl₃, 400MHz): 0.85 (3H, s), 1.05-1.15 (2H, m), 1.20 (3H, t), 1.40-1.52 (2H, m), 1.60-1.75 (4H, m), 1.90-2.00 (2H, m), 2.00-2.10 (2H, m), 2.18-2.30 (2H, m), 2.25 (3H, s), 2.85-2.92 (2H, m), 3.00-3.35 (5H, m), 3.50 (2H, q), 6.44-6.50 (2H, m), 6.62 (1H, d).

### Description 107. 1-[trans-4-(Ethyloxy)-1-methylcyclohexyl]-N-(5-methyl-2-nitrophenyl)-4-piperidinamine (D107)

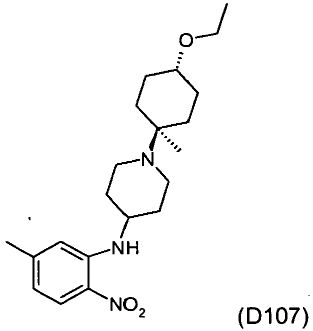

A stirred solution of 1-[*trans*-4-(ethyloxy)-1-methylcyclohexyl]-4-piperidinamine (D58 free base, 200mg, 0.83mmol) in dimethylformamide (5ml) at room temperature under argon was treated with diisopropylethylamine (0.28ml, 1.6mmol) and 3-fluoro-4-nitrotoluene (170mg, 1.1 mmol) and heated at 80°C for 18hrs. The mixture was concentrated under vacuum and the residue treated with 10% Na₂CO₃ solution and extracted with dichloromethane. The extract was dried (Na₂SO₄), concentrated under vacuum and the residue chromatographed on silica gel (10g) eluting with 0-10% methanol/dichloromethane to afford the title compound as an yellow oil (180mg, 58%). MH⁺ 376.
¹H NMR δ (CDCl₃, 400 MHz): 0.93 (3H, s), 1.20 (3H, t), 1.40-1.70 (8H, m), 1.80-1.90 (2H, m), 2.02-2.12 (2H, m), 2.28-2.38 (2H, m), 2.33 (3H, s), 2.90-2,98 (2H, m), 3.34-3.42 (1H, m), 3.45-3.58 (3H, q + m), 6.42 (1H, dd), 6.62 (1H, s), 8.05 (1H, d), 8.17 (1H, d).

### Description 108. N²-{1-[trans-4-(Ethyloxy)-1-methylcyclohexyl]-4-piperidinyl}-4-methyl-1,2-benzenediamine (D108)

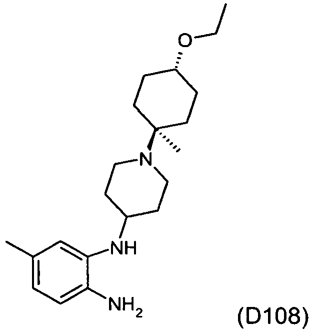

A stirred solution of 1-[*trans*-4-(ethyloxy)-1-methylcyclohexyl]-*N*-(5-methyl-2-nitrophenyl)-4-piperidinamine (D107, 180mg, 0.48mmol) in ethanol (12ml) at room temperature under argon was treated with Raney Nickel (∼20mg) followed by hydrazine hydrate (0.15ml, 4.8mmol) and maintained for 2 hrs. The mixture was filtered through Kieselguhr to remove catalyst and the filtrate concentrated under vacuum to afford the title compound as a very pale yellow oil (160mg, 97%yield). MH⁺ 346.
¹H NMR δ (CDCl₃, 400MHz): 0.92 (3H, s), 1.20 (3H, t), 1.38-1.57 (6H, m), 1.62-1.72 (2H, m), 1.80-1.90 (2H, m), 2.07 (2H, br d), 2.22-2.32 (2H, m), 2.24 (3H, s), 2.96 (2H, br d), 3.00-3.30 (4H, m), 3.33-3.38 (1H, m), 3.48 (2H, q), 6.42-6.50 (2H, m), 6.62 (1H, d).

### Description 109. cis/trans 1,1-Dimethylethyl {1-[1-methyl-4-(propyloxy)cyclohexyl]-4-piperidinyl}carbamate (D109)

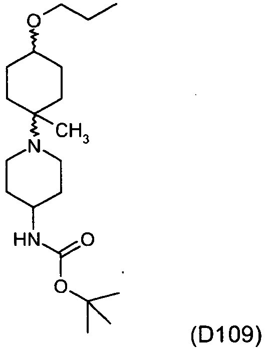

A three-necked flask was equipped with a Dean-Stark trap and an argon-connected condenser. The flask was charged with 1,1-dimethylethyl 4-piperidinylcarbamate (1.1 eq., 7 mmol, 1.4 g), 4-(propyloxy)cyclohexanone (D19, 1 eq., 6.4 mmol, 1 g), 1,2,3-triazole (1.2 eq., 7.7 mmol, 0.53 g) and 50 ml of dry toluene. The mixture was refluxed at 130°C for 6 hours under argon then cooled to room temperature and added to a solution of methylmagnesium bromide (3M in Et₂O) (4 eq., 25.6 mmol, 8.5 ml) at < 25°C over 20 minutes. Precipitation of a solid was observed: 50 ml extra of tetrahydrofuran were added and the temperature was rigorously kept at 10°C by an ice bath. The mixture was stirred at room temperature overnight. The mixture was then cooled to 0°C and it was slowly quenched with an aqueous saturated solution of ammonium chloride. The mixture was subsequently brought to room temperature, diluted with EtOAc and the two phases were separated. The aqueous phase was the extracted with EtOAc (2x), the organics were combined, dried over sodium sulphate, filtered and the solvent was evaporated to afford the crude product that was subsequently purified by silica gel chromatography (MeOH-NH₃-DCM) to afford of the title compound as a yellow oil (0.66 g, 30%). M⁺ + H = 355.

### Description 110. cis/trans 1-[1-Methyl-4-(propyloxy)cyclohexyl]-4-piperidinamine dihydrochloride (D110)

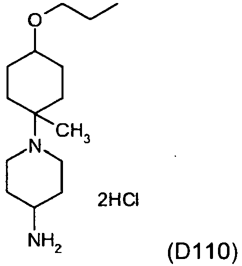

*cis*/*trans* 1,1-dimethylethyl {1-[1-methyl-4-(propyloxy)cyclohexyl]-4-piperidinyl}carbamate (D109, 0.66g, 1.8 mmol) was dissolved in Et₂O (10 ml) and HCl (5 ml of a 4M solution in 1,4-dioxane) was added at room temperature. The mixture was stirred at room temperature for 6 hours. The solvent was subsequently evaporated to afford the crude product (440 mg) which was directly used for the next step. M⁺ + H = 255.

### Description 111. cis and trans N-(5-Methyl-2-nitrophenyl)-1-[1-methyl-4-(propyloxy)cyclohexyl]-4-piperidinamine (cis = D111a; trans = D111b)

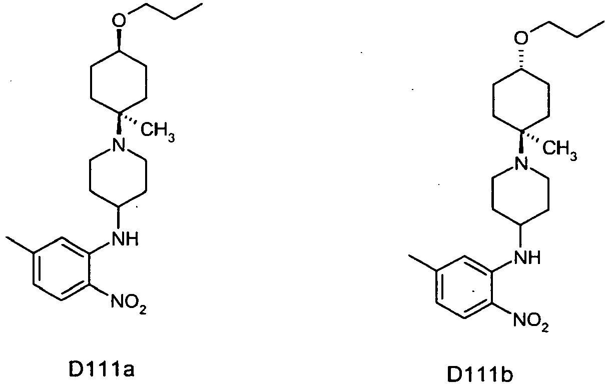

*cis*/*trans* 1-[1-methyl-4-(propyloxy)cyclohexyl]-4-piperidinamine dihydrochloride (D110, 1 eq., 440 mg, 1.3 mmol) was dissolved in dimethylformamide (4 ml) and Hunigh base (3 eq., 0.68 ml) followed by 2-fluoro-4-methyl-1-nitrobenzene (1 eq., 200 mg) were added at room temperature and the mixture was stirred at 100°C overnight. The crude mixture was then cooled to room temperature and poured onto water/brine. The aqueous solution was extracted with EtOAc (3x) and organics were alternatively washed with water (1x) and brine (1x). The organics were combined, dried over Na₂SO₄, filtered and the solvent was evaporated to afford the crude product that was purified by chromatography and SCX cartridge to yield the two separate title compound isomers, (*cis*, 57 mg) and (*trans,* 49 mg). M⁺ + H = 390.
cis isomer (D111a): ¹HNMR δ (DMSO, 500 MHz): 0.868 (6H, m), 1.392 (2H, m broad), 1.478 (8H, m), 1.739 (2H, m), 1.992 (2H, d), 2.294 (5H, m), 2.821 (2H, d), 3.300 (2H under water peak), 3.359 (1H, s broad), 3.646 (1H, s broad), 6.504 (1H, d), 6.932 (1H, s), 7.954 (1H, d), 8.023 (1H, d).
trans isomer (D111b): ¹HNMR δ (DMSO, 500 MHz): 0.849 (6H, m), 1.107 (2H, t), 1.499 (8H, m), 1.869 (2H, d), 1.993 (2H, d), 2.886 (5H, m), 2.790 (2H, d), 3.197 (1H, m), 3.315 (2H under the water peak), 3.652 (1H, m broad), 6.505 (1H, d), 6.936 (1H, s), 7.957 (1H, d), 8.040 (1H, d).

### Description 112. {2-Amino-5-methylphenyl){1-[cis-1-methyl-4-(propyloxy)cyclohexyl]-4-piperidinyl}amine (D112)

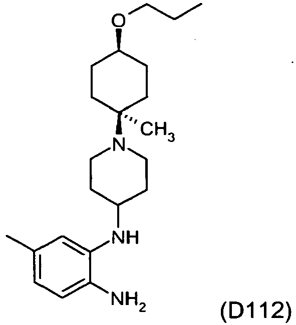

*N*-(5-Methyl-2-nitrophenyl)-1-[*cis*-1-methyl-4-(propyloxy)cyclohexyl]-4-piperidinamine (D111a, 57 mg, 0.147mmol) was dissolved into EtOH (5 ml) and Raney-Ni (0.2 ml of a 50% aqueous suspension) was added at room temperature. The mixture was heated to 60°C and hydrazine hydrate (10 eq., 1.47 mmol, 0.046 ml) was added dropwise at 60°C. The reaction was stirred at 60°C for 4 hours. The mixture was cooled to room temperature and it was filtered through celite. The solvent was subsequently evaporated to afford the title compound (53 mg, complete conversion). M⁺ + H = 360.
¹HNMR δ (DMSO, 400 MHz): 0.861 (6H, m), 1.413 (10H, m), 1.723 (2H, dd), 1.939 (2H, d), 2.100 (3H, s), 2.151 (2H, t), 2.881 (2H, d), 3.177 (1H, s broad), 3.306 (nH under the water peak), 4.043 (1H, d), 4.253 (1H, s broad), 6.178 (1H, d), 6.263, (1H, s), 6.413 (1H, d).

### Description 113. (2-Amino-5-methylphenyl){1-[trans-1-methyl-4-(propyloxy)cyclohexyl]-4-piperidinyl}amine (D113)

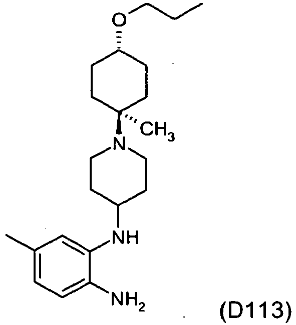

*N*-(5-Methyl-2-nitrophenyl)-1-[*trans*-1-methyl-4-(propyloxy)cyclohexyl]-4-piperidinamine (D111b, 49mg, 0.126 mmol) was dissolved into EtOH (5 ml) and Raney-Ni (0.2 ml of a 50% aqueous suspension) was added at room temperature. The mixture was heated to 60°C and hydrazine hydrate (10 eq., 1.26mmol, 0.039ml) was added dropwise at 60°C. The reaction was stirred at 60°C for 4 hours. The mixture was cooled to room temperature and it was filtered through celite. The solvent was subsequently evaporated to afford the title compound (45 mg, complete conversion). M⁺ + H = 360.
¹HNMR δ (DMSO, 400 MHz): 0.841 (6H, m), 1.086 (2H, t), 1.335 (2H, q), 1.481 (6H, m), 1.871 (2H, d), 1.942 (2H, d), 2.121 (5H, m), 2.855 (2H, d), 3.186 (2H, m broad), 3.326 (2H under water peak), 4.074 (1H, d), 4.258 (1H, s broad), 6.177 (1H, d), 6.268 (1H, s), 6.412 (1H, d).

### Description 114. cis-Ethyl 1-ethyl-4-(tert-butyldimethylsilyloxy)-cyclohexanecarboxylate (D114)

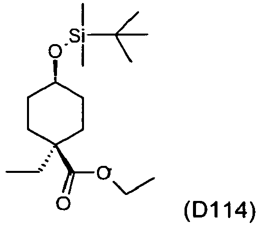

To a solution of diisopropylamine (5.7ml, 40mmol) in THF (150ml) at 0°C was added n-butyllithium (16ml of 2.5M in hexane, 40mmol). After 10min more at 0°C, it was cooled to -70°C and ethyl 4-(tert-butyldimethylsilyloxy)cyclohexanecarboxylate (D50, 8.6g) in tetrahydrofuran (30ml) was added. After 1 h at -70°C iodoethane (7.2ml) was added and the solution was allowed to warm to room temperature. After 2h at room temperature it was partitioned between aqueous ammonium chloride / ethyl acetate and the organic layer dried, evaporated, and purified by chromatography (50g silica, 0 to 10% ethyl acetate in hexane) to give the title compound, 6.2g.

### Description 115. cis-Ethyl 1-ethyl-4-hydroxycyclohexanecarboxylate (D115)

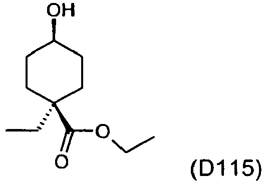

A mixture of *cis*-ethyl 1-ethyl-4-(tert-butyldimethylsilyloxy)-cyclohexanecarboxylate (D114, 6.2g, 20mmol) and tetrabutylammonium fluoride (25ml of 1 M in tetrahydrofuran, 25mmol) was stirred for 18h at room temperature, then partitioned between aqueous citric acid /diethyl ether and the organic layer dried, evaporated, and purified by chromatography (20g silica, 0 to 50% ethyl acetate in hexane) to give the title compound, 4.0g.

### Description 116. trans-Ethyl 1-ethyl-4-(chloroacetoxy)cyclohexanecarboxylate (D116)

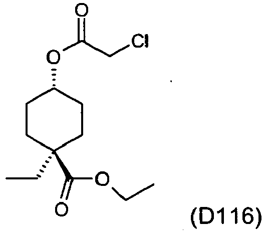

To a mixture of cis-ethyl 1-ethyl-4-hydroxycyclohexanecarboxylate (D115, 4.0g, 20mmol), triphenylphosphine (6.5g, 25mmol), chloroacetic acid (2.4g, 25mmol), and THF (100ml) at 0°C was added diisopropyl azodicarboxylate (5.0ml, 25mmol) dropwise. After a further 76h at room temperature the solution was evaporated and purified by chromatography (50g silica, 10 to 50% ethyl acetate in hexane) to give the title compound, 3.0g.

### Description 117. trans-Ethyl 1-ethyl-4-hydroxycyclohexanecarboxylate (D117)

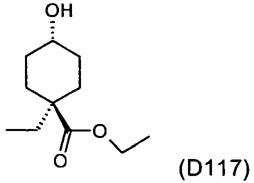

A mixture of *trans*-ethyl 1-ethyl-4-(chloroacetoxy)-cyclohexanecarboxylate (D116, 3.0g, 12mmol) and potassium carbonate (1.7g, 12mmol) in methanol (50ml) was stirred 1h at room temperature then partitioned aqueous ammonium chloride / ethyl acetate and the organic layer dried, evaporated, and purified by chromatography (20g silica, 0 to 50% ethyl acetate in hexane) to give the title compound, 1.5g.

### Description 118. trans-Ethyl 1-ethyl-4-(tert-butyldimethylsilyloxy)-cyclohexanecarboxylate (D118)

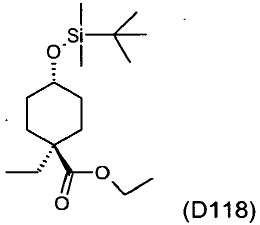

A mixture of *trans*-ethyl 1-ethyl-4-hydroxycyclohexanecarboxylate (D117, 1.5g, 7.5mmol), tert-butyldimethylsilyl chloride (1.2g, 8.0mmol), imidazole (1.1g, 16.0mmol), and dimethylformamide (10ml) was stirred overnight at room temperature then partitioned water / ethyl acetate and the organic layer dried, evaporated, and purified by chromatography (20g silica, 0 to 10% ethyl acetate in hexane) to give the title compound, 1.8g.

### Description 119. trans-Ethyl 1-ethyl-4-propoxycyclohexanecarboxylate (D119)

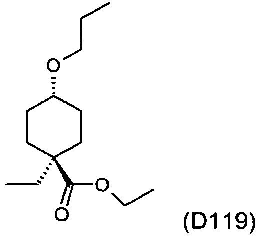

To a mixture of *trans*-ethyl 1-ethyl-4-(tert-butyldimethylsilyloxy)-cyclohexanecarboxylate (D118, 1.8g, 6.0mmol), triethylsilane (1.3ml, 8.0mmol), bismuth tribromide (0.3g, 0.6mmol), and acetonitrile (25ml) at 0°C was added propanal (0.6ml, 8.0mmol). The mixture was stirred 30min at room temperature then partitioned aqueous sodium bicarbonate / ethyl acetate and the organic layer dried and evaporated, giving the title compound as a 1:1 mixture with tert-butyldimethylsilyl triethyl siloxane, 2.7g.

### Description 120. trans-1-Ethyl-4-propoxycyclohexanecarboxylic acid (D120)

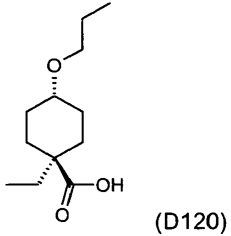

A mixture of *trans*-ethyl 1-ethyl-4-propoxycyclohexanecarboxylate (D119, 2.7g, 5.5mmol), methanol (50ml), THF (50ml) and 12.5M aqueous sodium hydroxide (4.0ml, 50mmol) was stirred overnight at 50°C then concentrated under vacuo and partitioned water/ethyl acetate. The aqueous layer was acidified and extracted with diethyl ether which was dried and evaporated, giving the title compound, 450mg.

### Description 121. trans-1-Ethyl-4-propoxycyclohexyl isocyanate (D121)

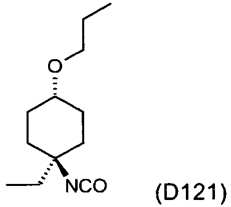

A mixture of *trans*-1-ethyl-4-propoxycyclohexanecarboxylic acid (D120, 450mg, 2.1mmol), diphenylphosphoryl azide (0.4ml, 1.8mmol), triethylamine (0.27ml, 0.2mmol), and toluene (10ml) was heated for 30min at 100°C, then cooled, washed with water, and purified by chromatography (10g silica, 0 to 20% ethyl acetate in hexane) to give the title compound, 240mg.

### Description 122. trans-1-Ethyl-4-propoxycyclohexylamine (D122)

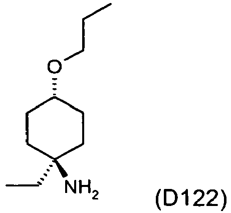

A mixture of *trans*-1-ethyl-4-propoxycyclohexyl isocyanate (D121, 240mg, 1.1mmol), THF (4ml), and 5M aqueous hydrochloric acid (1ml, 5.0mmol) was stirred at room temperature for 2h then purified by ion exchange chromatography using SCX resin to give the title compound, 90mg.

### Description 123. trans-1-[4-(Propoxy)-1-ethylcyclohexyl]-4-piperidone (D123)

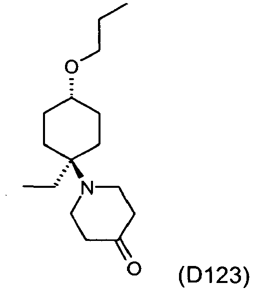

A mixture of *trans*-1-ethyl-4-propoxycyclohexylamine (D122, 90mg, 0.5mmol), 1-ethyl-1-methyl-4-oxopiperidinium iodide (D44, 210mg, 0.8mmol), potassium carbonate (10mg, 0.05mmol), water (2ml), and ethanol (4ml) was heated for 1h at 80°C, then cooled, partitioned water / dichloromethane, and purified by chromatography (10g silica, 0 to 10% methanol in dichloromethane containing 0.2M ammonia) to give the title compound, 30mg.

### Description 124. trans-1-[4-(Propoxy)-1-ethylcyclohexyl]-4-piperidinamine (D124)

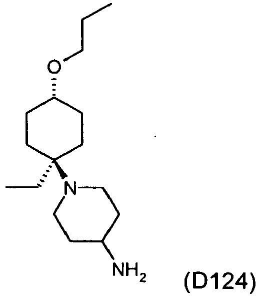

A mixture of *trans*-1-[4-(propoxy)-1-ethylcyclohexyl]-4-piperidone (D123, 30mg, 0.11mmol), 2M ammonia in methanol (20ml, 40mmol), and 10% palladium on carbon paste (30mg) was hydrogenated at 50psi at room temperature overnight then filtered and evaporated to give the title compound, 30mg.

### Description 125. trans-N-(5-Methyl-2-nitrophenyl)-1-(1-ethyl-4-propoxycyclohexyl)-4-piperidinamine (D125)

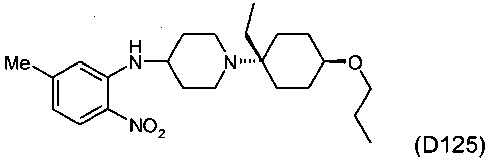

A stirred solution of 3-fluoro-4-nitrotoluene (30mg, 0.20mmol) in dimethylformamide (1ml) at room temperature under argon was treated with diisopropylethylamine (0.1ml, 0.60mmol) and *trans*-1-[4-(propoxy)-1-ethylcyclohexyl]-4-piperidinamine (D124, 30mg, 0.11mmol) and heated at 80°C for 18h. The cooled reaction was diluted with ethyl acetate and washed three times with water then dried, evaporated and chromatographed (5g silica, 0-5% methanol in dichloromethane containing 0.2M ammonia) to give the title compound (40mg).

### Description 126. trans-5-Methyl-N-[1-(1-ethyl-4-propoxycyclohexyl)-4-piperidinyl]-1,2-benzenediamine (D126)

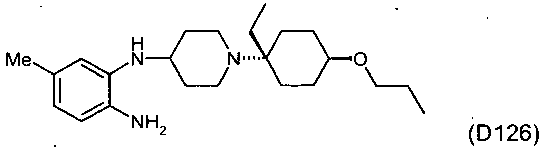

A stirred suspension of *trans*-*N*-(5-methyl-2-nitrophenyl-(1-ethyl-4-propoxycyclohexyl)-4-piperidinamine (D125, 40mg, 0.10mmol) in ethanol (5ml) at 50°C was treated with Raney nickel (0.5ml of 10% aqueous suspension) followed by hydrazine hydrate (0.1ml, 2.0mmol). The mixture was heated at the same temperature for 1 h, then filtered through Celite and the filtrate evaporated and re-evaporated from toluene then diethyl ether to afford the title compound, 40mg.

### Description 127. 3-({1-[trans-4-(Ethyloxy)cyclohexyl]-4-piperidinyl}amino)-4-nitrobenzonitrile (D127)

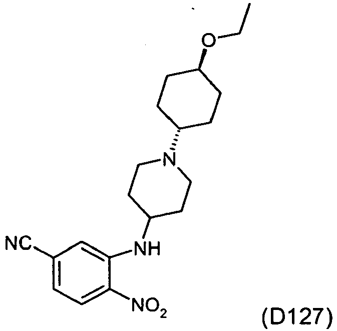

A stirred solution of 3-fluoro-4-nitrobenzonitrile (133mg, 0.80mmol) in dimethylformamide (4ml) at room temperature under argon was treated with diisopropylethylamine (0.46ml, 2.6mmol) followed by 1-[*trans*-4-(ethyloxy)cyclohexyl]-4-piperidinamine dihydrochloride (D12, 225mg, 0.75mmol) and heated at 70°C for 6hrs. The solution was concentrated under vacuum and the residue treated with 10% Na₂CO₃ solution (10ml) and extracted with dichloromethane (2x15ml). The combined extract was dried (Na₂SO₄), concentrated under vacuum and the residual solid chromatographed on silica gel (5g) eluting with 0-10% methanol/dichloromethane to give an orange solid, which was recrystallised from MeOH (8ml) to afford the title compound as an orange solid (175mg, 63%). MH⁺ 373.
¹H NMR δ (CDCl₃, 400 MHz): 1-18-1.40 (4H, m), 1.21 (3H, t), 1.60-1.72 (2H, m), 1.90-1.98 (2H, m), 2.03-2.18 (4H, m), 2.32-2.50 (3H, m), 2.88-2.98 (2H, m), 3.13-3.23 (1H, m), 3.42-3.58 (3H, q + m), 6.84 (1H, dd), 7.15 (1H, d), 8.08 (1H, d), 8.25 (1H, d).

### Description 128. 4-Amino-3-({1-[trans-4-(ethyloxy)cyclohexyl]-4-piperidinyl}amino)benzonitrile (D128)

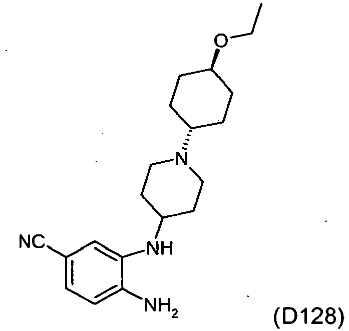

A stirred suspension of 3-({1-[*trans*-4-(ethyloxy)cyclohexyl]-4-piperidinyl}amino)-4-nitrobenzonitrile (D127, 145mg, 0.39mmol) in ethanol (8ml) and water (2ml) at room temperature under argon was treated with iron powder (220mg), then conc. HCl acid (0.2ml) and maintained for 1.5hrs. The mixture was concentrated under vacuum and the residue treated with NaHCO₃ solution (15ml) and EtOAc (20ml), then shaken well and filtered through Kieselguhr. The EtOAc layer was isolated, dried (Na₂SO₄) and concentrated under vacuum to afford the title compound as a green/grey solid (120mg, 90%). MH⁺ 343.
¹H NMR δ (CDCl₃, 400MHz): 1.15-1.40 (7H, m), 1.42-1.55 (2H, m), 1.80-1.98 (assume 2H, m), 2.00-2.20 (assume 4H, m), 2.28-2.50 (assume 3H, m), 2.85-2.95 (2H, m), 3.00-3.30 (3H, m), 3.51 (2H, q), 3.78 (2H, s), 6.69 (1H, d), 6.85 (1H, d), 6.99 (1H, dd).

### Description 129. 3-({1-[trans-4-(Ethyloxy)-1-methylcyclohexyl]-4-piperidinyl}amino)-4-nitrobenzonitrile (D129)

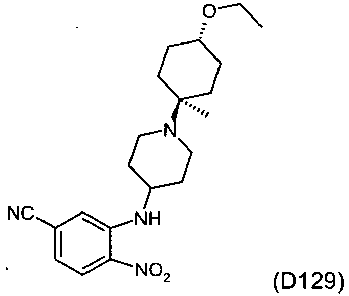

A solution of 3-fluoro-4-nitrobenzonitrile (111mg, 0.67mmol) and 1-[*trans*-4-(ethyloxy)-1-methylcyclohexyl]-4-piperidinamine dihydrochloride (D58, 200mg, 0.61mmol) in dimethylformamide (3ml) under argon was treated with diisopropylethylamine (0.3ml, 1.38 mmol) and the reaction mixture heated at 110°C for 20mins in a microwave reactor. The resulting mixture was then concentrated under vacuum, the residue treated using aqueous NaHCO₃ solution (30ml) and then extracted using ethyl acetate (2x40ml). The combined extract was dried (MgSO₄) and concentrated under vacuum to give crude material as an orange residue. The residue was then chromatographed on silica eluting 0-10% methanol/dichloromethane to yield the title compound (200mg, 80%) as an orange oil. M⁺ + H = 387

### Description 130. 4-Amino-3-({1-[trans-4-(ethyloxy)-1-methylcyclohexyl]-4-piperidinyl}amino)benzonitrile (D130)

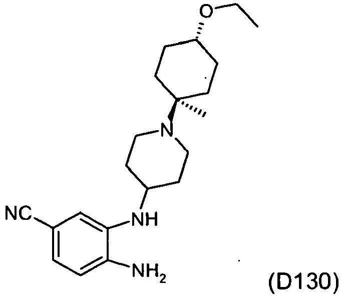

A stirred solution of 3-({1-[*trans*-4-(ethyloxy)-1-methylcyclohexyl]-4-piperidinyl}amino)-4-nitrobenzonitrile (110mg, 0.285mmol) in ethanol/Water (10ml/2ml) at room temperature under argon was treated with iron powder (250mg), followed by conc. HCl (0.3ml) and the reaction mixture maintained for 30mins. The homogeneous mixture was then concentrated under vacuum, the resultant residue treated with aqueous NaHCO₃ solution (50ml) and ethyl acetate (50ml). The biphasic mixture was then well shaken before filtration through Kieselguhr, the organic phase was then separated, dried (MgSO₄) and volatiles removed by evaporation under vacuum to afford crude product (90mg, 58% by NMR). M⁺ + H = 357.

### Description 131. 8-(2-Propyn-1-yloxy)-1,4-dioxaspiro[4.5]decane (D131)

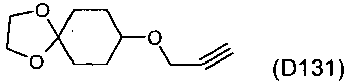

A stirred solution of 1,4-dioxaspiro[4.5]decan-8-ol (D1, 5.0g), propargyl bromide (4.6g) in N-methylpyrrolidinone (25ml) at ice bath temperature under argon and was treated portionwise with sodium hydride (2.6g of 60% oil dispersion), then the mixture was allowed to warm to room temperature overnight. The mixture was treated with water was added and the mixture extracted with a hexane - ethyl acetate - ether mixture. The organic extract as washed with sodium bicarbonate, then dried (Na₂SO₄) and solvent removed and the residue was chromatographed on silica gel eluted with hexane/ethyl acetate 10-30% to give the title compound as oil (3.11g)
¹HNMR δ (CDCl₃, 400 MHz): 1.55 (2H, m), 1.7-1.9 (6H, m), 2.41 (1H, m), 3.55 (1H, m), 3.92 (4H, m), 4.17 (2H, m).

### Description 132. 4-(2-Propyn-1-yloxy)cyclohexanone (D132)

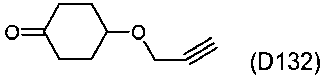

A solution of 8-(2-propyn-1-yloxy)-1,4-dioxaspiro[4.5]decane (D131, 3.1g) in tetrahydrofuran (5ml) was treated with 5M HCl acid (20ml) and stirred for 2h. The resulting mixture was diluted with water and extracted with dichloromethane (2 x). The combined extract was washed with brine and dried (Na₂SO₄) and concentrated under vacuum to afford the title compound as oil (2.37g).
¹H NMR δ (CDCl₃, 400 MHz): 2.00 (2H, m), 2.12 (2H, m), 2.3 (2H, m), 2.46 (1H, m) 2.6 (2H, m), 3.96 (1H, m), 4.25 (2H, s).

### Description 133. cis/trans-1,1-Dimethylethyl {1-[4-(2-propyn-1-yloxy)cyclohexyl]-4-piperidinyl}carbamate (D133)

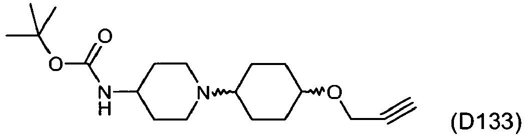

A stirred solution of 4-(2-propyn-1-yloxy)cyclohexanone (D132, 1g) in 1,2-dichloroethane (50ml) under argon was treated with 1,1-dimethylethyl 4-piperidinylcarbamate (1.4g), sodium triacetoxyborohydride (2.0g), then the resulting mixture stirred at room temperature for 2 hours. The reaction mixture was diluted with dichloromethane then washed with Na₂CO₃ solution, brine, then dried (Na₂SO₄) and concentrated under vacuum. The crude product which was purified by chromatography (ethyl acetate/n-hexane) on a Biotage KP-NH^{™}-silica column to yield, as a mixture of cis and trans isomers (1.1g). MH⁺ = 337

### Description 134. cis/trans-1-[4-(2-Propyn-1-yloxy)cyclohexyl]-4-piperidinamine dihydrochloride (D134)

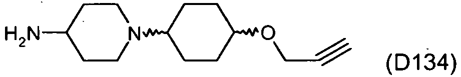

cis/trans-1,1-Dimethylethyl{1-[4-(2-propyn-1-yloxy)cyclohexyl]-4-piperidinyl}carbamate (D133, 1.1 g) was stirred in a solution of ethanol saturated with hydrogen chloride (25ml) for 2 hours. The solvent was evaporated to give the title compound as a white solid (1.27g). M⁺ + H = 253.

### Description 135. N-(5-Methyl-2-nitrophenyl)-1-[trans-4-(2-propyn-1-yloxy)cyclohexyl]-4-piperidinamine (D135a) and N-(5-methyl-2-nitrophenyl)-1-[cis-4-(2-propyn-1-yloxy)cyclohexyl]-4-piperidinamine (D135b)

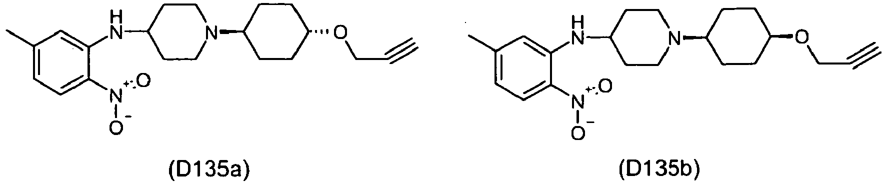

A solution of cis/trans-1-[4-(2-propyn-1-yloxy)cyclohexyl]-4-piperidinamine dihydrochloride (D134, 0.20g) diisopropylethylamine (1ml) and 3-fluoro-4-nitrotoluene (0.2g) in dry dimethylformamide (3 ml), was heated at 200°C for 20 minutes in a microwave reactor. The crude mixture was then cooled to room temperature, the solvent was evaporated and the residue was loaded onto a SCX cartridge. The cartridge was eluted with methanol followed by 2M methanolic ammonia. The methanolic ammonia fraction was evaporated to afford the crude product. The above was repeated multiple times. The separate cis and trans isomers of the title compound were obtained by repeated chromatography on a Biotage KP-NH^{™}-silica using a ethyl acetate/n-hexane gradient.

Fast eluting isomer (cis isomer; D135b) was obtained as a yellow solid. MH⁺ = 372.
¹H NMR δ (CDCl₃, 400 MHz): 0.94 (2H, m), 1.05-1.25 (obs, m), 1.95-2.14 (4H , m), 1.55-1.70 (obs, m), 2.33 (3H, t), 2.35-2.55 (4H, m), 2.88 (2H, m), 3.15 (1H, m), 3.22 (1H, m), 4.14 (2H, m), 3.52 (2H, m), 6.14 (1H, d), 6.61 (1H, s) 8.04 (1H, d), 8.19 (1H, d).

Slow eluting isomer (trans isomer; D135a) was obtained as a white solid. MH⁺ = 372.
¹H NMR δ (CDCl₃, 400 MHz): 1.2-1.4 (4H, m), 1.65 (2H, m), 1.95 (2H, m), 2.05-2.18 (4H, m), 2.33 (3H, t), 2.35-2.5 (4H, m), 2.88 (2H, m), 3.42 (1H, m), 2.55 (1H, m), 4.19 (2H, m), 6.42 (1H, d), 6.61 (1H, s) 8.07 (1H, d), 8.2 (1H, d).

### Description 136. 4-Methyl-N²-{1-[cis-4-(2-propyn-1-yloxy)cyclohexyl]-4-piperidinyl}-1,2-benzenediamine (D136)

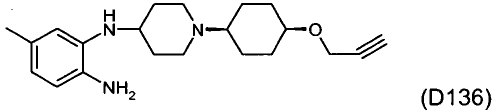

A solution of *N*-(4-fluoro-5-methyl-2-nitrophenyl)-1-[*cis*-1-methyl-4-(propyloxy)cyclohexyl]-4-piperidinamine (D135b, 0.18mg) in ethanol (7ml) was added over 10 min to a solution of tin (II) chloride ( 0.45g) in conc hydrochloric acid (5ml) stirred at 60°C. The mixture was heated at 60°C for 0.5hr then poured into a mixture of dilute sodium hydroxide solution and dichloromethane. The dichloromethane layer was separated, washed with brine and dried with sodium sulfate and the solvent was removed to give the title compound as a oil (0.2g). MH⁺ = 342.

### Description 137. 4-Methyl-N²-{1-[trans-4-(2-propyn-1-yloxy)cyclohexyl]-4-piperidinyl}-1,2-benzenediamine (D137)

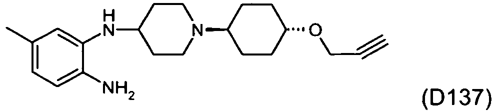

A solution of *N*-(4-fluoro-5-methyl-2-nitrophenyl)-1-[*trans*-1-methyl-4-(propyloxy)cyclohexyl]-4-piperidinamine (D135a, 0.269mg) in ethanol (15ml) was added over 5 min to a solution of tin (II) chloride (0.8g) in conc hydrochloric acid (8ml) stirred at 60°C. The mixture was heated at 60°C for 0.5hr then poured into a mixture of dilute sodium hydroxide solution and dichloromethane. The dichloromethane layer was separated, washed with brine and dried with sodium sulfate and the solvent was removed to give the title compound as a glass (0.28g). MH⁺ = 342.

### Description 138. Ethyl 4-{[2-nitro-5-(trifluoromethyl)phenyl]amino}-1-piperidinecarboxylate (D138)

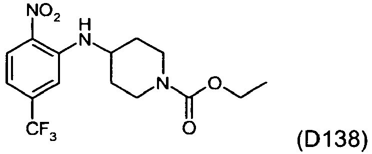

Ethyl-4-amino-1-piperidine-carboxylate (16.0 g, 92 mmol) was added to a stirred suspension of 3-chloro-4-nitrobenzotrifluoride (19.0 g, 84 mmol), sodium carbonate (8.9 g, 84 mmol) and potassium iodide (0.14 g, 0.84 mmol) in dimethylformamide (200 mL). The mixture was heated to 90 °C, stirred for 15 hours and then allowed to cool. The reaction mixture was diluted with water (200ml) and ethyl acetate (200ml). The aqueous phase was separated and the organic phase was extracted with water (2 x 200ml). The organic phase was dried over MgSO₄ and evaporated under reduced pressure. The residue was purified by column chromatography on silica, eluting with ethyl acetate/hexane (1:5) to give the title compound (12.0 g, 40% yield) as an orange solid. (R_{f}: 0.4 (ethyl acetate/hexane 1:5))

### Description 139. Ethyl 4-{[2-amino-5-(trifluoromethyl)phenyl]amino}-1-piperidinecarboxylate (D139)

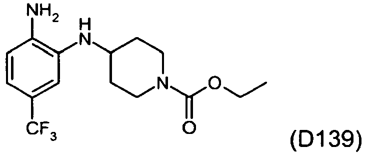

A suspension of ethyl 4-{[2-nitro-5-(trifluoromethyl)phenyl]amino}-1-piperidinecarboxylate (D138, 18.0 g, 50 mmol) and 5% Pd/C (1.7 g) in methanol (400ml) in an autoclave (1000ml) was put under an atmosphere of hydrogen (∼ 30 atm). The mixture was stirred at room temperature for 3 hours and then filtered through a pad of celite. The filtrate was concentrated under reduced pressure to give the title compound (15.9 g, 96% yield) as a brown solid that was used without further purification. (R_{f}: 0.3 (ethyl acetate/hexane 7:8))

### Description 140. Ethyl 4-[2-oxo-6-(trifluoromethyl)-2,3-dihydro-1H-benzimidazol-1-yl]-1-piperidinecarboxylate (D140).

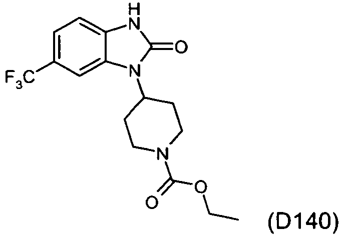

1,1'-Carbonyldiimidazole (9.9 g, 61 mmol) was added portionwise over 10 minutes to a stirred solution of ethyl 4-{[2-amino-5-(trifluoromethyl)phenyl]amino}-1-**piperidinecarboxylate** (D139, 12.7 g, 38 mmol) in acetonitrile (200ml). The reaction mixture stirred at room temperature for 5 hours and then the solvent was removed under reduced pressure. The residue was diluted with dichloromethane (200ml) and extracted with a 10% aqueous solution of citric acid (2 x 100ml). The organic phase was dried over MgSO₄ and evaporated under reduced pressure to give the title compound (13.5 g, 99% yield) as a brown solid that was used without further purification. (R_{f}: 0.2 (ethyl acetate))

### Description 141. 1-(4-Piperidinyl)-6-(trifluoromethyl)-1,3-dihydro-2H-benzimidazol-2-one (D141).

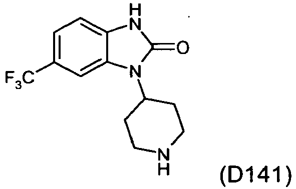

Iodotrimethylsilane (17ml, 120 mmol) was added to a stirred solution of ethyl 4-[2-oxo-6-(trifluoromethyl)-2,3-dihydro-1*H*-benzimidazol-1-yl]-1-piperidinecarboxylate (D140, 13.5 g, 38 mmol) in chloroform (150ml) under argon. The reaction mixture was refluxed for 15 hours and then allowed to cool. Methanol (30ml) was added and the reaction mixture was stirred for 30 minutes. The precipitate was collected by filtration and washed with chloroform (2 x 20ml). The solid was dissolved in water (150ml) and the solution basified to pH 13 with solid sodium hydroxide. After stirring for 30 minutes, 6 M HCl was added until pH 8.5 was reached and the resulting precipitate was collected by filtration. The solid was dried under vacuum at 40 °C for 15 hours to give the title compound (11.1 g, 97% yield) as a colourless solid (R_{f}: Baseline (ethyl acetate/methanol (19:1)).
¹H-NMR (300 MHz, DMSO-*d*₆): δ 7.60 (1H, s), 7.34 (1H, d), 7.14 (1H, d), 4.55 (1H, m), 3.40 (2H, d), 3.05 (2H, t), 2.50 (2H, m), 1.88 (2H, m); m.p. >200 °C (decomp.).

### Description 142. 1,1-Dimethylethyl 4-[(5-methyl-2-nitrophenyl)amino]-1-piperidinecarboxylate (D142)

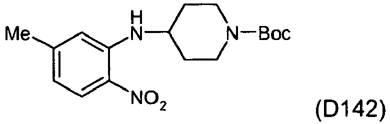

A stirred solution of 3-fluoro-4-nitrotoluene (10g, 0.064mol) in dimethylformamide (40ml) at room temperature under argon was treated with diisopropylethylamine (14ml), 0.080mol) followed by 1,1-dimethylethyl 4-amino-1-piperidinecarboxylate (11.3g, 0.064mol) and then heated at 90°C for 18hrs. The mixture was concentrated under vacuum and the residue treated with 10% Na₂CO₃ solution (100ml) and extracted with dichloromethane (2 x 150ml). The combined extract was washed with water (150ml), dried (Na₂SO₄) and concentrated under vacuum. The residue was treated with Et₂O (200ml) and allowed to stand overnight. The crystals which had formed were filtered off, washed with Et₂O and dried to afford the title compound as an orange solid (16g, 74%).
¹H NMR δ (CDCl₃, 400MHz): 1.45-1.65 (2H, m), 1.48 (9H, s), 2.00-2.10 (2H, m), 2.35 (3H, s), 3.00-3.15 (2H, m), 3.62-3.72 (1H, m), 3.92-4.12 (2H, br m), 6.46 (1H, dd), 6.63 (1H, s), 8.07 (1H, d), 8.15 (1H, d).

### Description 143. 1,1-Dimethylethyl 4-[(2-amino-5-methylphenyl)amino]-1-piperidinecarboxylate (D143)

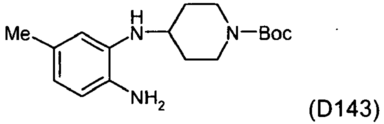

A stirred suspension of 1,1-dimethylethyl 4-[(5-methyl-2-nitrophenyl)amino]-1-**piperidinecarboxylate** (D142, 16g, 0.050mol) in ethanol (350ml) was treated with 10% Pd/C (1.5g) and maintained under a hydrogen atmosphere at room temperature and pressure for 16hrs. The mixture was filtered through Kieselguhr to remove the catalyst and the filtrate concentrated under vacuum to leave the title compound as a pale purple solid (13.5g, 93%).
¹H NMR δ (CDCl₃, 400MHz): 1.28-1.52 (2H, m), 1.48 (9H, s), 2.04 (2H, br d), 2.26 (3H, s), 2.90-3.02 (2H, m), 3.20 (3H, br s), 3.35-3.46 (1H, m), 4.02 (2H, br s), 6.46-6.50 (2H, m), 6.64 (1H, d).

### Description 144. 1,1-Dimethylethyl 4-(6-methyl-2-oxo-2,3-dihydro-1H-benzimidazol-1-yl)-1-piperidinecarboxylate (D144)

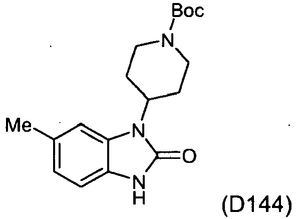

A stirred solution of 1,1-dimethylethyl 4-[(2-amino-5-methylphenyl)amino]-1-**piperidinecarboxylate** (13.5g, 0.044mol) in acetonitrile (300ml) at room temperature under argon was treated over 10 minutes with a solution of 1,1'-carbonyldiimidazole (11.4g, 0.070mol) in acetonitrile (150ml), then heated at 40°C for 6hrs followed by 18hrs at room temperature. The solid which had formed was filtered off, washed with acetonitrile and dried to afford 4.6g of title compound as a pale purple solid. The filtrate was concentrated under vacuum and the residue dissolved in dichloromethane (300ml) and washed with water (3 x 200ml), then dried (Na₂SO₄) and concentrated under vacuum to leave a brown oil. This was treated with 1:1 EtOAc/60-80 petrol (200ml) to cause crystallisation of more title compound (5.6g).
¹H NMR δ (CDCl₃, 400MHz): 1.52 (9H, s), 1.83 (2H, br d), 2.26-2.45 (2H, m), 2.39 (3H, s), 2.80-3.00 (2H, br m), 4.30 (2H, br s), 4.41-4.52 (1H, m), 6.87 (1H, d), 6.94 (1H, s), 6.98 (1H, d), 9.30 (1H, s).

### Description 145. 6-Methyl-1-(4-piperidinyl)-1,3-dihydro-2H-benzimidazol-2-one (D145)

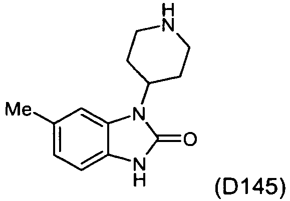

A stirred solution of 1,1-dimethylethyl 4-(6-methyl-2-oxo-2,3-dihydro-1*H*-benzimidazol-1-yl)-1-piperidinecarboxylate (D144, 10.2g, 0.031mol) in a mixture of dichloromethane (80ml) and MeOH (20ml) at room temperature under argon was treated with 4M HCl in dioxane (35ml, 0.14mol) and stirred at room temperature for 20hrs. The solid which had formed was filtered off, washed with Et₂O and dried to afford the HCl salt of the title compound as a pale grey solid (7.95g, 96%). Most of this material (7.80g) was treated with DCM (220ml) and 10% Na₂CO₃ solution (200ml), shaken well until solid had dissolved, then the DCM layer was separated, dried (Na₂SO₄) and concentrated under vacuum to afford the title compound as a beige solid (6.80g).
¹H NMR (free base) δ (CDCl₃, 400MHz): 1.64 (1H, br s), 1.80-1.90 (2H, m), 2.30 -2.45 (2H, m), 2.39 (3H, s), 2.75-2.86 (2H, m), 3.23-3.31 (2H, m), 4.38-4.50 (1H, m), 6.86 (1H, d), 6.98 (1H, d), 7.11 (1H, s), 9.50 (1H, br s).

### Description 146. 1-[trans-4-(Ethyloxy)-1-methylcyclohexyl]-N-(5-fluoro-2-nitrophenyl)-4-piperidinamine (D146)

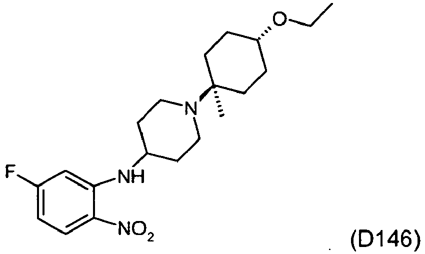

A solution of 2,4-difluoro-1-nitrobenzene (0.115ml, 1.06mmol) and 1-[*trans*-4-(ethyloxy)-1-methylcyclohexyl]-4-piperidinamine dihydrochloride (D58, 300mg, 0.962mmol) in N,N-dimethylformamide (5ml) under argon was treated with diisopropylethylamine (0.49ml, 2.88 mmol) and the reaction mixture heated at 110°C for 40mins in a microwave reactor. The resulting mixture was then concentrated under vacuum, the residue treated using dil. NaHCO₃ solution (30ml) and then extracted using ethyl acetate (2x50ml). The combined extract was dried (MgSO₄) and concentrated under vacuum to give crude material as an orange residue. The residue was chromatographed on silica gel eluting 0-10% methanol/dichloromethane to yield the title compound (130mg, 27%) as a yellow residue. MH⁺ = 380.

### Description 147. (2-Amino-5-fluorophenyl){1-[trans-4-(ethyloxy)-1-methylcyclohexyl]-4-piperidinyl}amine.

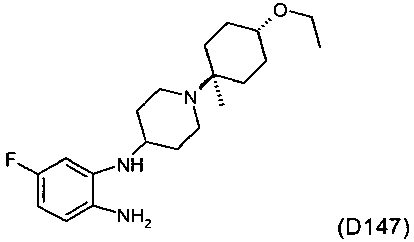

A stirred solution of the 1-[*trans*-4-(ethyloxy)-1-methylcyclohexyl]-*N*-(5-fluoro-2-nitrophenyl)-4-piperidinamine (D146, 130mg, 0.342mmol) in EtOH (10ml) at room temperature under argon was treated with Raney Nickel (25mg), followed by hydrazine hydrate (166µL, 3.42mmol). The reaction mixture left to stir for 2h when the initial yellow colour had been lost. The catalyst was removed by filtration through Kieselguhr and the filtrate concentrated under vacuum to leave the title compound as an off-white residue (97mg, 81 %). MH⁺ = 350.

### Description 148. cis/trans-1-Methyl-4-(methyloxy)cyclohexanecarboxylic acid (D148)

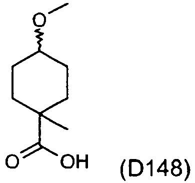

A stirred solution of diisopropylamine (16.1ml, 115 mmol, 2.1 eq.) in tetrahydrofuran (200ml) at 0°C under argon was treated with 2.5M n-butyllithium in hexane (1.05 eq., 110 mmol, 44ml) and it was then stirred at the same temperature for 10 minutes. The mixture was then treated with a solution of 4-(methyloxy)cyclohexanecarboxylic acid (1 eq., 55 mmol, 8.7g) in 50 ml of dry tetrahydrofuran. The resulting yellow solution was heated at 50°C for 2 hrs, then cooled to 0°C and treated with iodomethane (3 eq., 12 ml). The mixture was allowed to warm to room temperature and was stirred for 2 hrs. The mixture was partitioned between citric acid (10% aqueous solution) and Et₂O, the two phases were separated and the aqueous was extracted with Et₂O (2x). Organics were combined, dried (Na₂SO₄), filtered and concentrated under vacuum to leave a yellow solid (9.8g) a mixture of *cis:trans* isomers.
¹H NMR δ (d⁶DMSO, 400MHz): 1.078-2.018 (11 H, *cis*/*trans* isomers), 3.074 (1H, m single isomer), 3.193 (3H, s single isomer), 3.213 (3H, s single isomer), 3.606 (1H, s broad, single isomer), >12.5 (1H, s broad, *cis*/*trans* isomers).

### Description 149. trans-1-Methyl-4-(methyloxy)cyclohexanecarboxylic acid (D149)

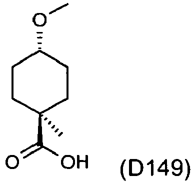

*cis*/*trans*-1-methyl-4-(methyloxy)cyclohexanecarboxylic acid (D148, 41.3 mmol, 7.7 g) was dissolved in thionyl chloride (15.3 eq., 631 mmol, 46 ml). The mixture was refluxed at 90°C for 4 hours. The solvent was evaporated and the crude product treated with toluene and concentrated in vacuo. The residual brown oil was treated with 5% Na₂CO₃ solution (400 ml) and tetrahydrofuran (40 ml) and the mixture was stirred at room temperature for 30 minutes. The aqueous solution was washed with Et₂O (2x) and the aqueous phase was acidified with 2M HCl acid and extracted with ethyl acetate (2x). The combined organics were dried over Na₂SO₄, filtered and the solvent was evaporated to afford the titled compound, 2.8 g, 40%.
¹H NMR δ (d⁶DMSO, 400MHz): 1.085 (3H, s), 1.419 (2H, m), 1.485 (4H, m), 1.701 (2H, m), 3.225 (4H, m), 3.342 (1H, s).

### Description 150. trans-1-Isocyanato-1-methyl-4-(methyloxy)cyclohexane (D150)

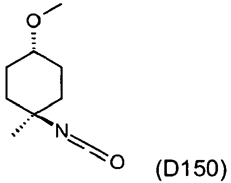

To a solution of *trans*-1-methyl-4-(methyloxy)cyclohexanecarboxylic acid (D149, 15.0 mmol, 2.8 g) in dry toluene (70 ml), Et₃N (1.3 eq., 19.6 mmol, 2.7 ml), and diphenylphosphoryl azide (1.0 eq., 15.0 mmol, 3.2 ml) were added at room temperature. The mixture was refluxed at 80°C for 2 hours. The mixture was cooled to room temperature and poured onto 1 M NaOH (70 ml); the aqueous solution was extracted with EtOAc (2x). The organics were combined dried over Na₂SO₄, filtered and the solvent was evaporated to afford the crude compound. The crude product was then purified by chromatography (EtOAc-nhex) on silica column to afford the title compound, 1.33 g, 48%.
¹H NMR δ (d⁶DMSO, 400MHz): 1.327 (3H, s), 1.61 (8H, m), 3.197 (3H, s) 3.355 (1H, m).

### Description 151. trans-1-Methyl-4-(methyloxy)cyclohexanamine monohydrochloride (D151)

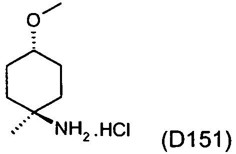

To a solution of *trans*-1-isocyanato-1-methyl-4-(methyloxy)cyclohexane (D150, 7.27 mmol, 1.33 g) in THF (30 ml), concentrated HCl (6 ml) was added at room temperature. The mixture was stirred for 4 hours at room temperature and concentrated HCl (a few drops) was added. The reaction was left overnight and the solvent evaporated to afford the title compound, 0.9 g, 69%.
¹H NMR δ (d⁶DMSO, 400MHz): 1.260 (3H, s), 1.39 (2H, m), 1.632 (4H, m), 1.87 (2H, m), 3.157 (1H, m), 3.217 (3H, s).

### Description 152. 1-[trans-1-Methyl-4-(methyloxy)cyclohexyl]-4-piperidinone (D152)

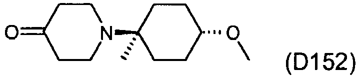

*Trans*-1-methyl-4-(methyloxy)cyclohexanamine monohydrochloride (D151, 7.69 mmol, 1.1 g), 1-ethyl-1-methyl-4-oxopiperidinium iodide (D49, 1.7 eq., 13.1 mmol), 3.5 g, K₂CO₃ (1.5 eq., 11.5 mmol, 1.5 g), water (40 ml) and ethanol (80 ml) were refluxed at 80°C until the starting material was not observed on TLC. The mixture was partitioned between NaHCO₃ and dichloromethane. Some product was lost due to mechanical spillage. The work up was repeated. The crude product was then purified by chromatography (MeOH-NH₃-dichloromethane) on silica column to afford the title compound, 650 mg, 35 %.
¹H NMR δ (d⁶DMSO, 400MHz) 0.85 (3H, s), 1.50 (8H, m)1.762 (2H, t), 2.301 (4H, t), 2.725 (4H, t), 3.207 (3H, s), 3.274 (1H, m).

### Description 153. 1-[trans-1-Methyl-4-(methyloxy)cyclohexyl]-4-piperidinamine (D153)

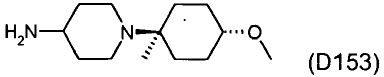

To a solution of 1-[*trans*-1-methyl-4-(methyloxy)cyclohexyl]-4-piperidinone (D152, 2.67 mmol, 650 mg) in 2M NH₃ in methanol (15.0 eq., 40.0 mmol, 20 ml), 10% Pd/C paste (65 mg) was added. The mixture hydrogenated at 50psi for 24 hours at room temperature. The mixture filtered through kieselguhr and washed with ethanol (100 ml). The solvent was evaporated to afford the crude product. The reaction was repeated on the crude product and left for 24 hours. The mixture was filtered through celite and washed with ethanol and the solvent was evaporated to afford the titled compound, 390 mg, 68%, M⁺ + H = 227.

### Description 154. N-(5-Fluoro-2-nitrophenyl)-1-[trans-1-methyl-4-(methyloxy)cyclohexyl]-4-piperidinamine (D154)

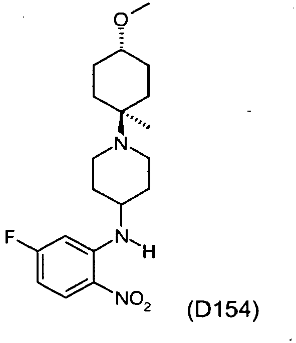

Diisopropylethylamine (0.55mL, 3.23mmol) and 2,4-difluoro-1-nitrobenzene (123µL, 1.12mmol) were added to a solution of 1-[*trans*-4-(methyloxy)-1-methylcyclohexyl]-4-piperidinamine dihydrochloride (D153, 306mg, 1.02mmol) in N,N-dimethylformamide (5ml) under argon. The reaction was heated at 100°C in a microwave reactor for 30min and then for a further 30min. The mixture was poured on to sat. NaHCO₃ (50mL) and extracted with EtOAc (3x). The combined organics were washed sequentially with brine, H₂O and brine, dried (Na₂SO₄) and concentrated via rotary evaporation. The crude residue was purified by SCX (5g) and then chromatographed (silica, CH₂Cl₂-0.5% NH₃ /9.5% MeOH /90% CH₂Cl₂) to give a yellow oil. The yellow oil was then chromatographed (amine doped silica, hexane-EtOAc) to yield the title compound (204mg, 55%) as a yellow oil. MH⁺ 366

### Description 155. (2-Amino-5-fluorophenyl){1-[trans-4-(methyloxy)-1-methylcyclohexyl]-4-piperidinyl}amine (D155)

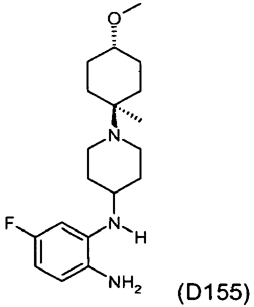

Raney Nickel (0.25mL, 10% sol. in H₂O) was added to a vigorously stirred solution of *N-*(5-fluoro-2-nitrophenyl)-1-[*trans*-1-methyl-4-(methyloxy)cyclohexyl]-4-piperidinamine (D154, 245mg, 0.67mmol) in EtOH (10mL) under argon. Hydrazine hydrate (220µL, 7.06mmol) was added dropwise followed by a second portion of Raney Nickel (0.25mL, 10% sol. in H₂O). The reaction was stirred for 1h and then filtered through Kieselguhr using EtOH. The solvent was removed via rotary evaporation to give the title compound (205mg, 91%) as a brown oil which solidified on standing to a brown solid. MH⁺ 336.

### Description D156. N-(5-Chloro-2-nitrophenyl)-1-[trans-1-methyl-4-(methyloxy)cyclohexyl]-4-piperidinamine (D156)

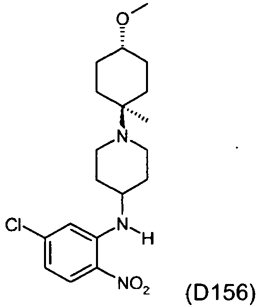

Diisopropylethylamine (0.53mL, 3.12mmol) and 6-chloro-2-fluoro-1-nitrobenzene (197mg, 1.12mmol) were added to a solution of 1-[*trans*-4-(methyloxy)-1-methylcyclohexyl]-4-piperidinamine dihydrochloride (D153, 318mg, 1.06mmol) in N,N-dimethylformamide (5ml) at r.t. under argon. The reaction was heated at 110°C in a microwave reactor for 30min and then for a further 30min after the addition of a second portion of 6-chloro-2-fluoro-1-nitrobenzene (81mg, 0.46mmol). The reaction was poured on to sat. NaHCO₃ (50mL) and extracted with EtOAc (3x). The combined organics were washed sequentially with brine, H₂O and brine, dried (Na₂SO₄) and concentrated via rotary evaporation. The crude residue was purified by SCX (5g) and then chromatographed (silica, CH₂Cl₂-0.5% NH₃ /9.5% MeOH /90% CH₂Cl₂) to yield the title compound (323mg, 85%) as a yellow oil.
M(Cl-35)⁺ 382, M(Cl-37)H⁺ 384.

### Description D157. (2-Amino-5-chlorophenyl){1-[trans-1-methyl-4-(methyloxy)cyclohexyl]-4-piperidinyl}amine (D157)

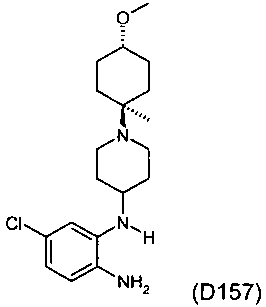

*N*-(5-Chloro-2-nitrophenyl)-1-[*trans*-1-methyl-4-(methyloxy)cyclohexyl]-4-piperidinamine (D156, 323mg, 0.85mmol) was stirred vigorously in EtOH (10mL) at r.t. under argon. Raney Nickel (0.25mL, 10% sol. in H₂O) was added, followed by the hydrazine hydrate (260µL, 8.35mmol). A second portion of Raney Nickel (0.25mL, 10% sol. in H₂O) was added and the reaction was stirred vigorously for 30min. The mixture was filtered through Kieselguhr using EtOH and concentrated by rotary evaporation to give the title compound (305mg) as a golden-brown solid.
M(Cl-35)⁺ 352, M(Cl-37)H⁺ 354

### Description 158. 2-Fluoro-4-ethoxy-1-nitrobenzene (D158)

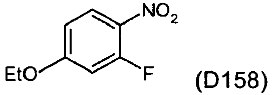

A solution of 3-fluoro-4-nitrophenol (3g), iodoethane (3ml) and potassium carbonate (5.5g) in butan-2-one (100ml) was heated at 85°C for 2.5 hours. The mixture was cooled, filtered and the filtrate was evaporated. Trituration with hexane gave the title compound as an off white solid (3g).

### Description 159. N-[5-(Ethyloxy)-2-nitrophenyl]-1-[trans-1-methyl-4-(methyloxy)cyclohexyl]-4-piperidinamine (D159)

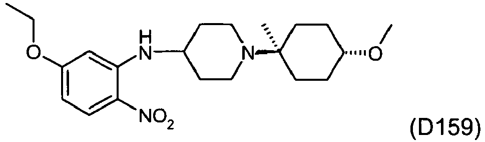

A microwave vessel was charged with 2-fluoro-4-ethoxy-1-nitrobenzene (D158, 0.3g), 1-[*trans*-1-methyl-4-(methoxy)cyclohexyl]-4-piperidinamine dihydrochloride (diHCl salt of D153, 0.3g), dimethylformamide (4ml) and diisopropylethylamine (1.0ml) and heated at 200°C for 20 minutes in a microwave reactor. The cooled reaction was evaporated, dissolved in methanol and loaded onto a SCX cartridge which was eluted with methanol then 2M methanolic ammonia. The methanolic ammonia fraction was evaporated and the residue was chromatographed on silica gel eluted with 0-5% dichloromethane - methanolic ammonia to give the title compound as a yellow glass (0.358g). MH⁺ = 392.

### Description 160. 4-(Ethyloxy)-N²-{1-[trans-1-methyl-4-(methyloxy)cyclohexyl]-4-piperidinyl}-1,2-benzenediamine (D160)

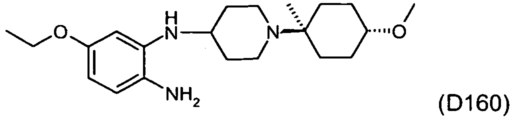

A solution of *N*-[5-(ethyloxy)-2-nitrophenyl]-1-[*trans*-1-methyl-4-(methyloxy) cyclohexyl]-4-piperidinamine (D159, 0.35g) in ethanol (50ml), Raney-nickel (∼2ml) and hydrazine hydrate (2ml) was stirred at 40°C for 30 minutes, then cooled, filtered and the filtrate was evaporated and azeotroped with ethanol to give the title compound as a glass (0.26g). MH⁺ = 362.

### Description 161. N-[5-(Ethyloxy)-2-nitrophenyl]-1-[trans-1-methyl-4-(ethyloxy)cyclohexyl]-4-piperidinamine (D161)

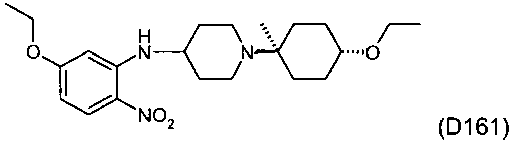

A microwave vessel was charged with 2-fluoro-4-ethoxy-1-nitrobenzene (D158, 0.3g), 1-[*trans*-4-(ethyloxy)-1-methylcyclohexyl]-4-piperidinamine dihydrochloride (D58, 0.312g), dimethylformamide (4ml) and diisopropylethylamine (1.0ml) and heated at 200°C for 20 minutes in a microwave reactor. The cooled reaction was evaporated, dissolved in methanol and loaded onto a SCX cartridge, which was eluted with methanol then 2M methanolic ammonia. The methanolic ammonia fraction was evaporated and the residue was chromatographed on silica gel eluted with 0-5% dichloromethane - methanolic ammonia to give the title compound as a yellow glass (0.123g). MH⁺ = 406.

### Description 162. 4-(Ethyloxy)-N²-{1-[trans-1-methyl-4-(ethyloxy)cyclohexyl]-4-piperidinyl}-1,2-benzenediamine (D162)

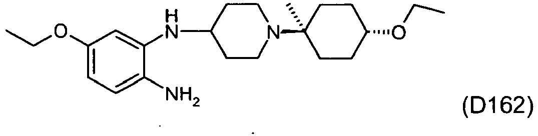

A solution of *N*-[5-(ethyloxy)-2-nitrophenyl]-1-[*trans*-1-methyl-4-(ethyloxy) cyclohexyl]-4-piperidinamine (D161, 0.123g) in ethanol (30ml), Raney-nickel (∼1ml) and hydrazine hydrate (1ml) was stirred at 40°C for 30 minutes then cooled, filtered and the filtrate was evaporated and azeotroped with ethanol to give the title compound as a glass (0.092g). MH⁺ = 376.

### Description 163. N-(5-Chloro-2-nitrophenyl)-1-[trans-4-(ethyloxy)-1-methylcyclohexyl]-4-piperidinamine (D163)

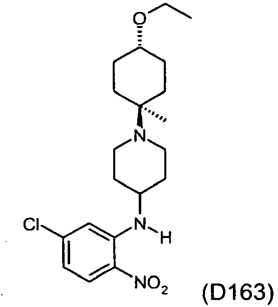

Diisopropylethylamine (0.52mL, 3.06mmol) and 4-chloro-2-fluoro-1-nitrobenzene (364mg, 2.07mmol) were added to a solution of 1-[*trans*-4-(ethyloxy)-1-methylcyclohexyl]-4-piperidinamine dihydrochloride (D58, 318mg, 1.02mmol) in N,N-dimethylformamide (5ml) at r.t. under argon. The reaction was heated at 110°C in a microwave reactor for 30min and then the mixture was poured on to sat. NaHCO₃ (50mL) and extracted with EtOAc (3x). The combined organics were washed sequentially with brine, H₂O and brine, dried (Na₂SO₄) and concentrated via rotary evaporation. The crude residue was purified by SCX (5g) and then chromatographed (silica, CH₂Cl₂-0.5% NH₃ /9.5% MeOH /90% CH₂Cl₂) to yield the title compound (153mg, 38%) as a yellow oil. M(Cl-35)⁺ 396, M(Cl-37)H⁺ 398.

### Description 164. (2-Amino-5-chlorophenyl){1-[trans-4-(ethyloxy)-1-methylcyclohexyl]-4-piperidinyl}amine (D164)

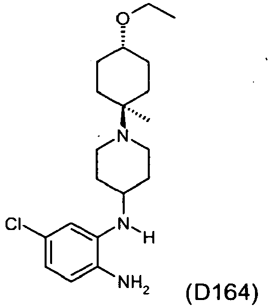

*N*-(5-Chloro-2-nitrophenyl)-1-[*trans*-4-(ethyloxy)-1-methylcyclohexyl]-4-piperidinamine (D163, 153mg, 0.40mmol) was stirred vigorously in EtOH (10mL) under argon. Raney Nickel (0.25mL, 10% sol. in H₂O) was added, followed by the hydrazine hydrate (130µL, 4.17mmol). A second portion of Raney Nickel (0.25mL, 10% sol. in H₂O) was added and the reaction was stirred vigorously for 30min. The mixture was filtered through Kieselguhr using EtOH and concentrated by rotary evaporation to give the title compound (139mg, 95%) as a yellow oil. M(Cl-35)⁺ 366, M(Cl-37)H⁺ 368.

### Example 1. 6-Methyl-1-[1-(cis-4-methoxycyclohexyl)-4-piperidinyl]-1,3-dihydro-2H-benzimidazol-2-one hydrochloride (E1)

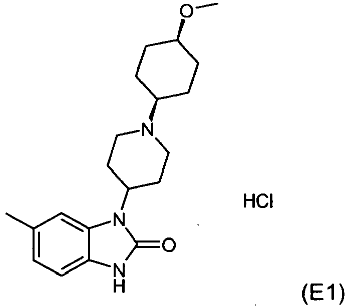

A solution of cis 5-methyl-*N*-[1-(4-methoxycyclohexyl)-4-piperidinyl]-1,2-benzenediamine (D7, 320mg; 0.9mmol) in dichloromethane (20ml) at 0°C was treated with triphosgene (110mg, 0.4eq) then diisopropylethylamine (0.23ml, 1.5eq), and then maintained at 0°C for 1h. The mixture was washed with water at pH9. Drying, evaporation, and chromatography (20g silica, 0-10% methanol in dichloromethane) gave 220mg (71%) of the free base compound from diethyl ether. This was then converted to 230mg of the hydrochloride salt from diethyl ether, 230mg.
¹H NMR (HCl salt) δ (d⁶DMSO): 1.4 (2H, m), 1.7 (2H, m), 1.8-2.1 (6H, m), 2.35 (3H, s), 2.9 (2H, m), 3.2 (3H, s), 3.2-3.6 (6H, m), 4.55 (1H, m), 6.8 (2H, ABq), 7.5 (1H, s), 10.55 (1H, br s), 10.8 (1H, s), MH+ 344.

### Example 2. 6-Methyl-1-[1-(trans-4-methoxycyclohexyl)-4-piperidinyl]-1,3-dihydro-2H-benzimidazol-2-one hydrochloride (E2)

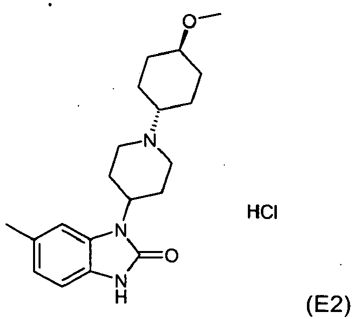

A solution of trans 5-methyl-*N*-[1-(4-methoxycyclohexyl)-4-piperidinyl]-1,2-benzenediamine (D8, 100mg, 0.3mmol) in dichloromethane (6ml) at 0°C was treated with triphosgene (40mg, 0.4eq) then diisopropylethylamine (0.08ml, 1.5eq), and then maintained at 0°C for 1h. The mixture was washed with water at pH9. Drying, evaporation and chromatography (10g silica, 0-10% methanol in dichloromethane) gave the title compound isolated as the hydrochloride salt from diethyl ether, 65mg.
¹H NMR (HCl salt) δ (d⁶DMSO): 1.2 (2H, m), 1.6 (2H, m), 1.9 (2H, m), 2.15 (4H, m), 2.35 (3H, s), 2.9 (2H, m), 3.25 (3H, s), 3.1-3.6 (6H, m), 4.55 (1H, m), 6.8 (2H, ABq), 7.5 (1H, s), 10.55 (1H, br s), 10.8 (1H, s), MH+ 344.

### Example 3. 1-{1-[trans-4-(Ethyloxy)cyclohexyl]-4-piperidinyl}-6-methyl-1,3-dihydro-2H-benzimidazol-2-one hydrochloride (E3)

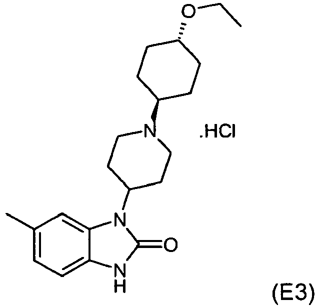

To a solution of (2-amino-5-methylphenyl){1-[*trans*-4-(ethyloxy)cyclohexyl]-4-piperidinyl}amine (D14, 110mg, 0.33 mmol) in dichloromethane (10 ml), diisopropylethylamine (0.085ml, 1.5eq., 0.50 mmol) was added at room temperature. The mixture was cooled to 0°C and bis(trichloromethyl)carbonate (40mg, 0.4eq., 0.13 mmol) was added. After 30 minutes the mixture was poured onto water; the aqueous solution was extracted with dichloromethane (2x) and the organics were alternatively washed with brine and water (2x). The organics were combined, dried over Na₂SO₄, filtered and the solvent was evaporated. The product was treated with HCl (3 ml of a 1 M solution in diethyl ether) to yield the title product (44 mgs, 37%). M⁺ - H = 356.
¹H NMR (HCl salt) δ (d⁶DMSO, 400MHz) 1.093 (3H, t), 1.221 (2H, m), 1.542 (2H, m) 1.887 (2H, m) 2.108 (3H, d) 2.333 (3H, s) 2.782 (2H, m), 3.202 (4H, m) 3.473 (4H, m) 4.542 (1H, m) 6.805 (1H, d) 6.871 (1H, d) 7.345 (1H, s), 10.00 (1H, br), 10.80 (1H, s).

### Example 4. 1-{1-[cis-4-(Ethyloxy)cyclohexyl]-4-piperidinyl}-6-methyl-1,3-dihydro-2H-benzimidazol-2-one hydrochloride (E4)

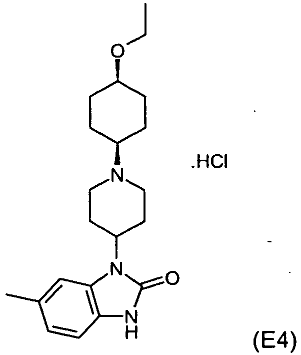

To a solution of (2-amino-5-methylphenyl){1-[*cis*-4-(ethyloxy)cyclohexyl]-4-piperidinyl}amine (D17, 147mg, 0.4 mmol) in dichloromethane (10 ml), diisopropylethylamine (0.09ml, 1.5eq., 0.7 mmol) was added at room temperature. The mixture was cooled to 0°C and bis(trichloromethyl)carbonate (0.053g, 0.4eq., 0.2 mmol) was added under argon. After 30 minutes waster was added; the aqueous solution was extracted with dichloromethane (2x) and the organics were alternatively washed with brine and water (2x). The organics were combined, dried over Na₂SO₄, filtered and the solvent was evaporated. The product was treated with HCl (3 ml of a 1 M solution in diethyl ether). The product was further purified by chromatography (EtOAc 60%: hexane), SCX followed by Waters Xbridge chromatography column eluting with acetonitrile/water/0/1% formic acid, to yield the title product (33 mgs, 21 %). M⁺ - H = 358.
¹H NMR (HCl salt) δ (d⁶DMSO 400MHz): 1.129 (3H, t), 1.441 (2H, t), 1.689 (2H, q), 1.887 (4H, t), 1.982 (2H, d), 2.331 (3H, s), 2.828 (2H, m), 3.247 (3H, m), 3.427 (2H, q), 3.492 (3H, m), 4.555 (1H, m), 6.801 (1H, d), 6.870 (1H, d), 7.462 (1H, s), 10.3 (1H, br s), 10.8 (1H, s).

### Example 5. 6-Methyl-1-{1-[cis-4-(propyloxy)cyclohexyl]-4-piperidinyl}-1,3-dihydro-2H-benzimidazol-2-one (E5)

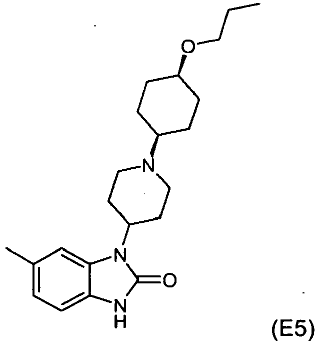

A stirred solution of (2-amino-5-methylpheny){1-[*cis*-4-(propyloxy)cyclohexyl]-4-piperidinyl}amine (D23, 400mg, 1.15mmol) and diisopropylethylamine (0.25ml, 1.4mmol, 1.5eq) in dichloromethane (35ml) at 0°C under argon was treated with solid triphosgene (138mg, 0.46mmol, 0.4eq) and maintained at room temperature for 3 days (64% of conversion). The mixture was treated with water (20ml) and extracted with dichloromethane (2 x 20ml). The combined extract was dried on MgSO₄ and concentrated under vacuum to leave a beige solid, which was chromatographed on silica column (40g of silica for 200mg of crude: 5% to 15% 0.4M NH₃ in methanol/dichloromethane 12CV) to give 150mg (35% yield) of title compound.
¹H NMR (free base) δ (CDCl₃ 400MHz): 0.9 (3H, t), 1.4 (2H, m), 1.6 (6H, m), 1.7 (2H, m), 1.85 (2H, m), 2.0 (2H, m), 2.4 (6H, m), 3.1 (2H, m), 3.35 (2H, t), 3.5 (1H, s), 4.3-4.4 (1H, m), 6.85 (1H, d), 6.9 (1H, d), 7.15 (1H, s), 8.3 (1H, s).
MH⁺ 372 and 373.

### Example 6. 6-Methyl-1-{1-[trans-4-(propyloxy)cyclohexyl]-4-piperidinyl}-1,3-dihydro-2H-benzimidazol-2-one hydrochloride (E6)

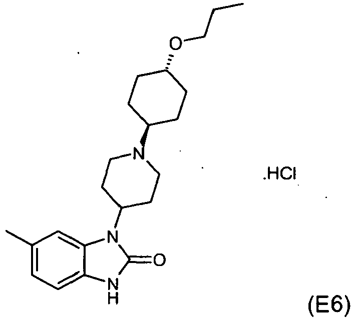

A stirred solution of (2-amino-5-methylphenyl){1-[*trans*-4-(propyloxy)cyclohexyl]-4-piperidinyl}amine (D24, 310mg, 0.89 mmol) and diisopropylethylamine (0.23ml, 0.36 mmol, 1.5eq) in dichloromethane (20ml) at 0°C under argon was treated with solid triphosgene (107mg,0.4eq) and maintained at 0°C for 30 minutes. The mixture was treated with water and extracted with dichloromethane. The combined extract was dried on MgSO₄ and concentrated under vacuum to leave 330mg of title compound as a brown solid. This material was dissolved in dichloromethane, treated with 1M HCl/diethyl ether (5ml) and concentrated under vacuum to give the hydrochloride salt as a white solid (360g, 99% yield).
¹H NMR (HCl salt) δ (CD₃OD 400MHz): 0.95 (3H, t), 1.3-1.4 (2H, m), 1.5-1.7 (4H, m), 2.05 -2.3 (6H, m), 2.4 (3H, m), 2.75-2.9 (2H, m), 3.25-3.4 (4H, m), 3.45 (2H, t), 3.6-3.7 (2H, m), 4.5-4.6 (1H, m), 6.9 (2H, m), 7.15 (1H, m).
MH⁺ 372 and 373.

### Example 7. 6-Methyl-1-(1-{trans-4-[(1-methylethyl)oxy]cyclohexyl}-4-piperidinyl)-1,3-dihydro-2H-benzimidazol-2-one hydrochloride (E7)

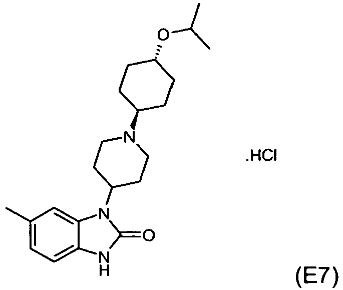

A stirred solution of (2-amino-5-methylphenyl)(1-{*trans*-4-[(1-methylethyl)oxy]cyclohexyl}-4-piperidinyl)amine (D31, 187mg, 0.54mmol) and diisopropylethylamine (0.144ml, 0.81mmol) in dichloromethane (15ml) at 0°C under argon was treated with solid triphosgene (64mg, 0.22mmol) and maintained at 0°C for 45 minutes. The mixture was treated with water (10ml) followed by dil. NaHCO₃ solution (15ml) and extracted with dichloromethane (2 x 20ml). The combined extract was dried (Na₂SO₄) and concentrated under vacuum. Trituration of the residue with 1:1 ethyl acetate/diethyl ether (8ml) produced a white solid which was filtered off after 15 minutes, washed with cold 1:1 ethyl acetate/diethyl ether and dried at 50°C under vacuum to afford a white solid. This material was dissolved in dichloromethane (2ml) and methanol (0.3ml), treated with 1M HCl/diethyl ether (0.60ml) and concentrated under vacuum. The residue was triturated with diethyl ether (5ml) to give a white solid, which was filtered off, washed with diethyl ether and dried to afford the title compound as a white solid.
¹H NMR (HCl salt) δ (d⁶DMSO, 400 MHz): 1.07 (6H, d), 1.12-1.28 (2H, m), 1.48-1.63 (2H, m), 1.78-1.90 (2H, m), 1.97-2.08 (2H, m), 2.10-2.20 (2H, m), 2.32 (3H, s), 2.82-3.00 (2H, m), 3.12-3.35 (4H, m), 3.42-3.55 (2H, m), 3.62-3.75 (1H, m), 4.50-4.65 (1H, m), 6.79 (1H, d), 6.86 (1H, d), 7.61 (1H, s), 10.78 (1H, s), 10.85 (1H, br s).

### Example 8. 6-Methyl-1-{1-[cis-1-methyl-4-(methyloxy)cyclohexyl]-4-piperidinyl}-1,3-dihydro-2H-benzimidazol-2-one hydrochloride (E8)

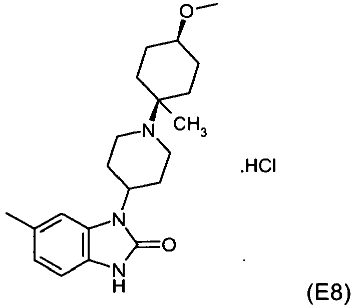

(2-Amino-5-methylphenyl){1-[*cis*-1-methyl-4-(methyloxy)cyclohexyl]-4-piperidinyl}amine (D36, 91 mg, 0.27 mmol) was dissolved in dichloromethane (5 ml) and cooled to 0 °C; bis(trichloromethyl) carbonate (0.4 eq., 0.11 mmol, 31 mg) was then added followed by Hunig's base diisopropylethylamine (1.5 eq., 68µl) at 0 °C; the mixture was stirred at the same temperature for 1 hour and then it was quenched with NaHCO₃ (aqueous saturated solution). The two phases were separated and the organic phase was dried over Na₂SO₄, filtered and the solvent was evaporated to afford the crude product, which was purified by chromatography (MeOH-NH₃-DCM) which gave the title compound, 76 mg, 80%, 99.7% = isomeric purity. The title compound was subsequently converted into the hydrochloride salt using HCl solution (1M in diethyl ether, 2 eq.).
¹H NMR δ (d⁶DMSO, 400 MHz) 0.851 (3H, s), 1.139 (2H, t), 1.585 (4H, m), 1.669 (2H, d), 1.884 (2H, d), 2.141 (2H, t), 2.262 (2H, t), 2.316 (3H, s), 3.011 (2H, d), 3.241 (3H, s), 4.020 (1H, m br), 6.768 (1H, d), 6.836 (1H, d), 6.994 (1H, s) 10.7 (1H, s br).

### Example 9. 6-Methyl-1-{1-[trans-1-methyl-4-(methyloxy)cyclohexyl]-4-piperidinyl}-1,3-dihydro-2H-benzimidazol-2-one hydrochloride (E9)

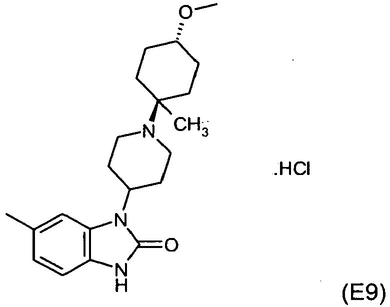

(2-Amino-5-methylphenyl){1-[*trans*-1-methyl-4-(methyloxy)cyclohexyl]-4-piperidinyl}amine (D37, 30 mg, 0.091 mmol) was dissolved in dichloromethane (2 ml) and cooled to 0°C. Bis(trichloromethyl) carbonate (0.4 eq., 0.036 mmol, 11 mg) was then added followed by Hunig's base diisopropylethylamine (1.5 eq., 23 µl) at 0°C. The mixture was stirred at the same temperature for 1 hour and then it was quenched with NaHCO₃ (aqueous saturated solution). The two phases were separated and the organic phase was dried over Na₂SO₄, filtered and the solvent was evaporated to afford the crude product, which was purified by chromatography (MeOH-NH₃-DCM) which gave the title compound, 9 mg, 28%, 97.8% = isomeric purity. The title compound was subsequently converted into the hydrochloride salt using HCl solution (1M in diethyl ether, 2 eq.).
¹H NMR δ (d⁶DMSO, 400 MHz) 0.851 (3H, s), 1.503 (6H, m), 1.663 (2H, d br), 1.794 (2H, m br), 2.157 (2H, t), 2.272 (2H, t), 2.320 (3H, s), 3.045 (2H, d), 3.161 (3H, s), 4.027 (1H, m br), 6.768 (1H, d), 6.836 (1H, d), 7.000 (1H, s), 10.7 (1H, s br).

### Example 10. 6-Methyl-1-(1-{cis-4-[(1-methylethyl)oxy]cyclohexyl}-4-piperidinyl)-1,3-dihydro-2H-benzimidazol-2-one hydrochloride (E10)

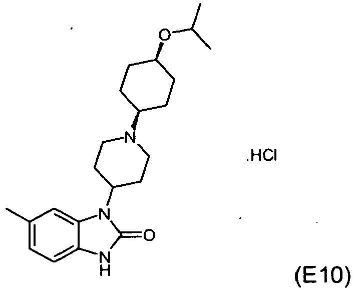

A stirred solution of (2-amino-5-methylphenyl)(1-{*cis*-4-[(1-methylethyl)oxy]cyclohexyl}-4-piperidinyl)amine (D39, 170mg, 0.49mmol) in dichloromethane (15ml) at 0°C under argon was treated with diisopropylethylamine (0.13ml), 0.74mmol) followed by solid triphosgene (57mg, 0.196mmol) and maintained at 0°C for 1h. The mixture was treated with water (10ml) followed by dil. NaHCO₃ solution (15ml) and extracted with dichloromethane (2 x 25ml). The combined extract was dried (Na₂SO₄) and concentrated under vacuum. The residue was dissolved in dichloromethane (4ml), treated with 1M HCl/diethyl ether (0.7ml) and concentrated under vacuum. The residue was triturated with diethyl ether (10ml) to give a white solid, which was filtered off, washed with diethyl ether and dried at 50°C under vacuum to afford the hydrochloride salt a white solid (170mg, 85%).
¹H NMR (HCl salt) δ (d⁶DMSO, 400 MHz): 1.10 (6H, d), 1.39-1.52 (2H, m), 1.62-1.80 (2H, m), 1.80-2.00 (6H, m), 2.32 (3H, s), 2.82-3.00 (2H, m), 3.18-3.32 (3H, m), 3.40-3.55 (2H, m), 3.55-3.70 (2H, m), 4.50-4.65 (1H, m), 6.80 (1H, d), 6.87 (1H, d), 7.60 (1H, s), 10.73 (1H, br s), 10.80 (1H, s).

### Examples 11 and 12. cis and trans 1-(1-[4-(Ethoxyl)cyclohexyl]-4-piperidinyl)-1,3-dihydro-2H-benzimidazol-2-one hydrochloride (cis = E11; trans = E12)

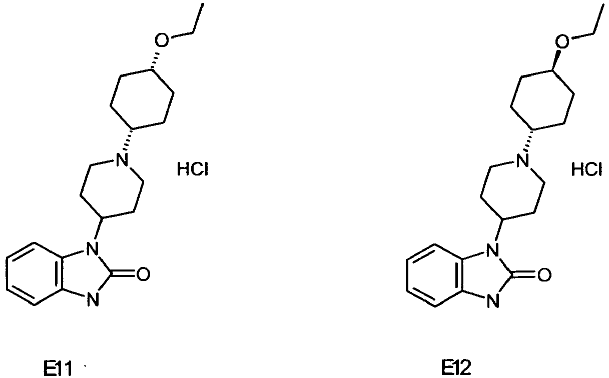

A microwave vessel was charged with 4-(2-oxo-1-benzimidazolinyl)piperidine (0.217g, 1mmol), 4-ethoxycyclohexanone (D10, 0.22g, 1.55mmol), acetic acid (0.2ml), dichloromethane (15ml) and (polystyrymethyl)trimethylammonium cyanoborohydride (4.3meq/g) (750mg) and heated at 110°C for 20 minutes in a microwave reactor. Further 4-ethoxycyclohexanone (146mg, 1.0mmol) and (polystyrymethyl)trimethylammonium cyanoborohydride (300mg) were added and mixture heated at 110°C for a further 20 minutes in a microwave reactor. The cooled mixture was filtered under vacuum and the filtrate loaded onto a 5g SCX cartridge, which was eluted with methanol followed by methanolic ammonia. The solvent was removed from the methanolic ammonia fraction to give 260mg of a clear oil, which was purified using a Waters Xbridge chromatography column and a gradient of aqueous ammonium bicarbonate (10mmolar; adjusted to pH10 with ammonia) and acetonitrile as the mobile phase to give the *cis* and *trans* isomers. Once obtained each isomer was redissolved in dichloromethane (2ml), and 1 molar HCl in diethyl ether (0.5ml) added, then the solvent was removed to afford the title compounds.

Fast eluting isomer (*trans* isomer; E12) was obtained as an off white solid (0.024g). MH⁺ = 344.
¹HNMR δ d⁶DMSO, 250MHz): 1.1-1.4 (assume 8H, m), 1.65 (assume 2H, m), 1.8 (assume 2H, m), 2.0 (assume 2H, m), 2.2-2.4 (assume 6H, m), 2.9 (assume 2H, d), 3.1-3.2 (assume 1H, m), 3.4-3.5 (assume 2H, q), 4.0-4.2 (assume 1H, m), 7.0 (assume 3H, m), 7.2 (assume 1H, m).

Slow eluting isomer (*cis* isomer; E11) was obtained as a white solid (0.026g). MH+ = 344.
¹HNMR δ CDCl3, 250MHz): 1.29 (assume 3H, t), 1.25-1.5 (assume 3H, m), 1.6-2.05 (m), 2.3-2.5 (assume 5H, m), 3.0-3.1 (assume 2H, m), 3.4-3.6 (assume 4H, m), 4.25-4.45 (assume 1H, m), 7.0-7.1 (assume 3H, m), 7.3 (assume 1H, m), 8.05 (assume 1H, s).

### Example 13. 1-{1-[trans-4-(Ethyloxy)cyclohexyl]-4-piperidinyl}-6-fluoro-1,3-dihydro-2H-benzimidazol-2-one hydrochloride (E13)

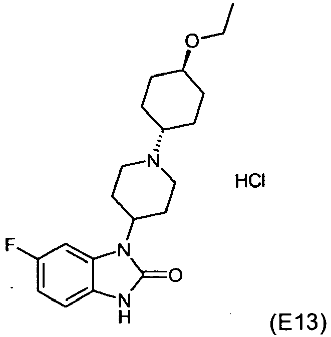

A stirred solution of *N*²-{1-[*trans*-4-(ethyloxy)cyclohexyl]-4-piperidinyl}-4-fluoro-1,2-benzenediamine (D72, ≤0.33mmol) in dichloromethane (10ml) at 0°C under argon was treated with diisopropylethylamine (0.087ml, 0.49mmol) followed by the addition of solid triphosgene (38mg, 0.13mmol) and maintained for 30mins. The mixture was treated with dil. NaHCO₃ solution (10ml) and extracted with dichloromethane (2x20ml). The combined extract was dried (Na₂SO₄) and concentrated under vacuum to leave a beige solid, which was recrystallised from EtOAc/Et2O to afford the free base of the title compound as a white solid (90mg, ≥76%). This was dissolved in dichloromethane (2ml), treated with 1 M HCl/Et₂O (0.30ml) and concentrated under a stream of argon to afford the title compound as a white solid MH⁺ = 362.
¹H NMR (free base) δ (CDCl₃, 400MHz): 1.15-1.42 (assume 7H, t + m), 1.82 (2H, br m), 1.92 (2H, br m), 2.12 (2H, br m), 2.30-2.50 (5H, m), 3.10-3.13 (2H, m), 3.13-3.35 (1H, m), 3.52 (2H, q), 4.25-4.40 (1H, m), 6.73-6.80 (1H, m), 6.96-7.02 (1H, m), 7.05 (1H, br d), 9.83 (1H, s).

### Example 14. 6-Bromo-1-{1-[trans-4-(ethyloxy)cyclohexyl]-4-piperidinyl}-1,3-dihydro-2H-benzimidazol-2-one hydrochloride (E14)

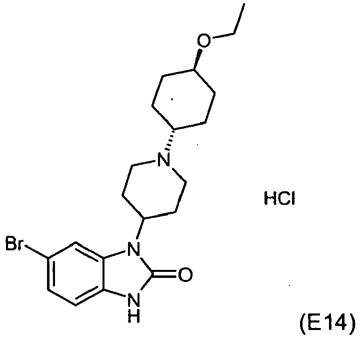

A stirred solution of 4-bromo-*N²*-{1-[*trans*-4-(ethyloxy)cyclohexyl]-4-piperidinyl}-1,2-benzenediamine (D74, 600mg, 1.5mmol) and diisopropylethylamine (0.4ml, 2.25mmol) in dichloromethane (50ml) at 0°C was treated with triphosgene (178mg, 0.6mmol) and stirred for 30mins. The mixture was washed with water and saturated NaHCO₃ solution, then extracted with dichloromethane. The extract was dried (Na₂SO₄) and concentrated under vacuum to leave a white solid, which was washed with 10% Et₂O/petrol ether to afford the free base of the title compound as a white solid (426mg, 67%). A portion of this (226mg) was dissolved in dichloromethane (10ml), treated with 1M HCl/Et₂O (5ml), stirred for 30mins, then concentrated under vacuum to afford the title compound as a white solid (220mg). MH+ = 423, 424.
¹H NMR δ (d⁶DMSO, 400MHz): 1.09 (3H, t), 1.12-1.30 (2H, m), 1.48-1.62 (2H, m), 1.90 (2H, br d), 2.05-2.18 (4H, m), 2.70-2.85 (2H, m), 3.15-3.30 (4H, m), 3.41-3.55 (4H, m), 4.53-4.65 (1H, m), 6.95 (1H, d), 7.16 (1H, dd), 7.66 (1H, d), 10.35 (1H, br m), 11.11 (1H, s).

### Example 15. 1-{1-[cis-4-(Ethyloxy)cyclohexyl]-4-piperidinyl}-6-(trifluoromethyl)-1,3-dihydro-2H-benzimidazol-2-one monohydrochloride (E15)

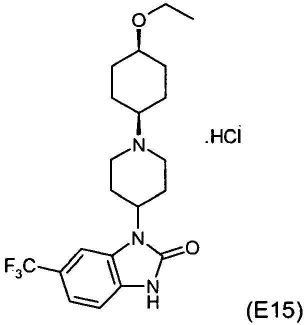

To a solution of 1-(4-piperidinyl)-6-(trifluoromethyl)-1,3-dihydro-2*H*-benzimidazol-2-one (D141, 0.63 mmol, 180 mg) in dichloroethane (5 ml), 4-(ethyloxy)cyclohexanone (D10, 4 eq., 2.52 mmol, 358 mg), Et₃N (a few drops) and 5A molecular sieves were added. The reaction was left stirring overnight. NaBH(OAc)₃ (1.5 eq., 0.9464 mmol, 0.2g) was added and the reaction left stirring for four overnights. The mixture was basified with 2M NaOH (1 ml). The reaction was quenched with NaHCO₃ and the aqueous solution was extracted with dichloromethane The organics were combined and washed with water (2x50ml), dried over Na₂SO₄, filtered and the solvent was evaporated to afford the crude product. The crude product was purified by chromatography (10% MeOH /dichloromethane) on silica column followed by another column chromatography (11% - 13% EtOAc in nhex) on Biotage KP-NH^{™}-silica column to yield to afford the free base of the title compound, 23 mg, 9%. M⁺ + H = 412. This was subsequently converted into the title compound using HCl solution (1 M in Et₂O) to afford 23 mg.
¹H NMR δ (d⁶DMSO, 400MHz) 1.111 (3H, t), 1.397 (2H, m), 1.694 (2H, q), 1.937 (6H, m), 2.782 (2H q), 3.240 (3H, m), 3.404 (2H, q), 3.536 (3H, m), 4.626 (1H m), 7.172 (1H, d), 7.373 (1H, d), 7.707 (1H, s), 10.05 (1H, s br), 11.45 (1H, s).

### Example 16 6-Ethyl-1-{1-[trans-4-(ethyloxy)cyclohexyl]-4-piperidinyl}-1,3-dihydro-2H-benzimidazol-2-one hydrochloride (E16)

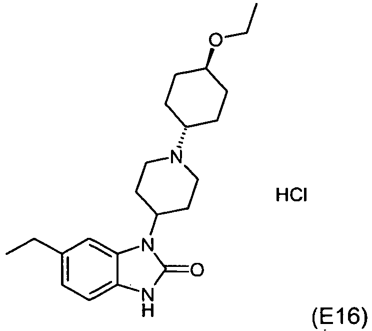

A stirred solution of 4-ethyl-*N*²-{1-[*trans*-4-(ethyloxy)cyclohexyl]-4-piperidinyl}-1,2-benzenediamine (D76, 348mg, 1mmol) and diisopropylethylamine (0.17ml, 1.5mmol) in dichloromethane (25ml) at 0°C was treated with triphosgene (118mg, 0.4mmol) and stirred at 0°C for 30mins. The mixture was washed with water and saturated NaHCO₃ solution, then extracted with dichloromethane. The extract was dried (Na₂SO₄) and concentrated under vacuum to leave a pale yellow solid. This was dissolved in dichloromethane (5ml), treated with HCl/Et₂O (3ml) and concentrated under vacuum to afford the title compound as a white solid (260mg, 65%). MH+ = 372.
¹H NMR δ (d⁶DMSO, 400MHz): 1.05-1.33 (2H, m), 1.10 (3H, t), 1.21 (3H, t), 1.47-1.63 (2H, m), 1.85 (2H, br d), 2.05-2.25 (4H, m), 2.50-2.68 (2H, m), 2.80-3.00 (2H, m), 3.13-3.30 (4H, m), 3.35-3.55 (4H, m), 4.50-4.62 (1H, m), 6.80-6.90 (2H, m), 7.54 (1H, s), 10.7 (1H, br m), 10.80 (1H, s).

### Example 17 6-Cyclopropyl-1-{1-[trans-4-(ethyloxy)cyclohexyl]-4-piperidinyl}-1,3-dihydro-2H-benzimidazol-2-one hydrochloride (E17)

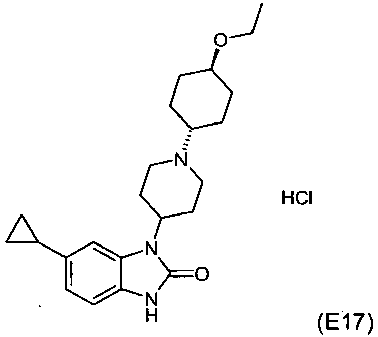

A stirred solution of 4-cyclopropyl-*N*²-{1-[*trans*-4-(ethyloxy)cyclohexyl]-4-piperidinyl}-1,2-benzenediamine (D78, 200mg, 0.56mmol) and diisopropylethylamine (0.14ml, 0.84mmol) in dichloromethane (20ml) at 0°C was treated with triphosgene (66mg, 0.23mmol) and stirred for 30mins at 0°C. The solution was washed with water and sat. NaHCO₃ solution, then extracted with dichloromethane. The extract was dried (Na₂SO₄) and concentrated under vacuum. The residue was dissolved in dichloromethane (10ml), treated with 1M HCl/Et₂O (3ml), stirred for 30mins, then concentrated under vacuum to afford the title compound as a white solid (165mg, 72%). MH+ = 384.
¹H NMR δ (d⁶DMSO, 400MHz): 0.70-0.77 (2H, m), 0.84-0.94 (2H, m), 1.09 (3H, t), 1.10-1.30 (2H, m), 1.48-1.62 (2H, m), 1.85 (2H, br d), 1.88-2.00 (1H, m), 2.04-2.20 (4H, m), 2.78-2.95 (2H, m), 3.15-3.30 (4H, m), 3.40-3.55 (4H, m), 4.50-4.62 (1H, m), 6.71 (1H, d), 6.85 (1H, d), 7.28 (1H, s), 10.6 (1H, br m), 10.78 (1H, s).

### Example 18. 1-{1-[trans-4-(Ethyloxy)cyclohexyl]-4-piperidinyl}-6-(methyloxy)-1,3-dihydro-2H-benzimidazol-2-one hydrochloride (E18)

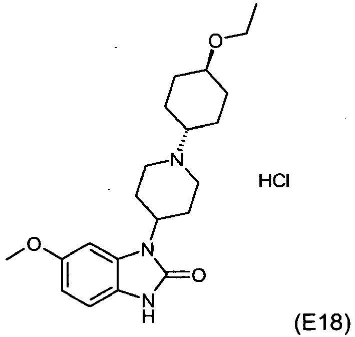

A stirred solution of *N*²-{1-[*trans*-4-(ethyloxy)cyclohexyl]-4-piperidinyl}-4-(methyloxy)-1,2-benzenediamine (D80, 120mg, 0.35mmol) in dichloromethane (10ml) at 0°C under argon was treated with diisopropylethylamine (0.092ml, 0.52mmol) followed by the addition of solid triphosgene (40mg, 0.14mmol) and maintained for 30mins. The mixture was treated with 10% Na₂CO₃ solution (10ml) and extracted with dichloromethane (2x15ml). The combined extract was dried (Na₂SO₄) and concentrated under vacuum to leave a beige solid, which was recrystallised from 1:1 EtOAc/Et₂O to afford the free base of the title compound as a white solid (105mg, 81%). This was dissolved in dichloromethane (5ml), treated with 1 M HCl/Et₂O (0.3ml) and concentrated under vacuum to afford the title compound as a white solid (110mg) MH⁺ = 374.
¹H NMR δ (d⁶DMSO, 400MHz): 1.07 (3H, t), 1.10-1.30 (4H, m), 1.47-1.67 (2H, m), 1.80-1.92 (2H, m), 2.03-2.23 (4H, m), 2.80-3.00 (2H, m), 3.12-3.32 (4H, m), 3.40-3.65 (assume 2H), 3.79 (3H, s), 4.50-4.67 (1H, m), 6.58 (1H, d), 6.87 (1H, d), 7.26 (1H, s), 10.71 (1H, s), 10.8 (1H, br m).

### Examples 19 and 20. cis and trans-1-{1-[4-(Ethoxy)cyclohexyl]-4-piperidinyl}-6-[(trifluoromethyl)oxy]-1,3-dihydro-2H-benzimidazol-2-one (cis = E19; trans = E20)

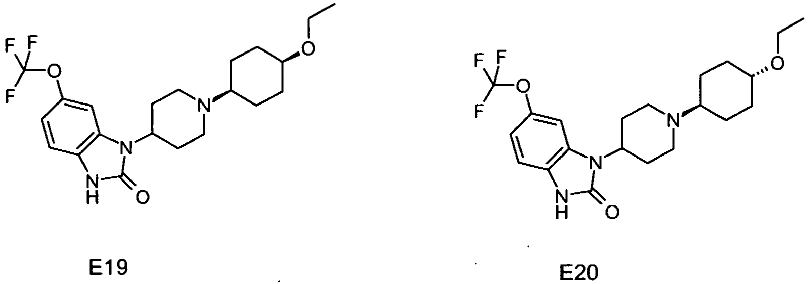

A microwave vessel was charged with 1-(4-piperidinyl)-6-[(trifluoromethyl)oxy]-1,3-dihydro-2*H*-benzimidazol-2-one (D83, 337mg), 4-(ethoxy)cyclohexanone (D10, 190mg) (polystyrylmethyl)trimethylammonium cyanoborohydride (4.3meq/g 0.6g), sodium acetate (100mg), dichloromethane (10ml), acetic acid (200µl) and heated at 110°C for 20 minutes in a microwave reactor. Further 4-(ethoxy)cyclohexanone (D10, 0.06g) and (polystyrylmethyl)trimethylammonium cyanoborohydride (4.3meq/g 0.6g) were added and heated at 110°C for 20 minutes in a microwave reactor. The cooled mixture was filtered and the filtrate was loaded onto a SCX cartridge which was eluted with methanol followed by methanolic ammonia. The methanolic ammonia fraction was evaporated and the resulting mixture was purified using a Waters Xbridge chromatography column. A gradient of aqueous ammonium bicarbonate (10mmolar adjusted to pH10 with ammonia) and acetonitrile was used as the mobile phase to give the *cis* and *trans* isomers. Once obtained each isomer was redissolved in dichloromethane (2ml), and 1 molar HCl in diethyl ether (0.5ml) added, the solvent was then removed.

Fast eluting isomer *(trans* isomer E20) was obtained as a white solid (0.035g). MH⁺ =.428
¹H NMR δ (DMSO, 250 MHz): 1.1 (3H, t), 1.1-1.3 (2H ,m), 1.4-1.7 (2H, m), 1.8-2.0 (2H, m), 2.0-2.2 (4H, m), 2.7-2.9 (2H, m), 3.1-3.4 (4H, m), 3.4-3.6 (4H, m), 4.5-4.7 (1H, br m), 6.95-7.1 (2H, m), 7.15 (1H, s), 10.4 (1H, br), 11.2 (1H, s).

Slow eluting isomer (cis isomer E19) was obtained as a white solid (0.015g). MH⁺ = 428
¹H NMR δ (DMSO, 250 MHz): 1.1 (3H, t), 1.35-1.5 (2H, m), 1.6-1.8 (2H, m), 1.8-2.1 (6H, m), 2.6-2.8 (2H, m), 3.1-3.6 (obs, m), 4.5-4.7 (1H, m), 7.0-7.1 (2H, m), 7.5 (1H, s), 9.6-9.8 (1H, br), 11.2 (1H, s).

### Example 21. cis/trans-6-Methyl-1-[1-(4-trifluoromethoxycyclohexyl)-4-piperidinyl]-1,3-dihydro-2H-benzimidazol-2-one hydrochloride (E21)

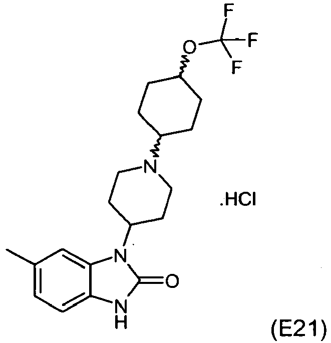

A mixture of 8-[(trifluoromethyl)oxy]-1,4-dioxaspiro[4.5]decane (D85, 400mg), THF (5ml), and 5M aqueous hydrochloric acid (5ml) was stirred at room temperature for 2h then partitioned dichloromethane / water. The organic layer was then dried and evaporated to give the crude ketone. This crude ketone was combined with 1-(4-piperidinyl)-6-methyl-1,3-dihydro-2*H*-benzimidazol-2-one (D145, 110mg, 0.5mmol), (polystyrylmethyl)trimethylammonium cyanoborohydride (600mg of 2.04mmol/g, 1.25mmol), dichloromethane (2.5ml), and acetic acid (0.15ml, 2.5mmol) and heated at 110°C for 10 minutes in a microwave reactor. The cooled mixture was filtered and the filtrate was loaded onto a SCX cartridge which was eluted with methanol followed by methanolic ammonia. The methanolic ammonia fraction was evaporated and the resulting mixture was purified using a Waters Xbridge chromatography column. A gradient using aqueous ammonium bicarbonate (10mmolar) adjusted to pH10 with ammonia and acetonitrile was used as the mobile phase to give the title compound, which was converted to the hydrochloride salt and crystallised from diethyl ether as a mixture of isomers, 42mg.
1 HNMR (HCl salt) δ (d⁶DMSO): 1.5-2.2 (9H, m), 2.35 (3H, s), 2.8 (2H, m), 3.2-3.6 (11H, m), 4.4 (0.4H, m), 4.55 (1H, m), 4.7 (0.6H,br s), 6.8-6.9 (2H, m), 7.5 (1H, 2s), 10.55 (0.6H, br s), 10.7 (0.4H, br s), 10.8 (1H, 2s), MH+ 398.

### Example 22. 1-(1-{cis-4-[(Cyclopropylmethyl)oxy]cyclohexyl}-4-piperidinyl)-6-methyl-1,3-dihydro-2H-benzimidazol-2-one hydrochloride (E22)

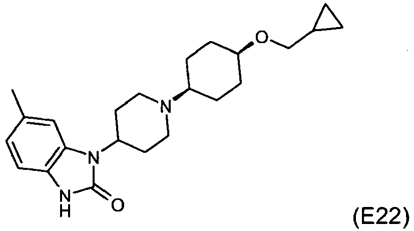

A stirred solution of *N*²-(1-{*cis*-4-[(cyclopropylmethyl)oxy]cyclohexyl}-4-piperidinyl)-4-methyl-1,2-benzenediamine (D91, 600mg) and diisopropylethylamine (0.6ml) in dichloromethane (20ml) at 0°C under argon was treated with solid triphosgene (0.2g, 0.67mmol) in one portion and stirred to room temperature overnight. The mixture was diluted with dichloromethane, washed with dil. NaHCO₃ solution, brine and dried with Na₂SO₄ and solvent removed and the residue was chromatographed on silica gel eluted with dichloromethane - methanolic ammonia 0-10%. The product was dissolved in methanol, treated with 1 M HCl/diethylether and concentrated under vacuum to give the hydrochloride salt of the title compound as a white solid (38mg). MH⁺ = 384.
¹H NMR δ (DMSO, 400 MHz): 0.2 (2H, m), 0.5 (2H, m), 1.05 (1H, m), 1.4 (2H , m), 1.7 (2H, m), 1.9 (4H, m), 2.0 (2H, m), 2.33 (3H, s), 2.75 (2H, m), 3.3 (obs, m), 3.55 (3H, m), 4.55, (1H, m), 6.79 (1H, d), 6.88 (1H, d) 7.33 (1H, s), 9.75 (1H, m), 10.8 (1H, s).

### Example 23. 1-(1-{trans-4-[(Cyclopropylmethyl)oxy]cyclohexyl}-4-piperidinyl)-6-methyl-1,3-dihydro-2H-benzimidazol-2-one (E23)

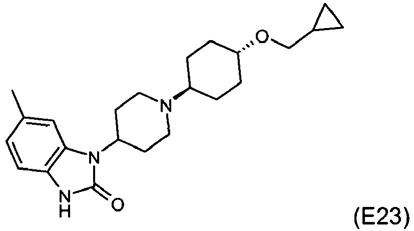

A stirred solution of *N*²-(1-{*trans*-4-[(cyclopropylmethyl)oxy]cyclohexyl}-4-piperidinyl)-4-methyl-1,2-benzenediamine (D92, 380mg, 1.06mmol) and diisopropylethylamine (0.3ml) in dichloromethane (20ml) at 0°C under argon was treated with solid triphosgene (126mg, 0.42mmol) and stirred to room temperature overnight. The mixture was diluted with dichloromethane, washed with dil. NaHCO₃ solution, brine and dried with Na₂SO₄ and solvent removed. The title compound was obtained as a white solid from ethanol (270mg). This material was dissolved in methanol/dichloromethane, treated with 1 M HCl/diethylether and concentrated under vacuum to give the hydrochloride salt of the title compound as a white solid (270mg). MH⁺ = 384.
¹H NMR δ (DMSO, 400 MHz): 0.15 (2H, m), 0.45 (2H, m), 0.95 (1H, m), 1.2 (2H , m), 1.55 (2H, m), 1.85 (2H, m), 2.1 (4H, m), 2.32 (3H, s), 2.85 (2H, m), 2.9 (2H, m), 3.22 (obs, m), 3.5 (2H, m), 4.55, (1H, m), 6.80 (1H, d), 6.87 (1H, d) 7.51 (1H, s), 10.6 (1H, m), 10.8 (1H, s).

### Example 24. trans-6-Methyl-1-[1-(4-cyclopropyloxycyclohexyl)-4-piperidinyl]-1,3-dihydro-2H-benzimidazol-2-one hydrochloride (E24)

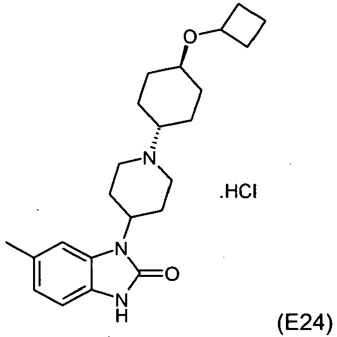

A solution of *trans*-5-methyl-*N*-[1-(4-cyclobutyloxycyclohexyl)-4-piperidinyl]-1,2-benzenediamine (D98, 50mg, 0.14mmol) in dichloromethane (3ml) at 0°C was treated with triphosgene (20mg, 0.4eq) then diisopropylethylamine (0.04ml, 1.5eq), and then maintained at 0°C for 1h. The mixture was partitioned between dichloromethane and washed with aqueous sodium bicarbonate. Drying, evaporation and crystallisation gave the title compound, isolated as the hydrochloride salt also crystallised from diethyl ether, 31mg.
1 HNMR (HCl salt) δ (d⁶DMSO): 1.2 (2H, m), 1.5 (4H, m), 1.9 (3H, m), 2.15 (5H, m), 2.35 (3H, s), 2.8 (2H, m), 3.2 (4H, m), 3.5 (2H, m), 4.0 (1H, m), 4.6 (1H, m), 6.8 (2H, m), 7.4 (1H, s), 10.2 (1H, br s), 10.8 (1H, s), MH+ 384.

### Examples 25 and 26. cis and trans-1-(1-[4-(Propyloxy)cyclohexyl]-4-piperidinyl)-1,3-dihydro-2H-benzimidazol-2-one hydrochloride (cis = E25; trans = E26)

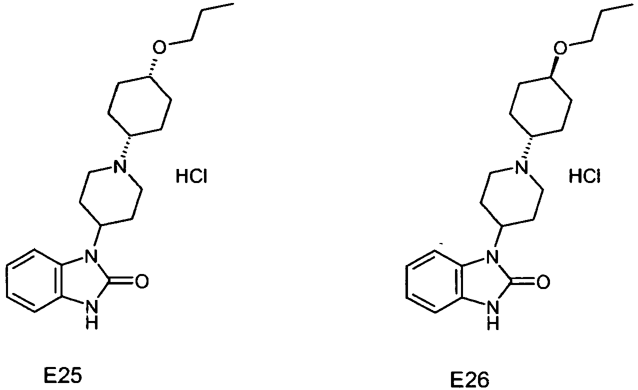

A microwave vessel was charged with 4-(2-oxo-1-benzimidazolinyl)piperidine (0.217g, 1mmol), 4-propoxycyclohexanone (D19, 0.22g, 1.41mmol), acetic acid (0.2ml), dichloromethane (15ml) and (polystyrymethyl)trimethylammonium cyanoborohydride (4.3meq/g) (0.75g) and heated at 110°C for 20 minutes in a microwave reactor. Further 4-propoxycyclohexanone (0.156g, 1.0mmol) and (polystyrymethyl)trimethylammonium cyanoborohydride (300mg) added and the mixture heated at 110°C for further 20 minutes in a microwave reactor. The cooled mixture was filtered under vacuum and the filtrate loaded onto a 5g SCX cartridge, which was eluted with methanol followed by methanolic ammonia. The solvent was removed from the methanolic ammonia fraction to give 300mg of clear crystals, which was purified using a Waters Xbridge chromatography column and a gradient of aqueous ammonium bicarbonate (10mmolar; adjusted to pH10 with ammonia) and acetonitrile as the mobile phase to give the *cis* and *trans* isomers. Once obtained each isomer was redissolved in dichloromethane (2ml), and 1 molar HCl in diethyl ether (0.5ml) added, then the solvent was removed to afford the title compounds.

Fast eluting isomer *(trans* isomer; E26) was obtained as a white solid (44mg). MH⁺ = 358
¹HNMR δ d⁶DMSO, 250MHz): 0.86 (3H, t), 1.1-1.3 (3H, m), 1.4-1.7 (4H, m), 1.8-2.0 (2H, m), 2.0-2.2 (4H, d), 2.7-2.9 (2H, m), 3.1-3.5 (assume 5H, m), 3.5-3.6 (2H, m), 4.5-4.7 (1H, m), 7.0 (3H, m), 7.4-7.6 (1H, m), 10.0 (1H, s), 10.9 (1H, s).

Slow eluting isomer (*cis* isomer; E25) was obtained as a white solid (15mg). MH⁺ = 358
¹HNMR *δ d⁶DMSO, 250MHz): 0.9 (3H, t), 1.35-2.1 (assume 13H, br m), 2.6-2.9 (2H, br s), 3.1-3.45 (assume 5H, br m), 3.5 (2H, br s), 4.45-4.7 (1H, br m), 7.0 (3H, s), 7.5 (1H, br m), 10.0 (1H, brs), 10.9 (1H, s).

### Example 27 6-Bromo-1-{1-[trans-4-(propyloxy)cyclohexyl]-4-piperidinyl}-1,3-dihydro-2H-benzimidazol-2-one hydrochloride (E27)

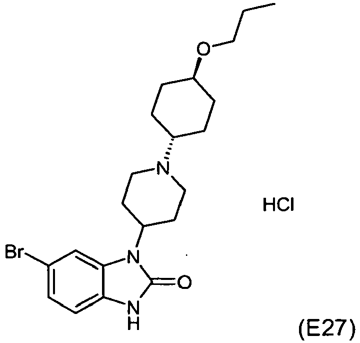

A stirred solution of 4-bromo-*N*²-{1-[*trans*-4-(propyloxy)cyclohexyl]-4-piperidinyl}-1,2-benzenediamine (D100, 574mg, 1.4mmol) and diisopropylethylamine (0.35ml, 2.1mmol) in dichloromethane (50ml) at 0°C was treated with triphosgene (166mg, 0.56mmol) and stirred at 0°C for 30mins. The mixture was washed with water and sat. NaHCO₃ solution, then extracted with dichloromethane. The extract was dried (Na₂SO₄) and concentrated under vacuum to leave a white solid, which was washed with a mixture of 10% Et₂O/petrol ether to afford the free base of the title compound (398mg, 65%). A portion of this (1 98mg) was dissolved in dichloromethane (10ml), treated with 1 M HCl/Et₂O (5ml), stirred for 30mins, then concentrated under vacuum to afford the title compound as a white solid (200mg). MH+ = 437, 438.
¹H NMR δ (d⁶DMSO, 400MHz): 0.87 (3H, t), 1.12-1.30 (2H, m), 1.42-1.63 (4H, m), 1.90 (2H, br d), 2.05-2.20 (4H, m), 2.70-2.85 (2H, m), 3.10-3.30 (4H, m), 4.43-4.54 (2H, m), 4.50-4.65 (1H, m), 6.95 (1H, d), 7.16 (1H, dd), 7.68 (1H, s), 10.32 (1H, br m), 11.12 (1H, s).

### Example 28 6-Ethyl-1-{1-[trans-4-(propyloxy)cyclohexyl]-4-piperidinyl}-1,3-dihydro-2H-benzimidazol-2-one hydrochloride (E28)

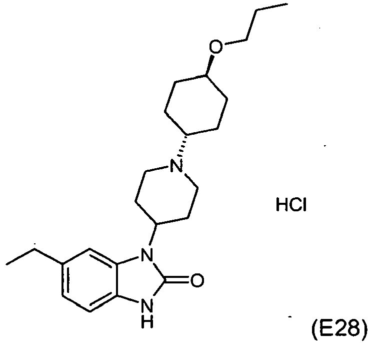

A stirred solution of 4-ethyl-*N*²-{1-[*trans*-4-(propyloxy)cyclohexyl]-4-piperidinyl}-1,2-benzenediamine (D102, 180mg, 0.5mmol) and diisopropylethylamine (0.085ml, 0.75mmol) in dichloromethane (15ml) at 0°C was treated with triphosgene (59.2mg, 0.12mmol) and stirred at 0°C for 30mins. The mixture was washed with water and sat. NaHCO₃ solution, then extracted with dichloromethane. The extract was dried (Na₂SO₄) and concentrated under vacuum to leave a white solid. This was dissolved in dichloromethane (5ml), treated with HCl/Et₂O (3ml) and concentrated under vacuum to afford the title compound as a white solid (200mg, 95%). MH+ = 386.
¹H NMR δ (d⁶DMSO, 400MHz): 0.86 (3H, t), 1.15-1.30 (5H, t + m), 1.42-1.62 (4H, m), 1.82-1.92 (2H, m), 2.08-2.18 (4H, m), 2.61 (2H, q), 2.76-2.90 (2H, m), 3.14-3.3 (4H, m), 3.38 (2H, t), 3.50 (2H, br d), 4.50-4.62 (1H, m), 6.83 (1H, d), 6.89 (1H, d), 7.43 (1H, s), 10.35 (1H, br m), 10.78 (1H, s).

### Example 29 6-Cyclopropyl-1-{1-[trans-4-(propyloxy)cyclohexyl]-4-piperidinyl}-1,3-dihydro-2H-benzimidazol-2-one hydrochloride (E29)

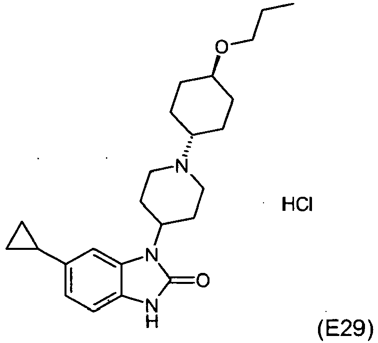

A stirred solution of 4-cyclopropyl-*N*²-{1-[*trans*-4-(propyloxy)cyclohexyl]-4-piperidinyl}-1,2-benzenediamine (D104, 70mg, 0.2mmol) and diisopropylethylamine (0.5ml) in dichloromethane (10ml) at 0°C was treated with triphosgene (28mg, 0.1 mmol) and stirred at 0°C for 30mins. The solution was washed with water and sat. NaHCO₃ solution, then extracted with dichloromethane. The extract was dried (Na₂SO₄) and concentrated under vacuum. The residue was dissolved in dichloromethane (5ml), treated with 1 M HCl/Et₂O (1ml), stirred for 30mins and concentrated under vacuum to afford the title compound (70mg, 81 %). MH+ = 398.
¹H NMR δ (d⁶DMSO, 400MHz): 0.70 -0.78 (2H, m), 0.83-0.91 (5H, t + m), 1.12-1.28 (2H, m), 1.42-1.62 (4H, m), 1.82 (2H, br d), 1.90-2.00 (1H, m), 2.10 (2H, br d), 2.18 (2H, br d), 2.85-3.00 (2H, m), 3.12-3.30 (4H, m), 3.37 (2H, t), 3.48 (2H, br d), 4.52-4.62 (1H, m), 6.70 (1H, dd), 6.85 (1H, d), 7.38 (1H, s), 10.78 (1H, s), 11.04 (1H, br m).

### Examples 30 and 31. cis and trans-1-{1-[4-(Propyloxy)cyclohexyl]-4-piperidinyl}-6-[(trifluoromethyl)oxy]-1,3-dihydro-2H-benzimidazol-2-one (cis = E30; trans = E31)

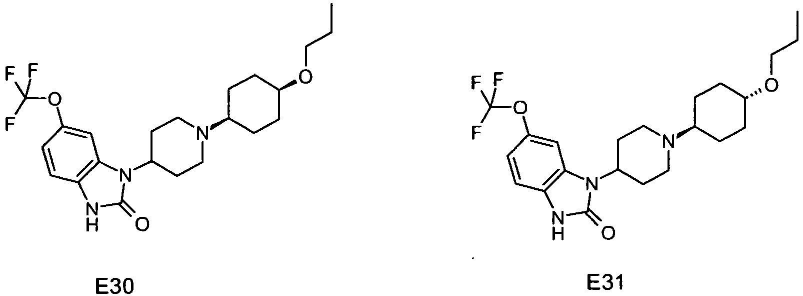

A microwave vessel was charged with 1-(4-piperidinyl)-6-[(trifluoromethyl)oxy]-1,3-dihydro-2*H*-benzimidazol-2-one (D83, 337mg), 4-(propyloxy)cyclohexanone (D19, 190mg) (polystyrylmethyl)trimethylammonium cyanoborohydride (4.3meq/g 0.6g), sodium acetate (100mg), dichloromethane (10ml), acetic acid (200µl) and heated at 110°C for a total of 30 minutes in a microwave reactor. The cooled mixture was filtered and the filtrate was loaded onto a SCX cartridge which was eluted with methanol followed by methanolic ammonia. The methanolic ammonia fraction was evaporated and the resulting mixture was purified using a Waters Xbridge chromatography column. A gradient using aqueous ammonium bicarbonate (10mmolar adjusted to pH10 with ammonia) and acetonitrile was used as the mobile phase to give the *cis* and *trans* isomers.

Fast eluting isomer *(trans* isomer; E31) was obtained as a white solid (0.071g). MH⁺ = 442.
¹H NMR δ (CDCl₃, 400 MHz): 0.91 (3H, t), 1.2-1.4 (5H , m), 1.85 (2H, m), 1.94 (2H, m), 2.11 (2H, m), 2.4 (4H, m), 3.07 (2H, m), 3.15 (1H, m), 3.41 (2H, t), 4.30 (1H, m), 6.93 (1H, d), 7.02 (1H, d) 7.16 (1H, m), 9.02 (1H, s).
¹⁹F NMR δ (d⁶DMSO) -58.24ppm

Slow eluting isomer (*cis* isomer: E30) was obtained as a white solid (0.077g). MH⁺ = 442.
¹H NMR δ (CDCl₃, 400 MHz): 0.91 (3H, t), 1.4 (2H , m), 1.55-1.75 (6H, m), 1.82 (2H, m), 2.00 (2H, m), 2.3-2.5 (4H, m), 3.07 (2H, m), 3.15 (1H, m), 3.33 (2H, t), 3.45 (1H, m), 4.30 (1H, m), 6.93 (1H, d) 7.04 (1H, d), 7.17 (1H, m), 9.16 (1H, s).
¹⁹F NMR δ (d⁶DMSO) -58.23ppm

### Example 32. 1-{1-[cis-4-(Ethyloxy)-1-methylcyclohexyl]-4-piperidinyl}-6-methyl-1,3-dihydro-2H-benzimidazol-2-one hydrochloride (E32)

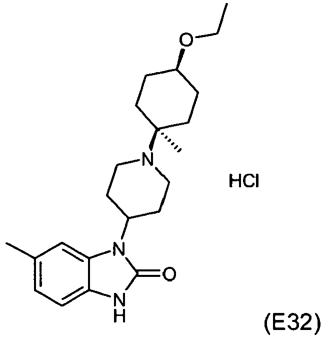

A stirred solution of *N*²-{1-[*cis*-4-(ethyloxy)-1-methylcyclohexyl]-4-piperidinyl}-4-methyl-1,2-benzenediamine (D106, 150mg, 0.44mmol) in dichloromethane (10ml) at 0°C under argon was treated with diisopropylethylamine (0.116ml, 0.65mmol) followed by the addition of solid triphosgene (51mg, 0.174mmol) and maintained at 0°C for 1.5hrs. The mixture was treated with dil. NaHCO₃ solution (10ml) and extracted with dichloromethane (2x10ml). The combined extract was dried (Na₂SO₄), concentrated under vacuum and the solid residue recrystallised from dichloromethane /EtOAc to afford the free base of the title compound as a white solid (115mg, 71 %). A portion of this material (84mg) was dissolved in a mixture of dichloromethane (2ml) and MeOH (5ml), treated with 1 M HCl/Et₂O (0.35ml) and concentrated under vacuum. Trituration with Et₂O gave a solid which was filtered off, washed with Et₂O and dried to afford the title compound as a white solid (86mg) MH⁺ = 372.
¹H NMR δ (d⁶DMSO, 400MHz): 1.13 (3H, t), 1.36 (3H, s), 1.48-1.62 (2H, m), 1.67-1.77 (2H, m), 1.80-1.92 (4H, br m), 1.92-2.05 (2H, m), 2.33 (3H, s), 2.82-3.00 (2H, m), 3.13-3.27 (2H, m), 3.41 (2H, q), 3.50 (1H, s), 3.67 (2H, br d), 4.55-4.67 (1H, m), 6.79 (1H, d), 6.87 (1H, d), 7.55 (1H, s), 9.95 (1H, br m), 10.80 (1H, s).

### Example 33. 1-{1-[trans-4-(ethyloxy)-1-methylcyclohexyl]-4-piperidinyl}-6-methyl-1,3-dihydro-2H-benzimidazol-2-one hydrochloride (E33)

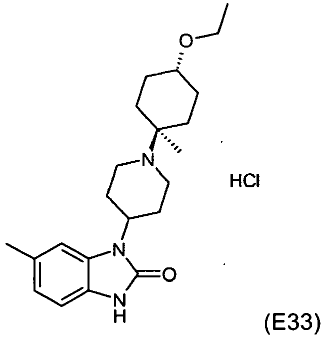

A stirred solution of *N*²-{1-[*trans*-4-(ethyloxy)-1-methylcyclohexyl]-4-piperidinyl}-4-methyl-1,2-benzenediamine (D108, 160mg, 0.464mmol) in dichloromethane (10ml) at 0°C under argon was treated with diisopropylethylamine (0.124ml, 0.696mmol) followed by the addition of solid triphosgene (55mg, 0.186mmol) and maintained at 0°C for 1.5hrs. The mixture was treated with dil. NaHCO₃ solution (10ml) and extracted with dichloromethane (2x10ml). The combined extract was dried (Na₂SO₄), concentrated under vacuum and the residue crystallised from 1:1 Et₂O/EtOAc to afford the free base of the title compound as a white solid (120mg, 70%). A portion of this material (85mg) was dissolved in a mixture of dichloromethane (3ml) and MeOH (5ml), treated with 1 M HCl/Et₂O (0.35ml) and concentrated under vacuum. Trituration of the residue with Et₂O gave a solid which was filtered off, washed with Et₂O and dried to afford the title compound as a white solid (86mg) MH⁺ = 372.
¹H NMR δ (d⁶DMSO, 400MHz): 1.11 (3H, t), 1.22-1.40 (2H, m), 1.34 (3H, s), 1.85 (2H, br d), 1.90-2.05 (6H, m), 2.32 (3H, s), 2.86-3.01 (2H, m), 3.12-3.38 (3H, m), 3.47 (2H, q), 3.65 (2H, br d), 4.54-4.68 (1H, m), 6.79 (1H, d), 6.86 (1H, d), 7.65 (1H, s), 10.40 (1H, br m), 10.76 (1H, s).

### Example 34. 6-Methyl-1-{1-[cis-1-methyl-4-(propyloxy)cyclohexyl]-4-piperidinyl}-1,3-dihydro-2H-benzimidazol-2-one monohydrochloride (E34)

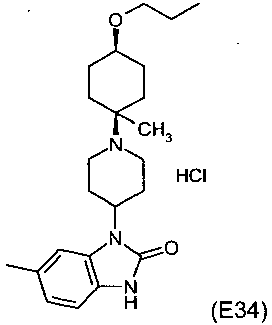

(2-Amino-5-methylphenyl){1-[*cis*-1-methyl-4-(propyloxy)cyclohexyl]-4-piperidinyl}amine (D112, 53 mg, 0.128 mmol) was dissolved in dichloromethane (5 ml) and Hunigh base diisopropylethylamine (1.5 eq., 32 microliters) was added. The mixture was then cooled to 0°C and bis(trichloromethyl) carbonate (0.4 eq., 0.05 mmol, 15 mg) was then added at 0°C portionwise. The mixture was stirred at the same temperature for 1.5 hours and then it was quenched with brine, basified to pH=10 and the aqueous obtained was extracted with EtOAc (2x). The organics were combined, dried (Na₂SO₄), filtered and the solvent was evaporated to afford the crude product which was purified by silica chromatography (MeOH-NH₃- dichloromethane) to afford the free base of the title compound (40mg, 75%). This was subsequently dissolved in dichloromethane (2 ml) and treated at room temperature with HCl solution (1M in Et₂O, 0.2 ml). The mixture was stirred at room temperature overnight and the solvent was then evaporated to afford the title compound (43 mgs, complete conversion). M⁺ + H = 385.
¹HNMR δ (DMSO, 500 MHz): 0.900 (3H, t), 1.351 (3H, s), 1.524 (4H, m), 1.728 (2H, d), 1.909 (6H, m), 2.341 (3H, s), 2.809 (2H, q), 3.177 (2H, q), 3.329 (2H, t), 3.467 (1H, s br), 3.667 (2H, d), 3.567 (1H, m), 6.805 (1H, m), 6.872 (1H, d), 7.361 (1H, s), 9.461 (1H, t broad), 10.774 (1H, s).

### Example 35. 6-Methyl-1-{1-[trans-1-methyl-4-(propyloxy)cyclohexyl]-4-piperidinyl}-1,3-dihydro-2H-benzimidazol-2-one monohydrochloride (E35)

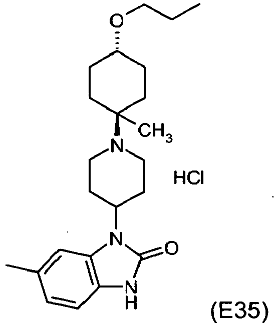

(2-Amino-5-methylphenyl){1-[*trans*-1-methyl-4-(propyloxy)cyclohexyl]-4-piperidinyl}amine (D113, 45 mg, 0.128 mmol) was dissolved in dichloromethane (5 ml) and Hunigh base (1.5 eq., 32 microliters) was added. The mixture was then cooled to 0°C and bis(trichloromethyl) carbonate (0.4 eq., 0.05 mmol, 15 mg) was then added at 0°C portionwise. The mixture was stirred at the same temperature for 1.5 hours and then it was quenched with brine, basified to pH=10 and the aqueous obtained was extracted with EtOAc (2x). The organics were combined, dried over Na₂SO₄, filtered and the solvent was evaporated to afford the crude product that was purified by silica chromatography (MeOH-NH₃- dichloromethane) to afford the free base of the title compound (50 mg, complete conversion). This was subsequently dissolved in dichloromethane (2 ml) and treated at room temperature with HCl solution (1M in Et₂O, 0.2 ml). The mixture was stirred at room temperature overnight, then the solvent was evaporated to afford the title compound (55 mgs, complete conversion). M⁺ + H = 385.
¹HNMR δ (DMSO, 500 MHz): 0.444 (3H, t), 1.287 (5H, m), 1.485 (2H, m), 1.919 (8H, m), 2.326 (3H, s), 2.903 (2H, q), 3.190 (3H, m), 3.376 (2H under the water peak), 3.640 (2H, d), 4.589 (1H, m), 6.793 (1H, d), 6.862 (1H, d), 7.578 (1H, s), 10.203 (1H, t broad), 10.760 (1H, s).

### Example 36. trans 6-Methyl-1-[1-(1-ethyl-4-propoxycyclohexyl)-4-piperidinyl]-1,3-dihydro-2H-benzimidazol-2-one hydrochloride (E36)

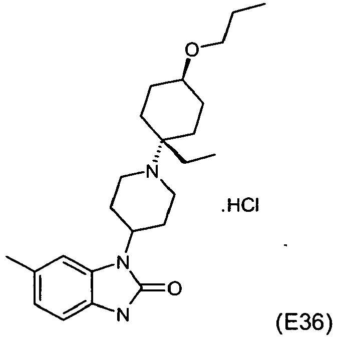

A solution of trans 5-methyl-*N*-[1-(1-ethyl-4-propoxycyclohexyl)-4-piperidinyl]-1,2-benzenediamine (D126, 40mg, 0.1mmol) in dichloromethane (3ml) at 0°C was treated with triphosgene (12mg, 0.04mmol) then diisopropylethylamine (0.03ml, 0.15mmol), and then maintained at 0°C for 30min. The mixture was partitioned between dichloromethane and aqueous sodium bicarbonate. Drying, evaporation and crystallisation from diethyl ether gave the title compound as a free base. This was converted to the hydrochloride salt which was isolated also from diethyl ether, 20mg. 1 HNMR (HCl salt) δ (d⁶DMSO): 0.85 (3H, t), 1.05 (3H, t), 1.3 (2H, m), 1.5 (2H, m), 1.9 (4H, m), 2.05 (4H, m), 2.35 (3H, s), 2.8 (2H, m), 3.2 (2H, m), 3.6 (2H, m), 4.6 (1H, m), 6.8 (2H, m), 7.35 (1H, s), 9.1 (1H, br s), 10.8 (1H, s), MH+ 400.

### Example 37. 3-{1-[trans-4-(Ethyloxy)cyclohexyl]-4-piperidinyl}-2-oxo-2,3-dihydro-1H-benzimidazole-5-carbonitrile hydrochloride (E37)

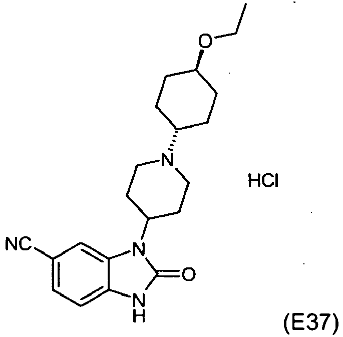

A stirred solution of 4-amino-3-({1-[*trans*-4-(ethyloxy)cyclohexyl]-4-piperidinyl}amino)benzonitrile (D128, 120mg, 0.35mmol) in dichloromethane (10ml) at 0°C under argon was treated with diisopropylethylamine (0.092ml, 0.52mmol) followed by the addition of solid triphosgene (40mg, 0.14mmol) and maintained for 1 hr. The mixture was treated with10% Na₂CO₃ solution (10ml) and extracted with dichloromethane (2x15ml). The combined extract was dried (Na₂SO₄), concentrated under vacuum and the residue was purified using a Waters Xbridge chromatography column, eluting with acetonitrile/water/0.1% formic acid, to afford the free base of the title compound as a white solid (50mg, 39%). This material (85mg) was dissolved in dichloromethane (5ml), treated with 1M HCl/Et₂O (0.2ml) and concentrated under vacuum to afford the title compound as a white solid (54mg) MH⁺ = 369.
¹H NMR δ (d⁶DMSO, 400MHz): 1.09 (3H, t), 1.10-1.30 (2H, m), 1.47-1.63 (2H, m), 1.92 (2H, br d), 2.05-2.18 (4H, m), 2.71-2.88 (2H, m), 3.18-3.40 (assume 4H, m), 3.4-3.55 (assume 4H, m), 4.57-4.70 (1H, m), 7.16 (1H, d), 7.48 (1H, d), 7.99 (1H, s), 10.26 (1H, br m), 11.55 (1H, s).

### Example 38. 3-{1-[trans-4-(Ethyloxy)-1-methylcyclohexyl]-4-piperidinyl}-2-oxo-2, 3-dihydro-1H-benzimidazole-5-carbonitrile hydrochloride (E38)

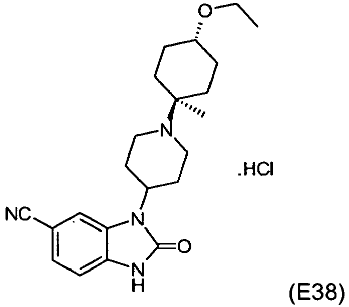

A stirred solution of the 4-amino-3-({1-[*trans*-4-(ethyloxy)-1-methylcyclohexyl]-4-piperidinyl}amino)benzonitrile (D130, 60mg, 0.168mmol) in dichloromethane (10ml) at 0°C under argon was treated with diisopropylethylamine (42µL, 0.252mmol, 32.5mg) followed by solid triphosgene (19mg, 0.067mmol) and maintained at 0°C for 1h. The mixture was treated with aqueous NaHCO₃ solution (10ml) and extracted with dichloromethane (2 x 15ml). The combined extract was dried (MgSO₄) and concentrated under vacuum to afford an orange residue. The residue was then purified using a Waters Xbridge chromatography column at high pH to afford the free base of the title compound (24mg, 38%), which was then treated with 1M HCl/diethyl ether (0.25ml) and solvents removed by evaporation to yield the title compound (24mg, 34%) as a white solid.
¹H NMR (free base) δ (CDCl₃, 400 MHz): 0.96 (3H, s), 1.21 (3H, t), 1.43-1.71 (6H, m), 1.84-1.94 (4H, m), 2.23-2.30 (4H, m), 3.18 (2H, d) 3.5 (1H, m), 3.52 (2H, q), 4.29 (1H, m), 7.15 (1H, d), 7.4 (1H, d), 7.52 (1H, s), 9.81 (1H, s).

### Example 39. 1-{1-[trans-4-(Ethyloxy)cyclohexyl]-4-piperidinyl}-6-(methylsulfonyl)-1,3-dihydro-2H-benzimidazol-2-one hydrochloride (E39)

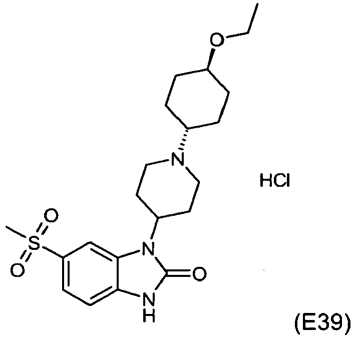

A stirred solution of 6-bromo-1-{1-[*trans*-4-(ethyloxy)cyclohexyl]-4-piperidinyl}-1,3-dihydro-2*H*-benzimidazol-2-one (free base of E14) (130mg, 0.31mmol) in DMSO (1ml) at room temperature under argon was treated with sodium methanesulphinate (35mg, 0.34mmol), copper (I) iodide (5.9mg, 0.031mmol) and L-proline sodium salt (8.5mg, 0.062mmol), then heated at 95°C for 18hrs. Further sodium sulphinate (70mg), copper (I) iodide (12mg) and L-proline sodium salt (17mg) were added and the mixture heated at 95°C for an additional 58hrs. The mixture was allowed to cool, then treated with 10% Na₂CO₃ solution (20ml) and EtOAc (25ml), shaken well and filtered through Kieselguhr. The EtOAc layer was isolated, dried (Na₂SO₄) and concentrated under vacuum. The residue was purified using a Waters Xbridge chromatography column column, eluting with acetonitrile/water/0.1% formic acid, to afford the free base of the title compound as a white solid (12mg). This was converted to the title compound as a yellow solid. MH+ = 422.
¹H NMR (free base) δ (CDCl₃, 400MHz): 1.20-1.40 (4H, m), 1.21 (3H, t), 1.80-2.05 (4H, m), 2.10-2.20 (2H, m), 2.34-2.52 (assume 5H, m), 3.02-3.12 (assume 2H, m), 3.10 (3H, s), 3.17-3.27 (1H, m), 3.52 (2H, q), 4.23-4.37 (1H, m), 7.24 (1H, d), 6.89 (1H, dd), 7.80 (1H, d), 10.20 (1H, br s).

### Example 40. 6-Methyl-1-{1-[cis-4-(2-propyn-1-yloxy)cyclohexyl]-4-piperidinyl}-1,3-dihydro-2H-benzimidazol-2-one (E40)

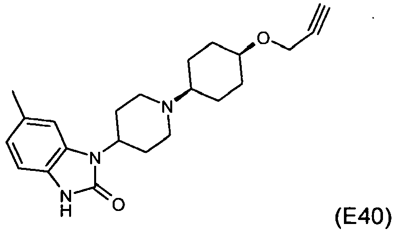

A stirred solution of 4-methyl-*N*²-{1-[*cis*-4-(2-propyn-1-yloxy)cyclohexyl]-4-piperidinyl}-1,2-benzenediamine (D136, 200mg, 0.58mmol) and diisopropylethylamine (200µl) in dichloromethane (10ml) at 0°C under argon was treated with solid triphosgene (0.07mg) and stirred for 2 hours. The mixture was diluted with dichloromethane, washed with dil. NaHCO₃ solution, dried with sodium sulfate and the solvent was removed. The title compound was obtained as a white solid (156mg) from ether. MH⁺ = 368.
¹H NMR δ (CDCl₃, 400 MHz): 1.45 (2H, m), 1.55-1.8 (obs, m), 1.85 (2H, m), 2.02 (2H , m), 2.4 (obs, m), 3.05 (2H, m), 3.75 (1H, m), 4.15 (2H, s), 4.34 (1H, m), 6.83 (1H, d), 6.92 (1H, d), 7.14 (1H, s), 8.44 (1H, s).

### Example 41. 6-Methyl-1-{1-[trans-4-(2-propyn-1-yloxy)cyclohexyl]-4-piperidinyl}-1,3-dihydro-2H-benzimidazol-2-one (E41)

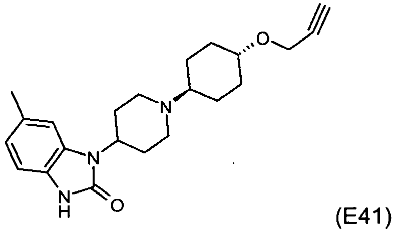

A stirred solution of 4-methyl-*N*²-{1-[*trans*-4-(2-propyn-1-yloxy)cyclohexyl]-4-piperidinyl}-1,2-benzenediamine (D137, 280mg) and diisopropylethylamine (0.3ml) in dichloromethane (20ml) at 0°C under argon was treated with solid triphosgene (97mg) and stirred for 1 hour. The mixture was diluted with dichloromethane, washed with dil. NaHCO₃ solution, dried with hydromatrix and the solvent was removed. The residue was chromatographed on silica gel eluted with dichloromethane - methanolic ammonia 0-10%. The title compound was obtained as a white solid (180mg). MH⁺ = 368.
¹H NMR δ (CDCl₃, 400 MHz): 1.2-1.4 (5H, m), 1.8 (2H, m), 1.95 (1H, m), 2.12 (2H , m), 2.4 (8H, m), 3.06 (2H, m), 3.45 (1H, m), 4.19 (2H, s), 4.33 (H, m), 6.83 (1H, d), 6.93 (1H, d), 7.12 (1H, s), 8.17 (1H, s).

### Example 42. 1-{1-[trans-4-(Ethyloxy)-1-methylcyclohexyl]-4-piperidinyl}-6-fluoro-1,3-dihydro-2H-benzimidazol-2-one hydrochloride (E42)

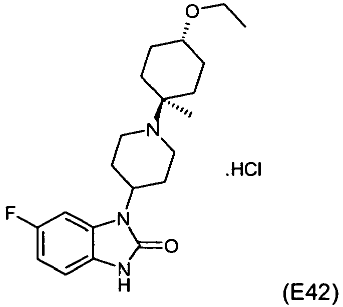

A stirred solution of (2-amino-5-fluorophenyl){1-[*trans*-4-(ethyloxy)-1-methylcyclohexyl]-4-piperidinyl}amine (D147, 97mg, 0.277mmol) in dichloromethane (7ml) at 0°C under argon was treated with diisopropylethylamine (70µL, 0.415mmol, 53mg) followed by solid triphosgene (33mg, 0.11mmol) and stirred at 0°C for 1h. The mixture was treated with dil. NaHCO₃ solution (20ml) and extracted with dichloromethane (2 x 25ml). The combined extract was dried (MgSO₄) and concentrated under vacuum to afford the free base of the title compound. This residue was then treated with Et₂O (5ml) and the resulting precipitate collected as the free base (31 mg). This was then treated with 1 M HCl/diethyl ether (0.25ml) and stirred for 20 mins, and the solid filtered to yield the title compound (38mg, 33%) as a white solid. MH⁺ 376.
¹H NMR (free base) δ (CDCl₃, 400 MHz): 0.94 (3H, s), 1.19-1.26 (4H, m), 1.48-1.52 (2H, m), 1.64-1.70 (2H, m), 1.82-1.92 (4H, m), 2.22-2.36 (4H, m), 3.14-3.16 (2H, d), 3.43-3.53 (4H, m), 4.23-4.28 (1H, m), 6.74- 6.79 (1H), 6.95-7.02 (2H, m), 8.63 (1H, s).

### Example 43. 6-Fluoro-1-{1-[trans-1-methyl-4-(methyloxy)cyclohexyl]-4-piperidinyl}-1,3-dihydro-2H-benzimidazol-2-one hydrochloride (E43)

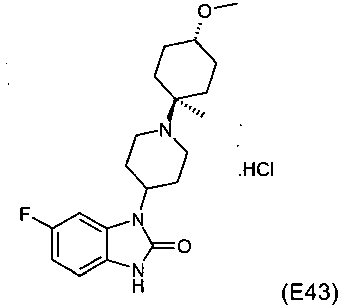

Diisopropylethylamine (210µL, 1.23mmol) was added to a solution of (2-amino-5-fluorophenyl){1-[*trans*-4-(methyloxy)-1-methylcyclohexyl]-4-piperidinyl}amine (D155, 205mg, 0.61mmol) in CH₂Cl₂ (10mL) at room temperature under argon. The reaction was cooled to 0°C and triphosgene (69mg, 0.23mmol) was added portion-wise. The reaction was stirred for 1h before the addition of 2M NaOH (10mL). The mixture was then diluted with CH₂Cl₂ (20mL) and sat. NaHCO₃ (20mL). The aqueous layer was extracted with CH₂Cl₂ (2x) and the combined organics were dried and concentrated via rotary evaporation to give a purple solid. This residue was treated with EtOAc-Et₂O (1:1, 4mL) to give a white solid which was azeotroped with toluene (2x5mL) to give the free base of the title compound as a white solid. The free base was treated with MeOH (2mL) and then 1 M HCl in Et₂O (0.58mL). The mixture was stirred for 30min and then the solvent was removed by rotary evaporation to give the title compound (103mg, 42%) as a pale yellow solid. MH⁺ 362.
¹H NMR δ (DMSO-*d*₆, 400 MHz): 1.22-1.38 (5 H, m), 1.78-2.08 (8 H, m), 2.73-2.88 (2 H, m), 3.07-3.30 (3 H, m), 3.23 (3 H, s), 3.62-3.71 (2 H, m), 4.62 (1H, m), 6.79-6.88 (1H, m), 6.94-7.00 (1H, m), 7.56 (1H, m), 9.80 (1H, m), 10.99 (1H, s).

### Example 44. 6-Chloro-1-{1-[trans-1-methyl-4-(methyloxy)cyclohexyl]-4-piperidinyl}-1,3-dihydro-2H-benzimidazol-2-one hydrochloride (E44)

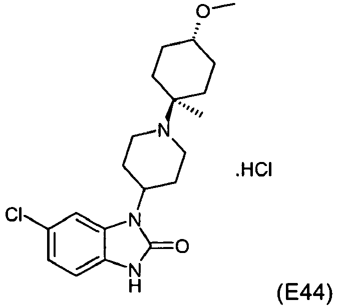

Diisopropylethylamine (290µL, 1.70mmol) was added to a solution of (2-amino-5-chlorophenyl){1-[*trans*-1-methyl-4-(methyloxy)cyclohexyl]-4-piperidinyl}amine (D157, 305mg, 0.87mmol) in CH₂Cl₂ (10mL) at room temperature under argon. The reaction was cooled to 0°C and triphosgene (86mg, 0.29mmol) was added portion-wise. The reaction was stirred for 1h before the addition of 2M NaOH (10mL). The mixture was then diluted with CH₂Cl₂ (20mL) and sat. NaHCO₃ (20mL). The aqueous layer was extracted with CH₂Cl₂ (2x) and the combined organics were dried and concentrated via rotary evaporation. The crude residue was treated with CH₂Cl₂ and filtered. The solid was triturated with EtOAc to give a pink solid, which was azeotroped with toluene (6x2mL) and then dissolved in hot MeOH-CH₂Cl₂ and the solvent was removed by rotary evaporation. The solid was dissolved a second time in hot MeOH-CH₂Cl₂ and the solvent was removed by rotary evaporation to give the free base of the title compound as an off-white solid. The free base was suspended in MeOH (2mL) and then 1 M HCl in Et₂O (0.45mL). The mixture was stirred for 30min and then the solvent was removed by rotary evaporation to give the title compound (92mg, 26%) as a pale pink solid. M(Cl-35)⁺ 378, M(Cl-37)H⁺ 380.
¹H NMR δ (DMSO-*d*₆, 400 MHz): 1.24-1.38 (5 H, m), 1.71-1.82 (2 H, m), 1.90-2.08 (6 H, m), 2.67-2.79 (2 H, m), 3.08-3.26 (3 H, m), 3.26 (3 H, s). 3.62-3.31 (2 H, m), 4.49 (1H, m), 6.98-7.07 (2 H, m), 7.52-7.58 (1H, m), 9.36 (1H, m), 11.12 (1H, s)

### Example 45. 6-(Ethyloxy)-1-{1-[trans-1-methyl-4-(methyloxy)cyclohexyl]-4-piperidinyl}-1,3-dihydro-2H-benzimidazol-2-one hydrochloride (E45)

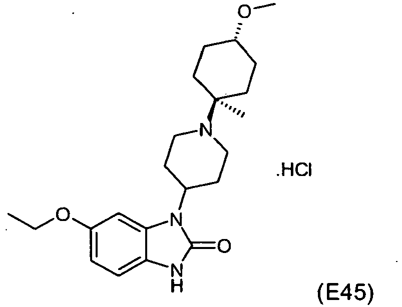

Triphoshene (0.082g) was added to a solution of 4-(ethyloxy)-*N*²-{1-[*trans*-1-methyl-4-(methyloxy)cyclohexyl]-4-piperidinyl}-1,2-benzenediamine (D160, 0.26g,), dichloromethane (20ml) and diisopropylethylamine (1ml) stirred at ice bath temperature. The solution was stirred to room temperature overnight then washed with saturated sodium bicarbonate solution and the solvent was removed. The residue was chromatographed on silica gel eluted with 0-10% dichloromethane - methanolic ammonia to give the free base of the title compound as a white solid (0.12g). This was dissolved in dichloromethane, treated with hydrogen chloride in ether and the solvent was removed to give the title compound as a white solid from diethyl ether (0.105g). MH⁺ = 388.
¹H NMR δ (d⁶DMSO, 400 MHz) (free base): 0.93 (3H, s), 1.45-1.7 (~11H, m), 1.8-2.0 (4H, m), 2.2-2.4 (4H, m), 3.1 (2H, m), 3.3 (obs, m), 4.05 (2H, m), 6.59 (1H, d of d), 6.86 (1H, d), 6.92 (1H, d), 8.65 (1H, s).

### Example 46. 6-(Ethyloxy)-1-{1-[trans-4-(ethyloxy)-1-methylcyclohexyl]-4-piperidinyl}-1,3-dihydro-2H-benzimidazol-2-one hydrochloride (E46)

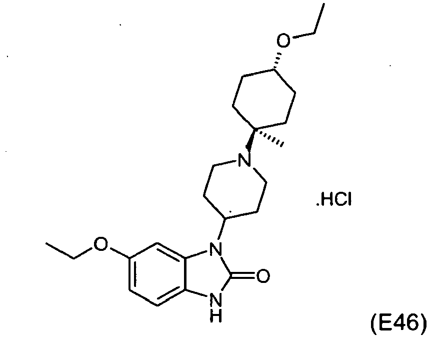

Triphoshene (0.02g) was added to a solution of 4-(ethyloxy)-*N*²-{1-[*trans*-1-methyl-4-(ethyloxy)cyclohexyl]-4-piperidinyl}-1,2-benzenediamine (D162, 0.09g), dichloromethane (10ml) and diisopropylethylamine (1ml) stirred at ice bath temperature. The solution was stirred to room temperature overnight then washed with saturated sodium bicarbonate solution and the solvent was removed. The residue was chromatographed on silica gel eluted with 0-10% dichloromethane - methanolic ammonia to give the free base of the title compound as a oil (0.08g). This was dissolved in dichloromethane, treated with hydrogen chloride in ether and the solvent was removed to give the title compound as a white solid from diethyl ether (0.067g). MH⁺ = 402.
¹H NMR δ (d⁶DMSO, 250 MHz) (free base): 0.94 (3H, s), 1.22 (3H, t), 1.4-1.7 (obs, m), 1.8-2.0 (4H, m), 2.2-2.4 (4H, m), 3.15 (2H, m), 34-3.6 (3H, m), 4.05 (2H, q), 4.25 (1H, m), 6.60 (1H, d of d), 6.80 (1H, d), 7.00 (1H, d), 9.8 (1H, s).

### Example 47. 6-Chloro-1-{1-[trans-4-(ethyloxy)-1-methylcyclohexyl]-4-piperidinyl}-1,3-dihydro-2H-benzimidazol-2-one hydrochloride (E47)

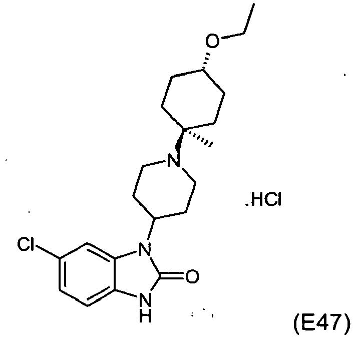

Solid-supported diisopropylbenzylamine (293mg, 1.04mmol) was added to a solution of (2-amino-5-chlorophenyl){1-[*trans*-4-(ethyloxy)-1-methylcyclohexyl]-4-piperidinyl}amine (D164, 139mg, 0.38mmol) in CH₂Cl₂ (10mL) at room temperature under argon. The reaction was cooled to 0°C and triphosgene (42mg, 0.14mmol) was added portion-wise. The reaction was stirred for 2h before the addition of 2M NaOH (10mL). The mixture was then filtered and washed with sat. NaHCO₃ solution. The aqueous layer was extracted with CH₂Cl₂ (2x) and the combined organics were dried (Na₂SO₄) and concentrated via rotary evaporation to give a brown solid. The solid was triturated with EtOAc to give a pale brown solid which was purified by chromatography (amine doped silica, hexane-EtOAc) to give the free base of the title compound as a pale brown solid. The free base was suspended in MeOH (2mL) and then 1M HCl in Et₂O (0.33mL) was added. The mixture was stirred for 30min and then filtered to give the title compound (63mg, 38%) as a pale pink solid.
M(Cl-35)⁺ 392, M(Cl-37)H⁺ 394.
¹H NMR δ (DMSO-*d*₆, 400 MHz): 1.10 (3 H, t, *J* 7), 1.28-1.40 (5 H, m), 1.76 (2 H, m), 1.90-2.05 (6 H, m), 2.71 (2 H, m), 3.15 (3 H, m), 3.48 (2 H, q, *J* 7), 3.66 (2 H, m), 4.58 (1H, m), 7.02 (2 H, m), 7.55 (1H, s), 9.34 (1H, m), 11.11 (1H, s)

## Claims

1. A compound of formula (I) or a salt or solvate thereof: 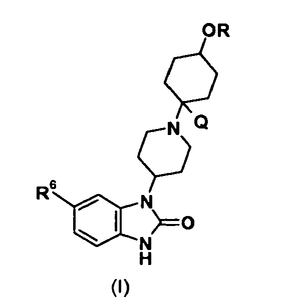 wherein:
• R⁶ is selected from hydrogen, cyano, halogen, C₁₋₆alkyl, C₁₋₆alkyl substituted with one or more fluorine atoms, C₁₋₆alkylsulfonyl, C₃₋₆cycloalkyl, C₃₋₆cycloalkyl substituted with one or more fluorine atoms, C₁₋₆alkoxy, and C₁₋₆alkoxy substituted with one or more fluorine atoms;
• R is selected from C₁₋₆alkyl, C₃₋₆cycloalkyl, C₃₋₆cycloalkylC₁₋₆alkyl and C₂₋₆alkynyl, any alkyl or cycloalkyl group being optionally substituted by one or more fluorine atoms; and
• Q is hydrogen or C₁₋₆alkyl.

2. A compound as claimed in claim 1, wherein R⁶ is selected from hydrogen, cyano, halogen, C₁₋₆alkyl, C₁₋₆alkyl substituted with one, two or three fluorine atoms, C₃₋₆cycloalkyl, C₁₋₆alkylsulfonyl, C₁₋₆alkoxy, and C₁₋₆alkoxy substituted with one, two or three fluorine atoms.

3. A compound as claimed in claim 2, wherein R⁶ is selected from hydrogen, cyano, fluoro, bromo, methyl, ethyl, methoxy, trifluoromethoxy, methylsulfonyl, trifluoromethyl and cyclopropyl.

4. A compound as claimed in any of claims 1-3, wherein R is selected from C₁₋₆alkyl, C₃-₆cycloalkyl, C₃₋₆cycloalkylC₁₋₆alkyl and C₂₋₄alkynyl, any alkyl or cycloalkyl group being optionally substituted by one, two or three fluorine atoms.

5. A compound as claimed in claim 4, wherein R is selected from methyl, ethyl, propyl, isopropyl, CF₃, cyclopropylmethyl, propynyl and cyclobutyl.

6. A compound as claimed in any of claims 1-5, wherein Q is selected from hydrogen and C₁₋₃alkyl.

7. A compound as claimed in claim 1, which is:
1. 6-Methyl-1-[1-(*cis*-4-methoxycyclohexyl)-4-piperidinyl]-1,3-dihydro-2*H*-benzimidazol-2-one
2. 6-Methyl-1-[1-(*trans*-4-methoxycyclohexyl)-4-piperidinyl]-1,3-dihydro-2*H-*benzimidazol-2-one
3. 1-{1-[*trans*-4-(Ethyloxy)cyclohexyl]-4-pipendinyl}-6-methyl-1,3-dihydro-2*H-*benzimidazol-2-one
4. 1-{1-[*cis*-4-(Ethyloxy)cyclohexyl]-4-piperidinyl}-6-methyl-1,3-dihydro-2*H-*benzimidazol-2-one
5. 6-Methyl-1-{1-[*cis*-4-(propyloxy)cyclohexyl]-4-piperidinyl}-1,3-dihydro-2*H-*benzimidazol-2-one
6. 6-Methyl-1-{1-[*trans*-4-(propyloxy)cyclohexyl]-4-piperidinyl}-1,3-dihydro-2*H-*benzimidazol-2-one
7. 6-Methyl-1-(1-{*trans*-4-[(1-methylethyl)oxy]cyclohexyl}-4-piperidinyl)-1,3-dihydro-2*H-*benzimidazol-2-one
8. 6-Methyl-1-{1-[*cis*-1-methyl-4-(methyloxy)cyclohexyl]-4-piperidinyl}-1,3-dihydro-2*H-*benzimidazol-2-one
9. 6-Methyl-1-{1-[*trans*-1-methyl-4-(methyloxy)cyclohexyl]-4-piperidinyl]-1,3-dihydro-2*H-*benzimidazol-2-one
10. 6-Methyl-1-(1-{*cis*-4-[(1-methylethyl)oxy]cyclohexyl}-4-piperidinyl)-1,3-dihydro-2*H-*benzimidazol-2-one
11. *cis*-(1-[4-(Ethoxyl)cyclohexyl]-4-piperidinyl)-1,3-dihydro-2H-benzimidazol-2-one
12. *trans* 1-(1-[4-(Ethoxyl)cyclohexyl]-4-piperidinyl)-1,3-dihydro-2H-benzimidazol-2-one
13. 1-{1-[*trans*-4-(Ethyloxy)cyclohexyl]-4-piperidinyl}-6-fluoro-1,3-dihydro-2*H-*benzimidazol-2-one
14. 6-Bromo-1-{1-[*trans*-4-(ethyloxy)cyclohexyl]-4-piperidinyl}-1,3-dihydro-2*H-*benzimidazol-2-one
15. 1-{1-[*cis*-4-(Ethyloxy)cyclohexyl]-4-piperidinyl}-6-(trifluoromethyl)-1,3-dihydro-2*H-*benzimidazol-2-one
16. 6-Ethyl-1-{1-[*trans*-4-(ethyloxy)cyclohexyl]-4-piperidinyl}-1,3-dihydro-2*H-*benzimidazol-2-one
17. 6-Cyclopropyl-1-{1-[*trans*-4-(ethyloxy)cyclohexyl]-4-piperidinyl}-1,3-dihydro-2*H-*benzimidazol-2-one
18. 1-{1-[*trans*-4-(Ethyloxy)cyclohexyl]-4-piperidinyl}-6-(methyloxy)-1,3-dihydro-2*H-*benzimidazol-2-one
19. *cis*-1-{1-[4-(Ethoxy)cyclohexyl]-4-piperidinyl}-6-[(trifluoromethyl)oxy]-1,3-dihydro-2H-benzimidazol-2-one
20. *trans*-1-{1-[4-(Ethoxy)cyclohexyl]-4-piperidinyl}-6-[(trifluoromethyl)oxy]-1,3-dihydro-2H-benzimidazol-2-one
21. 6-Methyl-1-[1-(4-trifluoromethoxycyclohexyl)-4-piperidinyl]-1,3-dihydro-2*H-*benzimidazol-2-one
22. 1-(1-{*cis*-4-[(Cyclopropylmethyl)oxy]cyclohexyl}-4-piperidinyl)-6-methyl-1,3-dihydro-2*H*-benzimidazol-2-one
23. 1-(1-{*trans*-4-[(Cyclopropylmethyl)oxy]cyclohexyl}-4-piperidinyl)-6-methyl-1,3-dihydro-2*H*-benzimidazol-2-one
24. *trans*-6-Methyl-1-[1-(4-cyclopropyloxycyclohexyl)-4-piperidinyl]-1,3-dihydro-2*H-*benzimidazol-2-one
25. *cis*-1-(1-[4-(Propyloxy)cyclohexyl]-4-piperidinyl)-1,3-dihydro-2H-benzimidazol-2-one
26. *trans*-1-(1-[4-(Propyloxy)cyclohexyl]-4-piperidinyl)-1,3-dihydro-2H-benzimidazol-2-one
27. 6-Bromo-1-{1-[*trans*-4-(propyloxy)cyclohexyl]-4-piperidinyl}-1,3-dihydro-2*H-*benzimidazol-2-one
28. 6-Ethyl-1-{1-[*trans*-4-(propyloxy)cyclohexyl]-4-piperidinyl}-1,3-dihydro-2*H-*benzimidazol-2-one
29. 6-Cyclopropyl-1-{1-[*trans*-4-(propyloxy)cyclohexyl]-4-piperidinyl}-1,3-dihydro-2*H-*benzimidazol-2-one
30. *cis-*1-{1-[4-(Propyloxy)cyclohexyl]-4-piperidinyl}-6-[(trifluoromethyl)oxy]-1,3-dihydro-2*H*-benzimidazol-2-one
31. *trans*-1-{1-[4-(Propyloxy)cyclohexyl]-4-piperidinyl}-6-[(trifluoromethyl)oxy]-1,3-dihydro-2*H*-benzimidazol-2-one
32. 1-{1-[*cis*-4-(Ethyloxy)-1-methylcyclohexyl]-4-piperidinyl}-6-methyl-1,3-dihydro-2*H-*benzimidazol-2-one
33. 1-{1-[*trans*-4-(ethyloxy)-1-methylcyclohexyl]-4-piperidinyl}-6-methyl-1,3-dihydro-2*H-*benzimidazol-2-one
34. 6-Methyl-1-{1-[*cis*-1-methyl-4-(propyloxy)cyclohexyl]-4-piperidinyl}-1,3-dihydro-2*H-*benzimidazol-2-one
35. 6-Methyl-1-{1-[*trans*-1-methyl-4-(propyloxy)cyclohexyl]-4-piperidinyl}-1,3-dihydro-2*H-*benzimidazol-2-one
36. *trans*-6-Methyl-1-[1-(1-ethyl-4-propoxycyclohexyl)-4-piperidinyl]-1,3-dihydro-2*H-*benzimidazol-2-one
37. 3-{1-[*trans*-4-(Ethyloxy)cyclohexyl]-4-piperidinyl}-2-oxo-2,3-dihydro-1*H-*benzimidazole-5-carbonitrile
38. 3-{1-[*trans*-4-(Ethyloxy)-1-methylcyclohexyl]-4-piperidinyl}-2-oxo-2, 3-dihydro-1*H-*benzimidazole-5-carbonitrile
39. 1-{1-[*trans*-4-(Ethyloxy)cyclohexyl]-4-piperidinyl}-6-(methylsulfonyl)-1,3-dihydro-2*H-*benzimidazol-2-one
40. 6-Methyl-1-{1-[*cis*-4-(2-propyn-1-yloxy)cyclohexyl]-4-piperidinyl}-1,3-dihydro-2*H-*benzimidazol-2-one
41. 6-Methyl-1-{1-[*trans*-4-(2-propyn-1-yloxy)cyclohexyl]-4-piperidinyl}-1,3-dihydro-2*H-*benzimidazol-2-one
42. 1-{1-[*trans*-4-(Ethyloxy)-1-methylcyclohexyl]-4-piperidinyl}-6-fluoro-1,3-dihydro-2*H-*benzimidazol-2-one
43. 6-Fluoro-1-{1-[*trans*-1-methyl-4-(methyloxy)cyclohexyl]-4-piperidinyl}-1,3-dihydro-2*H-*benzimidazol-2-one
44. 6-Chloro-1-{1-[*trans*-1-methyl-4-(methyloxy)cyclohexyl]-4-piperidinyl}-1,3-dihydro-2*H-*benzimidazol-2-one
45. 6-(Ethyloxy)-1-{1-[*trans*-1-methyl-4-(methyloxy)cyclohexyl]-4-piperidinyl)-1,3-dihydro-2*H*-benzimidazol-2-one
46. 6-(Ethyloxy)-1-{1-[*trans*-4-(ethyloxy)-1-methylcyclohexyl]-4-piperidinyl}-1,3-dihydro-2*H*-benzimidazol-2-one
47. 6-Chloro-1-{1-[*trans*-4-(ethyloxy)-1-methylcyclohexyl]-4-piperidinyl}-1,3-dihydro-2*H-*benzimidazol-2-one
or a salt or solvate thereof.

8. A pharmaceutical composition comprising a compound claimed in any one of claims 1 to 7 and a pharmaceutically acceptable carrier.

9. A compound as claimed in any one of claim 1 to 7 for use in therapy.

10. A compound as claimed in any one of claims 1 to 7 for use in the treatment of a condition which requires agonism of a muscarinic M₁ receptor.

11. A compound as claimed in any one of claims 1 to 7 for use in the treatment of a psychotic disorder or cognitive impairment.

12. Use of a compound as claimed in any one of claims 1 to 7 in the manufacture of a medicament for the treatment of a condition which requires agonism of a muscarinic M₁ receptor.

13. Use of a compound as claimed in any one of claims 1 to 7 in the manufacture of a medicament for the treatment of a psychotic disorder or cognitive impairment.

14. A process for preparing a compound as claimed in claim 1, which process is selected from:
(i) process (A1), which comprises coupling a compound of formula (II) 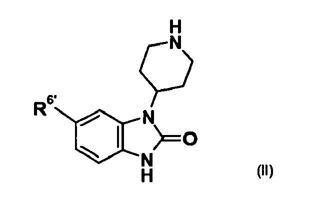 wherein R^{6'} is a group R⁶ as defined in claim 1, or a group convertible to R⁶, with a compound of formula (III): 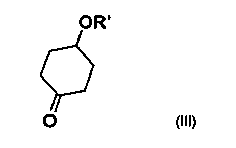 wherein R' is a group R as defined in claim 1, or a group convertible to R, under conditions suitable for reductive alkylation;
(ii) process (A2), which comprises reacting a compound of formula (II) as defined for process (A1), with a compound of formula (III) as defined for process (A1), in the presence of a source of cyanide to form the cyano intermediate (XXXX): 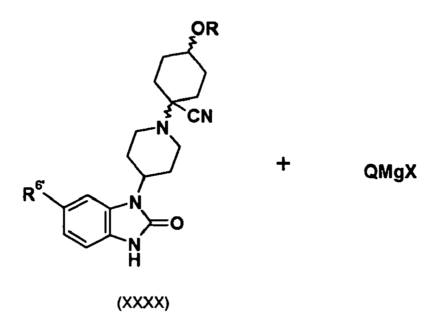 wherein R^{6'} is a group R⁶ as defined in claim 1, or a group convertible to R⁶, R' is a group R as defined in claim 1, or a group convertible to R, Q is C₁₋₆alkyl, and X is bromo, iodo or chloro, under conditions suitable for Grignard reactions;
(iii) process (B), which comprises reacting a compound of formula (IV): 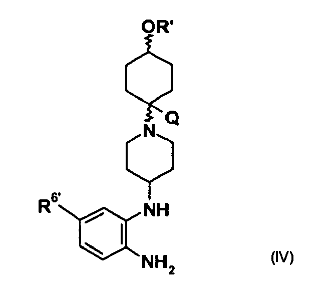 wherein R^{6'} is a group R⁶ as defined in claim 1, or a group convertible to R⁶, R' is a group R as defined in claim 1, or a group convertible to R, Q is as defined in claim 1, with a compound of formula (V): 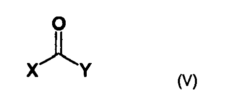 wherein X and Y both represent leaving groups optionally in an inert solvent, optionally in the presence of a base, and optionally with heating;
(iv) process (C), which comprises treatment of a compound of formula (VI): 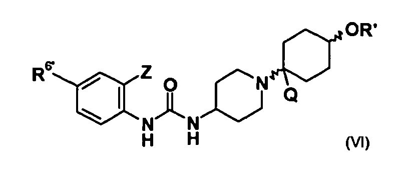 wherein R^{6'} is a group R⁶ as defined in claim 1, or a group convertible to R⁶, R' is a group R as defined in claim 1, or a group convertible to R, Q is as defined in claim 1, and Z is a leaving group such as bromo, iodo, chloro or triflate, with a palladium or copper catalyst (VII) to effect an intramolecular cyclisation;
(v) process (D), which comprises coupling a compound of formula (VIII): 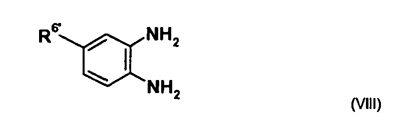 wherein R^{6'} is a group R⁶ as defined in claim 1, or a group convertible to R⁶, with a compound of formula (IX): 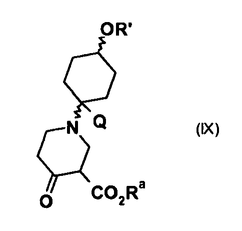 wherein R' is a group R as defined in claim 1, or a group convertible to R, Q is as defined in claim 1, and R^{a} is C₁₋₅alkyl, by heating in an inert solvent, for example xylene, followed by reduction of the piperidine double bond;
(vi) process (E), which comprises reaction of a compound of formula (X): 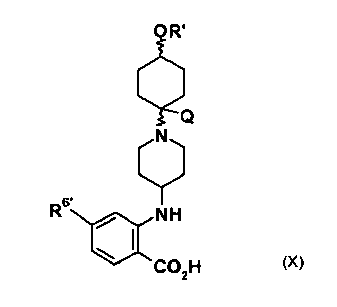 wherein R^{6'} is a group R⁶ as defined in claim 1, or a group convertible to R⁶, R' is a group R as defined in claim 1, or a group convertible to R, Q is as defined in claim 1, with a reagent/combination of reagents to effect a Curtius rearrangement of compound (X), followed by intramolecular cyclisation;
and
(vii) process (F), which comprises coupling a compound of formula (XI): 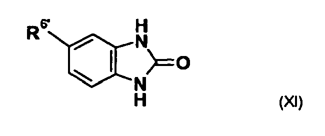 wherein R^{6'} is a group R⁶ as defined in claim 1, or a group convertible to R⁶, with a compound of formula (XII): 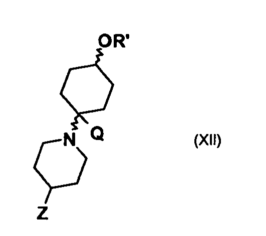
wherein R' is a group R as defined in claim 1, or a group convertible to R, Q is as defined in claim 1, and Z is hydroxy or a leaving group under alkylation or Mitsunobu reaction conditions;
and optionally thereafter, for any of the above processes:
- removing any protecting groups; and/or
- converting a compound of formula (I) or a salt or solvate thereof to another compound of formula (I) or a salt or solvate thereof.

15. A compound of formula (IV) or a salt or solvate thereof or formula (VI) or a salt or solvate thereof, or formula (X) or a salt or solvate thereof: 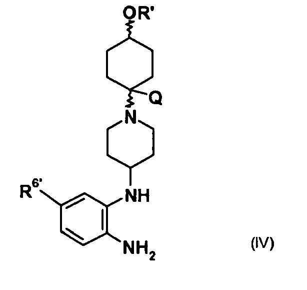 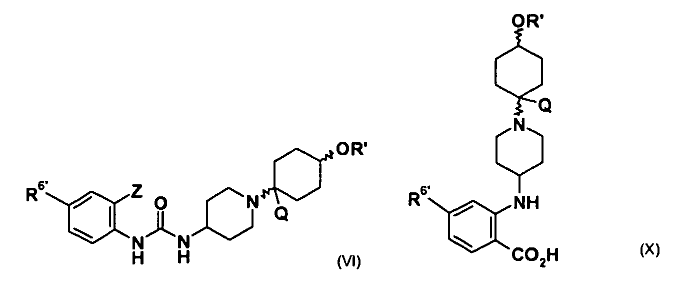 wherein in each of formula (IV), formula (VI), and formula (X), R^{6'} is a group R⁶ as defined in claim 1, and Z is bromo, iodo, chloro or triflate, R' is a group R as defined in claim 1 and Q is as defined in claim 1.

## Patentansprüche

1. Eine Verbindung der Formel (I) oder ein Salz oder Solvat davon: 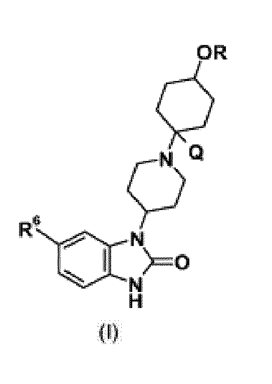 wobei:
• R⁶ aus Wasserstoff, Cyano, Halogen, C₁₋₆-Alkyl, C₁₋₆-Alkyl, substituiert mit einem oder mehreren Fluoratomen, C₁₋₆-Alkylsulfonyl, C₃₋₆-Cycloalkyl, C₃₋₆-Cycloalkyl, substituiert mit einem oder mehreren Fluoratomen, C₁₋₆-Alkoxy und C₁₋₆-Alkoxy, substituiert mit einem oder mehreren Fluoratomen, ausgewählt ist;
• R aus C₁₋₆-Alkyl, C₃₋₆-Cycloalkyl, C₃₋₆-Cycloalkyl-C₁₋₆-alkyl und C₂₋₆-Alkinyl ausgewählt ist, wobei jeder Alkyl- oder Cycloalkylrest gegebenenfalls mit einem oder mehreren Fluoratomen substituiert ist; und
• Q Wasserstoff oder C₁₋₆-Alkyl ist.

2. Eine Verbindung wie in Anspruch 1 beansprucht, wobei R⁶ aus Wasserstoff, Cyano, Halogen, C₁₋₆-Alkyl, C₁₋₆-Alkyl, substituiert mit einem, zwei oder drei Fluoratomen, C₃₋₆-Cycloalkyl, C₁₋₆-Alkylsulfonyl, C₁₋₆-Alkoxy und C₁₋₆-Alkoxy, substituiert mit einem, zwei oder drei Fluoratomen, ausgewählt ist.

3. Eine Verbindung wie in Anspruch 2 beansprucht, wobei R⁶ aus Wasserstoff, Cyano, Fluor, Brom, Methyl, Ethyl, Methoxy, Trifluormethoxy, Methylsulfonyl, Trifluormethyl und Cyclopropyl ausgewählt ist.

4. Eine Verbindung wie in einem der Ansprüche 1-3 beansprucht, wobei R aus C₁₋₆-Alkyl, C₃₋₆-Cycloalkyl, C₃₋₆-Cycloalkyl-C₁₋₆-alkyl und C₂₋₄-Alkinyl ausgewählt ist, wobei jeder Alkyl- oder Cycloalkylrest gegebenenfalls mit einem, zwei oder drei Fluoratomen substituiert ist.

5. Eine Verbindung wie in Anspruch 4 beansprucht, wobei R aus Methyl, Ethyl, Propyl, Isopropyl, CF₃, Cyclopropylmethyl, Propinyl und Cyclobutyl ausgewählt ist.

6. Eine Verbindung wie in einem der Ansprüche 1-5 beansprucht, wobei Q aus Wasserstoff und C₁₋₃-Alkyl ausgewählt ist.

7. Eine Verbindung wie in Anspruch 1 beansprucht, die
1. 6-Methyl-1-[1-(*cis*-4-methoxycyclohexyl)-4-piperidinyl]-1,3-dihydro-2*H-*benzimidazol-2-on
2. 6-Methyl-1-[1-(*trans*-4-methoxycyclohexyl)-4-piperidinyl]-1,3-dihydro-2*H-*benzimidazol-2-on
3. 1-{1-[*trans*-4-(Ethyloxy)cyclohexyl]-4-piperidinyl}-6-methyl-1,3-dihydro-2*H-*benzimidazol-2-on
4. 1-{1-[*cis*-4-(Ethyloxy)cyclohexyl]-4-piperidinyl}-6-methyl-1,3-dihydro-2*H-*benzimidazol-2-on
5. 6-Methyl-1-{1-[*cis*-4-(propyloxy)cyclohexyl]-4-piperidinyl}-1,3-dihydro-2*H-*benzimidazol-2-on
6. 6-Methyl-1-{1-[*trans*-4-(propyloxy)cyclohexyl]-4-piperidinyl}-1,3-dihydro-2*H-*benzimidazol-2-on
7. 6-Methyl-1-(1-{*trans*-4-[(1-methylethyl)oxy]cyclohexyl}-4-piperidinyl)-1,3-dihydro-2*H*-benzimidazol-2-on
8. 6-Methyl-1-{1-[*cis*-1-methyl-4-(methyloxy)cyclohexyl]-4-piperidinyl}-1,3-dihydro-2*H*-benzimidazol-2-on
9. 6-Methyl-1-{1[*trans*-1-methyl-4-(methyloxy)cyclohexyl]-4-piperidinyl}-1,3-dihydro-2*H*-benzimidazol-2-on
10. 6-Methyl-1-(1-{*cis*-4-[(1-methylethyl)oxy]cyclohexyl}-4-piperidinyl)-1,3-dihydro-2*H*-benzimidazol-2-on
11. *cis*-(1-[4-(Ethoxyl)cyclohexyl]-4-piperidinyl)-1,3-dihydro-2*H*-benzimidazol-2-on
*12. trans* 1-(1-[4-(Ethoxyl)cyclohexyl]-4-piperidinyl)-1,3-dihydro-2*H*-benzimidazol-2-on
13. 1-{1-[*trans*-4-(Ethyloxy)cyclohexyl]-4-piperidinyl}-6-fluor-1,3-dihydro-2*H-*benzimidazol-2-on
14. 6-Brom-1-{1-[*trans*-4-(ethyloxy)cyclohexyl]-4-piperidinyl}-1,3-dihydro-2*H-*benzimidazol-2-on
15. 1-{1-[*cis*-4-(Ethyloxy)cyclohexyl]-4-piperidinyl}-6-(trifluormethyl)-1,3-dihydro-2*H*-benzimidazol-2-on
16. 6-Ethyl-1-{1-[*trans*-4-(ethyloxy)cyclohexyl]-4-piperidinyl}-1,3-dihydro-2*H-*benzimidazol-2-on
17. 6-Cyclopropyl-1-{1-[*trans*-4-(ethyloxy)cyclohexyl]-4-piperidinyl}-1,3-dihydro-2*H*-benzimidazol-2-on
18. 1-{1-[*trans*-4-(Ethyloxy)cyclohexyl]-4-piperidinyl}-6-(methyloxy)-1,3-dihydro-2*H*-benzimidazol-2-on
19. *cis*-1-{1-[4-(Ethoxy)cyclohexyl]-piperidinyl}-6-[(trifluormethyl)oxy]-1,3-dihydro-2*H*-benzimidazol-2-on
20. *trans*-1-{1-[4-(Ethoxy)cyclohexyl]-4-piperidinyl}-6-[(trifluormethyl)oxy]-1,3-dihydro-2*H*-benzimidazol-2-on
21. 6-Methyl-1-[1-(4-trifluormethoxycyclohexyl)-4-piperidinyl]-1,3-dihydro-2*H-*benzimidazol-2-on
22. 1-(1-{*cis*-4-[(Cyclopropylmethyl)oxy]cyclohexyl}-4-piperidinyl)-6-methyl-1,3-dihydro-2*H*-benzimidazol-2-on
23. 1-(1-{*trans*-4-[(Cyclopropylmethyl)oxy]cyclohexyl}-4-piperidinyl)-6-methyl-1,3-dihydro-2*H*-benzimidazol-2-on
24. *trans*-6-Methyl-1-[1-(4-cyclopropyloxycyclohexyl)-4-piperidinyl]-1,3-dihydro-2*H-*benzimidazol-2-on
25. *cis*-1-(1-[4-(Propyloxy)cyclohexyl]-4-piperidinyl)-1,3-dihydro-2*H*-benzimidazol-2-on
26. *trans*-1-(1-[4-(Propyloxy)cyclohexyl]-4-piperidinyl)-1,3-dihydro-2*H-*benzimidazol-2-on
27. 6-Brom-1-{1-[*trans*-4-(propyloxy)cyclohexyl]-4-piperidinyl}-1,3-dihydro-2*H-*benzimidazol-2-on
28. 6-Ethyl-1-{1-[*trans*-4-(propyloxy)cyclohexyl]-4-piperidinyl}-1,3-dihydro-2*H-*benzimidazol-2-on
29. 6-Cyclopropyl-1-{1-[*trans*-4-(propyloxy)cyclohexyl]-4-piperidinyl}-1,3-dihydro-2*H*-benzimidazol-2-on
30. *cis*-1-{1-[4-(Propyloxy)cyclohexyl]-4-piperidinyl}-6-[(trifluormethyl)oxy]-1,3-dihydro-2*H*-benzimidazol-2-on
31. *trans*-1-{1-[4-(Propyloxy)cyclohexyl]-4-piperidinyl}-6-[(trifluormethyl)oxy]-1,3-dihydro-2*H*-benzimidazol-2-on
32. 1-{1-[*cis*-4-(Ethyloxy)-1-methylcyclohexyl]-4-piperidinyl}-6-methyl-1,3-dihydro-2*H*-benzimidazol-2-on
33. 1-{1-[*trans*-4-(Ethyloxy)-1-methylcyclohexyl]-4-piperidinyl}-6-methyl-1,3-dihydro-2*H*-benzimidazol-2-on
34. 6-Methyl-1-{1-[*cis*-1-methyl-4-(propyloxy)cyclohexyl]-4-piperidinyl}-1,3-dihydro-2*H*-benzimidazol-2-on
35. 6-Methyl-1-{1-[*trans*-1-methyl-4-(propyloxy)cyclohexyl]-4-piperidinyl}-1,3-dihydro-2*H*-benzimidazol-2-on
36. *trans*-6-Methyl-1-[1-(1-ethyl-4-propoxycyclohexyl)-4-piperidinyl]-1,3-dihydro-2*H*-benzimidazol-2-on
37. 3-{1-[*trans*-4-(Ethyloxy)cyclohexyl]-4-piperidinyl}-2-oxo-2,3-dihydro-1*H-*benzimidazol-5-carbonitril
38. 3-{1-[*trans*-4-(Ethyloxy)-1-methylcyclohexyl]-4-piperidinyl}-2-oxo-2,3-dihydro-1*H*-benzimidazol-5-carbonitril
39. 1-{1-[*trans*-4-(Ethyloxy)cyclohexyl]-4-piperidinyl}-6-(methylsulfonyl)-1,3-dihydro-2*H*-benzimidazol-2-on
40. 6-Methyl-1-{1-[*cis*-4-(2-propin-1-yloxy)cyclohexyl]-4-piperidinyl}-1,3-dihydro-2*H*-benzimidazol-2-on
41. 6-Methyl-1-{1-[*trans*-4-(2-propin-1-yloxy)cyclohexyl]-4-piperidinyl}-1,3-dihydro-2*H*-benzimidazol-2-on
42. 1-{1-[*trans*-4-(Ethyloxy)-1-methylcyclohexyl]-4-piperidinyl}-6-fluor-1,3-dihydro-2*H*-benzimidazol-2-on
43. 6-Fluor-1-{1-[*trans*-1-methyl-4-(methyloxy)cyclohexyl]-4-piperidinyl}-1,3-dihydro-2*H*-benzimidazol-2-on
44. 6-Chlor-1-{1-[*trans*-1-methyl-4-(methyloxy)cyclohexyl]-4-piperidinyl}-1,3-dihydro-2*H*-benzimidazol-2-on
45. 6-(Ethyloxy)-1-{1-[*trans*-1-methyl-4-(methyloxy)cyclohexyl]-4-piperidinyl}-1,3-dihydro-2*H*-benzimidazol-2-on
46. 6-(Ethyloxy)-1-{1-[*trans*-4-(ethyloxy)-1-methylcyclohexyl]-4-piperidinyl}-1,3-dihydro-2*H*-benzimidazol-2-on
47. 6-Chlor-1-{1-[*trans*-4-(ethyloxy)-1-methylcyclohexyl]-4-piperidinyl}-1,3-dihydro-2*H*-benzimidazol-2-on oder ein Salz oder Solvat davon ist.

8. Ein Arzneimittel, umfassend eine Verbindung wie in einem der Ansprüche 1 bis 7 beansprucht und einen pharmazeutisch verträglichen Träger.

9. Eine Verbindung wie in einem der Ansprüche 1 bis 7 beansprucht zur Verwendung bei der Therapie.

10. Eine Verbindung wie in einem der Ansprüche 1 bis 7 beansprucht zur Verwendung bei der Behandlung eines Zustandes, der einen Agonismus eines Muskarin-M₁-Rezeptors erfordert.

11. Eine Verbindung wie in einem der Ansprüche 1 bis 7 beansprucht zur Verwendung bei der Behandlung einer psychotischen Störung oder kognitiven Beeinträchtigung.

12. Verwendung einer Verbindung wie in einem der Ansprüche 1 bis 7 beansprucht bei der Herstellung eines Medikaments zur Behandlung eines Zustandes, der einen Agonismus eines Muskarin-M₁-Rezeptors erfordert.

13. Verwendung einer Verbindung wie in einem der Ansprüche 1 bis 7 beansprucht bei der Herstellung eines Medikaments zur Behandlung einer psychotischen Störung oder kognitiven Beeinträchtigung.

14. Ein Verfahren zur Herstellung einer Verbindung wie in Anspruch 1 beansprucht, wobei das Verfahren aus:
(i) Verfahren (A1), welches das Kuppeln einer Verbindung der Formel (II) 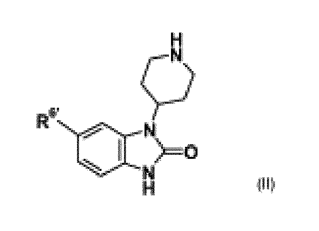 wobei R^{6'} ein wie in Anspruch 1 definierter Rest R⁶ ist oder ein Rest ist, der in R⁶ umgewandelt werden kann, mit einer Verbindung der Formel (III): 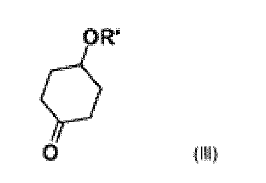 wobei R' ein wie in Anspruch 1 definierter Rest R ist oder ein Rest ist, der in R umgewandelt werden kann, bei Bedingungen, die zu reduktiver Alkylierung geeignet sind, umfasst;
(ii) Verfahren (A2), welches das Umsetzen einer Verbindung der Formel (II), wie für Verfahren (A1) definiert, mit einer Verbindung der Formel (III), wie für Verfahren (A1) definiert, in Gegenwart einer Cyanidquelle zur Bildung des Cyano-Zwischenproduktes (XXXX): 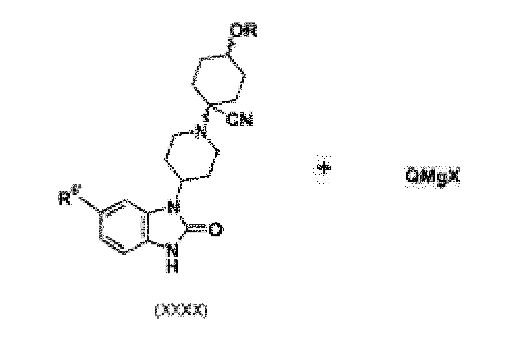 wobei R^{6'} ein wie in Anspruch 1 definierter Rest R⁶ ist oder ein Rest ist, der in R⁶ umgewandelt werden kann, R' ein wie in Anspruch 1 definierter Rest R ist oder ein Rest ist, der in R umgewandelt werden kann, Q C₁₋₆-Alkyl ist und X gleich Brom, Iod oder Chlor ist, bei Bedingungen, die für Grignard-Reaktionen geeignet sind, umfasst;
(iii) Verfahren (B), welches das Umsetzen einer Verbindung der Formel (IV): 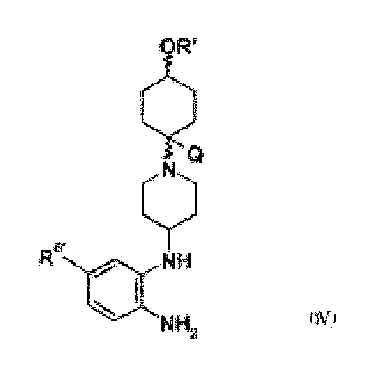 wobei R^{6'} ein wie in Anspruch 1 definierter Rest R⁶ ist oder ein Rest ist, der in R⁶ umgewandelt werden kann, R' ein wie in Anspruch 1 definierter Rest R ist oder ein Rest ist, der in R umgewandelt werden kann, Q wie in Anspruch 1 definiert ist, mit einer Verbindung der Formel (V): 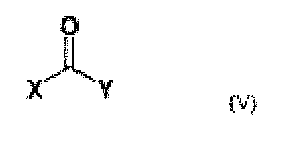 wobei X und Y beide für Abgangsgruppen stehen, gegebenenfalls in einem inerten Lösungsmittel, gegebenenfalls in Gegenwart einer Base und gegebenenfalls unter Erwärmen, umfasst;
(iv) Verfahren (C), welches das Behandeln einer Verbindung der Formel (VI): 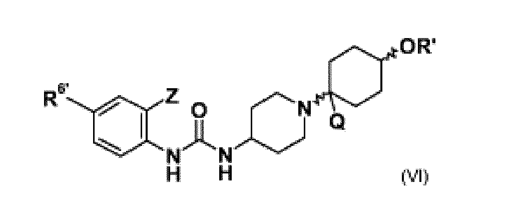 wobei R^{6'} ein wie in Anspruch 1 definierter Rest R⁶ ist oder ein Rest ist, der in R⁶ umgewandelt werden kann, R' ein wie in Anspruch 1 definierter Rest R ist oder ein Rest ist, der in R umgewandelt werden kann, Q wie in Anspruch 1 definiert ist und Z eine Abgangsgruppe wie etwa Brom, Iod, Chlor oder Triflat ist, mit einem Palladium- oder Kupferkatalysator (VII) zum Herbeiführen einer intramolekularen Cyclisierung umfasst;
(v) Verfahren (D), welches das Kuppeln einer Verbindung der Formel (VIII): 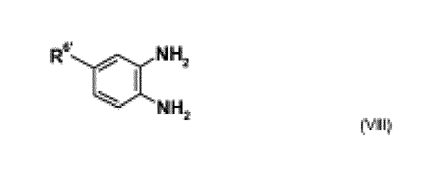 wobei R^{6'} ein wie in Anspruch 1 definierter Rest R⁶ ist oder ein Rest ist, der in R⁶ umgewandelt werden kann, mit einer Verbindung der Formel (IX): 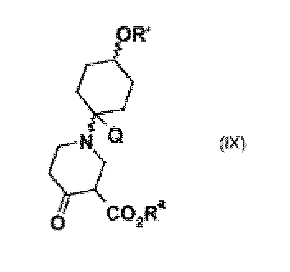 wobei R' ein wie in Anspruch 1 definierter Rest R ist oder ein Rest ist, der in R umgewandelt werden kann, Q wie in Anspruch 1 definiert ist und R^{a} C₁₋₅-Alkyl ist, durch Erwärmen in einem inerten Lösungsmittel, beispielsweise Xylol, gefolgt von Reduzieren der Piperidin-Doppelbindung, umfasst;
(vi) Verfahren (E), welches das Umsetzen einer Verbindung der Formel (X): 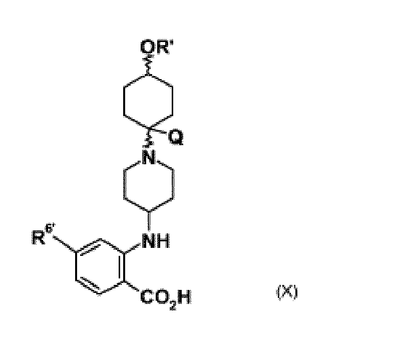 wobei R^{6'} ein wie in Anspruch 1 definierter Rest R⁶ ist oder ein Rest ist, der in R⁶ umgewandelt werden kann, R' ein wie in Anspruch 1 definierter Rest R ist oder ein Rest ist, der in R umgewandelt werden kann, Q wie in Anspruch 1 definiert ist, mit einem/-r Reagens/Kombination von Reagenzien zum Herbeiführen einer Curtius-Umlagerung der Verbindung (X), gefolgt von intramolekularer Cyclisierung, umfasst;
und
(vii) Verfahren (F), welches das Kuppeln einer Verbindung der Formel (XI): 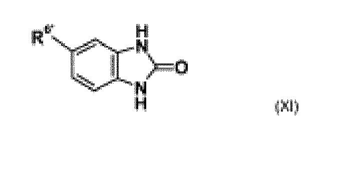 wobei R^{6'} ein wie in Anspruch 1 definierter Rest R⁶ ist oder ein Rest ist, der in R⁶ umgewandelt werden kann, mit einer Verbindung der Formel (XII): 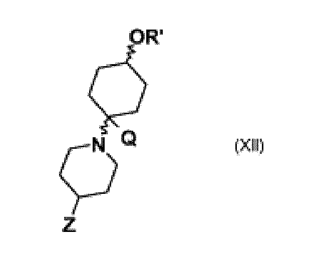 wobei R' ein wie in Anspruch 1 definierter Rest R ist oder ein Rest ist, der in R umgewandelt werden kann, Q wie in Anspruch 1 definiert ist und Z Hydroxy oder eine Abgangsgruppe ist, unter Alkylierungs- oder Mitsunobu-Reaktionsbedingungen umfasst; ausgewählt ist
und gegebenenfalls anschließend bei einem der oben genannten Verfahren:
- Entfernen von Schutzgruppen; und/oder
- Umwandeln einer Verbindung der Formel (I) oder eines Salzes oder Solvats davon in eine andere Verbindung der Formel (I) oder ein Salz oder Solvat davon.

15. Eine Verbindung der Formel (IV) oder ein Salz oder Solvat davon oder der Formel (VI) oder ein Salz oder Solvat davon oder der Formel (X) oder ein Salz oder Solvat davon: 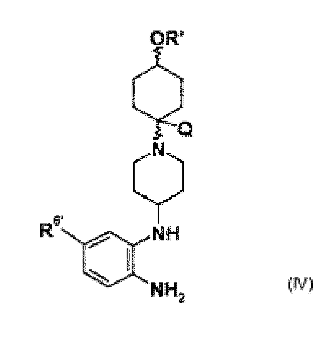 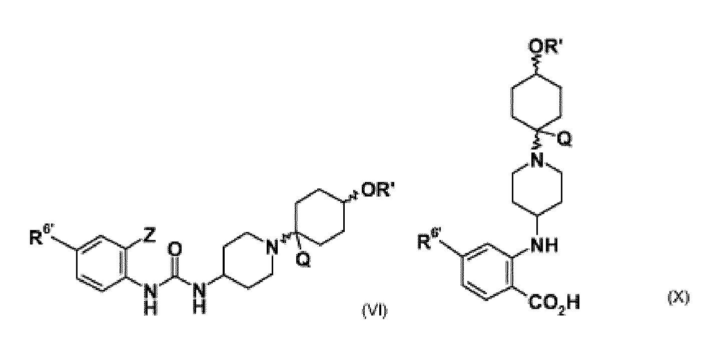 wobei in jeder Formel (IV), Formel (VI) und Formel (X) R^{6'} ein wie in Anspruch 1 definierter Rest R⁶ ist und Z Brom, Iod, Chlor oder Triflat ist, R' ein wie in Anspruch 1 definierter Rest R ist und Q wie in Anspruch 1 definiert ist.

## Revendications

1. Composé de formule (I) ou un de ses sels ou produits de solvatation : 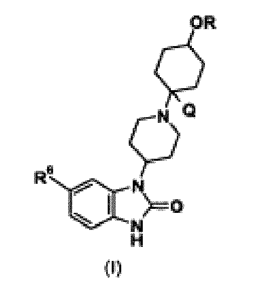 formule dans laquelle :
• R⁶ est choisi entre un atome d'hydrogène, des groupes cyano, halogéno, alkyle en C₁ à C₆, alkyle en C₁ à C₆ substitué avec un ou plusieurs atomes de fluor, alkylsulfonyle en C₁ à C₆, cycloalkyle en C₃ à C₆, cycloalkyle en C₃ à C₆ substitué avec un ou plusieurs atomes de fluor, alkoxy en C₁ à C₆ et alkoxy en C₁ à C₆ substitué avec un ou plusieurs atomes de fluor ;
• R est choisi entre des groupes alkyle en C₁ à C₆, cycloalkyle en C₃ à C₆, (cycloalkyle en C₃ à C₆)(alkyle en C₁ à C₆) et alcynyle en C₂ à C₆, n'importe quel groupe alkyle ou cycloalkyle étant facultativement substitué avec un ou plusieurs atomes de fluor ; et
• Q représente un atome d'hydrogène ou un groupe alkyle en C₁ à C₆.

2. Composé suivant la revendication 1, dans lequel R⁶ est choisi entre un atome d'hydrogène, des groupes cyano, halogéno, alkyle en C₁ à C₆, alkyle en C₁ à C₆ substitué avec un, deux ou trois atomes de fluor, cycloalkyle en C₃ à C₆, alkylsulfonyle en C₁ à C₆, alkoxy en C₁ à C₆ et alkoxy en C₁ à C₆ substitué avec un, deux ou trois atomes de fluor.

3. Composé suivant la revendication 2, dans lequel R⁶ est choisi entre un atome d'hydrogène, des groupes cyano, fluoro, bromo, méthyle, éthyle, méthoxy, trifluoro-méthoxy, méthylsulfonyle, trifluorométhyle et cyclopropyle.

4. Composé suivant l'une quelconque des revendications 1 à 3, dans lequel R est choisi entre des groupes alkyle en C₁ à C₆, cycloalkyle en C₃ à C₆, (cycloalkyle en C₃ à C₆) - (alkyle en C₁ à C₆) et alcynyle en C₂ à C₄, n'importe quel groupe alkyle ou cycloalkyle étant facultativement substitué avec un, deux ou trois atomes de fluor.

5. Composé suivant la revendication 4, dans lequel R est choisi entre des groupes méthyle, éthyle, propyle, isopropyle, CF₃, cyclopropylméthyle, propynyle et cyclobutyle.

6. Composé suivant l'une quelconque des revendications 1 à 5, dans lequel Q est choisi entre un atome d'hydrogène et un groupe alkyle en C₁ à C₃.

7. Composé suivant la revendication 1, qui est :
1. la 6-méthyl-1-[1-(*cis*-4-méthoxycyclohexyl)-4-pipéridinyl]-1,3-dihydro-2*H*-benzimidazole-2-one
2. la 6-méthyl-1-[1-(*trans*-4-méthoxycyclohexyl)-4-pipéridinyl]-1,3-dihydro-2*H*-benzimidazole-2-one
3. la 1-{1-[*trans*-4-(éthyloxy)cyclohexyl]-4-pipéridinyl}-6-méthyl-1,3-dihydro-2H-benzimidazole-2-one
4. la 1-{1-[*cis*-4-(éthyloxy)cyclohexyl]-4-pipéridinyl}-6-méthyl-1,3-dihydro-2H-benzimidazole-2-one
5. la 6-méthyl-1-[1-(*cis*-4-(propyloxy)cyclohexyl)-4-pipéridinyl]-1,3-dihydro-2*H*-benzimidazole-2-one
6. la 6-méthyl-1-[1-(*trans*-4-(propyloxy)CyClohexyl)-4-pipéridinyl]-1,3-dihydro-2*H*-benzimidazole-2-one
7. la 6-méthyl-1-(1-{*trans*-4-[(1-méthyléthyl)oxy]cyclohexyl}-4-pipéridinyl)-1,3-dihydro-2*H*-benzimidazole-2-one
8. la 6-méthyl-1-{1-[*cis*-1-méthyl-4-(méthyloxy)cyclohexyl]-4-pipéridinyl}-1,3-dihydro-2*H*-benzimidazole-2-one
9. la 6-méthyl-1-{1-[*trans*-1-méthyl-4-(méthyloxy)cyclohexyl]-4-pipéridinyl}-1,3-dihydro-2*H*-benzimidazole-2-one
10. la 6-méthyl-1-(1-{*cis*-4-[(1-méthyléthyl)oxy]cyclohexyl}-4-pipéridinyl)-1,3-dihydro-2*H*-benzimidazole-2-one
11. la *cis-*(1-[4-(éthoxyl)cyclohexyl]-4-pipéridinyl)-1,3-dihydro-2H-benzimidazole-2-one
12. la *trans*-1-(1-[4-(éthoxyl)cyclohexyl]-4-pipéridinyl)-1,3-dihydro-2H-benzimidazole-2-one
13. la 1-{1-[*trans*-4-(éthyloxy)cyclohexyl]-4-pipéridinyl}-6-fluoro-1,3-dihydro-2*H*-benzimidazole-2-one
14. la 6-bromo-1-{1-[*trans*-4-(éthyloxy)cyclohexyl]-4-pipéridinyl}-1,3-dihydro-2*H*-benzimidazole-2-one
15. la 1-{1-[*cis*-4-(éthyloxy)cyclohexyl]-4-pipéridinyl}-6-(trifluorométhyl)-1,3-dihydro-2*H*-benzimidazole-2-one
16. la 6-éthyl-1-{1-[*trans*-4-(éthyloxy)cyclohexyl]-4-pipéridinyl}-1,3-dihydro-2*H*-benzimidazole-2-one
17. la 6-cyclopropyl-1-{1-[*trans*-4-(éthyloxy)cyclohexyl]-4-pipéridinyl}-1,3-dihydro-2*H*-benzimidazole-2-one
18. la 1-{1-[*trans*-4-(éthyloxy)cyclohexyl]-4-pipéridinyl}-6-(méthyloxy)-1,3-dihydro-2*H*-benzimidazole-2-one
19. la *cis*-1-{1-[4-(éthoxy)cyclohexyl]-4-pipéridinyl}-6-[(trifluorométhyl)oxy]-1,3-dihydro-2H-benzimidazole-2-one
20. la *trans*-1-{1-[4-(éthoxy)cyclohexyl]-4-pipéridinyl}-6-[(trifluorométhyl)oxy]-1,3-dihydro-2H-benzimidazole-2-one
21. la 6-méthyl-1-[(4-trifluorométhoxycyclohexyl)-4-pipéridinyl]-1,3-dihydro-2*H*-benzimidazole-2-one
22. la 1-(1-{*cis*-4-[(cyclopropylméthyl)oxy]cyclohexyl}-4-pipéridinyl)-6-méthyl-1,3-dihydro-2*H*-benzimidazole-2-one
23. la 1-(1-{*trans*-4-[(cyclopropylméthyl)oxy]cyclohexyl}-4-pipéridinyl)-6-méthyl-1,3-dihydro-2*H*-benzimidazole-2-one
24. la *trans*-6-méthyl-1-[1-(4-cyclopropyloxycyclohexyl)-4-pipéridinyl]-1,3-dihydro-2*H*-benzimidazole-2-one
25. la *cis*-1-(1-[4-(propyloxy)cyclohexyl]-4-pipéridinyl)-1,3-dihydro-2H-benzimidazole-2-one
26. la *trans*-1-(1-[4-(propyloxy)cyclohexyl]-4-pipéridinyl)-1,3-dihydro-2H-benzimidazole-2-one
27. la 6-bromo-1-{1-[*trans*-4-(propyloxy)cyclohexyl]-4-pipéridinyl}-1,3-dihydro-2*H*-benzimidazole-2-one
28. la 6-éthyl-1-{1-[*trans*-4-(propyloxy)cyclohexyl]-4-pipéridinyl}-1,3-dihydro-2*H*-benzimidazole-2-one
29. la 6-cyclopropyl-1-{1-[*trans*-4-(propyloxy)cyclohexyl]-4-pipéridinyl}-1,3-dihydro-2*H*-benzimidazole-2-one
30. la *cis*-1-{1-[4-(propyloxy)cyclohexyl]-4-pipéridinyl}-6-[(trifluorométhyl)oxy]-1,3-dihydro-2*H*-benzimidazole-2-one
31. la *trans*-1-{1-[4-(propyloxy)cyclohexyl]-4-pipéridinyl}-6-[(trifluorométhyl)oxy]-1,3-dihydro-2*H*-benzimidazole-2-one
32. la 1-{1-[*cis*-4-(éthyloxy)-1-méthylcyclohexyl]-4-pipéridinyl}-6-méthyl-1,3-dihydro-2*H*-benzimidazole-2-one
33. la 1-{1-[*trans*-4-(éthyloxy)-1-méthylcyclohexyl]-4-pipéridinyl}-6-méthyl-1,3-dihydro-2*H*-benzimidazole-2-one
34. la 6-méthyl-1-{1-[*cis*-1-méthyl-4-(propyloxy)cyclohexyl]-4-pipéridinyl}-1,3-dihydro-2*H*-benzimidazole-2-one
35. la 6-méthyl-1-{1-[*trans*-1-méthyl-4-(propyloxy)cyclohexyl]-4-pipéridinyl}-1,3-dihydro-2*H*-benzimidazole-2-one
36. la *trans*-6-méthyl-1-[1-(1-éthyl-4-propoxycyclohexyl)-4-pipéridinyl]-1,3-dihydro-2*H*-benzimidazole-2-one
37. le 3-{1-[*trans*-4-(éthyloxy)cyclohexyl]-4-pipéridinyl}-2-oxo-2,3-dihydro-1*H*-benzimidazole-5-carbonitrile,
38. le 3-{1-[*trans*-4-(éthyloxy)méthylcyclohexyl]-4-pipéridinyl}-2-oxo-2,3-dihydro-1*H*-benzimidazole-5-carbonitrile,
39. la 1-{1-[*trans*-4-(éthyloxy)cyclohexyl]-4-pipéridinyl}-6-(méthylsulfonyl)-1,3-dihydro-2*H*-benzimidazole-2-one
40. la 6-méthyl-1-{1-[*cis*-4-(2-propyn-1-yloxy)cyclohexyl]-4-pipéridinyl}-1,3-dihydro-2*H*-benzimidazole-2-one,
41. la 6-méthyl-1-{1-[*trans*-4-(2-propyn-1-yloxy)cyclohexyl]-4-pipéridinyl}-1,3-dihydro-2*H*-benzimidazole-2-one,
42. la 1-{1-[*trans*-4-(éthyloxy)-1-méthylcyclohexyl]-4-pipéridinyl}-6-fluoro-1,3-dihydro-2*H*-benzimidazole-2-one
43. la 6-fluoro-1-{1-[*trans*-1-méthyl-4-(méthyloxy)cyclohexyl]-4-pipéridinyl}-1,3-dihydro-2*H*-benzimidazole-2-one
44. la 6-chloro-1-{1-[*trans*-1-méthyl-4-(méthyloxy)cyclohexyl]-4-pipéridinyl}-1,3-dihydro-2*H*-benzimidazole-2-one
45. la 6-(éthyloxy)-1-{1-[*trans*-1-méthyl-4-(méthyloxy)-cyclohexyl]-4-pipéridinyl}-1,3-dihydro-2*H*-benzimidazole-2-one
46. la 6-(éthyloxy)-1-{1-[*trans*-4-(éthyloxy)-1-méthylcyclohexyl]-4-pipéridinyl}-1,3-dihydro-2*H*-benzimidazole-2-one
47. la 6-chloro-1-{1-[*trans*-4-(éthyloxy)-1-méthylcyclohexyl]-4-pipéridinyl}-1,3-dihydro-2*H*-benzimidazole-2-one,
ou un de ses sels ou produis de solvatation.

8. Composition pharmaceutique comprenant un composé suivant l'une quelconque des revendications 1 à 7 et un support pharmaceutiquement acceptable.

9. Composé suivant l'une quelconque des revendications 1 à 7, pour une utilisation en thérapie.

10. Composé suivant l'une quelconque des revendications 1 à 7, pour une utilisation dans le traitement d'une affection qui nécessite l'agonisme d'un récepteur muscarinique M₁.

11. Composé suivant l'une quelconque des revendications 1 à 7, pour une utilisation dans le traitement d'un trouble psychotique ou d'une altération cognitive.

12. Utilisation d'un composé suivant l'une quelconque des revendications 1 à 7, dans la production d'un médicament pour le traitement d'une affection qui nécessite l'agonisme d'un récepteur muscarinique M₁.

13. Utilisation d'un composé suivant l'une quelconque des revendications 1 à 7, dans la production d'un médicament pour le traitement d'un trouble psychotique ou d'une altération cognitive.

14. Procédé pour préparer un composé suivant la revendication 1, procédé qui est choisi entre :
(i) le procédé (A1), qui comprend le couplage d'un composé de formule (II) 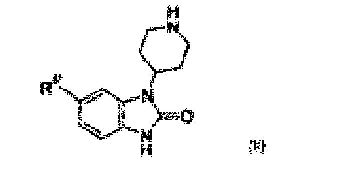 dans laquelle R^{6'} représente un groupe R⁶ tel que défini dans la revendication 1, ou un groupe pouvant être converti en un groupe R⁶, avec un composé de formule (III) : 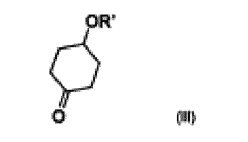 dans laquelle R' représente un groupe R tel que défini dans la revendication 1, ou un groupe pouvant être converti en un groupe R, dans des conditions convenables pour une alkylation réductrice ;
(ii) le procédé (A2), qui comprend la réaction d'un composé de formule (II) tel que défini pour le procédé (A1), avec un composé de formule (III) tel que défini pour le procédé (A1), en présence d'une source de cyanure pour former l'intermédiaire à fonction cyano (XXXX) 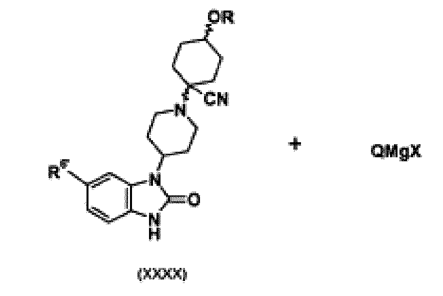 formules dans lesquelles R^{6'} représente un groupe R⁶ tel que défini dans la revendication 1, ou un groupe pouvant être converti en un groupe R⁶, R' représente un groupe R tel que défini dans la revendication 1, ou un groupe pouvant être converti en un groupe R, Q représente un groupe alkyle en C₁ à C₆ et X représente un groupe bromo, iodo ou chloro, dans des conditions convenables pour des réactions de Grignard ;
(iii) le procédé (B), qui comprend la réaction d'un composé de formule (IV) : 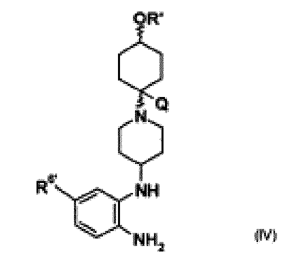 dans laquelle R^{6'} représente un groupe R⁶ tel que défini dans la revendication 1, ou un groupe pouvant être converti en un groupe R⁶, R' représente un groupe R tel que défini dans la revendication 1, ou un groupe pouvant être converti en un groupe R, Q est tel que défini dans la revendication 1, avec un composé de formule (V) : 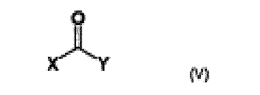 dans laquelle X et Y représentent tous deux des groupes partants, éventuellement dans un solvant inerte, éventuellement en présence d'une base, et éventuellement à chaud ;
(iv) le procédé (C), qui comprend le traitement d'un composé de formule (VI) : 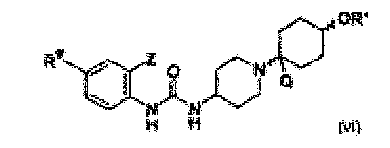 dans laquelle R^{6'} représente un groupe R⁶ tel que défini dans la revendication 1, ou un groupe pouvant être converti en un groupe R⁶, R' représente un groupe R tel que défini dans la revendication 1, ou un groupe pouvant être converti en un groupe R, Q est tel que défini dans la revendication 1, et Z représente un groupe partant tel qu'un groupe bromo, iodo, chloro ou triflate, avec un catalyseur au palladium ou au cuivre (VII) pour effectuer une cyclisation intramoléculaire ;
(v) le procédé (D), qui comprend le couplage d'un composé de formule (VIII) : 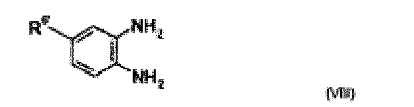 dans laquelle R^{6'} représente un groupe R⁶ tel que défini dans la revendication 1, ou un groupe pouvant être converti en un groupe R⁶, avec un composé de formule (IX) : 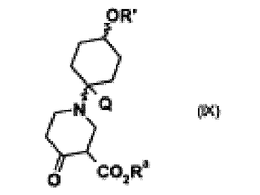 dans laquelle R' représente un groupe R tel que défini dans la revendication 1, ou un groupe pouvant être converti en un groupe R, Q est tel que défini dans la revendication 1, et R^{a} représente un groupe alkyle en C₁ à C₅, par chauffage dans un solvant inerte, par exemple le xylène, avec ensuite la réduction de la double liaison de la pipéridine ;
(vi) le procédé (E), qui comprend le couplage d'un composé de formule (X) : 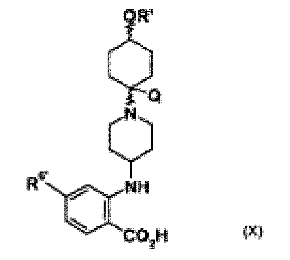 dans laquelle R^{6'} représente un groupe R⁶ tel que défini dans la revendication 1, ou un groupe pouvant être converti en un groupe R⁶, R' représente un groupe R tel que défini dans la revendication 1, ou un groupe pouvant être converti en un groupe R, Q est tel que défini dans la revendication 1, avec un réactif/une association de réactifs pour effectuer un réarrangement de Curtius du composé (X), avec ensuite une cyclisation intramoléculaire ;
et
(vii) le procédé (F), qui comprend le couplage d'un composé de formule (XI) : 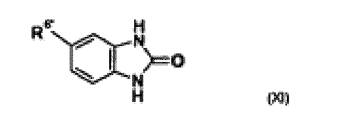 dans laquelle R^{6'} représente un groupe R⁶ tel que défini dans la revendication 1, ou un groupe pouvant être converti en un groupe R⁶, avec un composé de formule (XII) : 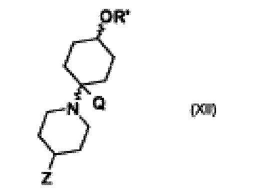 dans laquelle R' représente un groupe R tel que défini dans la revendication 1, ou un groupe pouvant être converti en un groupe R, Q est tel que défini dans la revendication 1, et Z représente un groupe hydroxy ou un groupe partant, dans des conditions de réaction d'alkylation ou de Mitsunobu ;
et ensuite, facultativement, pour n'importe lequel des procédés précités ;
- l'élimination de n'importe quel groupe protecteur ; et/ou
- la conversion d'un composé de formule (I) ou d'un de ses sels ou produits de solvatation en un autre composé de formule (I) ou un de ses sels ou produits de solvatation.

15. Composé de formule (IV) ou un de ses sels ou produits de solvatation, ou de formule (VI) ou un de ses sels ou produits de solvatation, ou de formule (X) ou un de ses sels ou produits de solvatation : 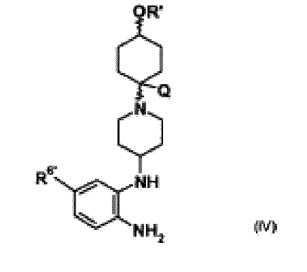 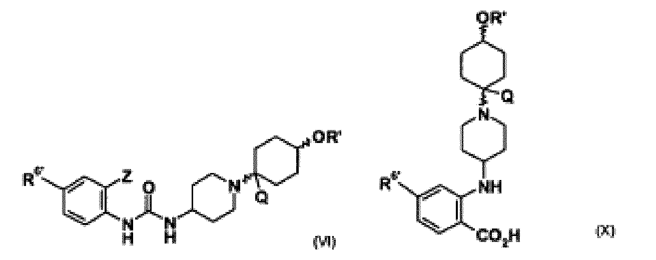 où dans chacune des formules (IV), (VI) et (X), R^{6'} représente un groupe R⁶ tel que défini dans la revendication 1, et Z représente un groupe bromo, iodo, chloro ou triflate, R' représente un groupe R tel que défini dans la revendication 1, et Q est tel que défini dans la revendication 1.
